# EUROPEAN PATENT SPECIFICATION

(11) **EP 3 300 736 B1**
(45) Date of publication and mention of the grant of the patent: **05.05.2021**
(21) Application number: 16306260.7
(22) Date of filing: 30.09.2016
(51) Int. Cl.: A61K 31/551, A61K 31/165, A61K 31/407, A61K 31/424, A61K 31/427, A61K 31/43, A61K 31/4439, A61K 31/496, A61K 31/5377, A61K 31/545, A61K 31/546, A61K 31/665, A61K 31/675, A61K 31/7036, A61K 31/7048, A61K 31/7056, A61K 38/14, A61K 38/12, C07D 471/08, A61P 31/04

(54) **COMPOSITION COMPRISING ANTIBIOTIC COMPOUND AND AN HETEROCYCLIC COMPOUND AND THEIR USE IN PREVENTING OR TREATING BACTERIAL INFECTIONS**
ZUSAMMENSETZUNG MIT EINER ANTIBIOTISCHEN VERBINDUNG UND HETEROCYCLISCHEN VERBINDUNG UND DEREN VERWENDUNG BEI DER VORBEUGUNG ODER BEHANDLUNG BAKTERIELLER INFEKTIONEN
COMPOSITION COMPRENANT UN COMPOSÉ ANTIBIOTIQUE ET UN COMPOSÉ HÉTÉROCYCLIQUE ET LEUR UTILISATION POUR PRÉVENIR OU TRAITER DES INFECTIONS BACTÉRIENNES

(43) Date of publication of application: 04.04.2018
(73) Proprietor: Mutabilis, 93230 Romainville (FR)
(72) Inventor: BONNARD, Damien, 75019 Paris (FR); LE ROUZIC, Erwann, 75011 Paris (FR); MOREAU, François, 91400 Orsay (FR)
(74) Representative: Lavoix

(56) References cited:
- EP-A1- 3 091 018
- WO-A1-2013/150296
- WO-A1-2014/141132
- WO-A1-2016/156344
- WO-A1-2016/156346
- WO-A1-2016/156348
- WO-A1-2016/156597
- WO-A1-2016/177862
- WO-A2-02/100860

## Description

The present invention relates to a composition comprising at least one antibiotic compound and at least one heterocyclic compound, their process of preparation, and use thereof for the prevention or treatment of bacterial infections. The present invention also relates to the use of these compounds as beta-lactamase inhibitors and/or antibacterial agent.

It has been described that there is a continuous evolution of antibacterial resistance which could lead to bacterial strains against which known antibacterial compounds are inefficient. There is thus a need to provide novel composition that can overcome bacterial antibiotic resistance and give enhanced treatment.

The objective of the present invention is to provide a composition that can be used as antibacterial agent and/or beta-lactamase inhibitor. An objective of the present invention is also to provide a composition that can be used for the prevention or treatment of bacterial infections. Another objective of the present invention is to provide such composition which can overcome bacterial antibiotic resistance. Other objectives will appear throughout the following description of the invention.

The invention is defined by the claims. Any subject-matter falling outside the scope of the claims is provided for information purposes only. Furthermore, any references in the description to methods of treatment refer to the compounds, pharmaceutical compositions and medicaments of the present invention for use in a method of treatment of the human or animal body by therapy.

The present invention relates to a composition comprising:
- at least one antibiotic chosen among: Amikacin, Ciprofloxacin, Clindamycin, Chloramphenicol, Colistin, Daptomycin, Erythromycin, Fosfomycin, Linezolid, Rifampicine, Synercid, Tigecycline, Vancomycin, Sulbactam; and
- at least one compound of formula (I) 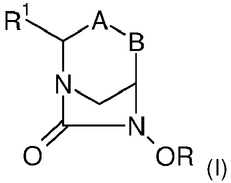 in which
   - R is chosen among -SO₃H or CF₂CO₂H;
   - R¹ is chosen among H, CH₂-NH₂, CONH₂ or a 5-membered heteroaryl comprising at least two nitrogen;
   - A-B is 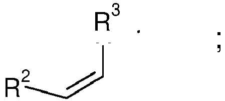 R² is a hydrogen atom or a saturated, partially or totally unsaturated or aromatic 4- to 10-membered heterocycle, comprising at least one nitrogen atom, unsubstituted or substituted by one or more T¹, preferably unsubstituted;
      R³ is H, or oxazolyl; R⁴ is -(CH₂)₂NH₂ or -CH₂CONH₂;
      T¹, identical or different, independently represents a fluorine atom ; -(CH₂)ₘOQ¹ ; - (CH₂)ₘ-CN ; -(CH₂)ₘ-OC(O)Q¹ ; -(CH₂)ₘ-C(O)OQ¹ ; -(CH₂)ₘ-OC(O)OQ¹ ; -(CH₂)ₘ-OC(O)NQ¹Q² ; -(CH₂)ₘ-C(O)NQ¹Q² ; -(CH₂)ₘ-C(O)ONQ¹Q² ; -(CH₂)ₘ-C(O)NQ¹OQ²; - (CH₂)ₘ-C(O)NQ¹-NQ¹Q² ; -(CH₂)ₘ-NQ¹C(O)Q² ; -(CH₂)ₘ-NQ¹S(O)₂NQ¹Q² ; -(CH₂)ₘ-NQ¹S(O)₂Q² ; -(CH₂)ₘ-S(O)₂NQ¹Q² ; -(CH₂)ₘ-NQ¹C(O)OQ² ; -(CH₂)ₘ-NQ¹C(O)NQ¹Q² ; - (CH₂)ₘ-NQ¹Q² ; -(CH₂)ₘ-NH-C(NHQ³)=NQ⁴ ; -(CH₂)ₘ-NH-CH=NQ³ ; -(CH₂)ₘ-C(NHQ³)=NQ⁴ ; -(X)-(CH₂)ₚOQ¹ ; -(X)-(C_{H2})ₙ-CN ; -(X)-(CH₂)ₚ-OC(O)Q¹ ; -(X)-(CH₂)ₙ-C(O)OQ¹ ; -(X)-(CH₂)ₚ-OC(O)OQ¹ ; -(X)-(CH₂)ₚ-OC(O)NQ¹Q²; -(X)-(CH₂)ₙ-C(O)NQ¹Q² ; -(X)-(CH₂)ₙ-C(O)ONQ¹Q² ; -(X)-(CH₂)ₙ-C(O)NQ¹OQ² ; -(X)-(CH₂)ₙ-C(O)NQ¹-NQ¹Q² ;-(X)-(CH₂)ₚ-NQ¹C(O)Q² ; -(X)-(CH₂)ₚ-NQ¹S(O)₂NQ¹Q² ; -(X)-(CH₂)ₚ-NQ¹S(O)₂Q² ; -(X)-(CH₂)ₚ-S(O)₂NQ¹Q² ; -(X)-(CH₂)ₚ-NQ¹C(O)OQ² ; -(X)-(CH₂)ₚ-NQ¹C(O)NQ¹Q² ; -(X)-(CH₂)ₚ-NQ¹Q² ; -(X)-(CH₂)ₚ-NH-C(NHQ³)=NQ⁴ ; -(X)-(CH₂)ₚ-NH-CH=NQ³ ; -(X)-(CH₂)ₙ-C(NHQ³)=NQ⁴ ; -C(O)-(CH₂)ₙOQ¹ ; -C(O)-(CH₂)ₙ-CN ; -C(O)-(CH₂)ₙ-OC(O)Q¹ ; -C(O)-(CH₂)ₙ-C(O)OQ¹ ; -C(O)-(CH₂)ₙ-OC(O)OQ¹ ; -C(O)-(C_{H2})ₙ-OC(O)NQ¹Q²; -C(O)-(CH₂)ₙ-C(O)NQ¹Q² ; -C(O)-(CH₂)ₙ-C(O)ONQ¹Q² ; -C(O)-(CH₂)ₙ-C(O)NQ¹OQ² ; -C(O)-(CH₂)ₙ-C(O)NQ¹-NQ¹Q² ; -C(O)-(CH₂)ₙ-NQ¹C(O)Q² ; -C(O)-(CH₂)ₙ-NQ¹S(O)₂NQ¹Q² ; -C(O)-(CH₂)ₙ-NQ¹S(O)₂Q² ; -C(O)-(CH₂)ₙ-S(O)₂NQ¹Q²; -C(O)-(CH₂)ₙ-NQ¹C(O)OQ² ; -C(O)-(CH₂)ₙ-NQ¹C(O)NQ¹Q² ; -C(O)-(CH₂)ₙ-NQ¹Q² ; -C(O)-(CH₂)ₙ-NH-C(NHQ³)=NQ⁴ ; -C(O)-(CH₂)ₙ-NH-CH=NQ³ ; -C(O)-(CH₂)ₙ-C(NHQ³)=NQ⁴ or T¹, identical or different, independently represents an unsubstituted or substituted by one or more T², -(CH₂)ₘ-(4-, 5- or 6-membered saturated, partially or totally unsaturated or aromatic heterocycle) ; -(X)-(CH₂)ₘ-(4-, 5- or 6-membered saturated, partially or totally unsaturated or aromatic heterocycle) ; (C₁-C₃)-alkyl ; (C₁-C₃)-fluoroalkyl ; -(X)-(C₁-C₃)-alkyl ; -(X)-(C₁-C₃)-fluoroalkyl ; -(CH₂)ₘ-(C₃-C₆)-cycloalkyl ; -(X)-(CH₂)ₘ-(C₃-C₆)-cycloalkyl ; -(CH₂)ₘ-(C₃-C₆)-cyclofluoroalkyl ; -(X)-(CH₂)ₘ-(C₃-C₆)-cyclofluoroalkyl ; - C(O)-(CH₂)ₘ-(4-, 5- or 6-membered saturated, partially or totally unsaturated or aromatic heterocycle) ; -C(O)-(C₁-C₃)-alkyl ; -C(O)-(C₁-C₃)-fluoroalkyl ; -C(O)O-(C₁-C₃)-fluoroalkyl ; -C(O)-(CH₂)ₘ-(C₃-C₆)-cycloalkyl; -C(O)-(CH₂)ₘ-(C₃-C₆)-cycloalkyl ; -C(O)-(CH₂)ₘ-(C₃-C₆)-cyclofluoroalkyl ; -C(O)-(CH₂)ₘ-(C₃-C₆)-cyclofluoroalkyl ;
      Q¹ and Q², identical or different, independently represent a hydrogen atom ; -(CH₂)ᵣ-NHQ³ ; -(CH₂)ᵣ-NH-C(NHQ³)=NQ⁴ ; -(CH₂)ᵣ-NH-CH=NQ³ ; (CH₂)ₙ-C(NHQ³)=NQ⁴ ; - (CH₂)ᵣ-OQ³ ; -(CH₂)ₙ-CONHQ³ ; or an unsubstituted or substituted by one or more T², (C₁-C₃)-alkyl ; (C₁-C₃)-fluoroalkyl ; saturated, partially or totally unsaturated or aromatic-(CH₂)ₘ-(4-, 5- or 6-membered heterocycle comprising at least one nitrogen atom) ; or Q¹, Q² and the nitrogen atom to which they are bonded, form together an unsubstituted or substituted by one or more T², saturated or partially unsaturated 4-, 5- or 6-membered heterocycle comprising 1, 2 or 3 heteroatoms ;
      Q³ and Q⁴, identical or different, independently represent a hydrogen atom or (C₁-C₃)-alkyl;
      T², identical or different, independently represents -OH ; -NH₂ ; -CONH₂;
      m, identical or different, independently represents 0, 1, 2 or 3 ;
      n, identical or different, independently represents 1, 2 or 3 ;
      p, identical or different, independently represents 2 or 3 ;
      r is 1, 2 or 3 when the (CH₂)ᵣ is directly linked to a carbon atom or 2 or 3 otherwise, preferably r is 2 or 3;
      X, identical or different, independently represents O ; S ; S(O) ; S(O)₂ or N(Q³);
   - at least one of R² and R³ is different from H and R² and R³ are not simultaneously H ; and a racemate, an enantiomer, a diastereoisomer, a geometric isomer or a pharmaceutically acceptable salt of the compound of formula (I).
The present invention relates to a composition comprising:
- at least one antibiotic chosen among: Amoxicillin, Amikacin, Aztreonam, Ciprofloxacin, Clindamycin, Chloramphenicol, Colistin, Cefpirome, Ceftriaxone, Cefotaxime, Daptomycin, Erythromycin, Cefepime, Cefixime, Fosfomycin, Cefuroxime, Imipenem, Linezolid, Meropenem, Piperacillin, Cefpodoxime, Rifampicine, Synercid, Tigecycline, Vancomycin, Sulbactam, Ceftaroline, Cefiderocol (S-649266) or BAL30072; and
- at least one compound selected among : 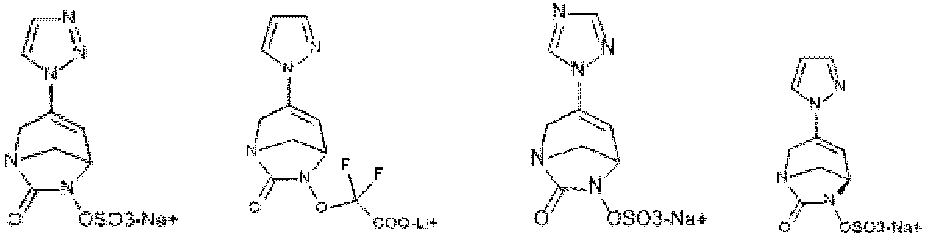 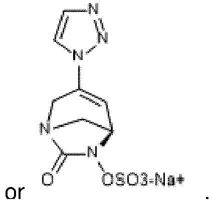
The compounds of formula (I) can be prepared according to methods described in application EP15305479.6; EP15305473.9; EP15305701.3; EP15305508.2; EP15307125.3; EP16305073.5; EP16305144.4; EP16305059.4; PCT/EP2016/056845; PCT/EP2016/056847; PCT/EP2016/060142 and PCT/EP2016/057274.

In the composition of the invention, compound of formula (I) can be specifically a compound of formula (I*): 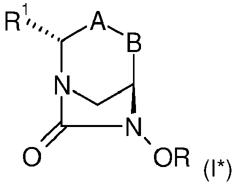 wherein R¹, A, B and R are as defined for formula (I). It should be understood that when A-B is 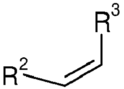 A is CR² and B is CR³.
The composition is preferably a pharmaceutical composition and preferably comprises at least one pharmaceutical excipient.

Preferably, in the compounds of formula (I) or (I*) according to the invention R² is chosen among H, or a 5 to 6-membered heterocycle, preferably monocycle, saturated, partially or totally unsaturated or aromatic, preferably aromatic, comprising at least one nitrogen atom.

Preferably, in the compounds of formula (I) or (I*) according to the invention R² is chosen among H or an heterocycle chosen among oxazolyl, pyrazolyl, pyridinyl or triazolyl.

Preferably, the compounds of formula (I) or (I*) are chosen among: 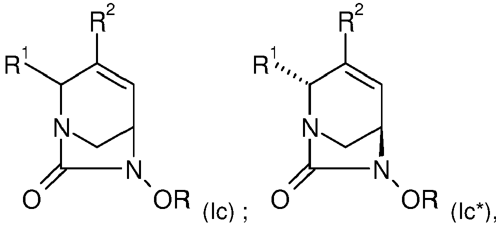 wherein R, R¹ and R² are as defined above, preferably R¹ is CH₂NH₂ or H and preferably R² is , a 5 to 6-membered heterocycle, preferably monocycle, saturated, partially or totally unsaturated or aromatic, preferably aromatic, comprising at least one nitrogen atom, preferably R² is an heterocycle chosen among oxazolyl, pyrazolyl, pyridinyl or triazolyl; or 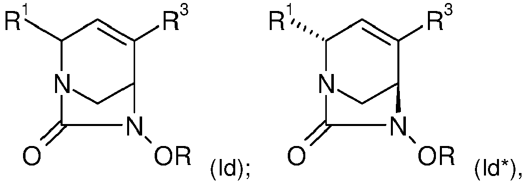 wherein R, R¹ and R³ are as defined above, preferably R¹ is CH₂NH₂.

The following compounds of formula (I) or (I*) are disclosed herein:
sodium and 2,2,2-trifluoroacetate [*trans*-2-(azaniumylmethyl)-4-oxazol-5-yl-7-oxo-1,6-diazabicyclo[3.2.1]oct-3-en-6-yl] sulfate
sodium and 2,2,2-trifluoroacetate [(2S, 5S)-4-(2-ammoniumethoxyimino)-2-carbamoyl-7-oxo-1,6-diazabicyclo[3.2.1]octan-6-yl] sulfate
sodium and 2,2,2-trifluoroacetate [5-(2-azaniumethyl)-4,9-dioxo-3-thia-5,8,10-triazatricyclo[6.2.1.0^{2,6}]undec-2(6)-en-10-yl] sulfate
sodium and 2,2,2-trifluroacetate [(2S,5S)-4-(2-amino-2-oxo-ethoxy)imino-2-(azaniumylmethyl)-7-oxo-1,6-diazabicyclo[3.2.1]octan-6-yl] sulfate
sodium and 2,2,2-trifluoroacetate [(2*S*,5*R*)-2-(azaniumylmethyl)-7-oxo-3-oxazol-2-yl-1,6-diazabicyclo[3.2.1]oct-3-en-6-yl] sulfate
sodium and 2,2,2-trifluoroacetate [*trans*-7-(1*H*-imidazol-3-ium--2-yl)-9-oxo-3-thia-5,8,10-triaza-tricyclo[6.2.1.0^{2,6}]undeca-2(6),4-dien-10-yl]sulfate
lithium difluoro-(3-oxazol-3-yl-7-oxo-1,6-diazabicyclo[3.2.1]oct-3-en-6-yloxy]-acetate sodium [3-(3-pyridyl)-7-oxo-1,6-diazabicyclo[3.2.1]oct-3-en-6-yl] sulfate
sodium [(5*R*)-3-(oxazol-2-yl)-7-oxo-1,6-diazabicyclo[3.2.1]oct-3-en-6-yl] sulfate [5-(3-aminopropyl)-4,9-dioxo-3-thia-5,8,10-triaza-tricyclo[6.2.1.0^{2,6}]undec-2(6)-en-10-yl] hydrogen sulfate
sodium (7-oxo-3-pyrazol-1-yl-1,6-diazabicyclo[3.2.1]oct-3-en-6-yl) sulfate
sodium [7-oxo-3-(triazol-1-yl)-1,6-diazabicyclo[3.2.1]oct-3-en-6-yl] sulfate
lithium difluoro-(7-oxo-3-pyrazol-1-yl-1,6-diaza-bicyclo[3.2.1]oct-3-en-6-yloxy)-acetate
sodium [7-oxo-3-(1,2,4-triazol-1-yl)-1,6-diazabicyclo[3.2.1]oct-3-en-6-yl] sulfate
sodium [(5*R*)-7-oxo-3-pyrazol-1-yl-1,6-diazabicyclo[3.2.1]oct-3-en-6-yl]sulfate
sodium [(5*R*)-7-oxo-3-(triazol-1-yl)-1,6-diazabicyclo[3.2.1]oct-3-en-6-yl] sulfate.
Preferably, in the composition of the invention, the antibiotic is chosen among Amoxicillin, Amikacin, Aztreonam, Ciprofloxacin, Clindamycin, Chloramphenicol, Colistin, Cefpirome, Ceftriaxone, Cefotaxime, Daptomycin, Erythromycin, Cefepime, Cefixime, Fosfomycin, Cefuroxime, Imipenem, Linezolid, Meropenem, Piperacillin, Cefpodoxime, Rifampicine, Synercid, Tigecycline, Vancomycin, Sulbactam, Ceftaroline.

Preferably, in the composition of the invention, the antibiotic is chosen among Amoxicillin, Aztreonam, Cefpirome, Ceftriaxone, Cefotaxime, Cefepime, Cefixime, Fosfomycin, Cefuroxime, Imipenem, Meropenem, Piperacillin, Cefpodoxime, Sulbactam.

Preferably, in the composition of the invention, the antibiotic is chosen among Meropenem, Aztreonam, Piperacillin, Fosfomycin, Cefepime, Cefixime or Sulbactam.

Preferably, in the composition of the invention:
R² is chosen among H, or a 5 to 6-membered heterocycle comprising at least one nitrogen atom; and
the antibiotic is chosen among Amikacin, Ciprofloxacin, Clindamycin, Chloramphenicol, Colistin, Daptomycin, Erythromycin, Fosfomycin, Linezolid, Rifampicine, Synercid, Tigecycline, Vancomycin, Sulbactam; or
the antibiotic is chosen among Fosfomycin, , Sulbactam.

Preferably, in the composition of the invention:
R² is chosen among H or an heterocycle chosen among oxazolyl, pyrazolyl, pyridinyl or triazolyl; and
the antibiotic is chosen among Amikacin, Ciprofloxacin, Clindamycin, Chloramphenicol, ColistinDaptomycin, Erythromycin, Fosfomycin, Linezolid, Rifampicine, Synercid, Tigecycline, Vancomycin, Sulbactam; or
the antibiotic is chosen among Fosfomycin, Sulbactam.

All the preferences of compounds of formula (I) or (I*) and of the antibiotic can be combined to describe preferred composition of the invention.

The term "alkyl", as used herein, refers to an aliphatic-hydrocarbon group which may be straight or branched, having 1 to 3 carbon atoms in the chain unless specified otherwise. Preferred alkyl groups have 1 or 2 carbon atoms in the chain. Specific examples of alkyl groups include, but are not limited to, methyl, ethyl, n-propyl, iso propyl. Preferably, the alkyl group is methyl or ethyl.

The term "fluoroalkyl", as used herein, refers to an alkyl group substituted with at least one fluorine atom. The term "alkyl" is as defined above. Specific examples of fluoroalkyl groups include but are not limited to trifluoromethyl, difluoromethyl, fluoromethyl.

The term "cycloalkyl" refers to a saturated monocyclic or bicyclic non-aromatic hydrocarbon ring of 3 to 6 carbon atoms, preferably 3 to 4 carbon atoms, which can comprise one or more unsaturation. Specific examples of monocyclic cycloalkyl groups include cyclopropyl, cyclobutyl, cyclopentyl, cyclohexyl. Preferably, the cycloalkyl group is cyclopropyl or cyclobutyl.

The term "cyclofluoroalkyl" refers to a cycloalkyl group substituted with at least one fluorine atom. The term "cycloalkyl" is as defined above. Specific examples of cyclofluoroalkyl groups include fluorocyclopropyl, difluorocyclopropyl, fluorocyclobutyl, difluorocyclobutyl.

The term "heterocycle", as used herein and without contrary definition specifically mentioned, either alone or in combination with another radical, refers to a monocyclic or bicyclic saturated, partially or totally unsaturated or aromatic hydrocarbon radical, preferably 4 to 10-membered, comprising at least one heteroatom, such as N, O, S, S(O) or S(O)₂. Preferably, the heterocycle is a monocyclic saturated, partially or totally unsaturated or aromatic hydrocarbon radical, preferably 4, 5- or 6-membered, comprising at least one nitrogen atom and which can comprise at least one further heteroatom, such as N, O, S, S(O) or S(O)₂, the carbon atoms of the heterocycle can also be oxidized as C(O). Preferably, the number of heteroatom in the cycle is comprised between 1 and 4, preferably between 2 and 3. Suitable heterocycles are also disclosed in the Handbook of Chemistry and Physics, 76th Edition, CRC Press, Inc., 1995-1996, pages 2 25 to 2-26. Examplary heterocycle groups include, but are not limited to, azetidinyl, oxetanyl, oxazolyl, oxazolidinyl, oxadiazolyl, pyrrolyl, pyrrolidinyl, pyridyl, tetrahydropyridinyl, piperidinyl, morpholinyl, pyrazolyl, pyrimidinyl, pyrazinyl, tetrazolyl, imidazolyl, thienyl, thiazolyl, furanyl, thiadiazolyl, isothiazolyl, triazolyl, tetrazolyl, pyrazolyl, isoxazolyl, 2-pyrrolidinonyl, imidazol-2,4-dione, 1,2,4-oxadiazolyl-5-one, 1,5-dihydropyrrolyl-2-one, pyrazinone, pyridazinone, pyridone, pyrimidone, dioxanyl, pyrrolidinyl, imidazolidinyl, pyranyl, tetrahydrofuranyl, dioxolanyl, tetrahydropyranyl.

The term heteroaryl relates to aromatic heterocycle.

The term heteroatom relates to an atom chosen among O, N or S.

Moreover some compounds according to this invention may contain a basic amino group and thus may form an inner zwitterionic salt (or zwitterion) with the acidic group (R) -OSO₃H, or - OCF₂CO₂H and such inner zwitterionic salts are also included in this invention.

The term "optionally substituted" means "non-substituted or substituted".

The term "racemate" is employed herein to refer to an equal amount of two specific enantiomers.

The term "enantiomer" is employed herein to refer to one of the two specific stereoisomers which is a non-superimposable mirror image with one other but is related to one other by reflection.

The compounds of the invention can possess one or more asymmetric carbon atoms and are thus capable of existing in the form of optical isomers as well as in the form of racemic or non-racemic mixtures thereof. The compounds of the invention can be used in the present invention as a single isomer or as a mixture of stereochemical isomeric forms. Diastereoisomers, i.e., nonsuperimposable stereochemical isomers can be separated by conventional means such as chromatography, distillation, crystallization or sublimation. The optical isomers (enantiomers) can be obtained by using optically active starting materials, by resolution of the racemic mixtures according to conventional processes, for example by formation of diastereoisomeric salts by treatment with an optically active acid or base or by using chiral chromatography column.

The expression "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the expression "pharmaceutically acceptable salts" refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids or aminohydroxyl-*O*-sulfonic acid; and the like. The pharmaceutically acceptable salts of the present invention can be synthesized from the parent compound which comprises a basic or an acidic moiety, by conventional chemical methods. Furthermore, the expression "pharmaceutically acceptable salt" refers to relatively non-toxic, inorganic and organic acid or base addition salts of the compounds of the present invention. These salts can be prepared in situ during the final isolation and purification of the compounds. In particular, the acid addition salts can be prepared by separately reacting the purified compound in its purified form with an organic or inorganic acid and by isolating the salt thus formed. Among the examples of acid addition salts are the hydrobromide, hydrochloride, hydroiodide, sulfamate, sulfate, bisulfate, phosphate, nitrate, acetate, propionate, succinate, oxalate, valerate, oleate, palmitate, stearate, laurate, borate, benzoate, lactate, tosylate, citrate, maleate, fumarate, tartrate, naphthylate, mesylate, glucoheptanate, glucoronate, glutamate, lactobionate, malonate, salicylate, methylenebis-b-hydroxynaphthoate, gentisic acid, isethionate, di-p-toluoyltartrate, ethanesulfonate, benzenesulfonate, cyclohexyl sulfamate, quinateslaurylsulfonate salts, and the like. Examples of base addition salts include ammonium salts such as tromethamine, meglumine, epolamine, etc, metal salts such as sodium, lithium, potassium, calcium, zinc or magnesium salts with organic bases such as dicyclohexylamine salts, N-methyl-D-glucamine. Lists of suitable salts may be found in Remington's Pharmaceutical Sciences, 17th ed., Mack Publishing Company, Easton, PA, 1985, p. 1418, P.H. Stahl, C.G. Wermuth, Handbook of Pharmaceutical salts - Properties, Selection and Use, Wiley-VCH, 2002 and S.M. Berge et al. "Pharmaceutical Salts" J. Pharm. Sci, 66: p.1-19 (1977).

Compounds according to the invention also include isotopically-labeled compounds wherein one or more atoms is replaced by an atom having the same atomic number, but an atomic mass or mass number different from the atomic mass or mass number usually found in nature. Examples of isotopes suitable for inclusion in the compounds described above and are not limited to ²H, ³H, ¹¹C, ¹³C, ¹⁴C,¹⁹F, ¹⁸F, ¹⁵N, ¹³N, ³³S, ³⁴S, ³⁵S, ³⁶S, ¹⁷O or ¹⁸O. In one embodiment, isotopically-labeled compounds are useful in drug and/or substrate tissue distribution studies. In another embodiment, substitution with heavier isotopes such as deuterium (²H) affords greater metabolic stability (for example increased in vivo half-life or reduced dosage requirements). Isotopically-labeled compounds are prepared by any suitable method or by processes using an appropriate isotopically-labeled reagent in place of the non-labeled reagent otherwise employed.

The term "pharmaceutically acceptable carrier" or "pharmaceutically acceptable excipient" is employed for any excipient, solvent, dispersion medium, absorption retardant, diluent or adjuvant etc., such as preserving or antioxidant agents, fillers, binders, disintegrating agents, wetting agents, emulsifying agents, suspending agents, solvents, dispersion media, coatings, antibacterial agents, isotonic and absorption delaying agents and the like, that does not produce a secondary reaction, for example an allergic reaction, in humans or animals. Typical, non-limiting examples of excipients include mannitol, lactose, magnesium stearate, sodium saccharide, talcum, cellulose, sodium croscarmellose, glucose, gelatin, starch, lactose, dicalcium phosphate, sucrose, kaolin, magnesium carbonate, wetting agents, emulsifying agents, solubilizing agents, sterile water, saline, pH buffers, non-ionic surfactants, lubricants, stabilizing agents, binding agents and edible oils such as peanut oil, sesame oils and the like. In addition, various excipients commonly used in the art may be included. Pharmaceutically acceptable carriers or excipients are well known to a person skilled in the art, and include those described in Remington's Pharmaceutical Sciences (Mack Publishing Company, Easton, USA, 1985), Merck Index (Merck & Company, Rahway, N.J.), Gilman et al (Eds. The pharmacological basis of therapeutics, 8th Ed., pergamon press., 1990). Except insofar as any conventional media or adjuvant is incompatible with the active ingredient according to the invention, its use in the therapeutic compositions is contemplated.

It is also described a kit comprising:
- at least one antibiotic as defined above; and
- at least one compound of formula (I) or formula (I*) as defined above,
for the treatment or prevention of bacterial infections by its simultaneous, separate or sequential administration to a patient in need thereof.

It is also described a kit comprising:
- at least one pharmaceutical composition comprising at least one antibiotic as defined above and optionally an acceptable pharmaceutically excipient; and
- at least one pharmaceutical composition comprising at least one compound of formula (I) or formula (I*) as defined above and optionally an acceptable pharmaceutically excipient;
for the treatment or prevention of bacterial infections by its simultaneous, separate or sequential administration to a patient in need thereof.

The present invention also relates to a kit comprising :
- at least one antibiotic chosen among: Amikacin, Ciprofloxacin, Clindamycin, Chloramphenicol, Colistin, Daptomycin, Erythromycin, Fosfomycin, Linezolid, Rifampicine, Synercid, Tigecycline, Vancomycin, Sulbactam; and
- at least one compound of formula (I) 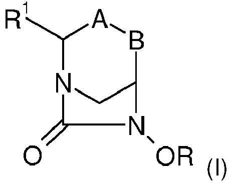 in which
   - R is chosen among -SO₃H or CF₂CO₂H;
   - R¹ is chosen among H, CH₂-NH₂, CONH₂ or a 5-membered heteroaryl comprising at least two nitrogen;
   - A-B is 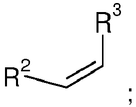 R² is a hydrogen atom or a saturated, partially or totally unsaturated or aromatic 4- to 10-membered heterocycle, comprising at least one nitrogen atom, unsubstituted or substituted by one or more T¹, preferably unsubstituted;
      R³ is H, or oxazolyl;
      T¹, identical or different, independently represents a fluorine atom ; -(CH₂)ₘOQ¹ ; - (CH₂)ₘ-CN ; -(CH₂)ₘ-OC(O)Q¹; -(CH₂)ₘ-C(O)OQ¹ ; -(CH₂)ₘ-OC(O)OQ¹ ; -(CH₂)ₘ-OC(O)NQ¹Q² ; -(CH₂)ₘ-C(O)NQ¹Q² ; -(CH₂)ₘ-C(O)ONQ¹Q² ; -(CH₂)ₘ-C(O)NQ¹OQ²; - (CH₂)ₘ-C(O)NQ¹-NQ¹Q² ; -(CH₂)ₘ-NQ¹C(O)Q² ; -(CH₂)ₘ-NQ¹S(O)₂NQ¹Q² ; -(CH₂)ₘ-NQ¹S(O)₂Q² ; -(CH₂)ₘ-S(O)₂NQ¹Q² ; -(CH₂)ₘ-NQ¹C(O)OQ² ; -(CH₂)ₘ-NQ¹C(O)NQ¹Q² ; - (CH₂)ₘ-NQ¹Q² ; -(CH₂)ₘ-NH-C(NHQ³)=NQ⁴ ; -(CH₂)ₘ-NH-CH=NQ³ ; -(CH₂)ₘ-C(NHQ³)=NQ⁴ ; -(X)-(CH₂)ₚOQ¹ ; -(X)-(CH₂)ₙ-CN ; -(X)-(CH₂)ₚ-OC(O)Q¹ ; -(X)-(CH₂)ₙ-C(O)OQ¹ ; -(X)-(CH₂)ₚ-OC(O)OQ¹ ; -(X)-(CH₂)ₚ-OC(O)NQ¹Q² ; -(X)-(CH₂)ₙ-C(O)NQ¹Q² ; -(X)-(CH₂)ₙ-C(O)ONQ¹Q² ; -(X)-(CH₂)ₙ-C(O)NQ¹OQ² ; -(X)-(CH₂)ₙ-C(O)NQ¹-NQ¹Q² ;-(X)-(CH₂)ₚ-NQ¹C(O)Q² ; -(X)-(CH₂)ₚ-NQ¹S(O)₂NQ¹Q² ; -(X)-(CH₂)ₚ-NQ¹S(O)₂Q² ; -(X)-(CH₂)ₚ-S(O)₂NQ¹Q² ; -(X)-(CH₂)ₚ-NQ¹C(O)OQ² ; -(X)-(CH₂)ₚ-NQ¹C(O)NQ¹Q² ; -(X)-(CH₂)ₚ-NQ¹Q² ; -(X)-(CH₂)ₚ-NH-C(NHQ³)=NQ⁴ ; -(X)-(CH₂)ₚ-NH-CH=NQ³ ; -(X)-(CH₂)ₙ-C(NHQ³)=NQ⁴ ; -C(O)-(CH₂)ₙOQ¹ ; -C(O)-(CH₂)ₙ-CN ; -C(O)-(CH₂)ₙ-OC(O)Q¹ ; -C(O)-(CH₂)ₙ-C(O)OQ¹ ; -C(O)-(CH₂)ₙ-OC(O)OQ¹ ; -C(O)-(CH₂)ₙ-OC(O)NQ¹Q²; -C(O)-(CH₂)ₙ-C(O)NQ¹Q² ; -C(O)-(CH₂)ₙ-C(O)ONQ¹Q² ; -C(O)-(CH₂)ₙ-C(O)NQ¹OQ² ; -C(O)-(CH₂)ₙ-C(O)NQ¹-NQ¹Q² ; -C(O)-(CH₂)ₙ-NQ¹C(O)Q² ; -C(O)-(CH₂)ₙ-NQ¹S(O)₂NQ¹Q² ; -C(O)-(CH₂)ₙ-NQ¹S(O)₂Q² ; -C(O)-(CH₂)ₙ-S(O)₂NQ¹Q²; -C(O)-(CH₂)ₙ-NQ¹C(O)OQ² ; -C(O)-(CH₂)ₙ-NQ¹C(O)NQ¹Q² ; -C(O)-(CH₂)ₙ-NQ¹Q² ; -C(O)-(CH₂)ₙ-NH-C(NHQ³)=NQ⁴ ; -C(O)-(CH₂)ₙ-NH-CH=NQ³ ; -C(O)-(CH₂)ₙ-C(NHQ³)=NQ⁴ or T¹, identical or different, independently represents an unsubstituted or substituted by one or more T², -(CH₂)ₘ-(4-, 5- or 6-membered saturated, partially or totally unsaturated or aromatic heterocycle) ; -(X)-(CH₂)ₘ-(4-, 5- or 6-membered saturated, partially or totally unsaturated or aromatic heterocycle) ; (C₁-C₃)-alkyl ; (C₁-C₃)-fluoroalkyl ; -(X)-(C₁-C₃)-alkyl ; -(X)-(C₁-C₃)-fluoroalkyl ; -(CH₂)ₘ-(C₃-C₆)-cycloalkyl ; -(X)-(CH₂)ₘ-(C₃-C₆)-cycloalkyl ; -(CH₂)ₘ-(C₃-C₆)-cyclofluoroalkyl ; -(X)-(CH₂)ₘ-(C₃-C₆)-cyclofluoroalkyl ; - C(O)-(CH₂)ₘ-(4-, 5- or 6-membered saturated, partially or totally unsaturated or aromatic heterocycle) ; -C(O)-(C₁-C₃)-alkyl ; -C(O)-(C₁-C₃)-fluoroalkyl ; -C(O)O-(C₁-C₃)-fluoroalkyl ; -C(O)-(CH₂)ₘ-(C₃-C₆)-cycloalkyl; -C(O)-(CH₂)ₘ-(C₃-C₆)-cycloalkyl ; -C(O)-(CH₂)ₘ-(C₃-C₆)-cyclofluoroalkyl ; -C(O)-(CH₂)ₘ-(C₃-C₆)-cyclofluoroalkyl ; Q¹ and Q², identical or different, independently represent a hydrogen atom ; -(CH₂)ᵣ-NHQ³ ; -(CH₂)ᵣ-NH-C(NHQ³)=NQ⁴ ; -(CH₂)ᵣ-NH-CH=NQ³ ; (CH₂)ₙ-C(NHQ³)=NQ⁴ ; - (CH₂)ᵣ-OQ³ ; -(CH₂)ₙ-CONHQ³ ; or an unsubstituted or substituted by one or more T², (C₁-C₃)-alkyl ; (C₁-C₃)-fluoroalkyl ; saturated, partially or totally unsaturated or aromatic-(CH₂)ₘ-(4-, 5- or 6-membered heterocycle comprising at least one nitrogen atom) ; or Q¹, Q² and the nitrogen atom to which they are bonded, form together an unsubstituted or substituted by one or more T², saturated or partially unsaturated 4-, 5- or 6-membered heterocycle comprising 1, 2 or 3 heteroatoms ; Q³ and Q⁴, identical or different, independently represent a hydrogen atom or (C₁-C₃)-alkyl;
      T², identical or different, independently represents -OH ; -NH₂ ; -CONH₂;
      m, identical or different, independently represents 0, 1, 2 or 3 ;
      n, identical or different, independently represents 1, 2 or 3 ;
      p, identical or different, independently represents 2 or 3 ;
      r is 1, 2 or 3 when the (CH₂)ᵣ is directly linked to a carbon atom or 2 or 3 otherwise, preferably r is 2 or 3;
   - X, identical or different, independently represents O ; S ; S(O) ; S(O)₂ or N(Q³);
   - at least one of R² and R³ is different from H and R² and R³ are not simultaneously H;
      and a racemate, an enantiomer, a diastereoisomer, a geometric isomer or a pharmaceutically acceptable salt of the compound of formula (I)
      for the treatment or prevention of bacterial infections by its simultaneous, separate or sequential administration to a patient in need thereof.
Preferably, the compound of formula (I) is a compound of formula (I*) 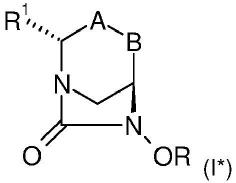 wherein R¹, A, B and R are as defined above.

The invention also relates to a kit comprising :
- at least one antibiotic chosen among: Amoxicillin, Amikacin, Aztreonam, Ciprofloxacin, Clindamycin, Chloramphenicol, Colistin, Cefpirome, Ceftriaxone, Cefotaxime, Daptomycin, Erythromycin, Cefepime, Cefixime, Fosfomycin, Cefuroxime, Imipenem, Linezolid, Meropenem, Piperacillin, Cefpodoxime, Rifampicine, Synercid, Tigecycline, Vancomycin, Sulbactam, Ceftaroline; Cefiderocol (S-649266), BAL30072; and
- at least one compound selected among : 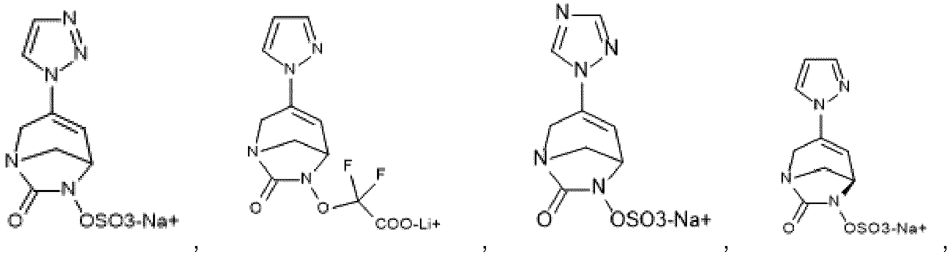 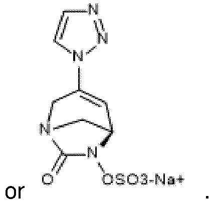 for the treatment or prevention of bacterial infections by its simultaneous, separate or sequential administration to a patient in need thereof.

The two composition can be prepared separately each with one specific pharmaceutically acceptable carrier, and can be mix especially extemporaneity.

The present invention also related to the composition according to the invention for its use as a medicine.
The present invention also relates to the use of the composition according to the invention or the kit according to the invention for the preparation of a medicine.

The present invention also relates to the composition according to the invention for its use as an antibacterial agent and/or as a β-lactamase inhibitor.
The present invention also relates to the use of the composition according to the invention or the kit according to the invention for the preparation of an antibacterial agent medicine.
The present invention also related to the use of the composition or kit according to the invention for the preparation of an inhibitor of beta-lactamase medicine.
The present invention also related to the use of the composition or kit according to the invention for the preparation of an antibacterial agent and inhibitor of beta-lactamase medicine.

The term "beta-lactam" or "β-lactam" refers to antibacterial compounds comprising a β-lactam unit, i.e. a group.

The present invention also relates to the composition according to the invention or the kit according to the invention for its use for treating or preventing a bacterial infection, preferably caused by bacteria producing one or more β-lactamase, preferably caused by a gram-positive bacteria or by gram-negative bacteria, preferably a bacterial infection caused by gram-negative bacteria.

In the present invention "Composition X for use for the treatment of Y" is equivalent to "Composition X for use in a method for the treatment of Y" or "Composition X for use in the therapy of Y".

The present invention also relates to the use of the composition according to the invention or kit according to the invention for the preparation of a medicine for the treatment or prevention of bacterial infection, preferably caused by bacteria producing one or more β-lactamase, preferably caused by a gram-positive bacteria or by gram-negative bacteria, preferably a bacterial infection caused by gram-negative bacteria.

The terms "prevention", "prevent" and "preventing" as used herein are intended to mean the administration of a compound or composition according to the invention in order to prevent infection by bacteria or to prevent occurrence of related infection and/or diseases. The terms "prevention", "prevent" and "preventing" also encompass the administration of a compound or composition according to the present invention in order to prevent at least one bacterial infection, by administration to a patient susceptible to be infected, or otherwise at a risk of infection by this bacteria.

The terms "treatment", "treat" and "treating" as used herein are intended to mean in particular the administration of a treatment comprising a compound or composition according to the present invention to a patient already suffering from an infection. The terms "treatment", "treat" and "treating" as used herein, also refer to administering a compound or composition according to the present invention, optionally with one or more antibacterial agent, in order to:
- reduce or eliminate either a bacterial infection or one or more symptoms associated with bacterial infection, or
- retard the progression of a bacterial infection or of one or more symptoms associated with bacterial infection, or
- reduce the severity of a bacterial infection or of one or more symptoms associated with the bacterial infection, or
- suppress the clinical manifestation of a bacterial infection, or
- suppress the manifestation of adverse symptoms of the bacterial infection.

The expression "infection" or "bacterial infection" as used herein, includes the presence of bacteria, in or on a subject, which, if its growth were inhibited, would result in a benefit to the subject. As such, the term "infection" or "bacterial infection" in addition to referring to the presence of bacteria also refers to normal flora, which is not desirable. The term "infection" includes infection caused by bacteria. Exemplary of such bacterial infection are urinary tract infection (UTI), kidney infections (pyelonephritis), gynecological and obstetrical infections, respiratory tract indection (RTI), acute exacerbation of chronic bronchitis (AECB), Community-acquired pneumonia (CAP), hospital-acquired pneumonia (HAP), ventilator associated pneumonia (VAP), intra-abdominal pneumonia (IAI), acute otitis media, acute sinusitis, sepsis, catheter-related sepsis, chancroid, chlamydia, skin infections, bacteremia.

The term "growth" as used herein, refers to the growth of one or more microorganisms and includes reproduction or population expansion of the microorganism, such as bacteria. The term also includes maintenance of on-going metabolic processes of a microorganism, including processes that keep the microorganism alive.

The bacteria are chosen amongst gram-positive bacteria or gram-negative bacteria, preferably the gram-negative bacteria.

The bacteria can be also chosen among bacteria producing "beta-lactamase" or "β-lactamase". These bacteria are well known by the skilled person.

The term "beta-lactamase" or "β-lactamase" as used herein, refers to any enzyme or protein or any other substance that is able to break down a beta-lactam ring. The term "beta-lactamase" or "β-lactamase" includes enzymes that are produced by bacteria and that have the ability to hydrolyze, either partially or completely, the beta-lactam ring present in a compound such as an antibacterial agent.

Among the gram-positive bacteria, the bacteria according to the invention is preferably chosen among *Staphylococcus, Streptococcus, Staphylococcus species* (including *Staphylococcus aureus, Staphylococcus epidermidis*), *Streptococcus species* (including *Streptococcus pneumonia, Streptococcus agalactiae), Enterococcus species* (including *Enterococcus faecalis* and *Enterococcus faecium*).

Among the gram-negative bacteria, the bacteria according to the invention is preferably chosen among *Acinetobacter* species (including *Acinetobacter baumannil*)*, Citrobacter* species, *Escherichia* species (including *Escherichia coli*), *Haemophilus influenza, Morganella morganii, Klebsiella* species (including *Klebsiella pneumonia*), *Enterobacter* species (including *Enterobacter cloacae*), *Neisseria gonorrhoeae, Burkholderia* species (including *Burkholderia cepacia*), (*Proteus* species (including *Proteus mirabilis), Serratia* species (including *Serratia marcescens*), *Pseudomonas aeruginosa.*

The present invention also refers to the kit as defined above, for a simultaneous, separated or sequential administration to a patient in need thereof for use for the treatment or prevention of bacterial infections, preferably caused by bacteria producing one or more beta-lactamase(s). Preferably, the bacteria are chosen amongst gram-positive bacteria or gram-negative bacteria, preferably gram-negative bacteria.

The present invention also relates to a method for the treatment or prevention of bacterial infections, preferably caused by bacteria producing one or more beta-lactamase(s) comprising the administration of a therapeutically effective amount of a composition according to the invention or a kit according to the invention to a patient in need thereof. Preferably, the bacteria are chosen amongst gram-positive bacteria or gram-negative bacteria, preferably gram-negative bacteria.

The term "patient" means a person or an animal at risk of being infected by bacteria or, a person or an animal being infected by bacteria, preferably by gram-positive and/or by gram-negative bacteria. As used herein, the term "patient" refers to a warm-blooded animal such as a mammal, preferably a human or a human child, who is afflicted with, or has the potential to be afflicted with one or more infections and conditions described herein. The identification of those subjects who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those subjects who are in need of such treatment.

The expression "therapeutically effective amount" or "pharmaceutically effective amount" as used herein, refer to an amount of a compound according to the invention, which when administered to a patient in need thereof, is sufficient to effect treatment for disease-states, conditions, or disorders for which the compound has utility. Such an amount would be sufficient to elicit the biological or medical response of a tissue system, or patient that is sought by a researcher or a clinician. The amount of a compound according to the invention which constitutes a "therapeutically effective amount" will vary, notably depending on the compound itself and its biological activity, the composition used for administration, the time of administration, the route of administration, the rate of excretion of the compound, the duration of the treatment, the type of disease-state or disorder being treated and its severity, drugs used in combination with or coincidentally with the compounds of the invention, and the age, body weight, general health, sex and diet of the patient. Such a "therapeutically effective amount" can be determined by one of ordinary skilled in the art having regard to its own knowledge, and this disclosure. Preferably, the compounds according to the invention are administered in an amount comprised between 0.1 to 30g per day.

The composition or kit according to the invention may be provided in an aqueous physiological buffer solution for parenteral administration.
The composition or kit according to the present invention are also capable of being administered in unit dose forms, wherein the expression "unit dose" means a single dose which is capable of being administered to a patient, and which can be readily handled and packaged, remaining as a physically and chemically stable unit dose comprising either the active compound itself, or as a pharmaceutically acceptable composition, as described hereinafter. Compounds provided herein can be formulated into pharmaceutical compositions by admixture with one or more pharmaceutically acceptable excipients. Such unit dose compositions may be prepared for use by oral administration, particularly in the form of tablets, simple capsules or soft gel capsules; or intranasally, particularly in the form of powders, nasal drops, or aerosols; or dermally, for example, topically in ointments, creams, lotions, gels or sprays, or via trans-dermal patches. The compositions or kit may conveniently be administered in unit dosage form and may be prepared by any of the methods well-known in the pharmaceutical art, for example, as described in Remington: The Science and Practice of Pharmacy, 20th ed.; Gennaro, A. R., Ed.; Lippincott Williams & Wilkins: Philadelphia, PA, 2000.
Preferred formulations include pharmaceutical compositions in which a compound of the present invention is formulated for oral or parenteral administration.
For oral administration, tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolved, modified-release or sustained-release preparations and formulations, and wherein such sustained-release formulations are preferably bi-modal. Preferred tablets contain lactose, cornstarch, magnesium silicate, croscarmellose sodium, povidone, magnesium stearate, or talc in any combination.
Liquid preparations for parenteral administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes.
Alternative modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavored base, such as sucrose or acacia, and other excipients such as glycocholate. Formulations suitable for rectal administration are preferably presented as unit-dose suppositories, with a solid based carrier, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.

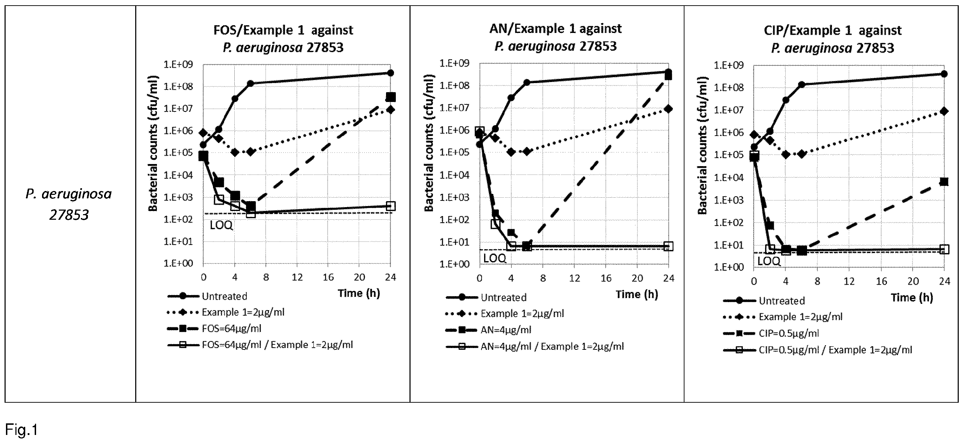

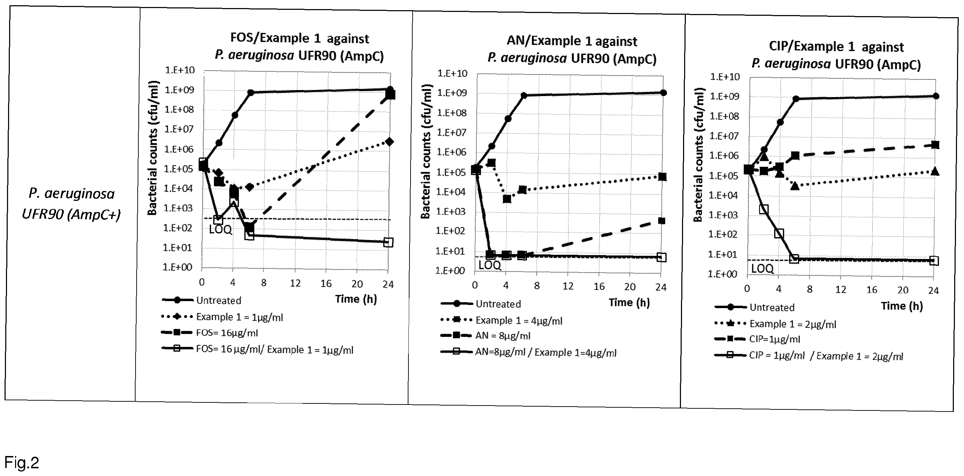

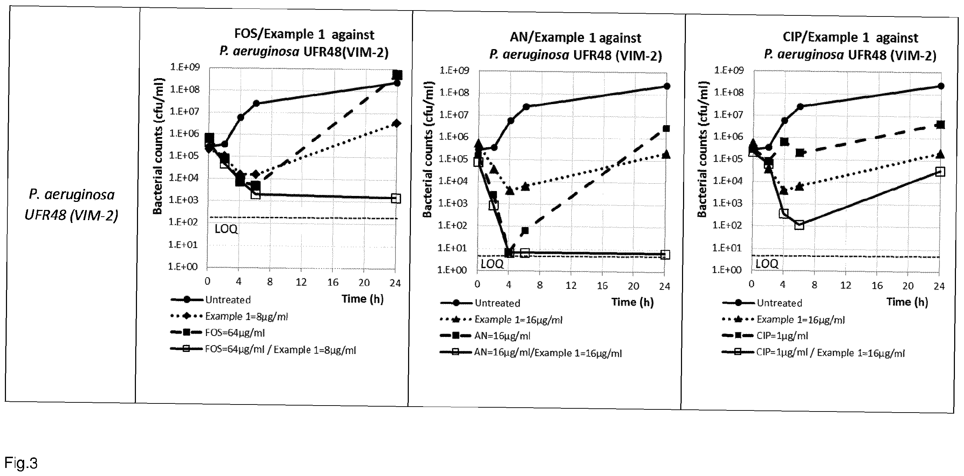

Figures 1 to 3 represent the time-kill kinetics of Example 1 combined with FOS, AN, CIP against 3 isolates of *P.aeruginosa.*

### Examples

The following examples are provided for the purpose of illustrating the present invention and by no means should be interpreted to limit the scope of the present invention.
The first part represents the preparation of the compounds (intermediates and final compounds) whereas the second part describes the evaluation of antibacterial activity of compounds according to the invention.

### Preparation of the compounds and biological activity:

### Abbreviations or symbols used herein include:

- AcCI:: acetyl chloride
- ACN:: acetonitrile
- ACHN:: 1,1'-azobis(cyclohexanecarbonitrile)
- AcOH:: acetic acid
- Bn:: benzyl
- Boc:: *tert*-butoxycarbonyl
- Boc₂O:: *tert*-butoxycarbonyl anhydride
- Boc-ON:: 2-(Boc-oxyimino)-2-phenylacetonitrile
- bs:: broad singlet
- Cbz:: carboxybenzyl
- CbzCl:: benzyl chloroformate
- CFU:: colony-forming units
- CLSI:: clinical laboratory standards institute
- Comins' Reagent:: *N*-(5-Chloro-2-pyridyl)bis(trifluoromethanesulfonimide)
- d:: doublet
- DBU:: 1,8-diazabicyclo[5.4.0]undec-7-ene
- DCM:: dichloromethane
- DCE:: 1,2-dichloroethane
- dd:: doublet of doublet
- ddd :: doublet of doublet of doublet
- ddt :: doublet of doublet of triplet
- dq:: doublet of quartet
- dt :: doublet of triplet
- DIAD:: di-*iso*-propyl azodicarboxylate
- DIPEA:: *N,N-*diisopropylethylamine
- DMF:: *N,N*-dimethylformamide
- DMSO:: dimethylsulfoxide
- DTA:: di-*tert*-butyl azodicarboxylate
- EtOAc:: ethyl acetate
- Et₂O:: diethyl ether
- h:: hours
- *i*PrOH:: isopropanol
- m :: multiplet
- min:: minutes
- MeOH:: methanol
- MeONa:: sodium methoxide
- MIC:: minimum inhibitory concentration
- MS:: mass spectrometry
- MsCl:: methanesulfonyl chloride
- NBS:: *N*-bromosuccinimide
- NCS:: *N*-chlorosuccinimide
- NMR:: nuclear magnetic resonance spectroscopy
- Nos:: nosyl, nitrobenzenesulfonyl
- Pd(Ph₃)₄:: tetrakis(triphenylphosphine)palladium(0)
- Pd(dppf)Cl₂:: [1,1'-Bis(diphenylphosphino)ferrocene]dichloropalladium(II)
- PG:: protective group
- PhSH:: thiophenol
- PMe₃:: trimethylphosphine
- PPh₃:: triphenylphosphine
- Ppm:: parts per million
- q:: quartet
- rt:: room temperature
- s:: singlet
- t:: triplet
- td:: triplet of doublet
- TBDMSCI:: *tert*-Butyldimethylsilyl chloride
- TBDMSOTf:: trifluoromethanesulfonic acid tert-butyldimethylsilyl ester
- *t*BuOK:: potassium *tert*-butoxide
- TEA:: triethylamine
- Tf:: trifluoromethanesulfonate
- TFA:: trifluoroacetic acid
- THF:: tetrahydrofuran
- TLC:: thin layer chromatography
- TMSI:: lodotrimethylsilane
- Tr:: trityl (triphenylmethyl)

### Example 1: synthesis of sodium and 2,2,2-trifluoroacetate [trans-2-(azaniumylmethyl)-4-oxazol-5-yl-7-oxo-1,6-diazabicyclo[3.2.1]oct-3-en-6-yl] sulfate

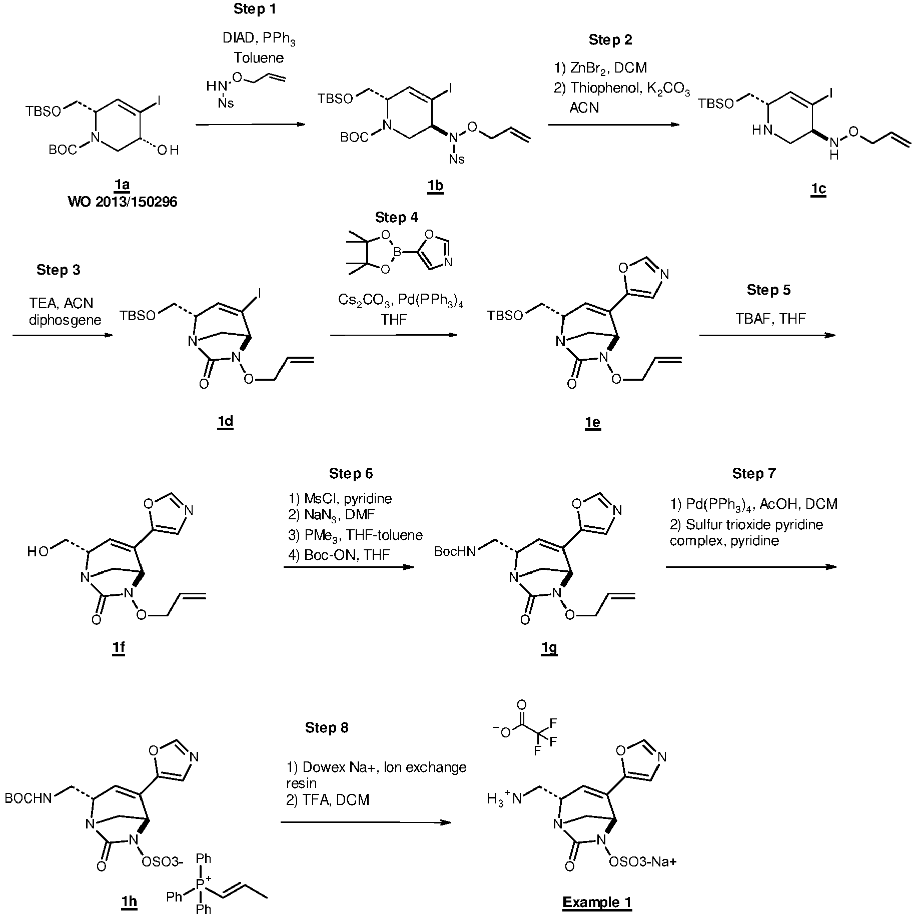

### Step 1: preparation of intermediate tert-butyl trans-3-[allyloxy-(2-nitrophenyl)sulfonyl-amino]-6-[[tert-butyl(dimethyl)silyl]oxymethyl]-4-iodo-3,6-dihydro-2H-pyridine-1-carboxylate (1b)

To a solution of *tert*-butyl *cis*-6-[[*tert*-butyl(dimethyl)silyl]oxymethyl]-3-hydroxy-4-iodo-3,6-dihydro-2*H*-pyridine-1-carboxylate (1a, prepared according to WO 2013/150296) (12.05 g, 25.67 mmol) in toluene (170 mL) at rt was added triphenylphosphine (8.08 g, 30.80 mmol), *N*-(allyloxy)-2-nitrobenzenesulfonamide (6.63 g, 25.67 mmol) and DIAD (6.06 mL, 30.80 mmol). The reaction mixture was stirred at rt overnight and concentrated *in vacuo.* The crude was purified by flash chromatography on silica gel (cyclohexane/EtOAc 100/0 to 85/15) to give *tert*-butyl *trans*-3-[allyloxy-(2-nitrophenyl)sulfonyl-amino]-6-[[*tert-*butyl(dimethyl)silyl]oxymethyl]-4-iodo-3,6-dihydro-2*H*-pyridine-1-carboxylate (1b) (17.0 g, 23.95 mmol, 93%).
¹H NMR (300 MHz, CDCl₃): *δ*(ppm) 0.03 (s, 6H), 0.88 (s, 9H), 1.35 (s, 9H), 3.16-3.75 (m, 3H), 3.93-4.78 (m, 5H), 5.12-5.38 (m, 2H), 5.68-5.89 (m, 1H), 6.73 (d, *J* = 4.1 Hz, 1H), 7.54-7.66 (m, 1H), 7.69-7.84 (m, 2H), 8.06-8.19 (m, 1H).

### Step 2: preparation of intermediate trans-N-allyloxy-6-[[tert-butyl(dimethyl)silyl]oxymethyl]-4-iodo-1,2,3,6-tetrahydropyridin-3-amine (1c)

To a solution of *tert*-butyl *trans*-3-[allyloxy-(2-nitrophenyl)sulfonyl-amino]-6-[[*tert-*butyl(dimethyl)silyl]oxymethyl]-4-iodo-3,6-dihydro-2*H*-pyridine-1-carboxylate (1b) (17.0 g, 23.95 mmol) in DCM (177 mL) was added ZnBr₂ (16.2 g, 71.86 mmol). The reaction mixture was stirred at rt overnight then diluted with DCM and successively washed with saturated saturated sodium bicarbonate and brine. The organic layer was dried over Na₂SO₄, filtered and concentrated *in vacuo.* The crude was diluted with ACN (177 mL). K₂CO₃ (16.6 g, 119.77 mmol) was added, followed by thiophenol (12.3 mL, 119.77 mmol). The reaction mixture was stirred at rt for 1 h and concentrated *in vacuo.* DCM was added and the resulting solids were removed by filtration. The filtrate was concentrated *in vacuo.* The crude was purified by flash chromatography on silica gel (DCM/MeOH 100/0 to 90/10) to give *trans-N-*allyloxy-6-[[*tert*-butyl(dimethyl)silyl]oxymethyl]-4-iodo-1,2,3,6-tetrahydropyridin-3-amine (1c) (7.99 g, 18.83 mmol, 78%).
MS *m*/*z* ([M+H]⁺) 425.
¹H NMR (300 MHz, CDCl₃) : *δ*(ppm) 0.06 (s, 6H), 0.89 (s, 9H), 1.82 (bs, 1H), 3.14 (dd, *J* = 12.6, 5.1 Hz, 1H), 3.21 (dd, *J* = 12.6, 3.9 Hz, 1H), 3.37-3.45 (m, 2H), 3.53-3.60 (m, 2H), 4.22 (dq, *J* = 6.0, 1.2 Hz, 2H), 5.18-5.25 (m, 1H), 5.25-5.35 (m, 1H), 5.88-5.35 (m, 2H), 6.53-6.56 (m, 1H).

### Step 3: preparation of intermediate trans-6-allyloxy-2-[[tert-butyl(dimethyl)silyl]oxymethyl]-4-iodo-1,6-diazabicyclo[3.2.1]oct-3-en-7-one (1d)

To a solution of *trans*-*N*-allyloxy-6-[[*tert*-butyl(dimethyl)silyl]oxymethyl]-4-iodo-1,2,3,6-tetrahydropyridin-3-amine (1c) (7.99 g, 18.83 mmol) in anhydrous ACN (980 mL) at 0°C under inert atmosphere was added TEA (10.56 mL, 75.31 mmol). A solution of diphosgene (1.14 mL, 9.41 mmol) in anhydrous ACN (20 mL) was added dropwise over 5 h. Once the addition finished, the reaction mixture was allowed to reach rt and stirred for 3 days. H₂O was added and the mixture was extracted with EtOAc. The organic layer was dried over Na₂SO₄, filtered and evaporated *in vacuo.* The crude was purified by flash chromatography on silica gel (cyclohexane/EtOAc 100/0 to 80/20) to give *trans*-6-allyloxy-2-[[*tert-*butyl(dimethyl)silyl]oxymethyl]-4-iodo-1,6-diazabicyclo[3.2.1]oct-3-en-7-one (1d) (7.25 g, 16.10 mmol, 85%).
MS *m*/*z* ([M+H]⁺) 451.
¹H NMR (300 MHz, CDCl₃) : *δ* (ppm) 0.06 (s, 6H), 0.88 (s, 9H), 3.19 (dd, *J* = 11.1, 3.0 Hz, 1H), 3.57 (d, *J* = 11.1 Hz, 1H), 3.80-3.90 (m, 3H), 4.05-4.08 (m, 1H), 4.35-4.53 (m, 2H), 5.28-5.34 (m, 1H), 5.34-5.43 (m, 1H), 5.97-6.12 (m, 1H), 6.37-6.41 (m, 1H).

### Step 4: preparation of intermediate trans-6-allyloxy-2-[[tert-butyl(dimethyl)silyl]oxymethyl]-4-oxazol-5-yl-1,6-diazabicyclo[3.2.1]oct-3-en-7-one (1e)

In a sealed flask, a mixture of *trans*-6-allyloxy-2-[[*tert*-butyl(dimethyl)silyl]oxymethyl]-4-iodo-1,6-diazabicyclo[3.2.1]oct-3-en-7-one (1d) (5.20 g, 11.55 mmol), 5-(4,4,5,5-tetramethyl-1,2,3-dioxaborolan-2-yl)oxazole (2.70 g, 13.86 mmol) and Cs₂CO₃ (7.52 g, 23.09 mmol) in anhydrous THF (100 mL) was degassed under argon for 5 min and Pd(PPh₃)₄ (400 mg, 0.35 mmol) was added. The mixture was heated at 60°C overnight. H₂O was added and the mixture was extracted with EtOAc. The organic layer was dried over Na₂SO₄, filtered and concentrated *in vacuo.* The crude was purified by flash chromatography on silica gel (cyclohexane/EtOAc 100/0 to 70/30) to give *trans*-6-allyloxy-2-[[*tert-*butyl(dimethyl)silyl]oxymethyl]-4-oxazol-5-yl-1,6-diazabicyclo[3.2.1]oct-3-en-7-one (1e) (3.64 g, 9.30 mmol, 80%).
MS *m*/*z* ([M+H]⁺) 392.
¹H NMR (300 MHz, CDCl₃) : *δ*(ppm) 0.07 (s, 6H), 0.88 (s, 9H), 3.37 (dd, *J =* 11.0, 3.0 Hz, 1H), 3.54 (d, *J* = 11.0 Hz, 1H), 3.87-4.06 (m, 3H), 4.11-4.14 (m, 1H), 4.33-4.50 (m, 2H), 5.27-5.40 (m, 2H), 5.92-6.08 (m, 1H), 6.15 (d, *J* = 3.0 Hz, 1H), 7.03 (s, 1H), 7.83 (s, 1H).

### Step 5: preparation of intermediate trans-6-allyloxy-2-(hydroxymethyl)-4-oxazol-5-yl-1,6-diazabicyclo[3.2.1]oct-3-en-7-one (1f)

To a solution of *trans*-6-allyloxy-2-[[*tert*-butyl(dimethyl)silyl]oxymethyl]-4-oxazol-5-yl-1,6-diazabicyclo[3.2.1]oct-3-en-7-one (1e) (3.64 g, 9.30 mmol) in THF (45 mL) at 0°C was added tetrabutylammonium fluoride (1M in THF) (13.9 mL, 13.94 mmol). The reaction mixture was stirred at 0°C for 1 h and concentrated *in vacuo.* The crude was purified by flash chromatography on silica gel (EtOAc 100%) to give *trans*-6-allyloxy-2-(hydroxymethyl)-4-oxazol-5-yl-1,6-diazabicyclo[3.2.1]oct-3-en-7-one (1f) (1.53 g, 5.52 mmol, 57%).
MS *m*/*z* ([M+H]⁺) 278.
¹H NMR (400 MHz, CDCl₃) : *δ*(ppm) 3.32 (d, *J =* 11.2 Hz, 1H), 3.40 (dd, *J =* 11.2, 2.9 Hz, 1H), 3.69-3.87 (m, 2H), 4.12-4.19 (m, 2H), 4.36-4.50 (m, 2H), 5.28-5.39 (m, 3H), 5.94-6.06 (m, 2H), 7.06 (s, 1H), 7.83 (s, 1H).

### Step 6: preparation of intermediate tert-butyl N-[[trans-6-allyloxy-4-oxazol-5-yl-7-oxo-1,6-diazabicyclo[3.2.1]oct-3-en-2-yl]methyl]carbamate (1g)

A solution of *trans*-6-allyloxy-2-(hydroxymethyl)-4-oxazol-5-yl-1 ,6-diazabicyclo[3.2.1]oct-3-en-7-one (1f) (1.53 g, 5.52 mmol) in pyridine (17 mL) was cooled to 0°C. Methanesulfonyl chloride (0.67 mL, 8.61 mmol) was added and the reaction mixture was stirred at the same temperature for 2 h. After concentrating *in vacuo,* the crude was dissolved in DCM and successively washed with a solution of 1N HCI and brine. The organic layer was dried over Na₂SO₄, filtered and evaporated *in vacuo.* The crude was dissolved in DMF (29 mL) and NaN₃ (1.79 g, 27.59 mmol) was added. The reaction mixture was heated at 65°C overnight and concentrated *in vacuo.* H₂O was added to the crude, which was extracted with EtOAc. The organic layer was washed with brine, dried over Na₂SO₄, filtered and concentrated *in vacuo.* The crude was dissolved in a mixture of THF and toluene (16.7 mL/16.7 mL) and trimethylphosphine (1M in THF) (8.28 mL, 8.28 mmol) was added at 0°C. After 1 h stirring at rt, the mixture was cooled to 0°C and a solution of Boc-ON (2.04 g, 8.28 mmol) in THF (11 mL) was dropwise added. The mixture was stirred at rt for 1 h and concentrated *in vacuo.* The crude was purified by flash chromatography on silica gel (cyclohexane/EtOAc 95/5 to 0/100) to give *tert*-butyl *N*-[[*trans*-6-allyloxy-4-oxazol-5-yl-7-oxo-1,6-diazabicyclo[3.2.1]oct-3-en-2-yl]methyl]carbamate (1g) (440 mg, 1.17 mmol, 21%).
¹H NMR (300 MHz, CDCl₃) : *δ*(ppm) 1.45 (s, 9H), 3.11-3.30 (m, 1H), 3.37 (dd, *J* = 11.3, 2.9 Hz, 1H), 3.53-3.67 (m, 1H), 3.98-4.07 (m, 1H), 4.15 (d, *J* = 2.9 Hz, 1H), 4.33-4.50 (m, 2H), 4.99-5.12 (m, 1H), 5.28-5.41 (m, 2H), 5.92-6.07 (m, 2H), 7.05 (s, 1H), 7.83 (s, 1H).
MS *m*/*z* ([M+H]⁺) 377.

### Step 7: preparation of intermediate triphenyl-[(E)-prop-1-enyl]phosphonium [trans-2-[(tert-butoxycarbonylamino)methyl]-4-oxazol-5-yl-7-oxo-1,6-diazabicyclo[3.2.1]oct-3-en-6-yl] sulfate (1h)

To a solution of *tert*-butyl *N*-[[*trans*-6-allyloxy-4-oxazol-5-yl-7-oxo-1,6-diazabicyclo[3.2.1]oct-3-en-2-yl]methyl]carbamate (1g) (440 mg, 1.17 mmol) and glacial acetic acid (134 µL, 2.34 mmol) in anhydrous DCM (13 mL) was added in one portion Pd(PPh₃)₄ (675 mg, 0.58 mmol). After stirring for 2 h, a solution of sulfur trioxide pyridine complex (753 mg, 4.73 mmol) in dry pyridine (15 mL) was added and the resulting mixture was stirred overnight. The reaction mixture was concentrated *in vacuo,* diluted with DCM and filtered. The filtrate was concentrated *in vacuo.* The crude was purified by flash chromatography on silica gel (DCM/acetone 97/3 to 20/80) to give triphenyl-[(*E*)-prop-1-enyl]phosphonium [*trans*-2-[(tert-butoxycarbonylamino)methyl]-4-oxazol-5-yl-7-oxo-1,6-diazabicyclo[3.2.1]oct-3-en-6-yl] sulfate (1h) (560 mg, 0.78 mmol, 67%).
¹H NMR (300 MHz, CDCl₃) : *δ*(ppm) 1.45 (s, 9H), 2.23-2.28 (m, 3H), 3.07-3.31 (m, 2H), 3.46-3.67 (m, 2H), 3.91-4.01 (m, 1H), 4.77 (bs, 1H), 5.10-5.27 (m, 1H), 5.85 (bs, 1H), 6.52-6.70 (m, 1H), 7.11-7.24 (m, 1H), 7.60-7.82 (m, 1H).

### Step 8: preparation of sodium and 2,2,2-trifluoroacetate [trans-2-(azaniumylmethyl)-4-oxazol-5-yl-7-oxo-1,6-diazabicyclo[3.2.1]oct-3-en-6-yl] sulfate (Example 1)

A solution of triphenyl-[(*E*)-prop-1-enyl]phosphonium [*trans*-2-[(tertbutoxycarbonylamino) methyl]-4-oxazol-5-yl-7-oxo-1,6-diazabicyclo[3.2.1]oct-3-en-6-yl] sulfate (1h) (560 mg, 0.78 mmol) dissolved in a mixture of H₂O/THF 7/3 (1 mL) was applied on a Dowex sodium form column (Dowex® 50WX8 hydrogen form stored with an aqueous solution of 2N NaOH and washed until neutral pH with H₂O). The fractions containing the desired compound were combined and concentrated *in vacuo.* The product was dissolved in ACN and the remaining precipitate was filtered off. The filtrate was concentrated *in vacuo.* The crude was dissolved in DCM (28 mL), cooled to 0°C, and trifluoroacetic acid (18.5 mL) was dropwise added. After 1 h stirring at the same temperature, the reaction mixture was concentrated *in vacuo,* dissolved in a minimum of H₂O, freezed and lyophilized to afford sodium and 2,2,2-trifluoroacetate [*trans*-2-(azaniumylmethyl)-4-oxazol-5-yl-7-oxo-1 ,6-diazabicyclo[3.2.1]oct-3-en-6-yl] sulfate (example 1) (350 mg, 0.77 mmol, 99%).
¹H NMR (300 MHz, DMSO-*d₆*) : *δ* (ppm) 3.10-3.26 (m, 1H), 3.29 (dd, *J* = 11.7, 2.9 Hz, 1H), 3.46 (d, *J* = 11.7 Hz, 1H), 4.02-4.11 (m, 1H), 4.59 (d, *J* = 2.2 Hz, 1H), 6.00 (d, *J* = 3.3 Hz, 1H), 7.30 (s, 1H), 8.09 (bs, 3H), 8.42 (s, 1H).

### Example 2: synthesis of sodium and 2,2,2-trifluoroacetate [(2S,5S)-4-(2-ammoniumethoxyimino)-2-carbamoyl-7-oxo-1,6-diazabicyclo[3.2.1]octan-6-yl] sulfate (reference example)

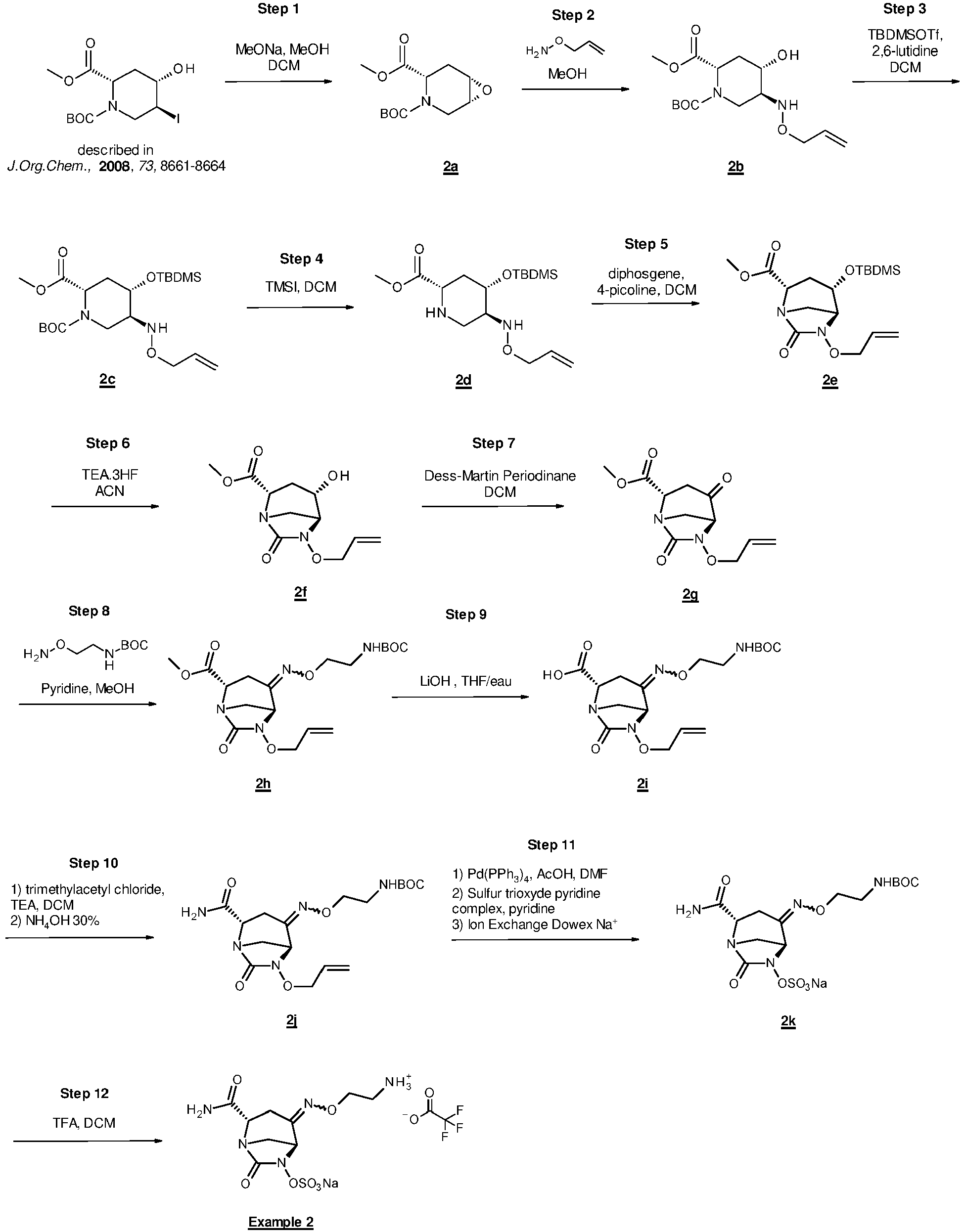

### Step 1: preparation of intermediate 4-tert-butyl ester 3-methyl ester (1R,4S,6S)-7-oxa-3-azabicyclo[4.1.0]heptane-3,4-dicarboxylate (2a)

To a solution of 1-*tert*-butyl ester 2-methyl ester (2S,4S,5S)-4-hydroxy-5-iodo-piperidine-1,2-dicarboxylate (prepared according to the procedure described in J.Org.Chem., 2008, 73, 8661-8664) (8.82 g, 22.90 mmol) in anhydrous DCM (100 mL) under inert atmosphere was added a MeONa solution 0.5M in MeOH (45.81 mL, 22.90 mmol). The reaction mixture was stirred 20 h at rt, then the solution was washed with NaOH 1N. The organic layer was dried over Na₂SO₄, filtered and evaporated *in vacuo.* The crude residue was solubilized in mixture of EtOAc/cyclohexane (50/50) and filtered on silica gel cake to provide 4-*tert*-butyl ester 3-methyl ester (1*R*,4*S*,6*S*)-7-oxa-3-aza-bicyclo[4.1.0]heptane-3,4-dicarboxylate (2a) (5.33 g, 20.71 mmol, 90%) as a yellow oil.
MS *m*/*z* ([M+Na]⁺) 280.
¹H NMR (400 MHz, CDCl₃): *δ*(ppm) 1.42 and 1.47 (2s, 9H), 2.18-2.26 (m, 1H), 2.85 (ddt, *J* = 13.4/11.0/2.4 Hz, 1H), 3.31-3.17 (m, 2H), 3.68-3.81 (m, 4H), 3.88-3.94 (m, 1H), 4.53 and 4.73 (2dd, *J* = 6.3/2.0 Hz, 1H).

### Step 2: preparation of intermediate 1-tert-butyl ester 2-methyl ester (2S,4S,5S)-5-allyloxyamino-4-hydroxy-piperidine-1,2-dicarboxylate (2b)

To a solution of 4-*tert*-butyl ester 3-methyl ester (1*R*,4*S*,6*S*)-7-oxa-3-azabicyclo[4.1.0]heptane-3,4-dicarboxylate (2a) (3.88 g, 15.09 mmol) in anhydrous MeOH (30 mL) under inert atmosphere was added O-allylhydroxylamine (5.51g, 75.45 mmol). The reactor was sealed and the reaction was stirred 3 days at 80°C. MeOH was evaporated and the residue was purified by flash chromatography on silica gel (heptane/EtOAc 80/20 to 0/100) to provide 1-*tert*-butyl ester 2-methyl ester (2S,4S,5S)-5-allyloxyamino-4-hydroxypiperidine-1,2-dicarboxylate (2b) (3.12 g, 9.44 mmol, 62%) as a colorless oil.
MS *m*/*z* ([M+H]⁺) 331.
¹H NMR (400 MHz, CDCl₃): *δ*(ppm) 1.45 (s, 9H), 1.94-1.98 (m, 1H), 2.21-2.28 (m, 2H), 3.10 (bs, 1H), 3.47 (d, *J* = 14.2 Hz, 1H), 3.73 (s, 3H), 3.90 (dd, *J* = 14.2/2.6 Hz, 1H), 3.98-4.02 (m, 1H), 4.18 (dq, *J* = 5.9/1.3 Hz, 2H), 4.72 (bs, 1H), 5.12-5.35 (m, 2H), 5.53 (s, 1H), 5.91 (ddt, J = 17.3/10.3/5.9 Hz, 1H).

### Step 3: preparation of intermediate 1-tert-butyl ester 2-methyl ester (2S,4S,5S)-5-allyloxyamino-4-(tert-butyl-dimethyl-silanyloxy)-piperidine-1,2-dicarboxylate (2c)

To a solution of 1-*tert*-butyl ester 2-methyl ester (2S,4S,5S)-5-allyloxyamino-4-hydroxy-piperidine-1,2-dicarboxylate (2b) (3.95 g, 11.97 mmol) in anhydrous DCM (50 mL) under inert atmosphere at 0°C was added 2,6-lutidine (1.67 mL, 14.36 mmol) followed by TBDMSOTf (2.88 mL, 12.58 mmol). The mixture was stirred for 3 h at 20°C and then the solution was extracted with DCM, washed with saturated sodium hydrogenocarbonate aqueous solution. The organic layer was dried over Na₂SO₄, filtered and evaporated. The product was purified by flash chromatography on silica gel (cyclohexane/EtOAc 90/10) to afford 1-*tert*-butyl ester 2-methyl ester (2S,4S,5S)-5-allyloxyamino-4-(tert-butyl-dimethyl-silanyloxy)-piperidine-1,2-dicarboxylate (2c) (4.55 g, 10.23 mmol, 85%) as a colorless oil.
MS *m*/*z* ([M+H]⁺) 445.
¹H NMR (300 MHz, CDCl₃): *δ*(ppm) 0.04 (2s, 6H), 0.86 (s, 9H), 1.46 (bs, 9H), 2.00-2.24 (m, 2H), 2.90-3.20 (m, 1H), 3.36-3.55 (m, 1H), 3.70 (s, 3H), 3.83-4.05 (m, 2H), 4.09-4.26 (m, 2H), 4.54-4.73 (2bs, 1H), 5.04-5.42 (m, 2H), 5.50 (bs, 1H), 5.91 (ddt, *J* = 17.3/10.4/5.9 Hz, 1H).

### Step 4: preparation of intermediate methyl (2S,4S,5S)-5-(allyloxyamino)-4-[tert-butyl(dimethyl)silyl]oxy-piperidine-2-carboxylate (2d)

To a solution of 1-*tert*-butyl ester 2-methyl ester (2*S*,4*S*,5*S*)-5-allyloxyamino-4-(*tert*-butyldimethyl-silanyloxy)-piperidine-1,2-dicarboxylate (2c) (0.368 g, 0.83 mmol) in anhydrous DCM (16 mL) under inert atmosphere at rt, was added slowly TMSI (177 µL, 1.24 mmol). The reaction mixture was stirred for 30 min at rt. The mixture was then quenched at 0°C with MeOH (1 mL). The mixture was evaporated under vacuum and the resulting residue was purified by flash chromatography on silica gel (DCM/MeOH 97/3) to provide methyl (2*S*,4*S*,5*S*)-5-(allyloxyamino)-4-[*tert*-butyl(dimethyl)silyl]oxy-piperidine-2-carboxylate (2d) (0.325 g, 0.829 mmol, quantitative yield) as a yellow solid.
MS *m*/*z* ([M+H]⁺) 345.
¹H NMR (400 MHz, CDCl₃): *δ*(ppm) 0.09 (2s, 6H), 0.88 (s, 9H), 2.15 (dt, *J* = 15.0/8.1 Hz, 1H), 2.46 (dt, *J* = 14.2/4.3 Hz, 1H), 3.20-3.32 (m, 2H), 3.79 (dd, *J* = 12.3, 3.0 Hz, 1H), 3.84 (s, 4H), 3.95-4.03 (m, 1H), 4.10-4.26 (m, 3H), 5.18-5.36 (m, 2H), 5.91 (ddt, *J* = 16.6/10.4/ 6.0 Hz, 1H).

### Step 5: preparation of intermediate methyl (2S,4S,5S)-6-allyloxy-4-[tert-butyl(dimethyl)silyl] oxy-7-oxo-1,6-diazabicyclo[3.2.1]octane-2-carboxylate (2e)

To a solution of (2S,4S,5S)-5-(allyloxyamino)-4-[*tert*-butyl(dimethyl)silyl]oxy-piperidine-2-carboxylate (2d) (0.325 g, 0.829 mmol) and 4-picoline (322 µL, 3.31 mmol) in anhydrous DCM (16 mL) at 0°C was added diphosgene (55 µL, 0.45 mmol) as a solution in DCM (4 mL, flow = 0.25 mL/min). Once addition was complete, the reaction was stirred at rt overnight. The mixture was washed with a saturated sodium hydrogenocarbonate aqueous solution then water, dried over Na₂SO₄, filtered and concentrated. The crude residue was purified by flash chromatography on silica gel (cyclohexane/EtOAc 90/10) to provide intermediate methyl (2S,4S,5S)-6-allyloxy-4-[*tert*-butyl(dimethyl)silyl] oxy-7-oxo-1,6-diazabicyclo[3.2.1]octane-2-carboxylate (2e) (0.176 g, 0.47 mmol, 57% over 2 steps).
MS *m*/*z* ([M+H]⁺) 371.
¹H NMR (300 MHz, CDCl₃): *δ* (ppm) 0.05 (2s, 6H), 0.86 (s, 9H), 2.21 (d, *J =* 15.8 Hz, 1H), 2.37 (ddd, *J* = 15.7/8.7/4.5 Hz, 1H), 2.99-3.08 (m, 1H), 3.52 (t, *J* = 3.7 Hz, 1H), 3.70 (d, *J =* 12.1 Hz, 1H), 3.77 (s, 3H), 4.05 (d, *J =* 8.5 Hz, 1H), 4.23-4.27 (m, 1H), 4.38-4.54 (m, 2H), 5.27-5.43 (m, 2H), 6.02 (ddt, *J* = 16.9/10.3/6.4 Hz, 1H).

### Step 6: preparation of intermediate methyl (2S,4S,5S)-6-allyloxy-4-hydroxy-7-oxo-1,6-diazabicyclo[3.2.1]octane-2-carboxylate (2f)

To a solution of methyl (2S,4S,5S)-6-allyloxy-4-[*tert*-butyl(dimethyl)silyl] oxy-7-oxo-1,6-diazabicyclo[3.2.1]octane-2-carboxylate (2e) (0.722 g, 1.95 mmol) in anhydrous ACN (20 mL) under inert atmosphere was added dropwise TEA.3HF (320 µL, 1.95 mmol). The reaction mixture was stirred to 60°C for 18 h. The mixture was diluted with EtOAc, washed with water, dried over Na₂SO₄, filtered and concentrated. The crude was purified by flash chromatography on silica gel (cyclohexane/EtOAc 50/50 to 0/100) to provide methyl (2S,4S,5S)-6-allyloxy-4-hydroxy-7-oxo-1,6-diazabicyclo[3.2.1]octane-2-carboxylate (2f) (0.361 g, 1.40 mmol, 72%) as a white solid.
MS *m*/*z* ([M+H]⁺) 257.
¹H NMR (400 MHz, CDCl₃): *δ* (ppm) 2.13 (dd, *J* = 16.3/2.0 Hz, 1H), 2.33 (ddd, *J* = 16.2/8.0/5.1 Hz, 1H), 2.95-3.04 (m, 1H), 3.36-3.45 (m, 2H), 3.67-3.76 (m, 1H), 3.80 (s, 3H), 4.08 (d, *J* = 7.9 Hz, 1H), 4.29 (q, *J* = 4.7 Hz, 1H), 4.31-4.51 (m, 2H), 5.23-5.39 (m, 2H), 5.98 (ddt, *J* = 16.9/10.3/6.4 Hz, 1H).

### Step 7: preparation of intermediate methyl (2S,5S)-6-allyloxy-4,7-dioxo-1,6-diazabicyclo[3.2.1]octane-2-carboxylate (2g)

To a solution of methyl (2S,4S,5S)-6-allyloxy-4-hydroxy-7-oxo-1,6-diazabicyclo[3.2.1]octane-2-carboxylate (2f) (0.361 g, 1.41 mmol) in anhydrous DCM (30 mL) under inert atmosphere at 0°C was added the reagent Dess-Martin periodinane (0.847g, 1.41 mmol). The reaction was stirred for 18 h at rt, then the mixture was washed with a saturated sodium hydrogenocarbonate aqueous solution, a 15% sodium thiosulfate solution, water, dried oved Na₂SO₄, filtered and concentrated under vacuum. The residue was purified by flash chromatography on silica gel (cyclohexane/EtOAc 80/20 to 0/100) to afford intermediate methyl (2S,5S)-6-allyloxy-4,7-dioxo-1,6-diazabicyclo[3.2.1]octane-2-carboxylate (2g) (0.333 g, 1.31 mmol, 92%) as a colorless oil.
MS *m*/*z* ([M+H]⁺) 255.
¹H NMR (400 MHz, CDCl₃): *δ*(ppm) 2.91-3.05 (m, 2H), 3.31 (d, *J* = 12.9 Hz, 1H), 3.56-3.66 (m, 1H), 3.79 (s, 3H), 3.99 (d, *J* = 3.8 Hz, 1H), 4.35-4.50 (m, 2H), 4.53 (ddd, *J* = 6.3/4.8/1.5 Hz, 1H), 5.28-5.41 (m, 2H), 5.89-6.04 (m, 1H).

### Step 8: preparation of intermediate methyl (2S,5S)-6-allyloxy-4-[2-(tert-butoxycarbonylamino)ethoxyimino]-7-oxo-1,6-diazabicyclo[3.2.1]octane-2-carboxylate (2h)

To a solution of methyl (2S,5S)-6-allyloxy-4,7-dioxo-1,6-diazabicyclo[3.2.1]octane-2-carboxylate (2g) (0.862 g, 3.39 mmol) in anhydrous MeOH (30 mL) under inert atmosphere was added successively pyridine (592 µL, 7.15 mmol) and *tert*-butyl *N*-(2-aminooxyethyl)carbamate (0.568 g, 3.22 mmol). The reaction mixture was stirred for 27 h at rt. The reaction mixture was concentrated under vacuum and the residue was purified by flash chromatography on silica gel (heptane/EtOAc : 80/20 to 0/100) to provide intermediate methyl (2S, 5S)-6-allyloxy-4-[2-(*tert*-butoxycarbonylamino)ethoxyimino]-7-oxo-1,6-diazabicyclo[3.2.1]octane-2-carboxylate (2h) (0.370 g, 0.897 mmol, ratio *Z*/*E* 50/50, 26%) as a colorless oil.
MS *m*/*z* ([M+H-Boc]⁺)/([M+H]⁺) 313/413.
¹H NMR (300 MHz, CDCl₃): *δ*(ppm) 1.44 (2s, 9H), 2.73-3.03 (m, 1H), 3.16 (t, *J =* 12.7 Hz, 1H), 3.29-3.56 (m, 3H), 3.80 (d, *J* = 0.7 Hz, 3H), 4.05-4.19 (m, 3.5H), 4.31-4.54 (m, 3H), 4.83 (d, *J* = 28.1 Hz, 1H), 5.08 (d, *J* = 3.6 Hz, 0.5H), 5.27-5.45 (m, 2H), 5.91-6.11 (m, 1H).

### Step 9: preparation of intermediate (2S,5S)-6-allyloxy-4-[2-(tert-butoxycarbonylamino) ethoxyimino]-7-oxo-1,6-diazabicyclo[3.2.1]octane-2-carboxylic acid (2i)

To solution of the intermediate (methyl *(2S,* 5S)-6-allyloxy-4-[2-(tert-butoxycarbonylamino)ethoxyimino]-7-oxo-1,6-diazabicyclo[3.2.1]octane-2-carboxylate (2h) (0.590 g, 1.43 mmol) in a mixture of acetone and water (1.44 mL/0.45 mL) at -10°C was added slowly LiOH 1N (1.43 mL, 1.43 mmol). The reaction was stirred 5 min at -10°C and the mixture was neutralised to pH = 7 with HCI 1N. The solution was washed with DCM. Aqueous layer was frozen and lyophilized to provided 393 mg of (2S, 5S)-6-allyloxy-4-[2-(tert-butoxycarbonylamino) ethoxyimino]-7-oxo-1,6-diazabicyclo[3.2.1]octane-2-carboxylic acid (2i) in mixture with (2S, 5S)-5-[allyloxy(methoxycarbonyl)amino]-4-[2-(tert-butoxycarbonylamino)ethoxyimino]piperidine-2-carboxylic acid after purification by flash chromatography on silica gel (DCM/propan-2-ol : 80/20 to 0/100). The mixture was used without further purification.
MS *m*/*z* ([M+H-Boc]⁺)/([M+H]⁺) 299/399.

### Step 10: preparation of intermediate tert-butyl N-[2-[(2S,5S)-6-allyloxy-2-carbamoyl-7-oxo-1,6-diazabicyclo[3.2.1]octan-4-ylidene]amino]oxyethyl]carbamate (2i)

To a solution of compound (2S, 5S)-6-allyloxy-4-[2-(tert-butoxycarbonylamino) ethoxyimino]-7-oxo-1,6-diazabicyclo[3.2.1]octane-2-carboxylic acid (2i) (0.300 g, 0.753 mmol) in anhydrous DCM (7 mL) under inert atmosphere at 0°C was added TEA (85 µL, 0.918 mmol) followed by trimethylacetyl chloride (97 µL, 0.790 mmol). The mixture was stirred for 30 min at 0°C then cooled at -20°C. Aqueous ammoniac solution 30% (127 µL, 3.03 mmol) was added and the reaction was stirred for 1h at -20°C. DCM was evaporated and the residue was purified by flash chromatography on silica gel (DCM/propan-2-ol: 100/0 to 0/100) to provide *N*-[2-[[(2S,5S)-6-allyloxy-2-carbamoyl-7-oxo-1,6-diazabicyclo[3.2.1]octan-4-ylidene]amino]oxyethyl]carbamate (2j) (0.202 g, 0.505 mmol, 35% over 2 steps) as a *Z*/*E* 50/50 mixture.
MS *m*/*z* ([M+H-Boc]⁺)/([M+H]⁺) 298/398.
¹H NMR (400 MHz, CDCl₃): *δ*(ppm) 1.41 (2s, 9H), 2.60-2.80 (m, 0.5H), 2.93 (t, *J* = 12.0 Hz, 0.5H), 3.17-3.51 (m, 3H), 3.71 (d, *J* = 19.0 Hz, 0.5H), 4.05 - 4.22 (m, 3H), 4.39 - 4.53 (m, 1.5H), 4.74 - 5.01 (m, 1H), 5.08 (d, *J* = 3.6 Hz, 0.5H), 5.27 - 5.44 (m, 2H), 5.64 (bs, 3H), 5.86-6.08 (m, 1.5H), 6.70 (d, *J* = 20.8 Hz, 1H)

### Step 11: preparation of sodium [(2S,5S)-4-[2-(tert-butoxycarbonylamino)ethoxyimino]-2-carbamoyl-7-oxo-1,6-diazabicyclo[3.2.1]octan-6-yl] sulfate (2k)

To a solution of (*N*-[2-[[(2*S*,5*S*)-6-allyloxy-2-carbamoyl-7-oxo-1,6-diazabicyclo[3.2.1]octan-4-ylidene]amino]oxyethyl] carbamate (2j) (0.237 g, 0.596 mmol) and glacial AcOH (0.134 mL, 1.71 mmol) in anhydrous DMF (9 mL) was added Pd(PPh₃)₄ (0.687 g, 0.594 mmol). After stirring for 48 h at rt, dry pyridine (5.94 mL) and sulfur trioxide pyridine complex (0.473 g, 2.97 mmol) were added to the mixture and the resulting solution was protected from light and stirred 16 h at rt then 26 h at 40°C until the sulfatation was completed. The reaction mixture was concentrated under flow of nitrogen, diluted with DCM and filtered. The filtrate was concentrated and purified by flash chromatography on silica gel (DCM/acetone/propan-2-ol : gradient 100/0/0 to 0/100/0 to 0/0/100) to provide 135 mg of triphenyl-(propenyl)-phosphonium [(2S, 5S)-4-[2-(*tert*-butoxycarbonylamino)ethoxyimino]-2-carbamoyl-7-oxo-1,6-diazabicyclo [3.2.1]octan-6-yl] sulfate. This oil was solubilized in a minimal volume of water and applied on a Dowex sodium form column (Dowex® 50WX8 hydrogen form stored with an aqueous solution of 2N NaOH and washed until neutral pH with water). The fractions containing the desired compound were combined, frozen and lyophilized to afford sodium [(2S, 5S)-4-[2-(*tert*-butoxycarbonylamino)ethoxyimino]-2-carbamoyl-7-oxo-1,6-diazabicyclo[3.2.1]octan-6-yl] sulfate (2k) (75 mg , 0.163 mmol, ratio *Z*/*E*: 40/60, 27%) as a white solid.
MS *m*/*z* ([M-H]⁻) 436.
¹H NMR (400 MHz, D₂O): *δ* (ppm) : 1.42 (s, 9H), 2.81-2.97 (m, 1H), 3.08 (dd, *J* = 17.3, 5.0 Hz, 0.5H), 3.27 (d, *J* = 12.6 Hz, 1H), 3.30-3.47 (m, 2.5H), 3.69 (ddd, *J* = 37.9/12.7/3.6 Hz, 1H), 4.07-4.20 (m, 2H), 4.29-4.36 (m, 1H), 4.57 (d, *J* = 3.4 Hz, 0.6H), 5.42 (d, *J =* 3.3 Hz, 0.4H)

### Step 12: preparation of sodium and 2,2,2-trifluoroacetate [(2S,5S)-4-(2-ammoniumethoxyimino)-2-carbamoyl-7-oxo-1,6-diazabicyclo[3.2.1]octan-6-yl] sulfate (Example 2)

To a solution of TFA (2mL, 26.13 mmol) in anhydrous DCM (2mL) at 0°C, was added a suspension of sodium [(2S, 5S)-4-[2-(*tert*-butoxycarbonylamino)ethoxyimino]-2-carbamoyl-7-oxo-1,6-diazabicyclo[3.2.1]octan-6-yl] sulfate (2k) (75 mg , 0.163 mmol) in anhydrous DCM (1mL). The mixture was stirred 20 min at 0°C and concentrated. The residual oil was diluted in water (3 mL), frozen and lyophilized to afford sodium and 2,2,2-trifluoroacetate [(2S,5S)-4-(2-ammonium-ethoxyimino)-2-carbamoyl-7-oxo-1,6-diazabicyclo[3.2.1]octan-6-yl] sulfate (Example 2) (56.3 mg, 0.118 mmol, ratio *Z*/*E*: 40/60, 72%) as a yellow solid.
MS *m*/*z* ([M+H]⁺) 338.
MS *m*/*z* ([M-H]⁻) 336.
¹H NMR (300 MHz, D₂O): *δ* (ppm) 2.80-3.01 (m, 1H), 3.03-3.56 (m, 4H), 3.59-3.91 (m, 1H), 4.23-4.45 (m, 3H), 4.59 (d, J= 3.4 Hz, 0.6H), 5.45 (d, *J* = 3.2 Hz, 0.4H)

### Example 3: synthesis of sodium and 2,2,2-trifluoroacetate [5-(2-azaniumethyl)-4,9-dioxo-3-thia-5,8,10-triaza-tricyclo[6.2.1.0^{2,6}]undec-2(6)-en-10-yl] sulfate (reference example)

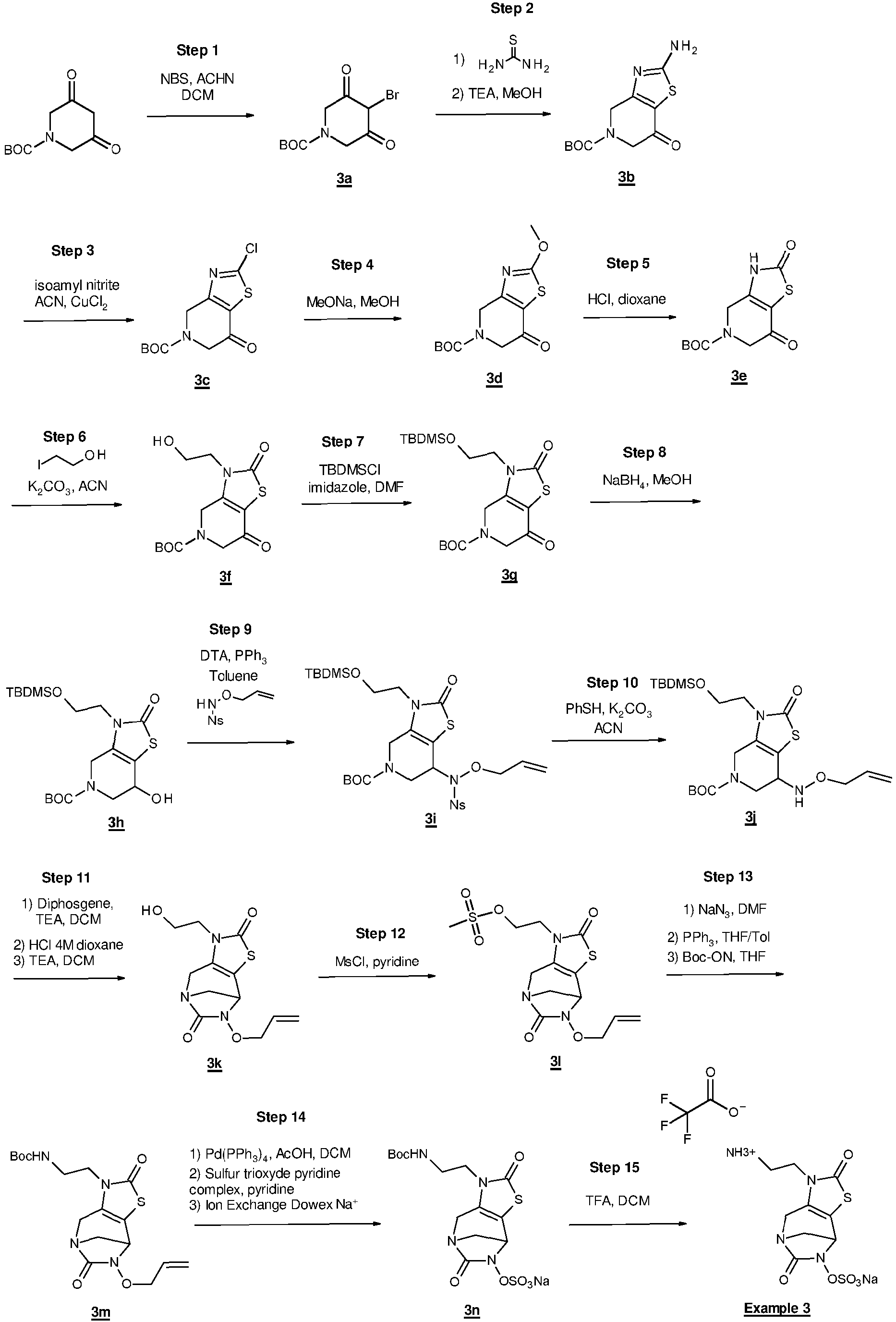

### Step 1: preparation of intermediate tert-butyl 4-bromo-3,5-dioxo-piperidine-1-carboxylate (3a)

To a solution of *tert*-butyl 3,5-dioxopiperidine-1-carboxylate (3 g, 14.07 mmol) in anhydrous DCM (60 mL) under inert atmosphere at 0°C was successively added NBS (2.5 g, 14,07 mmol) and ACHN (0.223 g, 0.91 mmol). The reaction mixture was stirred 2 h at 0°C. The solution was washed with water, then with NaCl aqueous solution. The organic layer was dried over Na₂SO₄ and evaporated *in vacuo* to give *tert*-butyl 4-bromo-3,5-dioxo-piperidine-1-carboxylate (3a) (4.11 g, 14.07 mmol, quantitative yield) as an off-white solid.
MS *m*/*z* ([M+H-*tert*butyl]⁺) 236/238.
MS *m*/*z* ([M-H]⁻) 290/292.
¹H NMR (400 MHz, CDCl₃): *δ*(ppm) 1.48 (s, 9H), 4.35 (bs, 4H).

### Step 2: preparation of intermediate tert-butyl 2-amino-7-oxo-6,7-dihydro-4H-thiazolo[4,5-c]pyridine-5-carboxylate (3b)

To a solution of *tert*-butyl 4-bromo-3,5-dioxo-piperidine-1-carboxylate (3a) (0.500 g, 1.71 mmol) in anhydrous MeOH (8 mL) under inert atmosphere was added thiourea (0.226 g, 3.42 mmol). After stirring 30 min at rt, TEA (0.477 mL, 3.42 mmol) was added and the mixture was refluxed 5 h. MeOH was evaporated and the residue was solubilized with EtOAc. The solution was washed with water, 10% of Na₂CO₃ aqueous solution and brine solution. The organic layer was dried over Na₂SO₄, filtered and evaporated. The solid was triturated with cyclohexane and filtered to give *tert*-butyl 2-amino-7-oxo-6,7-dihydro-4*H*-thiazolo[4,5-c]pyridine-5-carboxylate (3b) (0.350 g, 1.30 mmol, 76%) as an off white solid.
MS *m*/*z* ([M+H]⁺) 270.
MS *m*/*z* ([M-H]⁻) 268.
¹H NMR (400 MHz, CDCl₃): *δ*(ppm) 1.48 (s, 9H), 4.23 (s, 2H), 4.66 (s, 2H), 5.77 (bs, 2H).

### Step 3: preparation of intermediate tert-butyl 2-chloro-7-oxo-4,6-dihydrothiazolo[4,5-c]pyridine-5-carboxylate (3c)

To a solution of *tert*-butyl 2-amino-7-oxo-6,7-dihydro-4H-thiazolo[4,5-c]pyridine-5-carboxylate (3b) (0.473 g, 1.76 mmol) in anhydrous ACN (33 mL) under inert atmosphere at -20°C was added isoamyl nitrite (0.710 mL, 5.27 mmol). After 10 min at -20°C, Copper(II) chloride (0.473 g, 3.52 mmol) was added. The mixture was stirred for 1 h at -20°C, then 4 h at rt. The solution was extracted with DCM, washed with 10% of Na₂CO₃ aqueous solution. The organic layer was dried over Na₂SO₄, filtered and evaporated. The product was purified by flash chromatography on silica gel (DCM/EtOAc 98/2) to give *tert*-butyl 2-chloro-7-oxo-4,6-dihydrothiazolo[4,5-c]pyridine-5-carboxylate (3c) (0.458 g, 1.59 mmol, 90%) as an off-white solid.
MS *m*/*z* ([M+H-*tert*butyl]⁺) 233/235.
MS *m*/*z* ([M-H]⁻) 287/289.
¹H NMR (400 MHz, CDCl₃): *δ*(ppm) 1.48 (s, 9H), 4.31 (s, 2H), 4.83 (s, 2H).

### Step 4: preparation of intermediate tert-butyl 2-methoxy-7-oxo-6,7-dihydro-4H-thiazolo[4,5-c]pyridine-5-carboxylate (3d)

To a solution of *tert*-butyl 2-chloro-7-oxo-4,6-dihydrothiazolo[4,5-c]pyridine-5-carboxylate (3c) (3.75 g, 12.98 mmol) in anhydrous MeOH (97 mL) under inert atmosphere at -78°C was added dropwise a MeONa solution 0.5M in MeOH (28.6 mL, 14.30 mmol). The reaction mixture was stirred for 15 min at -78°C, then for 30 min at rt. MeOH was removed under vacuum and the resulting residue was diluted with DCM and filtered on a mixture of silica gel and celite®. The product was eluted with DCM/EtOAc 8/2, concentrated *in vacuo* and purified by flash chromatography on silica gel (cyclohexane/EtOAc 90/10 to 80/20) to provide *tert*-butyl 2-methoxy-7-oxo-6,7-dihydro-4*H*-thiazolo[4,5-c]pyridine-5-carboxylate (3d) (2.23 g, 7.84 mmol, 60%) as a yellow solid.
MS *m*/*z* ([M+H]⁺) 285.
¹H NMR (400 MHz, CDCl₃): *δ*(ppm) 1.49 (s, 9H), 4.17 (s, 3H), 4.26 (s, 2H), 4.70 (s, 2H).

### Step 5: preparation of intermediate tert-butyl 2,7-dioxo-4,6-dihydro-3H-thiazolo[4,5-c]pyridine-5-carboxylate (3e)

To a solution of *tert*-butyl 2-methoxy-7-oxo-6,7-dihydro-4*H*-thiazolo[4,5-c]pyridine-5-carboxylate (3d) (7.19 g, 25.31 mmol) in anhydrous dioxane (193 mL) was added drop by drop HCI 12 N (2.50 mL). The reaction mixture was stirred for 4 h at 70°C then concentrated under vacuum. The residue was diluted with EtOAc and washed with water. The organic layer was dried over Na₂SO₄, filtered and concentrated *in vacuo* to provide *tert*-butyl 2,7-dioxo-4,6-dihydro-3H-thiazolo[4,5-c]pyridine-5-carboxylate (3e) (3.43 g, 12.70 mmol, 50%) as an orange solid.
MS *m*/*z* ([M-tBu+H]⁺) 215.
MS *m*/*z* ([M-H]⁻) 269.

### Step 6: preparation of intermediate tert-butyl 3-(2-hydroxyethyl)-2,7-dioxo-4,6-dihydrothiazolo[4,5-c]pyridine-5-carboxylate (3f)

To a solution of *tert*-butyl 2,7-dioxo-4,6-dihydro-3*H*-thiazolo[4,5-c]pyridine-5-carboxylate (3e) (11 g, 40.70 mmol) in anhydrous ACN (90 mL) under inert atmosphere were added K₂CO₃ (7.3 g, 52.90 mmol) and iodoethanol (12.7 mL, 162 mmol) and the mixture was stirred for 7 h at 70°C and concentrated *in vacuo.* The residue was diluted with EtOAc and water. The aqueous layer was acidified (pH 2) with 1N HCI solution and extracted with EtOAc twice. The organic layers were combined, washed with water and brine, dried over Na₂SO₄, filtered and concentrated *in vacuo* to provide *tert*-butyl 3-(2-hydroxyethyl)-2,7-dioxo-4,6-dihydrothiazolo[4,5-c]pyridine-5-carboxylate (3f) (18 g) as a yellow oil. The crude was used in the next step without further purification.
MS *m*/*z* ([M+H]⁺) 315.
MS *m*/*z* ([M-H]⁻) 313.
¹H NMR (400 MHz, DMSO): δ (ppm) 1.42 (s, 9H), 3.60 (t, *J* = 4.9 Hz, 2H), 3.81 (t, *J* = 4.9 Hz, 2H), 4.18 (s, 2H), 4.77 (bs, 2H).

### Step 7: preparation of intermediate tert-butyl 3-[2-[tert-butyl(dimethyl)silyl]oxyethyl]-2,7-dioxo-4,6-dihydrothiazolo[4,5-c]pyridine-5-carboxylate (3g)

To a solution of crude compound *tert*-butyl 3-(2-hydroxyethyl)-2,7-dioxo-4,6-dihydrothiazolo[4,5-c]pyridine-5-carboxylate (3f) (2.70 g, 8.64 mmol) in anhydrous DMF under inert atmosphere were added TBDMSCI (1.43 g, 9.50 mmol) and imidazole (1.17 g, 17.18 mmol). The mixture was stirred for 1 h at rt then ice and EtOAc were added. The organic layer was separated, washed with brine, dried over Na₂SO₄, filtered and concentrated *in vacuo* to provide *tert*-butyl 3-[2-[*tert*-butyl(dimethyl)silyl]oxyethyl]-2,7-dioxo-4,6-dihydrothiazolo[4,5-c]pyridine-5-carboxylate (3g) (2.46 g, 5.74 mmol, 66% over 2 steps) as a brown oil which was used in the next step without further purification.
MS *m*/*z* ([M+H]⁺) 429.
MS *m*/*z* ([M-H]⁻) 427.
¹H NMR (400 MHz, CDCl₃): *δ*(ppm) 0.00 (s, 6H), 0.83 (s, 9H), 1.49 (s, 9H), 3.87 (s, 4H), 4.23 (s, 2H), 4.68 (bs, 2H).

### Step 8: preparation of intermediate tert-butyl 3-[2-[tert-butyl(dimethyl)silyl]oxyethyl]-7-hydroxy-2-oxo-6,7-dihydro-4H-thiazolo[4,5-c]pyridine-5-carboxylate (3h)

To a solution of *tert*-butyl 3-[2-[*tert*-butyl(dimethyl)silyl]oxyethyl]-2,7-dioxo-4,6-dihydrothiazolo[4,5-c]pyridine-5-carboxylate (3g) (15.09 g, 35.20 mmol) in anhydrous MeOH (525 mL) at 0°C under inert atmosphere was added sodium borohydride (2 g, 52.94 mmol) by portions. The reaction mixture was stirred for 1 h and then concentrated under vacuum. The residue was purified by flash chromatography on silica gel (DCM/MeOH 100/0 to 98/2) to provide *tert*-butyl 3-[2-[*tert*-butyl(dimethyl)silyl]oxyethyl]-7-hydroxy-2-oxo-6,7-dihydro-4*H-*thiazolo[4,5-c]pyridine-5-carboxylate (3h) (6.76 g, 15.6 mmol, 44%) as a yellow oil.
MS *m*/*z* ([M+H]⁺) 431.
¹H NMR (400 MHz, CDCl₃): *δ* (ppm) 0.00 (bs, 6H), 0.84 (s, 9H), 1.50 (s, 9H), 3.48 (dd, J = 3.4/13.7 Hz, 1H), 3.66-3.84 (m, 4H), 4.00-4.10 (m, 2H), 4.50-4.73 (m, 2H).

### Step 9: preparation of intermediate tert-butyl 7-[allyloxy-(2-nitrophenyl)sulfonyl-amino]-3-[2-[tert-butyl(dimethyl)silyl]oxyethyl]-2-oxo-6,7-dihydro-4H-thiazolo[4,5-c]pyridine-5-carboxylate (3i)

To a solution of *tert*-butyl 3-[2-[*tert*-butyl(dimethyl)silyl]oxyethyl]-7-hydroxy-2-oxo-6,7-dihydro-4*H*-thiazolo[4,5-c]pyridine-5-carboxylate (3h) (6.70 g, 15.56 mmol) in anhydrous toluene (170 mL) under inert atmosphere was added *N*-allyloxy-2-nitro-benzenesulfonamide (4.82 g, 18.67 mmol) and PPh₃ (4.08 g, 18.67 mmol). DTA (4.30 g, 18.6 mmol) was added by portion and the mixture was stirred at rt. After 1 h of reaction, magnesium chloride (3.60 g, 37.30 mmol) was added and the reaction mixture was stirred for 2 h at 60°C then overnight at rt. The solution was filtered and concentrated *in vacuo.* The residue was purified by flash chromatography on silica gel (toluene/acetone 100/0 to 90/10) to provide compound (3i) (5.54 g, 8.20 mmol, 53%) as a yellow oil.

### Step 10: preparation of intermediate tert-butyl 7-(allyloxyamino)-3-[2-[tert-butyl(dimethyl)silyl]oxyethyl]-2-oxo-6,7-dihydro-4H-thiazolo[4,5-c]pyridine-5-carboxylate(3j)

To a solution of *tert*-butyl 7-[allyloxy-(2-nitrophenyl)sulfonyl-amino]-3-[2-[*tert*-butyl (dimethyl)silyl]oxyethyl]-2-oxo-6,7-dihydro-4*H*-thiazolo[4,5-c]pyridine-5-carboxylate (3i) (5.2 g, 7.76 mmol) in anhydrous ACN (49 mL) under inert atmosphere was added successively PhSH (3.97 mL, 38.76 mmol) and K₂CO₃ (8.04 g, 58.09 mmol). The reaction mixture was stirred for 16 h at rt. The reaction mixture was concentrated under vacuum, diluted with DCM and filtered to eliminate salts. The residue was purified by flash chromatography on silica gel (cyclohexane/EtOAc 80/20 to 60/40) to provide *tert*-butyl 7-(allyloxyamino)-3-[2-[*tert-*butyl(dimethyl)silyl]oxyethyl]-2-oxo-6,7-dihydro-4*H*-thiazolo[4,5-c]pyridine-5-carboxylate (3j) (2.53 g, 5.21 mmol, 67%).

### Step 11: preparation of intermediate 10-Allyloxy-5-(2-hydroxy-ethyl)-3-thia-5,8,10-triazatricyclo[6.2.1.0^{2,6}]undec-2(6)-ene-4,9-dione (3k)

To a solution of *tert*-butyl 7-(allyloxyamino)-3-[2-[*tert*-butyl(dimethyl)silyl]oxyethyl]-2-oxo-6,7-dihydro-4*H*-thiazolo[4,5-c]pyridine-5-carboxylate (3j) (4.29 g, 8.83 mmol) in DCM (88.3 mL) were added TEA (2.46 mL, 17.7 mmol) and diphosgene (1.58 mL, 11.48 mmol). After 5 min, the solution was washed with brine. The organic layer was dried over Na₂SO₄, filtered and concentrated *in vacuo.* The yellow oil obtained was stirred with 4M HCI solution in dioxane (88.3 mL) during 1 h. The mixture was concentrated *in vacuo* and to the crude in DCM (88.4 mL) was dropwise added TEA (2.25 mL, 17.67 mmol). The reaction mixture was stirred at rt for 30 min then diluted with DCM and washed with water. The organic layer was dried over Na₂SO₄ and concentrated *in vacuo.* The crude was purified by flash chromatography on silica gel (DCM/MeOH 100/0 to 90/10) to afford 10-allyloxy-5-(2-hydroxy-ethyl)-3-thia-5,8,10-triazatricyclo[6.2.1.0^{2,6}]undec-2(6)-ene-4,9-dione (3k) (1.72 g, 5.78 mmol, 66%).
MS *m*/*z* ([M+H]⁺) 298.
¹H NMR (300 MHz, CDCl₃): *δ* (ppm) 3.27 (d, *J* = 10.8 Hz, 1H), 3.52-3.78 (m, 7H), 4.18 (s, 1H), 4.33-4.47 (m, 2H), 5.28-5.38 (m, 2H), 5.90-6.04 (m, 1H).

### Step 12: preparation of intermediate 10-allyloxy-5-(2-methylsulfonyloxyethyl)-3-thia-5.8.10-triaza-tricyclo[6.2.1.0^{2,6}]undec-2(6)-ene-4,9-dione (3l)

To a solution of 10-allyloxy-5-(2-hydroxy-ethyl)-3-thia-5,8,10-triaza-tricyclo[6.2.1.0^{2,6}]undec-2(6)-ene-4,9-dione (3k) (316 mg, 1.06 mmol) in anhydrous pyridine (5.3 mL) at 0°C was added MsCI (132 µL, 1.70 mmol). The mixture was stirred for 2 h at 0°C then concentrated *in vacuo.* The residue was diluted with DCM and washed with a 2N HCI solution. The organic layer was dried over Na₂SO₄ , filtered and concentrated *in vacuo* to provide 10-allyloxy-5-(2-methylsulfonyloxyethyl)-3-thia-5,8,10-triaza-tricyclo[6.2.1.0^{2,6}]undec-2(6)-ene-4,9-dione (31) which was used in the next step without further purification.
MS *m*/*z* ([M+H]⁺) 376.

### Step 13: preparation of intermediate 10-allyloxy-5-(2-(tert-butoxycarbonylamino)ethyl)-3-thia-5,8,10-triaza-tricyclo[6.2.1.0^{2,6}]undec-2(6)-ene-4,9-dione (3m)

In a sealed flask, to a solution of 10-allyloxy-5-(2-methylsulfonyloxyethyl)-3-thia-5,8,10-triazatricyclo[6.2.1.0^{2,6}]undec-2(6)-ene-4,9-dione (31) (1.06 mmol) in anhydrous DMF (5.30 mL) was added NaN₃ (345 mg, 5.31 mmol). The reaction mixture was stirred 18 h at 65°C before concentration *in vacuo.* The residue was dissolved in EtOAc and washed with NaCl aqueous solution. The organic layer was dried over Na₂SO₄, filtered and concentrated *in vacuo.* The yellow oil was dissolved in anhydrous THF (3.2 mL) and toluene (3.2 mL) and a solution of PMe₃ 1M in THF (1.6 mL) was added. The reaction mixture was stirred 1 h at rt then cooled to 0°C and a solution of Boc-ON (392 mg, 1.6 mmol) in anhydrous THF (2.2 mL) was slowly added. After 1 h at rt, the reaction mixture was concentrated *in vacuo* and purified by flash chromatography on silica gel (cyclohexane/EtOAc 100/0 to 0/100) to afford 10-allyloxy-5-(2-(*tert*-butoxycarbonylamino)ethyl)-3-thia-5,8,10-triaza-tricyclo[6.2.1.0^{2,6}]undec-2(6)-ene-4,9-dione (3m) (190 mg, 0.50 mmol, 45% over 2 steps) as a off white oil.
MS *m*/*z* ([M+H]⁺) 397.
¹H NMR (300 MHz, CDCl₃): *δ* (ppm) 1.41-1.42 (m, 9H), 3.27-3.36 (m, 2H), 3.47-3.55 (m, 1H), 3.65 (dd, *J* = 2.8/10.9 Hz, 1H), 3.72-3.81 (m, 1H), 4.15 (dd, *J* = 1.9/4.8 Hz, 2H), 4.34-4.48 (m, 2H), 4.37-4.90 (m, 1H), 5.28-5.38 (m, 2H), 5.91-6.05 (m, 1H), 7-13-7.24 (m, 1H).

### Step 14: preparation of sodium [5-(2-(tert-butoxycarbonylamino)ethyl)-4,9-dioxo-3-thia-5,8,10-triaza-tricyclo[6.2.1.0^{2,6}]undec-2(6)-en-10-yl] sulfate (3n)

To a solution of 10-allyloxy-5-(2-(*tert*-butoxycarbonylamino)ethyl)-3-thia-5,8,10-triazatricyclo[6.2.1.0^{2,6}]undec-2(6)-ene-4,9-dione (3m) (190 mg, 0.48 mmol) and glacial AcOH (26 µL, 0.85 mmol) in anhydrous DCM (1.7 mL) was added in one portion Pd(Ph₃)₄ (133 mg, 0.124 mmol). After stirring for 30 min at rt, dry pyridine (1.37 mL) and sulfur trioxide pyridine complex (182 mg, 0.65 mmol) were added to the mixture and the resulting solution was protected from light and stirred overnight at rt until sulfatation was completed. The reaction mixture was concentrated *in vacuo,* diluted with DCM and filtered. The filtrate was concentrated and purified on silica gel (DCM/acetone 100/0 to 60/40) to provide an oil. This oil was solubilized in a minimal volume of water and ACN and applied on a Dowex sodium form column (Dowex® 50WX8 hydrogen form stored with an aqueous solution of 2N NaOH and washed until neutral pH with water). The fractions containing the desired compound were combined, frozen and lyophilized to afford sodium [5-(2-(*tert*-butoxycarbonylamino)ethyl)-4,9-dioxo-3-thia-5,8,10-triaza-tricyclo[6.2.1.0^{2,6}]undec-2(6)-en-10-yl] sulfate (3n) (62.4 mg, 0.14 mmol, 29%) as a white solid.
MS *m*/*z* ([M-H]⁻) 435.
1H NMR (400 MHz, D₂O): *δ* (ppm) 1.36-1.38 (s, 9H), 3.24-3.40 (m, 2H), 3.44-3.53 (m, 1H), 3.62-3.67 (m, 2H), 3.82 (dd, *J* = 2.99/11.6 Hz, 1H), 4.20-4.35 (m, 2H), 4.76 (m, in D₂O peak, 1H).

### Step 15: preparation of sodium and 2,2,2-trifluoroacetate [5-(2-azaniumethyl)-4,9-dioxo-3-thia-5,8,10-triaza-tricyclo[6.2.1.0^{2,6}]undec-2(6)-en-10-yl] sulfate (Example 3)

At 0°C, a solution of TFA (155 µL) in DCM (105 µL) was prepared and added to a solution of sodium [5-(2-(*tert*-butoxycarbonylamino)ethyl)-4,9-dioxo-3-thia-5,8,10-triazatricyclo[6.2.1.0^{2,6}]undec-2(6)-en-10-yl] sulfate (3n) (5.7 mg, 0.013 mmol) in DCM (155 µL) at 0°C. The reaction mixture was stirred 30 min at this temperature then concentrated under nitrogen flux. The solid was dissolved in water (1 mL), filtered, frozen and lyophilized to afford sodium and 2,2,2-trifluoroacetate [5-(2-azaniumethyl)-4,9-dioxo-3-thia-5,8,10-triazatricyclo[6.2.1.0^{2,6}]undec-2(6)-en-10-yl] sulfate (Example 3) (3.2 mg, 0.007 mmol, 54%) as a white solid.
MS *m*/*z* ([M-H]⁻) 335.
1H NMR (300 MHz, D₂O): *δ* (ppm) 3.26 (t, *J* = 6.0 Hz, 2H), 3.54 (d, *J* = 11.5 Hz, 1H), 3.81 (dd, *J* = 3.0/11.5 Hz, 1H), 3.92-3.98 (m, 2H), 4.25 (d, *J* = 16.8 Hz, 1H), 4.38 (d, *J* = 16.9 Hz, 1H), 4.70-4.86 (m, in D₂O peak, 1H).

### Example 4: synthesis of sodium and 2.2.2-trifluroacetate [(2S,5S)-4-(2-amino-2-oxo-ethoxy)imino-2-(azaniumylmethyl)-7-oxo-1,6-diazabicyclo[3.2.1]octan-6-yl]sulfate (reference example)

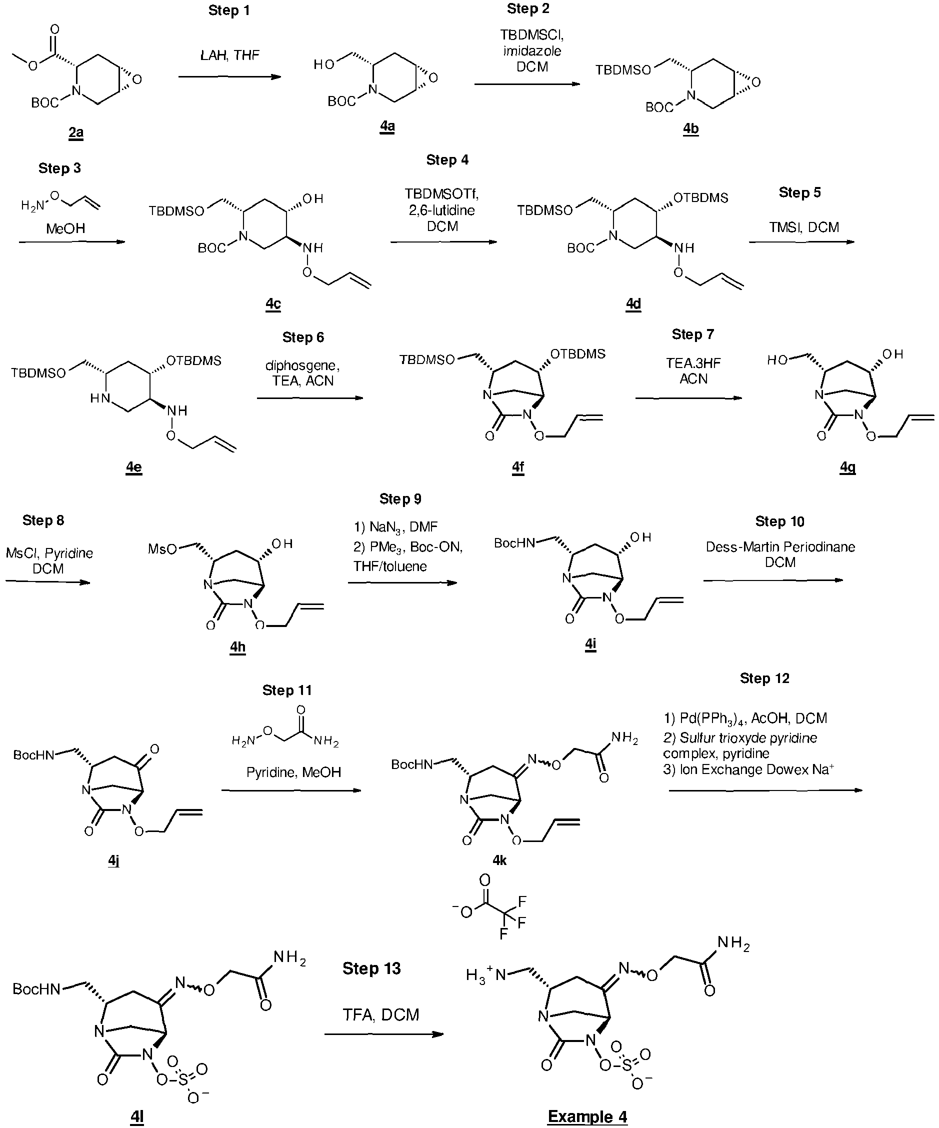

### Step 1 : preparation of intermediate tert-butyl (1S,3S,6R)-3-(hydroxymethyl)-7-oxa-4-azabicyclo[4.1.0]heptane-4-carboxylate (4a)

To a solution of 4-*tert*-butyl ester 3-methyl ester (1*R*,4*S*,6*S*)-7-oxa-3-azabicyclo[4.1.0]heptane-3,4-dicarboxylate (2a) (5.40 g, 21.01 mmol) in anhydrous THF (105 mL) under inert atmosphere to -78°C was added a solution of LiAlH₄ 2M in THF (10.5 mL, 21.01 mmol). The reaction was stirred 3 h between -78°C and -20°C. The reaction was quenched by adding of Na₂SO₄.10H₂O. The mixture was warmed to 20°C, stirred 30 min then filtered over Celite®. The filtrate was concentrated under vacuum and provided *tert-*butyl (1*S*,3*S*,6*R*)-3-(hydroxymethyl)-7-oxa-4-azabicyclo[4.1.0]heptane-4-carboxylate (4a) (3.76g, 16.39 mmol, 78%) as a colorless oil which was used for the next step without further purification.
MS *m*/*z* ([M+H-Boc]⁺) 130.

### Step 2 : preparation of intermediate tert-butyl (1S,3S,6R)-3-[[tert-butyl(dimethyl)silyl] oxymethyl]-7-oxa-4-azabicyclo[4.1.0]heptane-4-carboxylate (4b)

To a solution of *tert*-butyl (1*S*,3*S*,6*R*)-3-(hydroxymethyl)-7-oxa-4-azabicyclo[4.1.0]heptane-4-carboxylate (4a) (3.90 g, 17.03 mmol) in anhydrous DCM (170 mL) under inert atmosphere at rt was added imidazole (2.90 g, 42.57 mmol) followed by TBDMSCI (3.85 g, 25.55 mmol). The mixture was stirred for 2 h at 20°C and then the solution was quenched with saturated NaHCO₃ aqueous solution and extracted with DCM. The organic layer was dried over Na₂SO₄, filtered and evaporated. The product was purified by flash chromatography on silica gel (cyclohexane/EtOAc : from 90/10 to 50/50) to afford *tert*-butyl (1*S,*3*S,*6*R*)-3-[[*tert-*butyl(dimethyl)silyl]oxymethyl]-7-oxa-4-azabicyclo[4.1.0]heptane-4-carboxylate (4b) (3.79 g, 11.03 mmol, 64%) as a colorless oil.
MS *m*/*z* ([M+H-Boc]⁺) 244.
¹H NMR (400 MHz, CDCl₃): *δ* (ppm): 0.04 and 0.05 (s, 6H), 0.88 (s, 9H), 1.45 (s, 9H), 2.01 (ddd, *J* = 15.5/7.1/1.7 Hz, 1H), 2.25 (d, *J* = 15.4 Hz, 1H), 3.22-3.31 (m, 2H), 3.40-3.58 (m, 1H), 3.61 (dd, *J* = 9.7/7.7 Hz, 1H), 3.71 (t, *J* = 8.6 Hz, 1H), 3.95-4.30 (m, 2H)

### Step 3 : preparation of intermediate tert-butyl (2S,4S,5S)-5-(allyloxyamino)-2-[[tert-butyl(dimethyl)silyl]oxymethyl]-4-hydroxy-piperidine-1-carboxylate (4c)

To a solution of tert-butyl (1*S*,3*S*,6*R*)-3-[[*tert*-butyl(dimethyl)silyl]oxymethyl]-7-oxa-4-azabicyclo[4.1.0]heptane-4-carboxylate (4b) (4.35 g, 12.69 mmol) in anhydrous MeOH (25.4 mL) under inert atmosphere was added *O*-allylhydroxylamine (5.45g, 63.45 mmol). The reactor was sealed and the reaction was stirred 3 days at 80°C. MeOH was evaporated and the residue was purified by flash chromatography on silica gel (Cyclohexane/EtOAc: from 90/10 to 0/100) to provide *tert*-butyl (2*S*,4*S*,5*S*)-5-(allyloxyamino)-2-[[*tert-*butyl(dimethyl)silyl]oxymethyl]-4-hydroxy-piperidine-1-carboxylate (4c) (3.47 g, 8.32 mmol, 65%) as a colorless oil and starting material (4b) (1.05 g, 3.06 mmol).
MS *m*/*z* ([M+H]⁺) 417.
¹H NMR (300 MHz, CDCl₃): *δ* (ppm) 0.09 (s, 6H), 0.91 (s, 9H), 1.46 (s, 9H), 1.76 (dt, *J* = 14.6/4.1 Hz, 1H), 2.17 (ddd, *J* = 14.6/8.0/4.0 Hz, 1H), 3.11 (s, 1H), 3.36 (dd, *J* = 14.3/3.4 Hz, 1H), 3.60 (dd, *J* = 10.5/2.6 Hz, 1H), 3.70-3.83 (m, 1H), 3.90-4.06 (m, 2H), 4.06-4.21 (m, 3H), 4.31 (s, 1H), 5.12-5.30 (m, 2H), 5.50 (s, 1H), 5.82-6.02 (m, 1H)

### Step 4 : preparation of intermediate tert-butyl (2S,4S,5S)-5-(allyloxyamino)-4-[tert-butyl(dimethyl)silylloxy-2-[[tert-butyl(dimethyl)silyl]oxymethyl]piperidine-1-carboxylate (4d)

To a solution of *tert*-butyl (2*S*,4*S*,5*S*)-5-(allyloxyamino)-2-[[*tert*-butyl(dimethyl)silyl]oxymethyl]-4-hydroxy-piperidine-1-carboxylate (4c) (2.63 g, 6.32 mmol) in anhydrous DCM (25 mL) under inert atmosphere at 0°C was added 2,6-lutidine (0.883 mL, 7.58 mmol) followed by TBDMSOTf (1.52 mL, 6.64 mmol). The mixture was stirred for 2 h at 20°C and then the solution was extracted with DCM, washed with saturated sodium hydrogencarbonate aqueous solution. The organic layer was dried over Na₂SO₄, filtered and evaporated. The product was purified by flash chromatography on silica gel (cyclohexane/EtOAc: gradient 99/1 to 90/10) to afford *tert*-butyl (2*S*,4*S*,5*S*)-5-(allyloxyamino)-4-[*tert*-butyl(dimethyl)silyl]oxy-2-[[*tert*-butyl(dimethyl)silyl]oxymethyl] piperidine-1-carboxylate (4d) (2.92 g, 5.50 mmol, 87%) as a colorless oil.
MS *m*/*z* ([M+H]⁺) 531.
¹H NMR (300 MHz, CDCl₃): *δ* (ppm) 0.01 and 0.02 (s, 6H), 0.06 (s, 6H), 0.87 (s, 9H), 0.89 (s, 9H), 1.43 (s, 9H), 1.59-1.71 (m, 1H), 1.78-1.89 (ddd, *J* = 14.3/6.7/3.4 Hz 1H), 2.89-2.98 (m, 1H), 3.24 (dd, *J* = 14.2/3.1 Hz, 1H), 3.69 (dd, *J* = 10.2/6.4 Hz, 1H), 3.83-4.04 (m, 3H), 4.07-4.24 (m, 2H), 5.13-5.20 (m, 1H), 5.21-5.30 (m, 1H), 5.52 (d, *J* = 6.9 Hz, 1H), 5.91 (ddt, *J* = 17.3/10.3/5.9 Hz, 1H).

### Step 5: preparation of intermediate (3S,4S,6S)-N-allyloxy-4-[tert-butyl(dimethyl)silyl]oxy-6-[[tert-butyl(dimethyl)silyl]oxymethyl]piperidin-3-amine (4e)

To a solution of *tert*-butyl (2*S*,4*S*,5*S*)-5-(allyloxyamino)-4-[*tert*-butyl(dimethyl)silyl]oxy-2-[[*tert-*butyl(dimethyl)silyl]oxymethyl]piperidine-1-carboxylate (4d) (2.92 g, 5.51 mmol) in anhydrous DCM (110 mL) under inert atmosphere at rt, was added slowly TMSI (1.24 mL, 8.26 mmol). The reaction mixture was stirred for 2h30 at rt. The mixture was then quenched at 0°C with MeOH (10 mL). The mixture was quenched with Na₂S₂O₃ 15% (200 mL) and extracted with DCM (3 x 50 mL). The organic layer was dried over Na₂SO₄, filtered and evaporated and the resulting residue was purified by flash chromatography on silica gel (Cyclohexane/EtOAc : 90/10 to 0/100) to provide (3*S*,4*S*,6*S*)-*N*-allyloxy-4-[*tert*-butyl(dimethyl)silyl]oxy-6-[[*tert-*butyl(dimethyl)silyl]oxymethyl]piperidin-3-amine (4e) (1.88 g, 4.36 mmol, 79%) as a yellow solid.
MS *m*/*z* ([M+H]⁺) 431.
¹H NMR (400 MHz, CDCl₃): *δ* (ppm) 0.08 and 0.11 (s, 6H), 0.15 (s, 6H), 0.89 (s, 9H), 0.92 (s, 9H), 1.84 (q, *J* = 12.1 Hz, 1H), 1.98-2.08 (m, 1H), 3.00 (t, *J* = 11.9 Hz, 1H), 3.10-3.20 (m, 1H), 3.28-3.40 (m, 1H), 3.77 (dd, *J* = 12.5/4.1 Hz, 1H), 3.84-4.05 (m, 3H), 4.09-4.17 (m, 2H), 5.16-5.23 (m, 1H), 5.23-5.32 (m, 1H), 5.87 (ddt, *J* = 17.3/10.3/5.9 Hz, 1H), 6.11 (bs, 1H)

### Step 6: preparation of intermediate (2S,4S,5S)-6-allyloxy-4-[tert-butyl(dimethyl)silyl]oxy-2-[[tert-butyl(dimethyl)silyl]oxymethyl]-1,6-diazabicyclo[3.2.1]octan-7-one (4f)

To a solution of (3*S*,4*S*,6*S*)-*N*-allyloxy-4-[*tert*-butyl(dimethyl)silyl]oxy-6-[[*tert-*butyl(dimethyl)silyl]oxymethyl]piperidin-3-amine (4e) (1.88 g, 4.37 mmol) and TEA (2.43 mL, 17.48 mmol) in anhydrous ACN (360 mL) at 0°C was added diphosgene (0.290 mL, 2.40 mmol) as a solution in ACN (6 mL, flow = 0.15 mL/min). Once addition was completed, the reaction was stirred to rt for 1h. The mixture was washed with water (500 mL), extracted with EtOAc (3 x 200 mL) dried over Na₂SO₄,filtered and concentrated. The crude residue was purified by flash chromatography on silica gel (cyclohexane/EtOAc : gradient 100/0 to 0/100) to provide intermediate (2*S*,4*S*,5*S*)-6-allyloxy-4-[*tert*-butyl(dimethyl)silyl]oxy-2-[[*tert-*butyl(dimethyl)silyl]oxymethyl]-1,6-diazabicyclo[3.2.1]octan-7-one (4f) (1.22 g, 2.67 mmol, 61%) as a brown oil.
MS *m*/*z* ([M+H]⁺) 457.
¹H NMR (400 MHz, CDCl₃): *δ* (ppm) 0.02-0.12 (m, 12H), 0.88 and 0.89 (s, 18H), 1.73 (dt, *J* = 15.4/3.7 Hz, 1H), 2.06 (ddd, *J* = 15.4/7.6/5.4 Hz, 1H), 2.91 (dd, *J* = 11.6/3.3 Hz, 1H), 3.30-3.52 (m, 3H), 3.83 (dd, *J* = 6.5/1.9 Hz, 2H), 4.19-4.28 (m, 1H), 4.35-4.53 (m, 2H), 5.27-5.41 (m, 2H), 5.94-6.09 (m, 1H).

### Step 7: preparation of intermediate (2S,4S,5S)-6-allyloxy-4-hydroxy-2-(hydroxymethyl)-1,6-diazabicyclo[3.2.1]octan-7-one (4g)

To a solution of (2*S*,4*S*,5*S*)-6-allyloxy-4-[*tert*-butyl(dimethyl)silyl]oxy-2-[[*tert-*butyl(dimethyl)silyl]oxymethyl]-1,6-diazabicyclo[3.2.1]octan-7-one (4f) (1.22 g, 2.67 mmol) in anhydrous ACN (27 mL) under inert atmosphere was added dropwise TEA.3HF (0.436 mL, 2.67mmol). The reaction mixture was stirred at 70°C for 6 h. The mixture was concentrated under vacuum. The crude was purified by flash chromatography on silica gel (DCM/propan-2-ol: gradient 100/0 to 0/100) to provide (2*S*,4*S*,5*S*)-6-allyloxy-4-hydroxy-2-(hydroxymethyl)-1,6-diazabicyclo[3.2.1]octan-7-one (4g) (0.505 g, 2.21 mmol, 82%) as a colorless oil.
MS *m*/*z* ([M+H]⁺) 229.
¹H NMR (400 MHz, CDCl₃): *δ*(ppm) 1.60 (dt, *J* = 15.9/2.3 Hz, 1H), 2.27 (ddd, *J* = 15.8/8.3/2.3 Hz, 1H), 2.89 (dd, *J* = 12.3/3.7 Hz, 1H), 3.39-3.50 (m, 1H), 3.62-3.70 (m, 2H), 3.76-3.92 (m, 3H), 4.16-4.26 (m, 2H), 4.35-4.50 (m, 2H), 5.27-5.41 (m, 2H), 5.92-6.07 (m, 1H)

### Step 8: preparation of intermediate [(2S,4S,5S)-6-allyloxy-4-hydroxy-7-oxo-1,6-diazabicyclo[3.2.1]octan-2-yl]methyl methanesulfonate (4h)

A solution of (2S,4S,5S)-6-allyloxy-4-hydroxy-2-(hydroxymethyl)-1,6-diazabicyclo[3.2.1]octan-7-one (4g) (0.505 g, 2.21 mmol) in DCM (22 mL) was cooled to 0°C. Pyridine (0.21 mL, 2.65 mmol) and MsCI (0.28 mL, 2.32 mmol) were added and the reaction mixture was stirred at the same temperature for 18 h. After concentrating *in vacuo,* the crude was purified by flash chromatography on silica gel (DCM/MeOH: 100/0 to 90/10) to give the intermediate [(2*S*,4*S*,5*S*)-6-allyloxy-4-hydroxy-7-oxo-1,6-diazabicyclo[3.2.1]octan-2-yl]methyl methanesulfonate (4h) (0.72 g, 2.21 mmol quantitative yield) as an yellow oil.
MS *m*/*z* ([M+H]⁺) 307.
¹H NMR (400 MHz, CDCl₃): *δ* (ppm) 1.62 (dt, *J* = 16/2.9 Hz, 1H), 2.25 (ddd, *J* = 15.9/8.0/5.2 Hz, 1H), 2.97 (dd, *J* = 12.3, 3.5 Hz, 1H), 3.11 (s, 3H), 3.50 (d, *J* = 12.3 Hz, 1H), 3.61-3.74 (m, 2H), 4.30-4.52 (m, 5H), 4.59 (dd, *J =* 11.1/8.3 Hz, 1H), 5.28-5.43 (m, 2H), 5.93-6.08 (m, 1H) .

### Step 9: preparation of intermediate tert-butyl N-[[(2S,4S,5S)-6-allyloxy-4-hydroxy-7-oxo-1,6-diazabicyclo[3.2.1]octan-2-yl]methyl]carbamate (4i)

The intermediate [(2*S*,4*S*,5*S*)-6-allyloxy-4-hydroxy-7-oxo-1,6-diazabicyclo[3.2.1]octan-2-yl]methyl methanesulfonate (4h) (0.596 g, 1.95 mmol) was dissolved in DMF (7.8 mL) and NaN₃ (0.63 g, 9.74 mmol) was added. The reaction mixture was heated at 75°C overnight and then, concentrated *in vacuo.* The reaction mixture was quenched with water and extracted with EtOAc. The organic layer was washed with brine, dried over Na₂SO₄, filtered and concentrated *in vacuo.* The crude was dissolved in a mixture of THF and toluene (5 mL/5 mL) and PMe₃ (1M in THF) (2.92 mL, 2.92 mmol) was added at 0°C. After 30 min stirring at rt, the mixture was cooled to 0°C and a solution of Boc-ON (0.72 g, 2.92 mmol) in THF (5 mL) was dropwise added. The mixture was stirred at rt for 1h30 and concentrated *in vacuo.* The crude was purified by flash chromatography on silica gel (cyclohexane/EtOAc : 100/0 to 0/100) to give *tert*-butyl *N*-[[(2*S*,4*S*,5*S*)-6-allyloxy-4-hydroxy-7-oxo-1,6-diazabicyclo[3.2.1]octan-2-yl]methyl]carbamate (4i) (324 mg, 0.99 mmol, 50%) as a colorless oil.
MS *m*/*z* ([M+H]⁺) 328.
¹H NMR (400 MHz, CDCl₃) : *δ* (ppm) 1.43 (s, 9H), 1.57 (d, *J* = 16.2 Hz, 1H), 2.13-2.25 (m, 1H), 2.87 (dd, *J* = 12.1/3.6 Hz, 1H), 3.27-3.44 (m, 2H), 3.53-3.69 (m, 2H), 3.72 (t, *J* = 3.6 Hz, 1H), 4.32 (bs, 1H), 4.36-4.53 (m, 2H), 5.10 (bs, 1H), 5.27-5.42 (m, 2H), 5.94-6.09 (m, 1H).

### Step 10: preparation of intermediate tert-butyl N-[[(2S,5S)-6-allyloxy-4,7-dioxo-1,6-diazabicyclo[3.2.1]octan-2-yl]methyl]carbamate (4j)

To a solution of intermediate *tert*-butyl *N*-[[(2*S*,4*S*,5*S*)-6-allyloxy-4-hydroxy-7-oxo-1,6-diazabicyclo[3.2.1]octan-2-yl]methyl]carbamate (4i) (0.350 g, 1.07 mmol) in anhydrous DCM (21 mL) under inert atmosphere at 0°C was added the Dess-Martin periodinane reagent (0.680 g, 1.60 mmol). The reaction was stirred for 2h30 at rt then the mixture was washed with a saturated NaHCO₃ aqueous solution, a 15% Na₂S₂O₃ solution, water, dried oved Na₂SO₄, filtered and concentrated under vacuum. The residue was purified by flash chromatography on silica gel (cyclohexane/EtOAc : 100/0 to 0/100) to afford intermediate *tert*-butyl N-[[(2*S*,5*S*)-6-allyloxy-4,7-dioxo-1,6-diazabicyclo[3.2.1]octan-2-yl]methyl]carbamate (4j) (0.300 g, 0.92 mmol, 74%) as a colorless oil.
MS *m*/*z* ([M+H]⁺) 326.
¹H NMR (400 MHz, CDCl₃): *δ* (ppm) 1.41 (s, 9H), 2.26-2.37 (m, 1H), 2.90 (dd, *J* = 17.9/9.4 Hz, 1H), 3.19-3.41 (m, 3H), 3.41-3.51 (m, 1H), 3.84-3.99 (m, 2H), 4.34-4.49 (m, 2H), 4.89-4.97 (m, 1H), 5.26-5.40 (m, 2H), 5.97 (ddt, *J* = 16.9/10.3/6.5 Hz, 1H).

### Step 11: preparation of intermediate tert-butyl N-[[(2S,5S)-6-allyloxy-4-(2-amino-2-oxo-ethoxy)imino-7-oxo-1.6-diazabicyclo[3.2.1]octan-2-yl]methyl]carbamate (4k)

To a solution of *tert*-butyl *N*-[[(2*S*,5*S*)-6-allyloxy-4,7-dioxo-1,6-diazabicyclo[3.2.1]octan-2-yl]methyl]carbamate (4j) (0.107 g, 0.33 mmol) in anhydrous MeOH (3.3 mL) under inert atmosphere was added successively pyridine (56 µL, 0.69 mmol) and 2-(aminooxy)acetamide.HCl (0.041 g, 0.33 mmol). The reaction mixture was stirred for 30 min at rt, then concentrated under vacuum. The residue was purified by flash chromatography on silica gel (DCM/MeOH: 100/0 to 95/5) to provide intermediate *tert*-butyl *N*-[[(2*S*,5*S*)-6-allyloxy-4-(2-amino-2-oxo-ethoxy)imino-7-oxo-1,6-diazabicyclo[3.2.1] octan-2-yl]methyl]carbamate (4k) (0.101 g, 0.25 mmol, ratio *Z*/*E*: 60/40, 82%).
MS *m*/*z* ([M+H]⁺) 398.
¹H NMR (400 MHz, CDCl₃): *δ* (ppm) 1.39 (bs, 9H), 2.24-2.35 (m, 0.6H), 2.54-2.66 (m, 0.4H), 2.69-2.88 (m, 1H), 3.06-3.21 (m, 2H), 3.22-3.51 (m, 2H), 3.54-3.73 (m, 1H), 4.10-4.16 (m, 0.4H), 4.33 - 4.59 (m, 4H), 4.96-5.20 (m, 1.6H), 5.25-5.41 (m, 2H), 5.88-6.11 (m, 2H), 6.43 (s, 0.6H), 6.68 (s, 0.4H)

### Step 12: preparation of intermediate sodium [(2S,5S)-4-(2-amino-2-oxo-ethoxy)imino-2-[(tert-butoxycarbonylamino)methyl]-7-oxo-1.6-diazabicyclo[3.2.1]octan-6-yl] sulfate (41)

To a solution of intermediate *tert*-butyl *N*-[[(2*S*,5*S*)-6-allyloxy-4-(2-amino-2-oxo-ethoxy)imino-7-oxo-1,6-diazabicyclo[3.2.1]octan-2-yl]methyl]carbamate (4k) (0.101 g, 0.254 mmol) and glacial AcOH (29 µL, 0.509 mmol) in anhydrous DCM (2.5 mL) was added in one portion Pd(Ph₃)₄ (0.147 g, 0.127 mmol). After stirring for 1 h at rt, dry pyridine (2.5 mL) and sulfur trioxide pyridine complex (0.202 g, 1.27 mmol) were added to the mixture and the resulting solution was protected from light and stirred overnight at rt until the sulfatation was completed. The reaction mixture was concentrated under vacuum, diluted with DCM and filtered. The filtrate was concentrated and purified by flash chromatography on silica gel (DCM/acetone 100/0 to 0/100) to provide 25 mg of a colorless oil of triphenyl-(propenyl)-phosphonium [(2S,5S)-4-(2-amino-2-oxo-ethoxy)imino-2-[(*tert*-butoxycarbonylamino)methyl]-7-oxo-1,6-diazabicyclo[3.2.1]octan-6-yl] sulfate.
This oil was solubilized in a minimal volume of a mixture of water and acetone and applied on a Dowex sodium form column (Dowex® 50WX8 hydrogen form stored with an aqueous solution of 2N NaOH and washed until neutral pH with water). The fractions containing the desired compound were combined, frozen and lyophilized to afford compound sodium [(2*S*,5*S*)-4-(2-amino-2-oxo-ethoxy)imino-2-[(*tert*-butoxycarbonylamino)methyl]-7-oxo-1,6-diazabicyclo[3.2.1]octan-6-yl]sulfate(4l) (0.016 g, 0.036 mmol, ratio *Z*/*E*: 60/40, 14% over 2 steps) as a white solid.
MS *m*/*z* ([M-H]⁻) 436.
¹H NMR (400 MHz, D₂O): *δ* (ppm) 1.43 (s, 9H), 2.49 (dd, *J* = 17.3/5.5 Hz, 0.6H), 2.70-3.01 (m, 1H), 3.13- 3.59 (m, 4.4H), 3.68-3.75 (m, 1H), 4.49-4.65 (m, 2.4H), 5.46 (d, *J* = 2.9 Hz, 0.6H)

### Step 13: preparation of 2,2,2-trifluroacetate and sodium [(2S,5S)-4-(2-amino-2-oxo-ethoxy)imino-2-(azaniumylmethyl)-7-oxo-1,6-diazabicyclo[3.2.1]octan-6-yl] sulfate (Example 4)

To a suspension of sodium [(2*S*,5*S*)-4-(2-amino-2-oxo-ethoxy)imino-2-[(*tert-*butoxycarbonylamino)methyl]-7-oxo-1,6-diazabicyclo[3.2.1]octan-6-yl] sulfate (41) (0.0167 g , 0.036 mmol) in anhydrous DCM (1 mL) to 0°C was added a solution of TFA (0.4mL) in anhydrous DCM (0.5mL). The mixture was stirred 30 min to 0°C and concentrated. The oil obtained was diluted in water (3 mL), frozen and lyophilized to afford 2,2,2-trifluroacetate and sodium [(2S,5S)-4-(2-amino-2-oxo-ethoxy)imino-2-(azaniumylmethyl)-7-oxo-1,6-diazabicyclo[3.2.1]octan-6-yl] sulfate (Example 4) (0.0164 g, 0.034 mmol, ratio *Z*/*E*: 40/60, 95%) as an yellow solid.
MS *m*/*z* ([M-H]⁻) 336.
¹H NMR (400 MHz, D₂O): *δ* (ppm) 2.56 (dd, *J* = 17.4/7.5 Hz, 0.6H), 2.67 (dd, *J* = 19.7/4.2 Hz, 0.4H), 2.89 (dd, *J* = 17.4/8.0 Hz, 0.6H), 3.15-3.39 (m, 2.4H), 3.46 (dd, *J* = 12.8/2.7 Hz, 1H), 3.58 (dd, *J* = 12.9/3.1 Hz, 0.6H), 3.65 (dd, *J* = 12.7/3.5 Hz, 0.4H), 3.86-3.97 (m, 1H), 4.56-4.64 (m, 2.4H), 5.44 (d, *J* = 2.9 Hz, 0.6H)

### Example 5: synthesis of sodium and 2,2,2-trifluoroacetate [(2S,5R)-2-(azaniumylmethyl)-7-oxo-3-oxazol-2-yl-1,6-diazabicyclo[3.2.1]oct-3-en-6-yl] sulfate

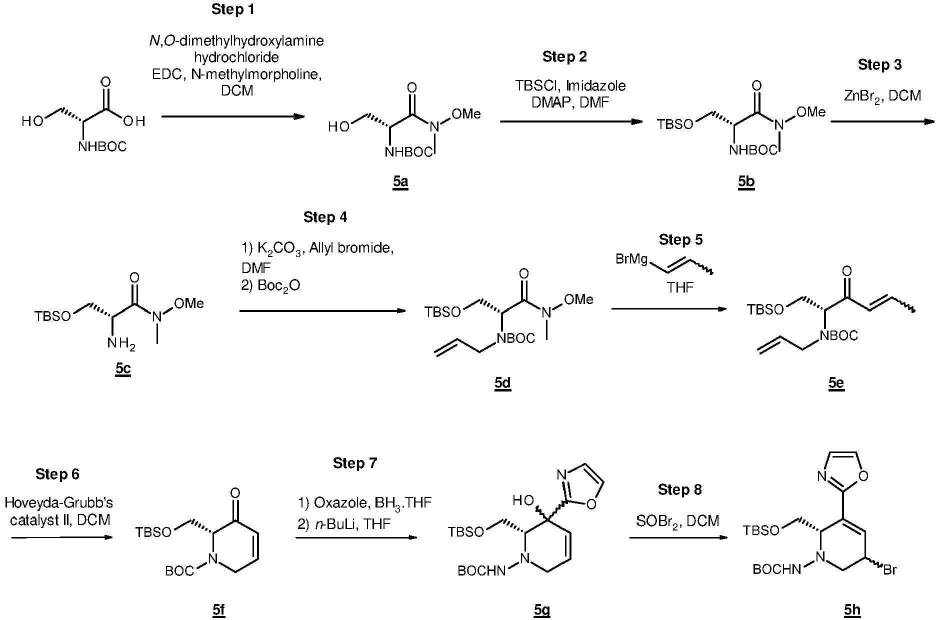

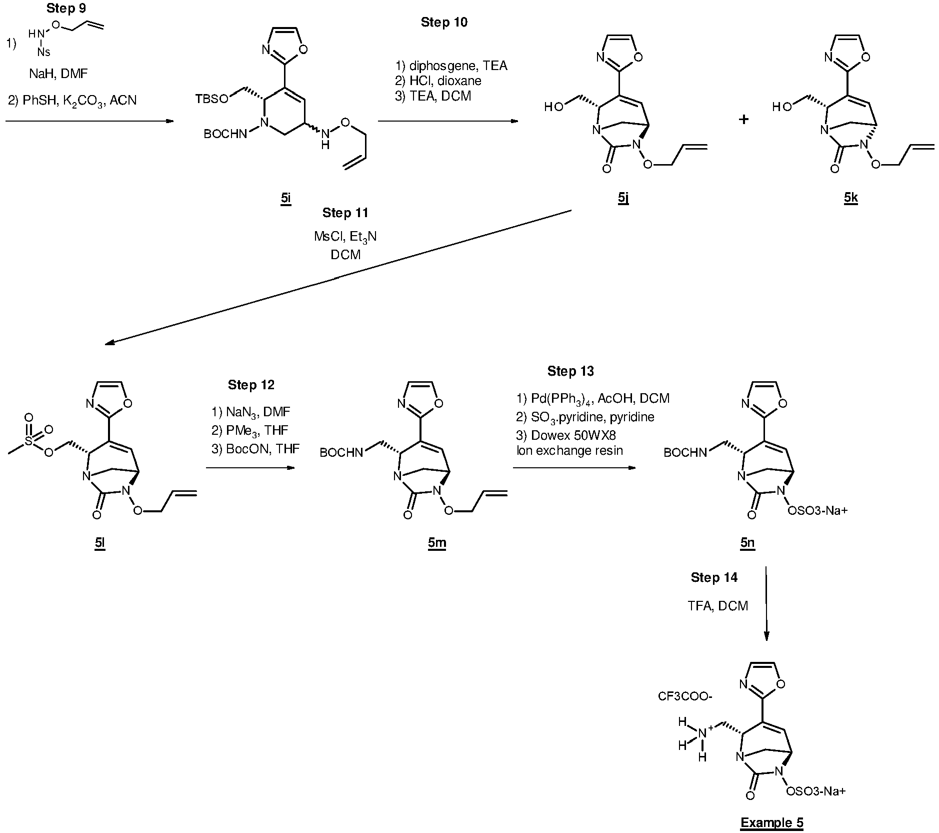

### Step 1: preparation of intermediate tert-butyl N[(1R)-1-(hydroxymethyl)-2-[methoxy(methyl)amino]-2-oxo-ethyl]carbamate (5a)

To a solution of Boc-D-Ser-OH (5 g, 24.37 mmol) in anhydrous DCM (100 mL) at -15°C were added *N*,*O*-dimethylhydroxylamine hydrochloride (2.54 g, 26.07 mmol) and N-methylmorpholine (2.87 mL, 26.07 mmol). *N*-(3-Dimethylaminopropyl)-*N*'-ethylcarbodiimide hydrochloride (5.00 g, 26.07 mmol) was then added portionwise (5 portions) over 20 min. The mixture was stirred at -15°C for 40 min. A 1M HCI solution (50 mL) was added. The mixture was extracted with DCM (2 x 25 mL). The organic layer was washed with a saturated solution of NaHCO₃ (50 mL), H₂O (50 mL), dried over Na₂SO₄ and concentrated *in vacuo* to provide the *tert*-butyl *N*[(1*R*)-1-(hydroxymethyl)-2-[methoxy(methyl)amino]-2-oxo-ethyl]carbamate (5a) (5.42 g, 21.83 mmol, 89%) as a white solid.
¹H NMR (400 MHz, CDCl₃) *δ*1.44 (s, 9H), 2.65 (s, 1H), 3.23 (s, 3H), 3.62-3.97 (m, 5H), 4.79 (s, 1H), 5.60 (d, *J* = 8.4 Hz, 1H).

### Step 2: preparation of intermediate tert-butyl N-[(1R)-1-[[tert-butyl(dimethyl)silyl]oxymethyl]-2-[methoxy(methyl)amino1-2-oxo-ethyl]carbamate (5b)

To a solution of *tert*-butyl *N*-[(1*R*)-1-(hydroxymethyl)-2-[methoxy(methyl)amino]-2-oxoethyl]carbamate (5a) (5.42 g, 21.8 mmol), imidazole (4.46 g, 65.5 mmol) and DMAP (133 mg, 1.1 mmol) in anhydrous DMF (17 mL) at rt was portionwise added tert-butyldimethylsilyl chloride (3.95 g, 26.2 mmol). The mixture was stirred for 2 h then poured in H₂O (50 mL). The aqueous layer was extracted with EtOAc (2 x 40 mL). The organic layer was washed with 1 M HCI (50 mL), brine (40 mL), dried over Na₂SO₄ and concentrated *in vacuo.* The residue was purified by flash chromatography on silica gel (cyclohexane/EtOAc 100/0 to 50/50) to provide *tert*-butyl *N*[(1*R*)-1-[[*tert*-butyl(dimethyl)silyl]oxymethyl]-2-[methoxy(methyl)amino]-2-oxo-ethyl]carbamate (5b) (7.09 g, 19.5 mmol, 89%) as a colorless oil.
MS *m*/*z* ([M+Na]⁺) 385, ([M+H]⁺) 363.
¹H NMR (400 MHz, CDCl₃) *δ* 0.03 (s, 6H), 0.87 (s, 9H), 1.44 (s, 9H), 3.21 (s, 3H), 3.64-3.94 (m, 5H), 4.75 (s, 1H), 5.35 (d, *J* = 9.0 Hz, 1H).

### Step 3: preparation of intermediate (2R)-2-amino-3-[tert-butyl(dimethyl)silyl]oxy-N-methoxy-N-methyl-propanamide (5c)

A solution of compound (5b) (4.60 g, 12.69 mmol) and ZnBr₂ (5.71 g, 25.38 mmol) in DCM (37 mL) was stirred at room temperature for 2h30. A 2M NaOH solution (25 mL) was added followed by H₂O (25 mL). The suspension was filtrated. The solid was washed with H₂O and DCM. The filtrate was extracted with DCM. The organic layer was washed with brine, dried over Na₂SO₄ and concentrated *in vacuo* to provide (2*R*)-2-amino-3-[*tert-*butyl(dimethyl)silyl]oxy-*N*-methoxy-*N*-methyl-propanamide (5c) (3.17 g, 12.08 mmol, 96%) as a colorless oil.
MS *m*/*z* ([2M+H]⁺) 525, ([M+H]⁺) 263.
¹H NMR (400 MHz, CDCl₃) *δ* 0.07 (s, 6H), 0.90 (s, 9H), 1.71 (bs, 2H), 3.23 (s, 3H), 3.63 (dd, *J* = 9.6, 6.6 Hz, 1H), 3.74 (s, 3H), 3.81 (dd, *J* = 9.6, 5.4 Hz, 1H), 3.87-3.96 (m, 1H).

### Step 4: preparation of intermediate tert-butyl N-allyl-N-[(1R)-1-[[tert-butyl(dimethyl)silyl]oxymethyl]-2-[methoxy(methyl)amino]-2-oxo-ethyl]carbamate (5d)

To a solution of compound (5c) (3.17 g, 12.08 mmol) in anhydrous DMF (24 mL) at 0°C was added K₂CO₃ (3.34 g, 24.16 mmol). The mixture was stirred at this temperature for 20 min before adding allyl bromide (1.15 mL, 13.29 mmol). The mixture was stirred for 1 h at 0°C then 2 h at rt. Boc₂O (3.95 g, 18.12 mmol) was added and the mixture maintained at rt overnight. H₂O (50 mL) was added. The mixture was extracted with EtOAc (2 x 30 mL). The organic layer was washed with brine (40 mL), dried over Na₂SO₄ and concentrated *in vacuo.* The residue was purified by flash chromatography on silica gel (cyclohexane/EtOAc : 90/10) to provide *tert*-butyl *N*-allyl-*N*-[(1*R*)-1-[[*tert*-butyl(dimethyl)silyl]oxymethyl]-2-[methoxy(methyl)amino]-2-oxo-ethyl]carbamate (5d) (2.06 g, 5.11 mmol, 42%) as a colorless oil.
MS *m*/*z* ([M+Na]⁺) 425, ([M+H]⁺) 403.
¹H NMR (400 MHz, CDCl₃) *δ* 0.05 (s, 6H), 0.87 (s, 9H), 1.38-1.60 (m, 9H), 3.16 (s, 3H), 3.73 (s, 3H), 3.78-4.07 (m, 4H), 4.87-5.40 (m, 3H), 5.68-5.96 (m, 1H).

### Step 5: preparation of intermediate tert-butyl N-allyl-N-[(1R)-1-[[tert-butyl(dimethyl)silyl]oxymethyl]-2-oxo-pent-3-enyl]carbamate (5e)

A solution of compound (5d) (1.84 g, 4.57 mmol) in anhydrous THF (5 mL) was dropwise added to a propen-1-ylmagnesium bromide solution 0.5M in THF (18.3 mL, 9.14 mmol) at 0°C under nitrogen atmosphere. The mixture was stired at 0°C for 20 min. H₂O (15 mL) and a saturated solution of NH₄Cl (15 mL) were added. The mixture was extracted with *tert*-butyl methyl ether (2 x 20 mL). The organic layer was washed with 1M HCI (20 mL), dried over Na₂SO₄ and concentrated *in vacuo* to provide *tert*-butyl *N*-allyl-*N*-[(1*R*)-1-[[*tert-*butyl(dimethyl)silyl]oxymethyl]-2-oxo-pent-3-enyl]carbamate (5e) (1.70 g, 4.43 mmol, 97%) which was used without further purification.
MS *m*/*z* ([M+Na]⁺) 406.

### Step 6: preparation of intermediate tert-butyl (2R)-2-[[tert-butyl(dimethyl)silyl]oxymethyl]-3-oxo-2,6-dihydropyridine-1-carboxylate (5f)

A solution of compound (5e) (2.48 g, 6.47 mmol) and (1,3-Bis-(2,4,6-trimethylphenyl)-2-imidazolidinylidene)dichloro(o-isopropoxyphenylmethylene)ruthenium (203 mg, 0.32 mmol) in DCM was refluxed for 1 h. The mixture was concentrated *in vacuo.* The residue was purified by flash chromatography on silica gel (DCM/EtOAc: 100/0 to 95/5) to provide *tert*-butyl (2*R*)-2-[[*tert*-butyl(dimethyl)silyl]oxymethyl]-3-oxo-2,6-dihydropyridine-1-carboxylate (5f) (2.07 g, 6.06 mmol, 93%) as a greenish solid.
¹H NMR (400 MHz, CDCl₃) *δ* -0.04 (s, 3H), -0.01 (s, 3H), 0.82 (s, 9H), 1.47 (s, 6H), 1.50 (s, 3H), 3.70-4.19 (m, 3H), 4.38-4.75 (m, 2H), 6.18 (d, *J* = 10.3, 1H), 6.82-7.08 (m, 1H).

### Step 7: preparation of intermediate tert-butyl (2R)-2-[[tert-butyl(dimethyl)silyl]oxymethyl]-3-hydroxy-3-oxazol-2-yl-2,6-dihydropyridine-1-carboxylate (5g)

To a solution of borane tetrahydrofuran complex solution 1.0 M in THF (19 mL, 19 mmol) under nitrogen atmosphere at rt, was dropwise added oxazole (1.24 mL, 18.89 mmol). The mixture was stirred at rt for 1 h then cooled down to -78°C. A n-butyllithium solution 1.6 M in hexanes (12.2 ml, 19.5 mmol) was dropwise added and the mixture maintained at this temperature for 30 min. A solution of compound (5f) (4.30 g, 12.6 mmol) in anhydrous THF (9 mL) was dropwise added. The mixture was stirred at -78°C for 90 min. Ethanol containing 5% AcOH (30 mL) was added and the mixture was stirred at rt for 18 h. Water (50 mL) was added. The aqueous layer was extracted with EtOAc (2 x 50 mL). The combined organic layers were washed with a saturated solution of NaHCO₃ (50 mL), dried over Na₂SO₄ and concentrated *in vacuo.* The residue was purified by flash chromatography on silica gel (cyclohexane/EtOAc: 80/20 to 40/60) to provide compound (5g) (1.12 g, 2.72 mmol, 21%) as a *cis*/*trans* mixture.
MS *m*/*z* ([M+H]⁺) 411.
1H NMR (400 MHz, CDCl₃) *δ* 0.10 and 0.11 (s, 6H), 0.90 (s, 9H), 1.33 and 1.37 (s, 9H), 3.40-3.78 (m, 2H), 4.00-4.08 (m, 1H), 4.26 and 4.38 (d, *J* = 19.4 Hz, 1H), 4.68 and 4.99 (bs, 1H), 4.88 and 4.74 (t, *J* = 7.2 Hz, 1H), 5.86-6.04 (m, 2H), 7.01 and 7.07 (s, 1H), 7.61 (d, *J* = 0.8 Hz, 1H).

### Step 8: preparation of intermediate tert-butyl (6S)-3-bromo-6-[[tert-butyl(dimethyl)silyl]oxymethyl]-5-oxazol-2-yl-3,6-dihydro-2H-pyridine-1-carboxylate (5h)

Thionyl bromide (0.24 mL, 3.0 mmol) was dropwise added to a solution of TEA (0.42 mL, 3.0 mmol) and compound (5g) (1.12 g, 2.73 mmol) in anhydrous DCM (12 mL) at 0°C. The mixture was stirred at 0°C for 20 min then poured in a mixture of ice and H₂O (50 mL). The layers were separated. The aqueous layer was extracted with DCM (2 x 15 mL). The combined organic layers were washed with brine (15 mL) dried over Na₂SO₄ and concentrated *in vacuo* to provide *tert*-butyl (6*S*)-3-bromo-6-[[*tert-*butyl(dimethyl)silyl]oxymethyl]-5-thiazol-2-yl-3,6-dihydro-2H-pyridine-1-carboxylate (5h) (1.25 g, 2.64 mmol, 96%) as a *cis*/*trans* mixture which was used without further purification.

### Step 9: preparation of intermediate tert-butyl (6S)-3-(allyloxyamino)-6-[[tert-butyl(dimethyl)silyl]oxymethyl]-5-oxazol-2-yl-3,6-dihydro-2H-pyridine-1-carboxylate (5i)

To a suspension of NaH 60% in oil (132 mg, 3.31 mmol) in anhydrous DMF (4 mL) at 0°C under nitrogen atmosphere was portionwise added *N*-allyloxy-2-nitro-benzenesulfonamide (856 mg, 3.31 mmol). The mixture was stirred at 0°C for 15 min then a solution of compound (5h) (1.25 g, 2.64 mmol) in anhydrous DMF (4 mL) was dropwise added. The mixture was stirred for 90 min at 0°C then H₂O (15 mL) was added. The mixture was extracted with EtOAc (2 x 15 mL). The organic layer was washed with brine (15 mL), dried over Na₂SO₄ and concentrated *in vacuo.* The residue was purified by flash chromatography on silica gel (DCM). The fractions containing the nosylated intermediate were combined and concentrated *in vacuo.* The residue was dissolved in ACN (20 mL) and K₂CO₃ (1.91 g, 13.81 mmol) and thiophenol (1.42 mL, 13.81 mmol) were added. The mixture was stirred at rt for 1 h then concentrated *in vacuo.* The residue was dissolved in EtOAc (15 mL), washed with a NaOH 2.0 M solution (15 mL), dried over Na₂SO₄ and concentrated *in vacuo.* The residue was purified by flash chromatography on silica gel (DCM/EtOAc: 100/0 to 70/30) to provide *tert-*butyl (6*S*)-3-(allyloxyamino)-6-[[*tert*-butyl(dimethyl)silyl]oxymethyl]-5-oxazol-2-yl-3,6-dihydro-2*H*-pyridine-1-carboxylate (5i) as a *trans*/*cis* mixture (38/62) (750 mg, 1.61 mmol, 61%).
MS *m*/*z* ([M+H]⁺) 466.

### Step 10: preparation of intermediates (2S,5R)-6-allyloxy-2-(hydroxymethyl)-3-oxazol-2-yl-1,6-diazabicyclo[3.2.1]oct-3-en-7-one (5i) and (2S,5S)-6-allyloxy-2-(hydroxymethyl)-3-oxazol-2-yl-1,6-diazabicyclo[3.2.1]oct-3-en-7-one (5k)

To a solution of *tert*-butyl (6*S*)-3-(allyloxyamino)-6-[[*tert*-butyl(dimethyl)silyl]oxymethyl]-5-oxazol-2-yl-3,6-dihydro-2H-pyridine-1-carboxylate (5i, *trans*/*cis* mixture 38/62) (750 mg, 1.61 mmol) in anhydrous DCM (8 mL) at 0°C under nitrogen were added TEA (0.45 mL, 2.88 mmol) and diphosgene (0.253 mL, 2.09 mmol). The mixture was stirred at 0°C for 30 min, diluted with DCM (9 mL) and washed with brine (10 mL). The organic layer was dried over Na₂SO₄ and concentrated *in vacuo.* The residue was dissolved in anhydrous dioxane (2 mL) and dropwise added to 4 M HCI solution in dioxane (9 mL). The mixture was stirred at rt for 1 h and concentrated *in vacuo.* The residue was dissolved in anhydrous DCM (16 mL) cooled at 0°C and TEA (0.89 mL, 6.45 mmol) was added. The mixture was stirred at rt for 15 min then washed with brine (9 mL). The organic layer was dried over Na₂SO₄ and concentrated *in vacuo.* The residue was purified by preparative TLCs on silica gel (EtOAc) to provide (2*S*,5*R*)-6-allyloxy-2-(hydroxymethyl)-3-thiazol-2-yl-1,6-diazabicyclo[3.2.1]oct-3-en-7-one (5j) (88 mg, 0.31 mmol, 20%) and (2S,5S)-6-allyloxy-2-(hydroxymethyl)-3-thiazol-2-yl-1,6-diazabicyclo[3.2.1]oct-3-en-7-one (5k) (201 mg, 0.72 mmol, 45%).
MS *m*/*z* ([M+H]⁺) 278.
*5j :*
1H NMR (400 MHz, CDCl₃) *δ* 3.33 (s, 2H), 3.51 (bs, 1H), 3.93 (dd, *J* = 11.4, 7.5 Hz, 1H), 4.03-4.08 (m, 1H), 4.22 (dd, *J* = 11.6, 4.7 Hz, 1H), 4.33-4.52 (m, 3H), 5.24-5.40 (m, 2H), 5.92-6.07 (m, 1H), 7.11 (s, 1H), 7.19 (d, *J* = 5.3 Hz, 1H), 7.58 (s, 1H).
*5k :*
¹H NMR (400 MHz, CDCl₃) *δ* 3.19 (dd, *J* = 14.1, 3.3 Hz, 1H), 3.90 (bs, 1H), 4.21 (t, *J* = 8.5 Hz, 1H), 4.32-4.47 (m, 3H), 4.86-4.98 (m, 1H), 5.04 (t, *J* = 9.0 Hz, 1H), 5.30 (dd, *J* = 10.4, 1.0 Hz, 1H), 5.35-5.45 (m, 1H), 5.55 (s, 1H), 5.99-6.09 (m, 1H), 6.86 (d, *J* = 5.9 Hz, 1H), 7.26 (d, *J* = 0.8 Hz, 1H), 7.76 (d, *J* = 0.8 Hz, 1H).

### Step 11: preparation of intermediate [(2S,5R)-6-allyloxy-7-oxo-3-oxazol-2-yl-1,6-diazabicyclo[3.2.1]oct-3-en-2-yl]methyl methanesulfonate (5l)

To a solution of compound (5j) (119 mg, 0.43 mmol) in anhydrous DCM (2 mL) at 0°C under atmosphere of nitrogen were successively added TEA (86 µL, 0.61 mmol) and MsCI (41µL, 0.507 mmol). The mixture was stirred at 0°C. H₂O (3 mL) was added. The layers were separated. The aqueous layer was extracted with DCM (2 x 3 mL). The combined organic layers were washed with a saturated solution of NaHCO₃ (5 mL), dried over Na₂SO₄ and concentrated *in vacuo* to provide [(2*S*,5*R*)-6-allyloxy-7-oxo-3-thiazol-2-yl-1,6-diazabicyclo[3.2.1]oct-3-en-2-yl]methyl methanesulfonate (51) (153 mg, 0.43 mmol, 100%) as a yellow oil which was used without further purification.
MS *m*/*z* ([M+H]⁺) 356.
1H NMR (400 MHz, CDCl₃) *δ*3.05 (s, 3H), 3.40 (ddd, *J* = 11.5, 2.7, 1.3 Hz, 1H), 3.48 (dd, *J* = 11.5, 0.8 Hz, 1H), 4.07 (dd, *J* = 5.2, 1.9 Hz, 1H), 4.35 - 4.50 (m, 2H), 4.66 (ddd, *J* = 6.4, 3.9, 1.8 Hz, 1H), 4.85-4.89 (m, 2H), 5.29-5.41 (m, 2H), 5.95-6.07 (m, 1H), 7.14 (d, *J* = 0.8 Hz, 1H), 7.27 (dt, *J* = 5.2, 1.4 Hz, 1H), 7.59 (d, *J* = 0.8 Hz, 1H).

### Step 12: preparation of intermediate tert-butyl N-[[(2S,5R)-6-allyloxy-7-oxo-3-oxazol-2-yl-1,6-diazabicyclo[3.2.1]oct-3-en-2-yl]methyl]carbamate (5m)

A mixture of compound (51) (153 mg, 0.43 mmol) and NaN₃ (137 mg, 2.13 mmol) in anhydrous DMF (1.6 mL) was stirred at 65°C for 20 h. The mixture was poured in H₂O (6 mL) and extracted with ethyl acetate (2 x 6 mL). The organic layer was washed with brine (6 mL), dried over Na₂SO₄ and concentrated *in vacuo.* The residue was dissolved in anhydrous THF (2 mL) and anhydrous toluene (2mL) and cooled at 0°C under nitrogen atmosphere. A trimethylphosphine solution 1M in THF (640 µL, 0.640 mmol) was dropwise added and the mixture was stirred at rt for 1 h. The mixture was cooled at 0°C and a solution of Boc-ON (157 mg, 0.640 mmol) in anhydrous THF (1 mL) was added. The mixture was stirred at rt for 3 h. H₂O (5 mL) was added and the layers separated. The aqueous layer was extracted with EtOAc (2 x 5 mL). The combined organic layers were washed with brine (5 mL), dried over Na₂SO₄ and concentrated *in vacuo.* The residue was purified by flash chromatography on silica gel (cyclohexane/EtOAc: 70/30 to 0/100) then by preparative TLC (cyclohexane/acetone 60/40) to provide *tert*-butyl *N*-[[(2*S*,5*R*)-6-allyloxy-7-oxo-3-oxazol-2-yl-1,6-diazabicyclo[3.2.1]oct-3-en-2-yl]methyl]carbamate (5m) (57 mg, 0.15 mmol, 35%).
MS *m*/*z* ([M+H]⁺) 377.
¹H NMR (400 MHz, CDCl₃) *δ* 1.43 (s, 9H), 3.19-3.37 (m, 3H), 3.97-4.10 (m, 2H), 4.32-4.49 (m, 3H), 5.15 (bs, 1H), 5.24-5.40 (m, 2H), 5.89-6.07 (m, 1H), 7.11 (s, 1H), 7.16 (d, *J* = 4.9, 1H), 7.56 (s, 1H).

### Step 13: preparation of intermediate sodium [(2S,5R)-2-[(tert-butoxycarbonylamino)methyl]-7-oxo-3-oxazol-2-yl-1,6-diazabicyclo[3.2.1]oct-3-en-6-yl] sulfate (5n)

To a solution of compound (5m) (57 mg, 0.15 mmol) in anhydrous DCM (1 mL) under nitrogen atmosphere were successively added AcOH (17 µL, 0.299 mmol) and Pd(PPh₃)₄ (86.2 mg, 0.074 mmol). The mixture was stirred at rt for 1 h then concentrated *in vacuo.* The residue was purified by flash chromatography on silica gel (DCM/acetone: 100/0 to 0/100) to provide a mixture of expected intermediate and triphenylphosphine oxide. The mixture was dissolved in pyridine (1 mL) and sulfur trioxide trimethylamine complex (213 mg, 1.53 mmol) was added. The mixture was stirred at rt overnight then concentrated *in vacuo.* DCM (3 mL) was added to the residue and the precipitate filtered. The filtrate was concentrated and the residue purified by flash chromatography on silica gel (DCM/acetone: 60/40 to 0/100). The fractions containing the expected intermediate were combined and concentrated *in vacuo.* The residue was dissolved in H₂O (1 mL) and converted after ion exchange (Dowex sodium form column, Dowex® 50WX8 hydrogen form stored with an aqueous solution of 2N NaOH and washed until neutral pH with H₂O) to sodium [(2*S*,5*R*)-2-[(*tert-*butoxycarbonylamino)methyl]-7-oxo-3-oxazol-2-yl-1,6-diazabicyclo[3.2.1]oct-3-en-6-yl] sulfate (5n) (28 mg, 0.064 mmol, 42%) as a white solid.
MS *m*/*z* ([M+H]⁺) 417.
MS *m*/*z* ([M-H]⁻) 415.
¹H NMR (400 MHz, D₂O) *δ* 1.43 (s, 9H), 3.41-3.58 (m, 2H), 3.64 (d, *J* = 11.9 Hz, 1H), 3.74 (dd, *J* = 14.8, 3.2 Hz, 1H), 4.40 (dd, *J* = 9.9, 3.7 Hz, 1H), 4.53 (dd, *J* = 5.3, 2.5 Hz, 1H), 7.22 (s, 1H), 7.27 (d, *J* = 5.2 Hz, 1H), 7.85 (s, 1H).

### Step 14: preparation of sodium and 2,2,2-trifluoroacetate [(2S,5R)-2-(azaniumylmethyl)-7-oxo-3-oxazol-2-yl-1,6-diazabicyclo[3.2.1]oct-3-en-6-yl]sulfate (Example 5)

A solution of compound (5n) (28 mg, 0.064 mmol) in anhydrous DCM (0.67 mL) was added to a mixture of DCM (0.77 mL) and TFA (0.77 mL) at 0°C. The mixture was stirred at 0°C for 30 min then concentrated *in vacuo.* The residue was co-evaporated three times with DCM (3 mL). The residue was dissolved in H₂O (2 mL) and lyophilized to provide sodium and 2,2,2-trifluoroacetate [(2*S*,5*R*)-2-(azaniumylmethyl)-7-oxo-3-oxazol-2-yl-1,6-diazabicyclo[3.2.1]oct-3-en-6-yl] sulfate (Example 2) (22.8 mg, 0.050 mmol, 78%) as an off white solid.
MS *m*/*z* ([M+H]⁺) 317.
MS *m*/*z* ([M-H]⁻) 315.
¹H NMR (300 MHz, D₂O) *δ*3.41 (dd, *J* = 13.5, 11.9 Hz, 1H), 3.57 (d, *J* = 1.6 Hz, 2H), 3.79 (dd, *J* = 13.8, 3.9 Hz, 1H), 4.54-4.66 (m, 2H), 7.24 (d, *J* = 0.9 Hz, 1H), 7.37 (dd, *J* = 5.3, 1.6 Hz, 1H), 7.86 (d, *J* = 0.9 Hz, 1H).

### Example 6: synthesis of sodium and 2,2,2-trifluoroacetate [trans-7-(1H-imidazol-3-ium--2-yl)-9-oxo-3-thia-5.8.10-triaza-tricyclo[6.2.1.0^{2,6}]undeca-2(6),4-dien-10-yl]sulfate (reference example)

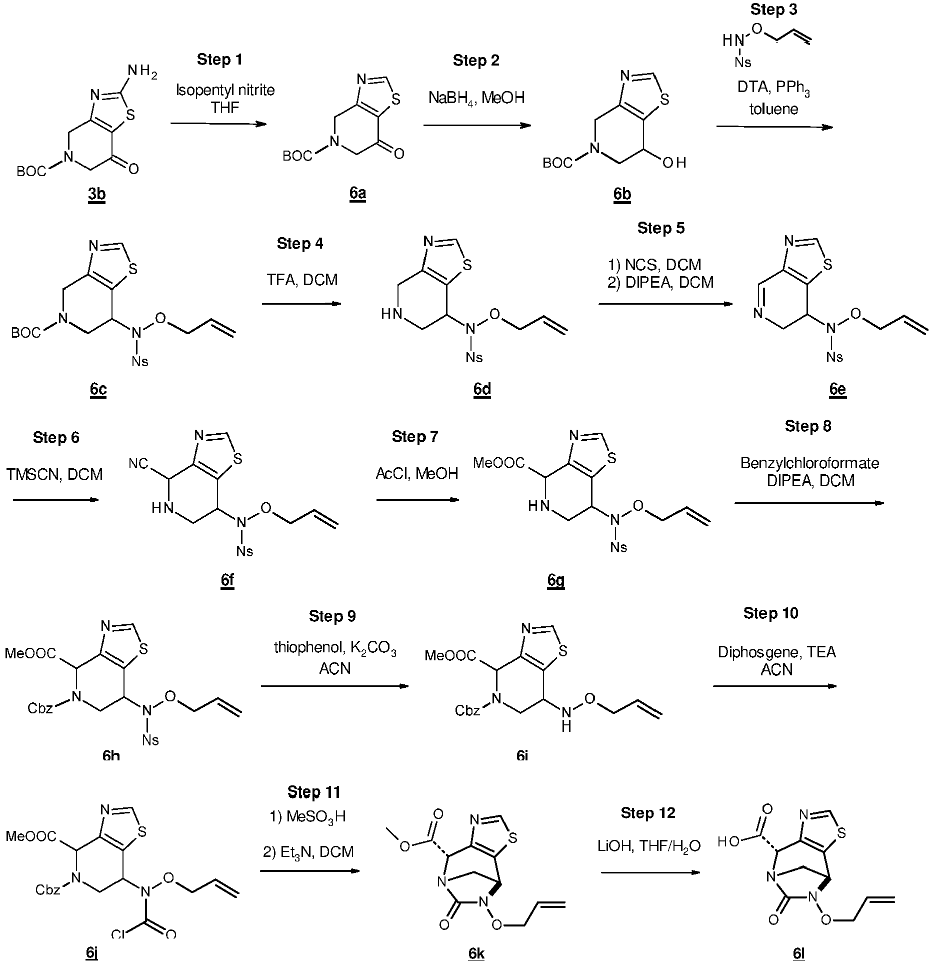

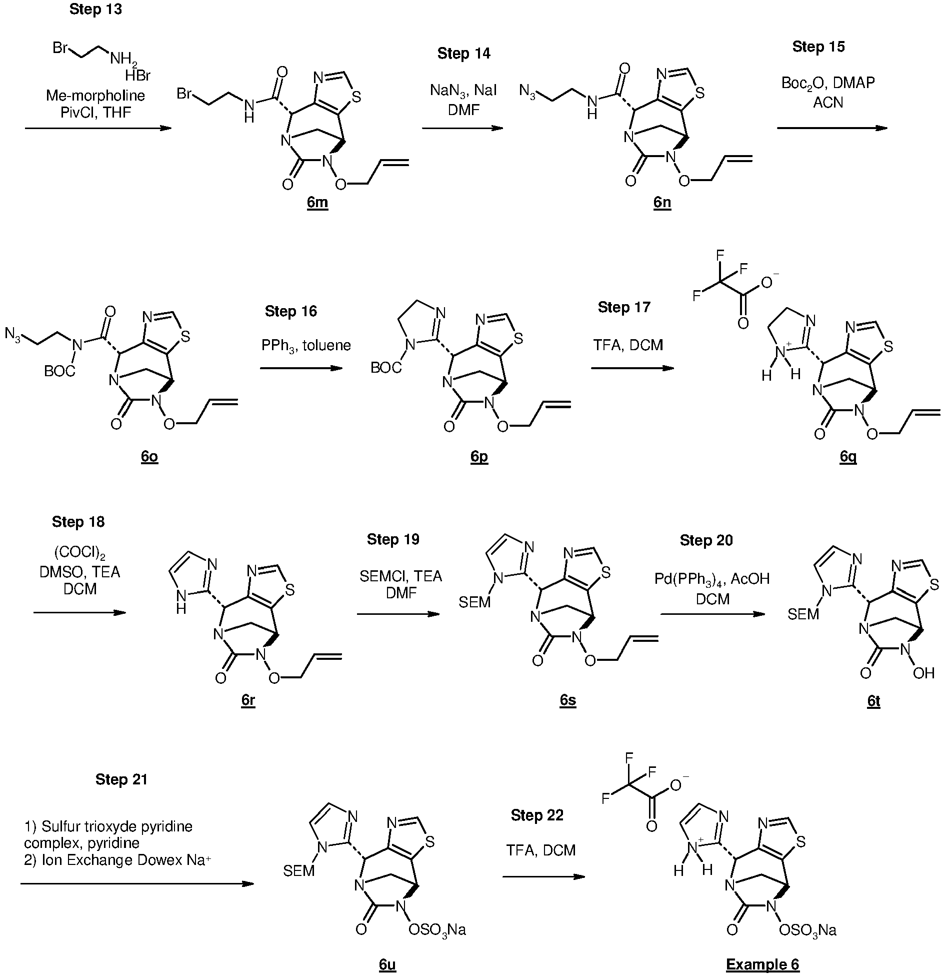

### Step 1: preparation of intermediate tert-butyl 7-oxo-4,6-dihydrothiazolo[4,5-c]pyridine-5-carboxylate (6a)

To a solution of *tert*-butyl 2-amino-7-oxo-4,6-dihydro-4*H*-thiazolo[4,5-c]pyridine-5-carboxylate (3b) (15.87 g, 58.9 mmol) in anhydrous THF (500 mL) under inert atmosphere was added isopentyl nitrite (40 mL, 294 mmol) under 4 days. The solvent was evaporated *in vacuo.* The residue was solubilized in DCM and washed with water then brine. The organic layer was dried over Na₂SO₄ and evaporated *in vacuo.* The red solid was purified by flash chromatography on silica gel (DCM) to give *tert*-butyl 7-oxo-4,6-dihydrothiazolo[4,5-c]pyridine-5-carboxylate (6a) (10.83 g, 42.58 mmol, 72%) as an off-white solid.
MS *m*/*z* ([M+H]) 255.
¹H NMR (400 MHz, CDCl₃): *δ*(ppm) 1.49 (s, 9H), 4.35 (s, 2H), 4.95 (s, 2H), 9.08 (s, 1H).

### Step 2: preparation of intermediate tert-butyl 7-hydroxy-6,7-dihydro-4H-thiazolof4,5-c]pyridine-5-carboxylate (6b)

To a solution of *tert*-butyl 7-oxo-4,6-dihydrothiazolo[4,5-c]pyridine-5-carboxylate (6a) (27.2 g, 107 mmol) in MeOH (1550 mL) under inert atmosphere at 0°C was portionwise added NaBH₄ (4.53 g, 120 mmol). The reaction mixture was stirred for 1 h at 0°C, then hydrolyzed with water and concentrated *in vacuo.* The residue was solubilized with DCM, washed with water and brine. The organic layer was dried over Na₂SO₄, evaporated *in vacuo* to give *tert*-butyl 7-hydroxy-6,7-dihydro-4*H*-thiazolo[4,5-c]pyridine-5-carboxylate (6b) (27.4 g, 106.9 mmol, quantitative yield) as a yellow solid.
MS *m*/*z* ([M+H]) 257.
¹H NMR (400 MHz, CDCl₃): *δ* (ppm) 1.49 (s, 9H), 3.68 (d, *J* = 12.4 Hz, 1H), 3.97 (dd, *J* = 13.6, 4.4 Hz, 1H), 4.50 (d, *J* = 17.2 Hz, 1H), 4.86 (d, *J* = 14.0 Hz, 1H), 4.97 (bs, 1H), 8.75 (s, 1H).

### Step 3: preparation of intermediate tert-butyl 7-[allyloxy-(2-nitrophenyl)sulfonyl-amino]-6,7-dihydro-4H-thiazolo[4.5-c]pyridine-5-carboxylate (6c)

To a solution of *tert*-butyl 7-hydroxy-6,7-dihydro-4*H*-thiazolo[4,5-c]pyridine-5-carboxylate (6b) (49.4 g, 193 mmol) in toluene (2140 mL) under inert atmosphere was added *N*-allyloxy-2-nitro-benzenesulfonamide (49.84 g, 193 mmol), PPh₃ (50.62 g, 193 mmol) and DTA (50.15 g, 218 mmol) portionwise. The reaction mixture was stirred for 24 h at rt. The precipitate was filtered and the filtrate was concentrated *in vacuo.* The residue was purified by flash chromatography on silica gel (petroleum ether/acetone 100/0 to 60/40) to give *tert*-butyl 7-[allyloxy-(2-nitrophenyl)sulfonyl-amino]-6,7-dihydro-4*H*-thiazolo[4,5-c]pyridine-5-carboxylate (6c) (80 g, 161.1 mmol, 83%) as a yellow oil.
MS *m*/*z* ([M+H]) 497.
¹H NMR (400 MHz, CDCl₃): *δ*(ppm) 1.44 and 1.45 (s, 9H), 3.50 (bs, 1H), 4.00-4.50 (m, 4H), 4.75-5.05 (m, 3H), 5.25-5.60 (m, 2H), 7.66 (d, *J* = 8.0 Hz, 1H), 7.75 (td, *J* = 7.7, 1.3 Hz, 1H), 7.84 (td, *J* = 7.8, 1.3 Hz, 1H), 8.13 (d, *J* = 8.0 Hz, 1H), 8.74 (s, 1H).

### Step 4: preparation of intermediate N-allyloxy-2-nitro-N-(4,5,6,7-tetrahydrothiazolo[4,5-c]pyridin-7-yl)benzenesulfonamide (6d)

To a solution of *tert*-butyl 7-[allyloxy-(2-nitrophenyl)sulfonyl-amino]-6,7-dihydro-4*H-*thiazolo[4,5-c]pyridine-5-carboxylate (6c) (20 g, 40.28 mmol) in anhydrous DCM (37 mL) under inert atmosphere at 0°C was dropwise added TFA (37 mL, 483 mmol). After stirring for 2 h at rt, the reaction mixture was cooled at 0°C and neutralized with ammonium hydroxide solution 28% until pH 8. The mixture was diluted with water and extracted with DCM. The organic layer was dried over Na₂SO₄, concentrated *in vacuo* to give *N*-allyloxy-2-nitro-*N-*(4,5,6,7-tetrahydrothiazolo[4,5-c]pyridin-7-yl)benzenesulfonamide (6d) (14.68 g, 37.02 mmol, 92%) as a yellow solid.
MS *m*/*z* ([M+H]) 397.
¹H NMR (400 MHz, CDCl₃): *δ*(ppm) 1.73 (bs, 1H), 3.03 (dd, *J* = 4.9/14.7 Hz, 1H), 3.19 (bs, 1H), 3.88 (d, *J* = 17.1 Hz, 1H), 4.02 (bs, 1H), 4.10 (d, *J* = 17.1 Hz, 1H), 4.37 (dd, *J* = 11.4/6.2 Hz, 1H), 5.02-5.13 (m, 2H), 5.14-5.20 (m, 1H), 5.47-5.59 (m, 1H), 7.65 (dd, *J* = 7.9/1.3 Hz, 1H), 7.75 (td, *J* = 7.7/1.4 Hz, 1H), 7.84 (td, *J* = 7.7/1.5 Hz, 1H), 8.14 (dd, *J* = 7.7/1.5 Hz, 1H), 8,77 (s, 1H).

### Step 5: preparation of intermediate N-allyloxy-N-(6,7-dihydrothiazolo[4,5-c]pyridin-7-yl)-2-nitro-benzenesulfonamide (6e)

To a solution of *N*-allyloxy-2-nitro-*N*-(4,5,6,7-tetrahydrothiazolo[4,5-c]pyridin-7-yl)benzenesulfonamide (6d) (14.68 g, 37.03 mmol) in anhydrous DCM (225 mL) under inert atmosphere at 0°C was dropwise added a solution of *N*-chlorosuccinimide (6.43 g, 48,14 mmol) in DCM (225 mL). After stirring for 4 h at 0°C, DIPEA (24 mL, 137,82 mmol) was added and the reaction mixture was stirred for 18 h at rt. The reaction mixture was cooled at 0°C, washed with a tartaric acid solution (2eq/DIPEA) and extracted with DCM. The organic layer was dried over Na₂SO₄ and concentrated *in vacuo* to give *N*-allyloxy-*N*-(6,7-dihydrothiazolo[4,5-c]pyridin-7-yl)-2-nitro-benzenesulfonamide (6e) (14.60 g, 37.03 mmol, quantitative yield) as an ochre solid.
MS *m*/*z* ([M+H]) 395.

### Step 6: preparation of intermediate N-allyloxy-N-(4-cyano-4,5,6,7-tetrahydrothiazolo[4,5-c]pyridin-7-yl)-2-nitro-benzenesulfonamide (6f)

To a solution of *N*-allyloxy-*N*-(6,7-dihydrothiazolo[4,5-c]pyridin-7-yl)-2-nitrobenzenesulfonamide (6e) (14.60 g, 37.01 mmol) in anhydrous DCM (260 mL) under inert atmosphere at 0°C was added TMSCN (47 mL, 377 mmol). The reaction mixture was stirred for 9 days at rt. The solvent was evaporated and the residue was purified by flash chromatography on silica gel (DCM/acetone 9/1) to give *N*-allyloxy-*N*-(4-cyano-4,5,6,7-tetrahydrothiazolo[4,5-c]pyridin-7-yl)-2-nitro-benzenesulfonamide (6f) (12.67 g, 30.06 mmol, 81%) as a beige solid.
MS *m*/*z* ([M+H]) 422.
¹H NMR (400 MHz, CDCl₃): *δ*(ppm) 2.33 (bs, 1H), 3.38 (bs, 2H), 4.04 (bs, 1H), 4.37 (dd, *J* = 6.2/11.4 Hz, 1H), 4.97-5.24 (m, 4H), 5.43-5.55 (m, 1H), 7.68 (dd, *J* = 8.0/0.8 Hz, 1H), 7.76 (td, *J* = 7.8/1.3 Hz, 1H), 7.86 (td, *J* = 7.8/1.3 Hz, 1H), 8.12 (dd, *J* = 8.0/0.8 Hz, 1H), 8.83 (s, 1H).

### Step 7: preparation of intermediate methyl 7-[allyloxy-(2-nitrophenyl)sulfonyl-amino]-4,5,6,7-tetrahydrothiazolo[4,5-c]pyridine-4-carboxylate (6g)

In MeOH (72 mL) at 0°C under inert atmosphere was added AcCI (30 mL, 422 mmol). After stirring for 2 h at 0°C then for 30 min at rt, a solution of *N*-allyloxy-*N*-(4-cyano-4,5,6,7-tetrahydrothiazolo[4,5-c]pyridin-7-yl)-2-nitro-benzenesulfonamide (6f) in MeOH (36 mL) was added. The mixture was heated at 50°C for 18 h. The solvent was evaporated. The residue was extracted with DCM and washed with a saturated solution of NaHCO₃. The organic layer was dried over Na₂SO₄ and concentrated *in vacuo.* The brown oil was purified by flash chromatography on silica gel (DCM/acetone 100/0 to 80/20) to give methyl 7-[allyloxy-(2-nitrophenyl)sulfonyl-amino]-4,5,6,7-tetrahydrothiazolo[4,5-c]pyridine-4-carboxylate (6g) (10.77 g, 23.70 mmol, 78%) as a yellow solid.
MS *m*/*z* ([M+H]) 455.
¹H NMR (400 MHz, CDCl₃): *δ*(ppm) 3.20-3.57 (m, 2H), 3.48 3.54 3.79 and 3.88 (s, 3H), 4.05-4.16 (m, 1H), 4.34 and 4.43 (dd, *J* = 11.1/6.6 Hz, 1H), 4.90 and 5.00 (s, 1H), 5.02-5.21 (m, 2H), 5.27-5.41 (m, 1H), 5.47-5.69 (m, 1H), 7.67 (t, *J* = 7.7 Hz, 1H), 7.74-7.81 (m, 1H), 7.82-7.89 (m, 1H), 8.13-8.19 (m, 1H), 8.83 and 8.84 (s, 1H).

### Step 8: preparation of intermediate O5-benzyl O4-methyl7-[allyloxy-(2-nitrophenyl)sulfonyl-amino]-6,7-dihydro-4H-thiazolo[4.5-c]pyridine-4,5-dicarboxylate (6h)

To a solution of methyl 7-[allyloxy-(2-nitrophenyl)sulfonyl-amino]-4,5,6,7-tetrahydrothiazolo[4,5-c]pyridine-4-carboxylate (6g) (10.77 g, 23.70 mmol) in anhydrous DCM (230 mL) under inert atmosphere at -78°C were successively added DIPEA (8.3 mL, 47,4 mmol) and benzyl chloroformate (4.06 mL, 28,44 mmol). The mixture was then stirred at rt for 2 h 30. Water was added. The aqueous layer was extracted with DCM. The organic layers were combined, washed with a saturated solution of sodium bicarbonate, dried over Na₂SO₄ and concentrated *in vacuo.* The residue was purified by flash chromatography on silica gel (DCM/acetone 100/0 to 90/10) to give *O*5-benzyl *O*4-methyl 7-[allyloxy-(2-nitrophenyl)sulfonyl-amino]-6,7-dihydro-4*H*-thiazolo[4,5-c]pyridine-4,5-dicarboxylate (6h) (13.08 g, 22.22 mmol, 93%) as a yellow solid.
MS *m*/*z* ([M+H]) 589.

### Step 9: preparation of intermediate O5-benzyl O4-methyl 7-(allyloxyamino)-6,7-dihydro-4H-thiazolo[4,5-c]pyridine-4,5-dicarboxylate (6i)

To a solution of *O*5-benzyl *O*4-methyl 7-[allyloxy-(2-nitrophenyl)sulfonyl-amino]-6,7-dihydro-4*H*-thiazolo[4,5-c]pyridine-4,5-dicarboxylate (6h) (13.08 g, 22.22 mmol) in ACN (145 mL) under inert atmosphere at 0°C were successively added PhSH (11.4 mL, 111 mmol) and potassium carbonate (23 g, 167 mmol). The mixture was stirred at rt for 18 h then filtered on Celite® and rinsed with DCM. The filtrate was concentrated *in vacuo.* The residue was purified by flash chromatography on silica gel (heptane/EtOAc 100/0 to 20/80) to give *O*5-benzyl *O*4-methyl 7-(allyloxyamino)-6,7-dihydro-4*H*-thiazolo[4,5-c]pyridine-4,5-dicarboxylate (6i) (7.87 g, 19.51 mmol, 87%) as an ochre oil.
MS *m*/*z* ([M+H]) 404.

### Step 10: preparation of intermediate O5-benzyl O4-methyl 7-[allyloxy(chlorocarbony)amino]-6,7-dihydro-4H-thiazolo[4,5-c]pyridine-4,5-dicarboxylate (6i)

To a solution of *O*5-benzyl *O*4-methyl 7-(allyloxyamino)-6,7-dihydro-4*H*-thiazolo[4,5-c]pyridine-4,5-dicarboxylate (6i) (7.87 g, 19.51 mmol) in ACN (365 mL) under inert atmosphere at -10°C was added TEA (8.16 mL, 58,53 mmol). A solution of diphosgene (3.06 mL, 25.3 mmol) diluted in ACN (45 mL) was dropwise added. After stirring for 10 min at 0°C, the solvent was evaporated and the residue was purified by flash chromatography on silica gel (heptane/EtOAc 100/0 to 50/50) to give *O*5-benzyl *O*4-methyl 7-[allyloxy(chlorocarbonyl)amino]-6,7-dihydro-4*H*-thiazolo[4,5-c]pyridine-4,5-dicarboxylate (6j) (8.44 g, 18.11 mmol, 93%) as a yellow oil.
MS *m*/*z* ([M+H]) 466/468.

### Step 11: preparation of intermediate methyl trans-10-allyloxy-9-oxo-3-thia-5.8.10-triazatricyclo[6.2.1.0^{2,6}]undeca-2(6),4-diene-7-carboxylate (6k)

To a solution of *O*5-benzyl *O*4-methyl 7-[allyloxy(chlorocarbonyl)amino]-6,7-dihydro-4*H-*thiazolo[4,5-c]pyridine-4,5-dicarboxylate (6j) (7.4 g, 15.88 mmol) in anhydrous DCM (100 mL) under inert atmosphere at 0°C was dropwise added methanesulfonic acid (25.8 mL, 397 mmol). After stirring for 2 h at rt, the reaction mixture was poured at -78°C in a solution of TEA (110 mL, 794 mmol) in DCM (74 mL) and stirred for 30 min. The mixture was washed with water and extracted with DCM. The organic layer was dried over Na₂SO₄ and concentrated *in vacuo.* The residue was purified by flash chromatography on silica gel (DCM/EtOAc 100/0 to 85/15) to give methyl *trans-*10-allyloxy-9-oxo-3-thia-5,8,10-triazatricyclo[6.2.1.0^{2,6}]undeca-2(6),4-diene-7-carboxylate (6k) (4.50 g, 15.24 mmol, 96%) as an off-white solid.
MS *m*/*z* ([M+H]) 296.
¹H NMR (300 MHz, CDCl₃): *δ*(ppm) 3.65 (dd, *J* = 11.6/2.8 Hz, 1H), 3.76 (dd, *J* = 11.6/0.8 Hz, 1H), 3.86 (s, 3H), 4.36-4.51 (m, 2H), 4.67 (dd, *J* = 2.8/0.8 Hz, 1H), 5.28-5.41 (m, 3H), 5.92-6.07 (m, 1H), 8.72 (s, 1H).

### Step 12: preparation of intermediate trans-10-allyloxy-9-oxo-3-thia-5,8,10-triazatricyclo[6.2.1.0^{2,6}]undeca-2(6),4-diene-7-carboxylic acid (61)

To a solution of methyl *trans*-10-allyloxy-9-oxo-3-thia-5,8,10-triaza-tricyclo[6.2.1.0^{2,6}]undeca-2(6),4-diene-7-carboxylate (6k) (1.6 g, 5.42 mmol) in a mixture of THF (30 mL) and water (19 mL) at 0°C was dropwise added a solution of lithium hydroxide 1N (5.4 mL, 5.42 mmol). After stirring for 24 h, the mixture was diluted with DCM and acidified with a solution of HCI 2N (6 mL) at 0°C. The product was extracted with DCM and the organic layer was dried over Na₂SO₄ before being concentrated *in vacuo.* The crude was triturated with Et₂O to give *trans-*10-allyloxy-9-oxo-3-thia-5,8,10-triaza-tricyclo[6.2.1.0^{2,6}]undeca-2(6),4-diene-7-carboxylic acid (61) (1.52 g, 5.42 mmol, quantitative yield) as a white solid.
MS *m*/*z* ([M+H]) 282.
¹H NMR (400 MHz, CDCl₃): *δ*(ppm) 3.59 (d, *J* = 11.7 Hz, 1H), 3.72 (dd, *J* = 11.7/2.8 Hz, 1H), 4.18 (bs, 1H), 4.38-4.50 (m, 2H), 4.67 (d, *J* = 2.6 Hz, 1H), 5.30-5.40 (m, 2H), 5.41 (s, 1H), 5.94-6.05 (m, 1H), 8.89 (s,1H).

### Step 13: preparation of intermediate trans-10-allyloxy-N-(2-bromoethyl)-9-oxo-3-thia-5,8,10-triaza-tricyclo[6.2.1.0^{2,6}]undeca-2(6),4-diene-7-carboxamide (6m)

To a solution of *trans*-10-allyloxy-9-oxo-3-thia-5,8,10-triaza-tricyclo[6.2.1.0^{2,6}]undeca-2(6),4-diene-7-carboxylic acid (61) (1.4 g, 4.98 mmol) in anhydrous THF (50 mL) under inert atmosphere at -50°C was added 4-Methylmorpholine (3.28 mL, 29.86 mmol). After stirring for 15 min trimethylacetyl chloride (0.920 mL, 7.47 mmol) was added and the mixture was maintained at -50°C for 1h. 2-Bromoethylamine hydrobromide (3.06 g, 14.94 mmol) was portiowise added and temperature was raised at -30°C over 2 h. The precipitate was filtered and washed with EtOAc. The filtrate was concentrated to give *trans-*10-allyloxy-*N*-(2-bromoethyl)-9-oxo-3-thia-5,8,10-triaza-tricyclo[6.2.1.0^{2,6}]undeca-2(6),4-diene-7-carboxamide (6m) (1.93 g, 4.98 mmol, quantitative yield) as a yellow oil which was used without further purification.
MS *m*/*z* ([M+H]) 387/389.
¹H NMR (400 MHz, CDCl₃): *δ*(ppm) 3.39 (d, *J* = 11.5 Hz, 1H), 3.43- 3.54 (m, 2H), 3.61- 3.73 (m, 2H), 3.75-3.85 (m, 1H), 4.38-4.49 (m, 2H), 4.66 (d, *J* = 2.4 Hz, 1H), 5.25 (s, 1H), 5.29-5.40 (m, 2H), 5.94-6.05 (m, 1H), 7.43 (t, *J* = 6.0 Hz, 1H), 8.75 (s, 1H).

### Step 14: preparation of intermediate trans-10-allyloxy-N-(2-azidoethyl)-9-oxo-3-thia-5,8,10-triaza-tricyclo[6.2.1.0^{2,6}]undeca-2(6),4-diene-7-carboxamide (6n)

To a solution of *trans-*10-Allyloxy-*N*-(2-bromoethyl)-9-oxo-3-thia-5,8,10-triazatricyclo[6.2.1.0^{2,6}]undeca-2(6),4-diene-7-carboxamide (6m) (1.93 g, 4.98 mmol) in anhydrous DMF (50 mL) under inert atmosphere were successively added sodium iodide (1.49 g, 9,96 mmol) and sodium azide (0.65 g, 9.96 mmol). The reaction mixture was stirred for 1 h at 40°C then at rt overnight. The solvent was evaporated. DCM was added to the residue. The solids were filtered off and rinsed with DCM. The filtrate was concentrated *in vacuo.* The residue was purified by flash chromatography on silica gel (DCM/acetone 100/0 to 80/20) then triturated in Et₂O to give *trans-*10-allyloxy-*N*-(2-azidoethyl)-9-oxo-3-thia-5,8,10-triazatricyclo[6.2.1.0^{2,6}]undeca-2(6),4-diene-7-carboxamide (6n) (1.15 g, 3.29 mmol, 66%) as a white solid.
MS *m*/*z* ([M+H]) 350.
¹H NMR (400 MHz, CDCl₃): *δ* (ppm) 3.39 (d, *J* = 11.5 Hz, 1H), 3.42-3.54 (m, 4H), 3.69 (dd, *J* = 11.5, 2.9 Hz, 1H), 4.37-4.48 (m, 2H), 4.65 (d, *J* = 2.7 Hz, 1H), 5.24 (s, 1H), 5.29-5.38 (m, 2H), 5.93-6.03 (m, 1H), 7.36 (bs, 1H), 8.74 (s, 1H).

### Step 15: preparation of intermediate tert-butyl N-(2-azidoethyl)-N-[trans-10-allyloxy-9-oxo-3-thia-5,8,10-triaza-tricyclo[6.2.1.0^{2,6}]undeca-2(6),4-diene-7-carbonyl)]carbamate (6o)

To a solution of *trans*-10-Allyloxy-*N*-(2-azidoethyl)-9-oxo-3-thia-5,8,10-triazatricyclo[6.2.1.0^{2,6}]undeca-2(6),4-diene-7-carboxamide (6n) (1.15 g, 3.29 mmol) in anhydrous ACN (33 mL) under inert atmosphere at rt were successively added Boc₂O (1.08 g, 4.94 mmol) and 4-dimethylaminopyridine (0.04 mg, 0.33 mmol). The mixture was stirred for 3 days. The solvent was evaporated *in vacuo* and the residue was purified by flash chromatography on silica gel (DCM/Et₂O 100/0 to 90/10) to give *tert*-butyl *N*-(2-azidoethyl)-*N-*[*trans*-10-allyloxy-9-oxo-3-thia-5,8,10-triaza-tricyclo[6.2.1.0^{2,6}]undeca-2(6),4-diene-7-carbonyl)]carbamate (6o) (1.10 g, 2.45 mmol, 74%) as a yellow solid.
MS *m*/*z* ([M+H]) 450
¹H NMR (400 MHz, CDCl₃): δ (ppm) 1.62 (s, 9H), 3.47 (t, *J* = 6.1 Hz, 2H), 3.52 (d, *J* = 11.4 Hz, 1H), 3.59 (dd, *J* = 11.5, 2.8 Hz, 1H), 3.89 (dt, *J* = 13.8, 5.8 Hz, 1H), 4.00 (dt, *J* = 14.0, 6.4 Hz, 1H), 4.37-4.50 (m, 2H), 4.64 (d, *J* = 2.7 Hz, 1H), 5.28-5.40 (m, 2H), 5.94-6.06 (m, 1H), 6.44 (s, 1H), 8.70 (s, 1H).

### Step 16: preparation of intermediate tert-butyl 2-(trans-10-allyloxy-9-oxo-3-thia-5,8,10-triazatricyclo[6.2.1.0^{2,6}]undeca-2(6),4-dien-7-yl)-4,5-dihydroimidazole-1-carboxylate (6p)

To a solution of *tert*-butyl *N*-(2-azidoethyl)-*N*-[*trans*-10-allyloxy-9-oxo-3-thia-5,8,10-triazatricyclo[6.2.1.0^{2,6}]undeca-2(6),4-diene-7-carbonyl)]carbamate (6o) (1.10 g, 2.45 mmol) in anhydrous toluene (41 mL) under inert atmosphere was added PPh₃ (0.64 g, 2.45 mmol). The mixture was stirred for 16 h at rt,, deposited on silica and purified by flash chromatography on silica gel (Heptane/Acetone 100/0 to 0/100) to give *tert*-butyl 2-(*trans*-10-allyloxy-9-oxo-3-thia-5,8,10-triaza-tricyclo[6.2.1.0^{2,6}]undeca-2(6),4-dien-7-yl)-4,5-dihydroimidazole-1-carboxylate (6p) (0.91 g, 2.24 mmol, 91%) as a white solid.
MS *m*/*z* ([M+H]) 406.
¹H NMR (400 MHz, CDCl₃): *δ* (ppm) 1.59 (s, 9H), 3.45 (dd, *J* = 11.2/2.9 Hz, 1H), 3.72-3.93 (m, 5H), 4.37-4.49 (m, 2H), 4.62 (d, *J=* 2.3 Hz, 1H), 5.28-5.38 (m, 2H), 5.94-6.06 (m, 1H), 6.34 (s, 1H), 8.70 (s, 1H).

### Step 17: preparation of intermediate trans-10-allyloxy-7-(4,5-dihydro-1H-imidazol-3-ium-2-yl)-3-thia-5,8,10-triaza-tricyclo[6.2.1.0^{2,6}]undeca-2(6),4-dien-9-one trifluoroacetate (6q)

To a solution of *tert-*butyl 2-(*trans*-10-allyloxy-9-oxo-3-thia-5,8,10-triaza-tricyclo[6.2.1.0^{2,6}]undeca-2(6),4-dien-7-yl)-4,5-dihydroimidazole-1-carboxylate (6p) (0.24 g, 0.59 mmol) in anhydrous DCM (4.3 mL) under inert atmosphere at 0°C was dropwise added TFA (4.3 mL, 56.15 mmol). The mixture was stirred at rt for 16 h then concentrated *in vacuo.* The residue was co-evaporated with toluene to give *trans*-10-allyloxy-7-(4,5-dihydro-1H-imidazol-3-ium-2-yl)-3-thia-5,8,10-triaza-tricyclo[6.2.1.0^{2,6}]undeca-2(6),4-dien-9-one trifluoroacetate (6q) (0.247 g, 0.59 mmol, quantitative yield).
MS *m*/*z* ([M+H]) 306.

### Step 18: preparation of trans-10-allyloxy-7-(1H-imidazol-2-yl)-3-thia-5,8,10-triaza-tricyclo[6.2.1.0^{2,6}]undeca-2(6),4-dien-9-one (6r)

To a solution of oxalyl chloride (0.1 mL, 1.18 mmol) in DCM (9 mL) under inert atmosphere at -65°C was dropwise added dimethyl sulfoxide (0.168 mL, 2.36 mmol). The mixture was stirred for 30 min then a solution of *trans-*10-allyloxy-7-(4,5-dihydro-1*H*-imidazol-3-ium-2-yl)-3-thia-5,8,10-triaza-tricyclo[6.2.1.0^{2,6}]undeca-2(6),4-dien-9-one trifluoroacetate (6q) (0.247 g, 0.59 mmol) in anhydrous DCM (9 mL) was dropwise added. After 5 min at -65°C, TEA (0.821 mL, 5.89 mmol) was added and the temperature was raised to -15°C over 3 h. The reaction mixture was quenched with water and the product was extracted with DCM. The brown oil was purified by flash chromatography on silica gel (heptane/acetone 100/0 to 0/100) to give *trans*-10-allyloxy-7-(1*H*-imidazol-2-yl)-3-thia-5,8,10-triaza-tricyclo[6.2.1.0^{2,6}]undeca-2(6),4-dien-9-one (6r) (0.059 g, 0.19 mmol, 32%) as an off-white solid.
MS *m*/*z* ([M+H]) 304.
¹H NMR (400 MHz, CDCl₃): δ (ppm) 3.49 (dd, *J* = 11.4/2.9 Hz, 1H), 3.76 (d, *J =* 11.4 Hz, 1H), 4.43 (ddt, *J* = 12.3/6.6/1.1 Hz, 1H), 4.49 (ddt, *J* = 12.4/6.0/1.2 Hz, 1H), 4.69 (d, *J* = 2.5 Hz, 1H), 5.30-5.41 (m, 2H), 5.86 (s, 1H), 5.95-6.07 (m, 1H), 7.04 (s, 2H), 8.74 (s, 1H), 10.47 (bs, 1H).

### Step 19: preparation of trans-10-allyloxy-7-[1-(2-trimethylsilylethoxymethyl)-1H-imidazol-2-yl]-3-thia-5,8,10-triaza-tricyclo[6.2.1.0^{2,6}]undeca-2(6),4-dien-9-one (6s)

To a solution of *trans-*10-allyloxy-7-(1*H*-imidazol-2-yl)-3-thia-5,8,10-triaza-tricyclo[6.2.1.0^{2,6}]undeca-2(6),4-dien-9-one (6r) (0.059 g, 0.194 mmol) in anhydrous DMF (2 mL) under inert atmosphere were successively added (2-(chloromethoxy)ethyl)trimethylsilane (0.103 mL, 0.583 mmol) and TEA (0.081 mL, 0.58 mmol). After stirring for 16 h at rt, the mixture was concentrated *in vacuo.* The residue was purified by flash chromatography on silica gel (DCM/Acetone 80/20 to 0/100) to give *trans-*10-allyloxy-7-[1-(2-trimethylsilylethoxymethyl)-1*H*-imidazol-2-yl]-3-thia-5,8,10-triaza-tricyclo[6.2.1.0^{2,6}]undeca-2(6),4-dien-9-one (6s) (0.043 g, 0.1 mmol, 51%) as a yellow oil.
MS *m*/*z* ([M+H]) 434.
¹H NMR (300 MHz, CDCl₃): *δ* (ppm) 0.00 (s, 9H), 0.87-1.08 (m, 2H), 3.35 (dd, *J* = 11.3/2.9 Hz, 1H), 3.60-3.68 (m, 2H), 3.78 (d, *J=* 11.6 Hz, 1H), 4.36-4.52 (m, 2H), 4.68 (d, *J=* 2.3 Hz, 1H), 5.27-5.43 (m, 3H), 5.66 (d, *J =* 10.7 Hz, 1H), 5.95 (s, 1H), 5.96-6.11 (m, 1H), 6.99 (d, *J* = 1.3 Hz, 1H), 7.11 (d*, J* = 1.3 Hz, 1H), 8.73 (s, 1H).

### Step 20: preparation of intermediate trans-10-Hydroxy-7-[1-(2-trimethylsilylethoxymethyl)-1H-imidazol-2-yl]-3-thia-5,8,10-triaza-tricyclo[6.2.1.0^{2,6}]undeca-2(6),4-dien-9-one(6t)

To a solution of *trans*-10-allyloxy-7-[1-(2-trimethylsilylethoxymethyl)-1*H*-imidazol-2-yl]-3-thia-5,8,10-triaza-tricyclo[6.2.1,0^{2,6}]undeca-2(6),4-dien-9-one (6s) (0.043 g, 0.1 mmol) in anhydrous DCM (2 mL) under inert atmosphere were successively added AcOH (0.011 mL, 0.20 mmol) and Pd(PPh₃)₄ (0.057 g, 0.05 mmol). After stirring for 20 min at rt, the mixture was concentrated *in vacuo.* The residue was purified by flash chromatography on silica gel (DCM/Acetone 80/20 to 0/100) to give *trans*-10-Hydroxy-7-[1-(2-trimethylsilylethoxymethyl)-1*H*-imidazol-2-yl]-3-thia-5,8,10-triaza-tricyclo[6.2.1.0^{2,6}]undeca-2(6),4-dien-9-one (6t) (0.018 g, 0.046 mmol, 46%).
MS *m*/*z* ([M+H]) 394.
¹H NMR (300 MHz, CDCl₃): *δ* (ppm) 0.00 (s, 9H), 0.89-1.07 (m, 2H), 3.30 (dd, *J* = 11.3/2.9 Hz, 1H), 3.60-3.71 (m, 3H), 4.52 (d, *J =* 2.7 Hz, 1H), 5.40 (d, *J* = 10.7 Hz, 1H), 5.68 (d, *J* = 10.7 Hz, 1H), 5.93 (s, 1H), 6.96 (d, *J=* 1.3 Hz, 1H), 7.12 (d, *J=* 1.3 Hz, 1H), 8.72 (s, 1H).

### Step 21: preparation of sodium {trans-9-oxo-7-[1-(2-trimethylsilylethoxymethyl)-1H-imidazol-2-yl]-3-thia-5,8,10-triaza-tricyclo[6.2.1.0^{2,6}]undeca-2(6),4-dien-10-yl}sulfate (6u)

To a solution of *trans*-10-Hydroxy-7-[1-(2-trimethylsilylethoxymethyl)-1*H*-imidazol-2-yl]-3-thia-5,8,10-triaza-tricyclo[6.2.1.0^{2,6}]undeca-2(6),4-dien-9-one (6t) (0.018 g, 0.0046 mmol) in anhydrous pyridine (0.5 mL) under inert atmosphere was added sulfur trioxide pyridine complex (0.03 g, 0.185 mmol). After stirring for 16 h, the heterogeneous mixture was concentrated *in vacuo.* DCM was added to the residue and the solids were filtered. The crude residue was applied on a Dowex sodium form column (Dowex® 50WX8 hydrogen form stored with an aqueous solution of 2N NaOH and washed until neutral pH with water). The fractions containing the desired compound were combined, freezed and lyophilized. The salt was triturated with DCM and filtered on a PTFE filter. The filtrate was eliminated and the solid was solubilized with water MilliQ®. The aqueous layer was freezed and lyophilized to give sodium {*trans*-9-oxo-7-[1-(2-trimethylsilylethoxymethyl)-1*H*-imidazol-2-yl]-3-thia-5,8,10-triaza-tricyclo[6.2.1.0^{2,6}]undeca-2(6),4-dien-10-yl}sulfate (6u) (0.01 g, 0.020 mmol, 43%) as a white powder.
MS *m*/*z* ([M+H]) 474.
¹H NMR (400 MHz, D₂O): *δ*(ppm) -0.01 (s, 9H), 0.90-1.08 (m, 2H), 3.58-3.67 (m, 2H), 3.71-3.81 (m, 2H), 5.28 (d, *J=* 1.5 Hz, 1H), 5.59 (d, *J=* 11.1 Hz, 1H), 5.73 (d, *J=* 11.1 Hz, 1H), 6.07 (s, 1H), 6.97 (d, *J* = 1.3 Hz, 1H), 7.41 (d, *J* = 1.3 Hz, 1H), 9.00 (s, 1H).

### Step 22: preparation of sodium and 2,2,2-trifluoroacetate [trans-7-(1H-imidazol-3-ium--2-yl)-9-oxo-3-thia-5,8,1 0-triaza-tricyclo[6.2.1.0^{2,6}]undeca-2(6),4-dien-10-yl]sulfate (Example 6)

Sodium {*trans*-9-oxo-7-[1-(2-trimethylsilylethoxymethyl)-1*H*-imidazol-2-yl]-3-thia-5,8,10-triaza-tricyclo[6.2.1.0^{2,6}]undeca-2(6),4-dien-10-yl}sulfate (6u) (0.01 g, 0.020 mmol) was dissolved in TFA (0.5 mL, 6.53 mmol) at 0°C under inert atmosphere. After stirring for 2 h at rt, the mixture was concentrated *in vacuo.* The residue was triturated several times in Et₂O and the filtrate was filtered on a PTFE filter. The solid was solubilized with water MilliQ®. The aqueous layer was freezed and lyophilized to give sodium and 2,2,2-trifluoroacetate [*trans*-7-(1*H*-imidazol-3-ium--2-yl)-9-oxo-3-thia-5,8,10-triaza-tricyclo[6.2.1.0^{2,6}]undeca-2(6),4-dien-10-yl]sulfate (Example 6) (0.06 g, 0.012 mmol, 62%) as a white solid.
MS *m*/*z* ([M+H]) 344.
¹H NMR (400 MHz, D₂O): *δ*(ppm) 3.41 (d, *J=* 12.2 Hz, 1H), 3.82 (dd, *J* = 12.2/2.9 Hz, 1H), 5.32 (d, *J=* 2.7 Hz, 1H), 6.25 (s, 1H), 7.50 (s, 2H), 9.11 (s, 1H).

### Example 7: synthesis of lithium difluoro-(3-oxazol-3-yl-7-oxo-1,6-diazabicyclo[3.2.1]oct-3-en-6-yloxy]-acetate

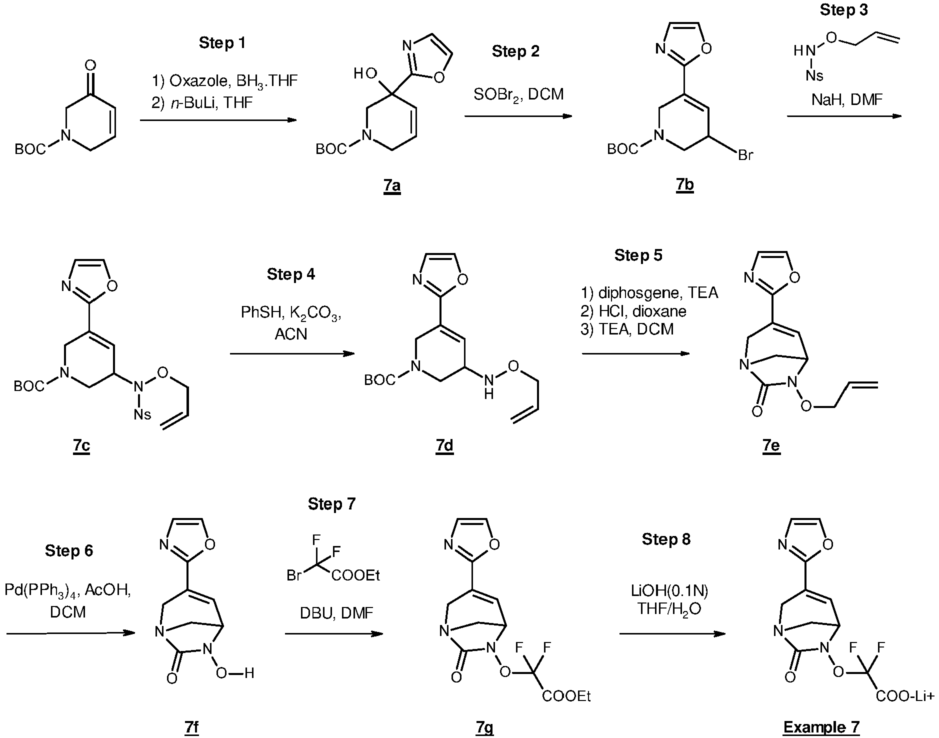

### Step 1: preparation of intermediate tert-butyl 3-hydroxy-3-oxazol-2-yl-2,6-dihydropyridine-1-carboxylate (7a)

To a solution of borane tetrahydrofuran complex solution 1.0 M in THF (2 mL, 2.0 mmol) under argon atmosphere at rt, was dropwise added oxazole (0.133 mL, 2.0 mmol). The mixture was stirred at rt for 1 h then cooled down to -78°C. A n-butyllithium solution 1.6 M in hexanes (1.33 ml, 2.13 mmol) was dropwise added and the mixture maintained at this temperature for 30 min. A solution of 1-methyl-2,6-dihydropyridin-3-one (200 mg, 1.01 mmol) in anhydrous THF (0.7 mL) was dropwise added. The mixture was stirred at -78°C for 2 h. EtOH containing 5% AcOH (2.6 mL) was added and the mixture was stirred at rt for 5 h. Water was added. The aqueous layer was extracted with EtOAc. The combined organic layers were washed with a saturated solution of NaHCO₃, dried over Na₂SO₄ and concentrated *in vacuo.* The residue was purified by flash chromatography on silica gel (petroleum ether/acetone: 100/0 to 80/20) to provide *tert*-butyl 3-hydroxy-3-oxazol-2-yl-2,6-dihydropyridine-1-carboxylate (7a) (68 mg, 0.26 mmol, 25%).
MS *m*/*z* ([M+H]⁺) 267.
1H NMR (400 MHz, CDCl₃) *δ*(ppm) 1.41-1.44 (m, 9H), 3.70-4.11 (m, 5H), 6.03 (s, 2H), 7.10 (s, 1H), 7.65 (s, 1H).

### Step 2: preparation of intermediate tert-butyl 3-bromo-5-oxazol-2-yl-3,6-dihydro-2H-pyridine-1 -carboxylate (7b)

Thionyl bromide (32 µL, 0.41 mmol) was dropwise added to a solution of TEA (58 µL, 0.41 mmol) and *tert-butyl* 3-hydroxy-3-oxazol-2-yl-2,6-dihydropyridine-1-carboxylate (7a) (100 mg, 0.38 mmol) in anhydrous DCM (1.38 mL) at 0°C. The mixture was stirred at 0°C for 50 min then poured in a mixture of ice and H₂O. The layers were separated. The aqueous layer was extracted with DCM. The combined organic layers were washed with brine, dried over Na₂SO₄ and concentrated *in vacuo* to provide *tert*-butyl 3-bromo-5-oxazol-2-yl-3,6-dihydro-2H-pyridine-1-carboxylate (7b) (124 mg, 0.38 mmol, 99%) as a brown oil which was used without further purification.
1H NMR (400 MHz, CDCl₃) *δ*(ppm) 1.51 (s, 9H), 3.78-3.89 (m, 2H), 3.98-4.06 (m, 1H), 4.20-4.36 (m, 1H), 4.80-4.83 (m, 1H), 6.90-6.91 (m, 1H), 7.18 (s, 1H), 7.63 (s, 1H).

### Step 3: preparation of intermediate tert-butyl 3-[allyloxy-(2-nitrophenyl)sulfonyl-amino]-5-oxazol-2-yl-3,6-dihydro-2H-pyridine-1-carboxylate (7c)

To a suspension of NaH 60% in oil (97 mg, 2.42 mmol) in anhydrous DMF (4 mL) at 0°C under nitrogen atmosphere was portionwise added *N*-allyloxy-2-nitro-benzenesulfonamide (624 mg, 2.42 mmol). The mixture was stirred at 0°C for 15 min then a solution of *tert*-butyl 3-bromo-5-oxazol-2-yl-3,6-dihydro-2*H*-pyridine-1-carboxylate (7b) (692 mg, 2.10 mmol) in anhydrous DMF (2 mL) was dropwise added. The mixture was stirred for 90 min at 0°C then H₂O was added. The mixture was extracted twice with EtOAc. The organic layer was washed with brine, dried over Na₂SO₄ and concentrated *in vacuo.* The residue was purified by flash chromatography on silica gel (petroleum ether/acetone: 100/0 to 80/20) to provide *tert*-butyl 3-[allyloxy-(2-nitrophenyl)sulfonyl-amino]-5-oxazol-2-yl-3,6-dihydro-2*H*-pyridine-1-carboxylate (7c) (513 mg, 1.01 mmol, 48%) as a solid.
MS *m*/*z* ([M+H]⁺) 507.
¹H NMR (400 MHz, CDCl₃) *δ*(ppm) 1.46 (s, 9H), 3.68-3.80 (m, 2H), 4.25-4.50 (m, 4H), 4.68-4.75 (m, 1H), 5.16-5.27 (m, 2H), 5.71-5.83 (m, 1H), 6.48 (bs, 1H), 7.13 (s, 1H), 7.57 (s, 1H), 7.64 (dd, *J* = 7.9/ 1.3 Hz, 1H), 7.75 (td, *J* = 7.7/ 1.4 Hz, 1H), 7.82 (td, *J* = 7.7/ 1.5 Hz, 1H), 8.16 (dd, *J* = 7.9/ 1.4 Hz, 1H).

### Step 4: preparation of intermediate tert-butyl 3-(allyloxyamino)-5-oxazol-2-yl-3,6-dihydro-2H-pyridine-1-carboxylate (7d)

The *tert-*butyl 3-[allyloxy-(2-nitrophenyl)sulfonyl-amino]-5-oxazol-2-yl-3,6-dihydro-2*H-*pyridine-1-carboxylate (7c) (512 mg, 1.01 mmol) was dissolved in ACN (6.3 mL) and K₂CO₃ (978 mg, 7.08 mmol) and thiophenol (415 µL, 4.04 mmol) were added. The mixture was stirred at rt for 5h and the mixture was diluted with DCM and filtered on a pad of silica gel to eliminate the excess of PhSH. Then the pad was washed with (9/1)DCM/MeOH and the filtrate was concentrated *in vacuo.* The residue was purified by flash chromatography on silica gel (DCM/acetone: 100/0 to 90/10) to provide *tert*-butyl 3-(allyloxyamino)-5-oxazol-2-yl-3,6-dihydro-2*H*-pyridine-1-carboxylate (7d) (263 mg, 0.82 mmol, 81%).
MS *m*/*z* ([M+H]⁺) 322.
¹H NMR (400 MHz, CDCl₃) *δ*(ppm) 1.50 (s, 9H), 3.35-3.95 (m, 3H), 4.17-4.27 (m, 3H), 4.35-4.60 (m, 1H), 5.17-5.23 (m, 1H), 5.25-5.33 (m, 1H), 5.44 (bs, 1H), 5.89-6.00 (m, 1H), 6.72 (s, 1H), 7.15 (s, 1H), 7.60 (s, 1H).

### Step 5: preparation of intermediate 6-allyloxy-3-oxazol-2-yl-1,6-diazabicyclo[3.2.1]oct-3-en-7-one (7e)

To a solution of of compound *tert*-butyl 3-(allyloxyamino)-5-oxazol-2-yl-3,6-dihydro-2*H-*pyridine-1-carboxylate (7d) (257 mg, 0.80 mmol) in anhydrous DCM (4 mL) at 0°C under argon were added TEA (223 µL, 1.60 mmol) and diphosgene (125.5 µL, 1.04 mmol). The mixture was stirred at 0°C for 1h, diluted with DCM and washed with brine. The organic layer was dried over Na₂SO₄ and concentrated *in vacuo.* The residue was dissolved in anhydrous dioxane (1 mL) and dropwise added to a 4 M HCI solution in dioxane (8 mL). The mixture was stirred at rt for 1 h and concentrated *in vacuo.* The residue was dissolved in anhydrous DCM (8 mL) cooled at 0°C and TEA (446 µL, 3.20 mmol) was added. The mixture was stirred at rt for 1h then washed with brine. The organic layer was dried over Na₂SO₄ and concentrated *in vacuo.* The residue was purified by flash chromatography on silica gel (DCM/Acetone: 100/0 to 80/20) to provide 6-allyloxy-3-oxazol-2-yl-1,6-diazabicyclo[3.2.1]oct-3-en-7-one (7e) (155 mg, 0.63 mmol, 78%).
MS *m*/*z* ([M+H]⁺) 248.
¹H NMR (400 MHz, CDCl₃): δ (ppm) 3.14 (d, *J* = 10.8 Hz, 1H), 3.54-3.59 (m, 1H), 4.05-4.12 (m, 2H), 4.36 (dd, *J* = 18.2/ 1.1 Hz, 1H), 4.37-4.49 (m, 2H), 5.28-5.40 (m, 2H), 5.96-6.07 (m, 1H), 7.11-7.15 (m, 2H), 7.58 (s, 1H).

### Step 6: preparation of intermediate 3-oxazol-2-yl-1,6-diazabicyclo[3.2.1]oct-3-en-7-one (7f)

To a solution of 6-allyloxy-3-oxazol-2-yl-1,6-diazabicyclo[3.2.1]oct-3-en-7-one (7e) (160 mg, 0.65 mmol) and glacial AcOH (59.5 µL, 1.04 mmol) in anhydrous DCM (6.5 mL) was added in one portion Pd(PPh₃)₄ (374 mg, 0.32 mmol) at rt. After stirring for 30 min, the mixture was concentrated under nitrogen flux. The residue was purified by chromatography on silica gel (DCM/Acetone: 100/0 to 50/50) to afford 3-oxazol-2-yl-1,6-diazabicyclo[3.2.1]oct-3-en-7-one (7f) (122 mg, 0.59 mmol, 91%).
MS *m*/*z* ([M+H]⁺) 208.

### Step 7: preparation of intermediate ethyl 2,2-difluoro-2-[(3-oxazol-2-yl-7-oxo-1,6-diazabicyclo[3.2.1]oct-3-en-6-yl)oxy]-acetate (7g)

3-oxazol-2-yl-1,6-diazabicyclo[3.2.1]oct-3-en-7-one (7f) (122 mg, 0.59 mmol) was solubilized in DMF (6.5 mL) at -20°C with DBU (97 µL, 0.65 mmol) and ethyl 2-bromo-2,2-difluoro-acetate (340 µL, 2.65 mmol). The reaction was stirred for 1h15 at -20°C. H₂O was added and the mixture was extracted twice with EtOAc. The organic layer was dried over Na₂SO₄, filtered, and concentrated *in vacuo.* The crude was purified by flash chromatography on silica gel (petroleum ether/acetone 100/0 to 70/30) to provide ethyl 2,2-difluoro-2-[(3-oxazol-2-yl-7-oxo-1,6-diazabicyclo[3.2.1]oct-3-en-6-yl)oxy]-acetate (7g) (121 mg, 0.37 mmol, 63.5%).
MS *m*/*z* ([M+H]⁺) 330.
¹H NMR (400 MHz, CDCl₃): *δ*(ppm) 1.37 (t, *J* = 7.2 Hz, 3H), 3.23 (d, *J=* 11.2 Hz, 1H), 3.67-3.70 (m, 1H), 4.17 (dd, *J* = 18.0/ 2.1 Hz, 1H), 4.28 (dd, *J* = 5.3/ 2.5 Hz, 1H), 4.33-4.41 (m, 2H), 4.45 (dd, *J=* 18.0/1.4 Hz, 1H), 7.08-7.11 (m, 1H), 7.15 (s, 1H), 7.62 (s, 1H).

### Step 8 : preparation of lithium 2,2-difluoro-2-[(3-oxazol-2-yl-7-oxo-1,6-diazabicyclo[3.2.1]oct-3-en-6-yloxy]acetate (Example 7)

Ethyl 2,2-difluoro-2-[(3-oxazol-2-yl-7-oxo-1,6-diazabicyclo[3.2.1]oct-3-en-6-yl)oxy]-acetate (7g) (10 mg, 0.03 mmol) was solubilized in THF (0.25 mL) and H₂O (2 µL) at 0 °C. A solution of LiOH 0.1N (320 µL, 0.73 mmol) was then dropwise added. The mixture was stirred for 2 h at 0 °C. H₂O was added (0.5 mL) and the aqueous layer was washed with EtOAc. The resulting aqueous layer was frozen and lyophilized to provide lithium 2,2-difluoro-2-[(3-oxazol-2-yl-7-oxo-1,6-diazabicyclo[3.2.1]oct-3-en-6-yloxy]acetate (Example 7) (8 mg, 0.03 mmol, 86%) as a white solid.
MS *m*/*z* ([M+H]⁺) 302.

MS *m*/*z* ([M-H]⁻) 300.
¹H NMR (400 MHz, D₂O): *δ*(ppm) 3.44 (d, *J=* 11.4 Hz, 1H), 3.67-3.71 (m, 1H), 4.21 (dd, *J* = 17.8/ 1.5 Hz, 2H), 4.28 (dd, *J* = 17.8/ 2.1 Hz, 1H), 4.48 (dd, *J* = 5.4/ 2.7 Hz, 1H), 7.19 (s, 1H), 7.20-7.24 (m, 1H), 7.83 (s, 1H).

### Example 8: synthesis of sodium [3-(3-pyridyl)-7-oxo-1,6-diazabicyclo[3.2.1]oct-3-en-6-yl] sulfate

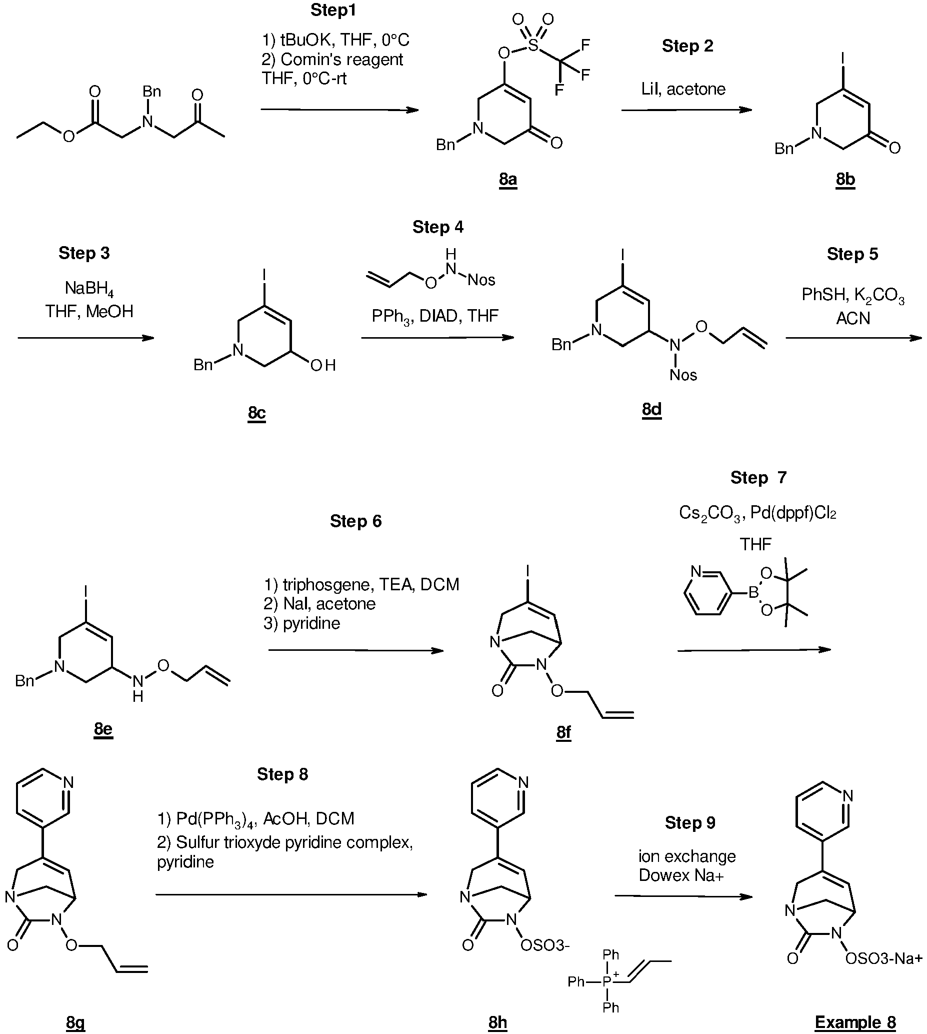

### Step 1: preparation of intermediate (1-benzyl-5-oxo-2,6-dihydropyridin-3-yl) trifluoromethanesulfonate (8a)

In a 500 mL round bottom flask, under nitrogen atmosphere, *t*BuOK (2.7 g, 24.07 mmol) was dissolved in anhydrous THF (180 mL) and the resulting solution was cooled at 0°C. Compound *N*-benzyl-*N*-acetonylglycinate (synthesized according to the procedures described in the litterature (J.Org.Chem. 2006, 71(21), 8256, J.Med.Chem. 2012, 55(11), 5403, WO2013/181741) (6 g, 24.07 mmol) dissolved in anhydrous THF (60 mL) was added with a dropping funnel over 5 min. The resulting viscous solution was stirred 30 min at 0°C (LC/MS showed the formation of the corresponding dione *m*/*z* ([M+H]⁺ 204, [M+H₂O+H]⁺ 222, [M-H]⁻ 202).
At 0°C, the Comins' Reagent (9.7 g, 24.07 mmol) dissolved in THF (20 mL) was added and the reaction was stirred for an additional 30 min. The reaction mixture was diluted with Et₂O and the solution was washed with H₂O. The organic layer was dried over Na₂SO₄, filtered and concentrated *in vacuo.* The residue was purified by chromatography on silica gel (toluene/acetone 100/0 to 95/5 or cyclohexane/EtOAc 100/0 to 50/50) to provide (1-benzyl-5-oxo-2,6-dihydropyridin-3-yl) trifluoromethanesulfonate (8a) which was triturated in a mixture of petroleum ether/ether (9/1) at -78°C. After filtration, compound (8a) was obtained as a white crystalline solid (5.80 g, 17.29 mmol, 71%) and stored in the freezer.
MS *m*/*z* ([M+H]⁺) 336.
¹H NMR (300 MHz, CDCl₃): *δ*(ppm) 3.27 (s, 2H), 3.49 (s, 2H), 3.73 (s, 2H), 6.17 (t, *J=* 1.3 Hz, 1H).

### Step 2: preparation of intermediate 1-benzyl-5-iodo-2,6-dihydropyridin-3-one (8b)

In a 1 L round bottom flask under nitrogen atmosphere, the vinyl triflate (8a) (16.1 g, 48.02 mmol) was diluted with acetone (480 mL). Anhydrous Lil was added (12.9 g, 96.03 mmol) and the resulting pale yellow solution was stirred for 3.5 h at 45°C. It was evaporated to dryness under reduced pressure. The residue was diluted with DCM (350 mL) making salts precipitate which were filtered over a pad of celite ®. The filtrate was washed with H₂O (2 x 100 mL), dried over Na₂SO₄. After concentration, the 1-benzyl-5-iodo-2,6-dihydropyridin-3-one (8b) (15.3 g, 15.0 g expected) was obtained as pale yellow solid once triturated.
MS *m*/*z* ([M+H]⁺) 314.
¹H NMR (300 MHz, CDCl₃): *δ*(ppm) 3.29 (bs, 2H), 3.66 (bs, 2H), 3.73 (bs, 2H), 6.89 (t, *J* = 1.7 Hz, 1H), 7.29-7.37 (m, 5H).

### Step 3: preparation of intermediate 1-benzyl-5-iodo-3,6-dihydro-2H-pyridin-3-ol (8c)

In a 1 L three-neck round bottom flask under nitrogen atmosphere, 1-benzyl-5-iodo-2,6-dihydropyridin-3-one (8b) (15.3 g, 48.02 mmol theoretical) was dissolved in a 5/1 MeOH/THF mixture (0.16 M) and cooled down to 0°C. After 15 min, NaBH₄ (2.1 g, 55.2 mmol) was added by small portions over 10 minutes. The reaction was completed within 10 min. The solvents were removed under reduced pressure at rt to a volume of approximatively 60 mL. The mixture was then diluted with DCM (500 mL) and washed with crushed ice/ water (100 mL). Aqueous layer was taken up with DCM (2 x 30 mL). The combined organic layers were dried over Na₂SO₄, evaporated to dryness and the crude 1-benzyl-5-iodo-3,6-dihydro-2*H*-pyridin-3-ol (8c) (15.4 g, 15.1 expected) was obtained as a pale solid and used in the next step without further purification.
MS *m*/*z* ([M+H]⁺) 316.
¹H NMR (400 MHz, CDCl₃): *δ*(ppm) 2.36 (bs, 1H), 2.57 (dd, *J* = 12.0/2.4 Hz, 1H), 2.86 (dd, *J* = 12.0/2.4 Hz, 1H), 3.04 (d, *J* = 16.3 Hz, 1H), 3.43 (d, *J* = 16.3 Hz, 1H), 3.61 (d, *J* = 11.5 Hz, 1H), 3.66 (d, *J* = 11.5 Hz, 1H), 3.99-4.06 (m, 1H), 6.52-6.57 (m, 1H), 7.28-7.38 (m, 5H).

### Step 4: preparation of intermediate N-allyloxy-N-(1-benzyl-5-iodo-3,6-dihydro-2H-pyridin-3-yl)-2-nitro-benzenesulfonamide (8d)

Using the procedure described in example 1 (step 1), the intermediate 1-benzyl-5-iodo-3,6-dihydro-2*H*-pyridin-3-ol (8c) (15.4 g, 48.02 mmol theoretical) was converted into *N*-allyloxy-*N-*(1-benzyl-5-iodo-3,6-dihydro-2*H*-pyridin-3-yl)-2-nitro-benzenesulfonamide (8d) after purification by flash chromatography on silica gel (petroleum ether/Et₂O 100/0 to 40/60) (39.0 g, 26.7 g expected) contaminated by an excess of unreacted *N*-allyloxy-2-nitrobenzenesulfonamide and reduced DIAD. The oily residue was covered with cold diisopropyl ether making reduced DIAD precipitate partially. After filtration of the white solid, 34 g were recovered and used as such in the next step.
MS *m*/*z* ([M+H]⁺) 556.

### Step 5: preparation of intermediate N-allyloxy-1-benzyl-5-iodo-3,6-dihydro-2H-pyridin-3-amine (8e)

Under nitrogen atmosphere, K₂CO₃ (50.0 g, 360.1 mmol) was added to a solution of N-allyloxy-*N*-(1-benzyl-5-iodo-3,6-dihydro-2*H*-pyridin-3-yl)-2-nitro-benzenesulfonamide (8d) (48.02 mmol theoretical) in ACN (400 mL) in the presence of PhSH (25.0 mL, 240.1 mmol). After stirring 3 h at rt, the reaction mixture was filtered on celite® and the cake was washed with DCM (3 x 150 mL). The filtrate was concentrated and the crude yellow slurry (60 g) was poured in heptane (500 mL) making reduced DIAD precipitate. After filtration and evaporation, clear yellow oil was obtained (51 g). A first purification by flash chromatography on silica gel (petroleum ether/ Et₂O100/0 to 40/60) followed by a second purification (DCM 100% then DCM/EtOAc 15/85) gave desired *N*-allyloxy-1-benzyl-5-iodo-3,6-dihydro-2*H-*pyridin-3-amine (8e) as a pale yellow solid after trituration (12.2 g, 68% over 4 steps).
MS *m*/*z* ([M+H]⁺) 371.
¹H NMR (400 MHz, CDCl₃): *δ*(ppm) 2.48 (dd, *J* = 11.7/3.4 Hz, 1H), 2.96-3.08 (m, 2H), 3.34 (d, *J=* 16.5Hz, 1H), 3.57 (bs, 1H), 3.60 (d, *J=* 13.5 Hz, 1H), 3.65 (d, *J=* 13.5 Hz, 1H), 4.09-4.22 (m, 2H), 5.15-5.30 (m, 2H), 5.73 (bs, 1H), 5.84-5.96 (m, 1H), 6.37-6.43 (m, 1H), 7.25-7.38 (m, 5H).

### Step 6: preparation of intermediate 6-allyloxy-3-iodo-1,6-diazabicyclo[3.2.1]oct-3-en-7-one (8f)

In a 2 L three neck round bottom flask under inert atmosphere with an addition funnel and a water condenser, *N*-allyloxy-1-benzyl-5-iodo-3,6-dihydro-2*H*-pyridin-3-amine (8e) (12.2 g, 32.96 mmol) was diluted in anhydrous DCE (350 mL). A solution of triphosgene (12.7 g, 42.84 mmol) in DCE (150 mL) was added at rt over 5 min and the solution was stirred until the pale yellow solution turned to a white suspension. The reaction mixture was then heated at 55°C for 20 min.
A solution of dry Nal (49.2 g, 329.6 mmol) in dry acetone (170 mL) was then added dropwise and the yellow suspension turned to a brown slurry which was heated at 65°C for 25 min. Pyridine (66 mL, 823.9 mmol) was carefully added dropwise over 10 min. The reaction was stirred for 30 min at 65°C. The reaction was cooled down to 0°C, diluted with DCM (600 mL), filtered on celite® and concentrated to dryness under reduced pressure. The brown residue was diluted with DCM (600 mL), filtered once more on celite® and washed with an aqueous 0.2M solution of NaH₂PO₄ (2 x 200 mL) and Na₂S₂O₃ 1M aqueous solution (2 x 200 mL). The organic layer was dried over Na₂SO₄, concentrated under reduced pressure. The crude compound (14.5 g) was purified by flash chromatography on silica gel (petroleum ether/ether 100/0 to 40/60) to give 6-allyloxy-3-iodo-1,6-diazabicyclo[3.2.1]oct-3-en-7-one (8f) (7.1 g, 23.2 mmol, 70%) as an orange oil. 400 mg of starting material (8e) were also recovered.
MS *m*/*z* ([M+H]⁺) 307.
¹H NMR (300 MHz, CDCl₃): *δ* (ppm) 3.21 (d, *J=* 10.8 Hz, 1H), 3.51-3.58 (m, 1H), 3.83-3.86 (m, 1H), 3.90 (dd, *J* = 18.0/2.2 Hz, 1H), 4.07 (dd, *J* = 18.0/1.4 Hz, 1H), 4.36-4.53 (m, 2H), 5.28-5.46 (m, 2H), 5.95-6.13 (m, 1H), 6.87-6.97 (m, 1H).

### Step 7: preparation of intermediate 6-allyloxy-3-(3-pyridyl)-1,6-diazabicyclo[3.2.1]oct-3-en-7-one (8g)

In a wheaton vial, 6-allyloxy-3-iodo-1,6-diazabicyclo[3.2.1]oct-3-en-7-one (8f) (200 mg, 0.653 mmol), pyridine-3-boronic acid pinacol ester (161 mg, 0.784 mmol), dry Cs₂CO₃ (426 mg, 1.31 mmol) were dissolved in anhydrous THF (6.5 mL). The solution was degassed under argon for 5 min and [1,1'-Bis(diphenylphosphino)ferrocene] dichloropalladium(II) (107 mg, 0.131 mmol) was added. The reaction was stirred at 60 °C overnight. The reaction mixture was filtered on isolute Si-TMT resin and concentrated under reduced pressure to afford a crude material which was purified by flash chromatography on C-18 reverse phase (H₂O/ACN 90/10 to 0/100) to give 6-allyloxy-3-(3-pyridyl)-1,6-diazabicyclo[3.2.1]oct-3-en-7-one (8g) (85 mg, 0.329 mmol, 50%) as a clear yellow gum.
MS *m*/*z* ([M+H]⁺) 258.
¹H NMR (300 MHz, CDCl₃): *δ*(ppm) 3.15 (d, *J=* 10.1 Hz, 1H), 3.55 (ddd, *J* = 10.7/ 2.9/ 1.2 Hz, 1H), 4.04 (ddd, *J=* 11.2/ 6.0/ 2.1 Hz, 2H), 4.24 (dd, *J* = 17.6/ 1.2 Hz, 1H), 4.35-4.50 (m, 2H), 5.26-5.32 (m, 1H), 5.36 (dq, *J* = 17.6/ 1.5 Hz, 1H), 5.94-6.10 (m, 1H), 6.64-6.70 (m, 1H), 7.25 (ddd, *J* = 8.0/ 4.8/ 0.8 Hz, 1H), 7.57 (ddd, *J* = 8.0/ 2.4/ 1.6 Hz, 1H), 8.52 (dd, *J* = 4.8/ 1.6 Hz, 1H), 8.56 (dd, *J* = 2.4/ 0.8 Hz, 1H).

### Step 8: preparation of triphenyl-(propenyl)-phosphonium [3-(3-pyridyl)-7-oxo-1,6-diazabicyclo[3.2.1]oct-3-en-6-yl] sulfate (8h)

To a solution of intermediate 6-allyloxy-3-(3-pyridyl)-1,6-diazabicyclo[3.2.1]oct-3-en-7-one (8g) (85 mg, 0.329 mmol) in anhydrous DCM (4 mL) were added glacial AcOH (38 µL, 0.655 mmol) and Pd(PPh₃)₄ (189 mg, 0.164 mmol). After 45 min of stirring at rt, pyridine (4 mL) and sulfur trioxide pyridine complex (260 mg, 1.64 mmol) were added to the reaction mixture. The resulting white suspension was protected from light and stirred overnight until the reaction was completed. The suspension was filtered, the solids were washed with DCM (3 x 10 mL), the filtrate was concentrated under *vacuum* and then purified by flash chromatography on silica gel (DCM/acetone: 100/0 to 0/100) to afford
triphenyl-(propenyl)-phosphonium [3-(3-pyridyl)-7-oxo-1,6-diazabicyclo[3.2.1]oct-3-en-6-yl] sulfate (8h).
MS *m*/*z* ([M-H]⁻) 296.
MS *m*/*z* ([M+H]⁺) 303 (triphenyl-propenyl-phosphonium).

### Step 9: preparation of sodium [3-(3-pyridyl)-7-oxo-1,6-diazabicyclo[3.2.1]oct-3-en-6-yl] sulfate (Example 8)

triphenyl-(propenyl)-phosphonium [3-(3-pyridyl)-7-oxo-1,6-diazabicyclo[3.2.1]oct-3-en-6-yl] sulfate (8h) dissolved in H₂O (200 µL) was applied on a Dowex sodium form column (Dowex® 50WX8 hydrogen form stored with an aqueous solution of 2N NaOH and washed until neutral pH with H₂O). The fractions containing the desired compound were combined, frozen and lyophilized to afford sodium [3-(3-pyridyl)-7-oxo-1,6-diazabicyclo[3.2.1]oct-3-en-6-yl] sulfate (Example 8) (19.8 mg, 0.062 mmol, 19% over 3 steps) as a white solid after lyophilization.
MS *m*/*z* ([M-H]⁻) 296.
¹H NMR (300 MHz, D₂O): *δ*(ppm) 3.43 (d, *J=* 11.2 Hz, 1H), 3.65-3.72 (m, 1H), 4.12 (d, *J*= 17.8 Hz, 1H), 4.28 (dd, *J* = 17.8/ 2.1 Hz, 1H), 4.50 (dd, *J* = 5.4/ 2.7 Hz, 1H), 6.72 (d, *J* = 5.3 Hz, 1H), 7.38 (dd, *J* = 8.2/ 4.9 Hz, 1H), 7.74 (dt, *J* = 8.2/ 2.0 Hz, 1H), 8.37-8.44 (m, 2H).

### Example 9: synthesis of sodium [(5R)-3-(oxazol-2-yl)-7-oxo-1,6-diazabicyclo[3.2.1]oct-3-en-6-yl] sulfate

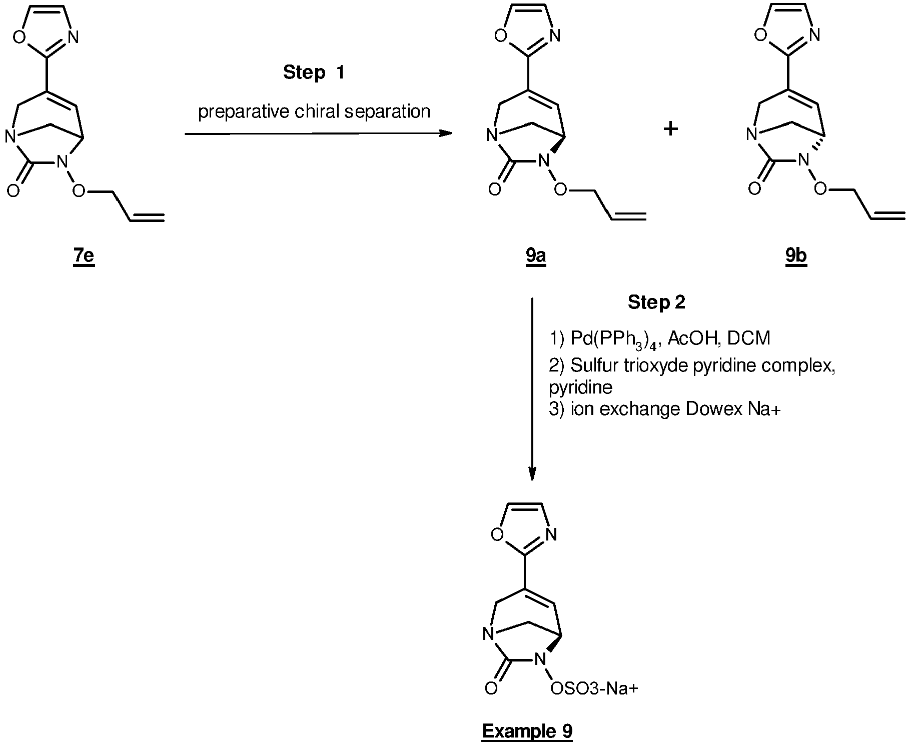

### Step 1: preparation of intermediates (5R)-6-allyloxy-3-oxazol-2-yl-1,6-diazabicyclo[3.2.1]oct-3-en-7-one (9a) and (5S)-6-allyloxy-3-oxazol-2-yl-1,6-diazabicyclo[3.2.1]oct-3-en-7-one (9b)

Both enantiomers of 6-allyloxy-3-oxazol-2-yl-1,6-diazabicyclo[3.2.1]oct-3-en-7-one (7e) (3.94 g, 15.9 mmol) were separated using preparative chiral chromatography (CHIRALPAK® ID 5 µm, 250*30 mm, Heptane/DCM 30/70, 42.5 mL/min) to provide (5*R*)-6-allyloxy-3-oxazol-2-yl-1.6-diazabicyclo[3.2.1]oct-3-en-7-one (9a) (1.70 g , 6.88 mmol, 43%, 98.7 ee) and (5*S*)-6-allyloxy-3-oxazol-2-yl-1,6-diazabicyclo[3.2.1]oct-3-en-7-one (9b) (1.55 g , 6.27 mmol, 39%, 99.4 ee).
MS *m*/*z* ([M+H]⁺) 248.
(9a) (9b) ¹H NMR (400 MHz, CDCl₃): *δ* (ppm) 3.17 (d, *J* = 10.9 Hz, 1H), 3.56-3.54 (m, 1H), 4.07-4.17 (m, 2H), 4.34-4.51 (m, 3H), 5.31-5.45 (m, 2H), 5.97-6.14 (m, 1H), 7.16 (d, *J=* 0.7 Hz, 1H), 7.17-7.19 (m, 1H), 7.62 (d, *J* = 0.7 Hz, 1H).

### Step 2: preparation of sodium [(5R)-3-(oxazol-2-yl)-7-oxo-1,6-diazabicyclo[3.2.1]oct-3-en-6-yl] sulfate (Example 9)

To a solution of (5*R*)-6-allyloxy-3-oxazol-2-yl-1,6-diazabicyclo[3.2.1]oct-3-en-7-one (9a) (1.60 g, 6.47 mmol)in anhydrous DCM (64 mL) with glacial AcOH (741µL, 12.9 mmol) was added in one portion Pd(PPh₃)₄ (3.74 g, 3.23 mmol). After stirring 30 min at rt under inert atmosphere the reaction was completed. To this solution was added anhydrous pyridine (64 mL) followed by sulfur trioxide pyridine complex (5.15 g, 32.36 mmol) and the resulting suspension was protected from light and stirred overnight until the sulfation was completed. The reaction mixture was filtered and concentrated under vacuum, diluted with DCM and filtered. The residue was taken up in a mixture H₂O/ACN (20/80, 6 mL) and applied on a Dowex sodium form column (Dowex® 50WX8 hydrogen form stored with an aqueous solution of 2N NaOH and washed until neutral pH with H₂O). The fractions containing the desired compound were combined, freezed and lyophilized to afford
sodium [(5*R*)-3-(oxazol-2-yl)-7-oxo-1,6-diazabicyclo[3.2.1]oct-3-en-6-yl] sulfate (example 9) (0.82 g, 2.65 mmol, 41%) as a beige solid.
MS *m*/*z* ([M-H]⁻) 286.
¹H NMR (400 MHz, D₂O): *δ*(ppm) 3.23 (d, *J=* 11.3 Hz, 1H), 3.45-3.53 (m, 1H), 3.96 (dd, *J* 17.8/1.5 Hz, 1H), 4.05 (dd, *J* 17.8/2.0 Hz, 1H), 4.34 (dd, *J* = 5.2/2.5 Hz, 1H), 6.97 (d, *J* = 0.8 Hz, 1H), 6.99-7.04 (m, 1H), 7.62 (d, *J =* 0.8 Hz, 1H).

### Example 10: synthesis of [5-(3-aminopropyl)-4,9-dioxo-3-thia-5,8,10-triaza-tricyclo[6.2.1.0^{2,6}]undec-2(6)-en-10-yl] hydrogen sulfate (reference example)

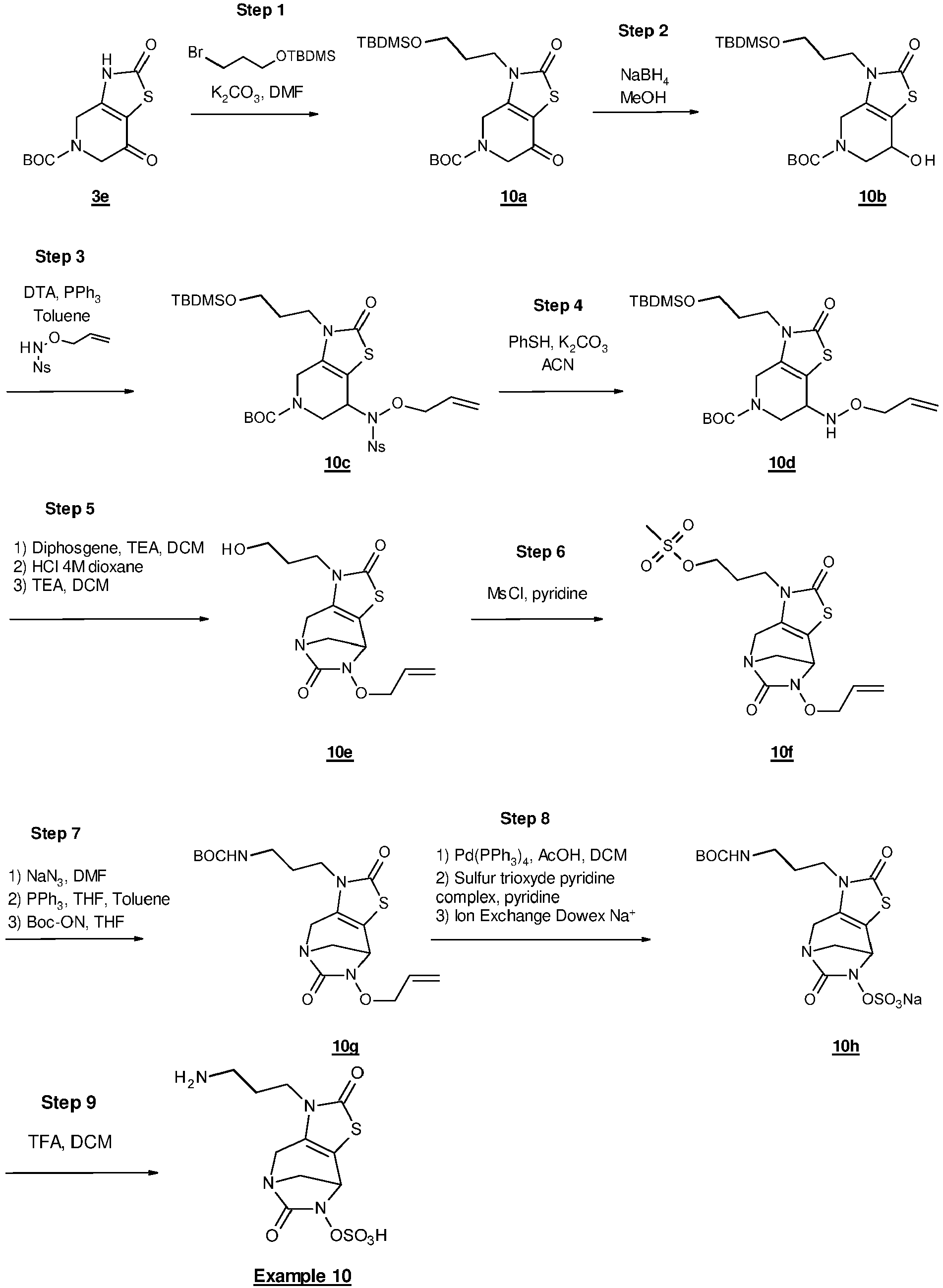

### Step 1: preparation of intermediate tert-butyl 3-[3-[tert-butyl(dimethyl)silyl]oxypropyl]-2,7-dioxo-4,6-dihydrothiazolo[4,5-c]pyridine-5-carboxylate (10a)

To a solution of *tert*-butyl 2,7-dioxo-4,6-dihydro-3*H*-thiazolo[4,5-*c*]pyridine-5-carboxylate (3e) (1.50 g, 5.55 mmol) in anhydrous DMF (55 mL) under inert atmosphere were added K₂CO₃ (0.81 g, 5.83 mmol) and (3-bromopropoxy)-*tert*-butyldimethylsilane (1.35 mL, 5.83 mmol). The mixture was stirred at 70°C overnight. Water was added and the mixture was extracted with EtOAc twice. The organic layers were combined, dried over Na₂SO₄, filtered and concentrated *in vacuo.* The crude was purified twice by flash chromatography on silica gel (petroleum ether/acetone 100/0 to 80/20 then heptane/acetone 100/0 to 60/40) to provide *tert*-butyl 3-[3-[*tert*-butyl(dimethyl)silyl]oxypropyl]-2,7-dioxo-4,6-dihydrothiazolo[4,5-c]pyridine-5-carboxylate (10a) (1.53 g, 3.46 mmol, 62%).
MS *m*/*z* ([M+H]⁺) 443.
¹H NMR (400 MHz, CDCl₃): δ (ppm) 0.06 (s, 6H), 0.89 (s, 9H), 1.49 (s, 9H), 1.86-1.98 (m, 2H), 3.68 (t, *J=* 5.6 Hz, 2H), 3.87 (t, *J=* 4.9 Hz, 2H), 4.24 (s, 2H), 4.65 (s, 2H).

### Step 2: preparation of intermediate tert-butyl 3-[3-[tert-butyl(dimethyl)silyl]oxypropyl]-7-hydroxy-2-oxo-6,7-dihydro-4H-thiazolo[4,5-c]pyridine-5-carboxylate (10b)

Using the procedure described in example 3 (step 8), *tert*-butyl 3-[3-[*tert-*butyl(dimethyl)silyl]oxypropyl]-2,7-dioxo-4,6-dihydrothiazolo[4,5-c]pyridine-5-carboxylate (10a) (1.58 g, 3.57 mmol) was converted to *tert*-butyl 3-[3-[*tert*-butyl(dimethyl)silyl]oxypropyl]-7-hydroxy-2-oxo-6,7-dihydro-4*H*-thiazolo[4,5-*c*]pyridine-5-carboxylate (10b) (1.13 g, 2.54 mmol, 71%) after a purification by flash chromatography on silica gel (DCM/EtOAc 100/0 to 90/10).
MS *m*/*z* ([M+H]⁺) 445.
¹H NMR (400 MHz, CDCl₃): *δ* (ppm) 0.05 (s, 3H), 0.06 (s, 3H), 0.89 (s, 9H), 1.49 (s, 9H), 1.82-1.92 (m, 2H), 3.53 (dd, *J* = 13.7, 3.5 Hz, 1H), 3.61-3.78 (m, 4H), 3.98-4.10 (m, 2H), 4.52 (bs, 2H).

### Step 3: preparation of intermediate tert-butyl 7-[allyloxy-(2-nitrophenyl)sulfonyl-aminol-3-[3-[tert-butyl(dimethyl)silyl]oxypropyl]-2-oxo-6,7-dihydro-4H-thiazolo[4,5-c]pyridine-5-carboxylate (10c)

Using the procedure described in example 3 (step 9), *tert*-butyl 3-[3-[*tert-*butyl(dimethyl)silyl]oxypropyl]-7-hydroxy-2-oxo-6,7-dihydro-4*H*-thiazolo[4,5-c]pyridine-5-carboxylate (10b) (1.13 g, 2.54 mmol) was converted to *tert*-butyl 7-[allyloxy-(2-nitrophenyl)sulfonyl-amino]-3-[3-[*tert*-butyl(dimethyl)silyl]oxypropyl]-2-oxo-6,7-dihydro-4*H-*thiazolo[4,5-c]pyridine-5-carboxylate (10c) (587 mg, 0.86 mmol, 34%) using *N*-allyloxy-2-nitro-benzenesulfonamide (656 mg, 2.54 mmol), PPh₃ (666 mg, 2.54 mmol) and DTA (661 mg, 2.54 mmol) and after a purification by flash chromatography on silica gel (DCM/EtOAc 100/0 to 90/10).
MS *m*/*z* ([M+H]⁺) 685.

### Step 4: preparation of intermediate tert-butyl 7-(allyloxyamino)-3-[3-[tert-butyl(dimethyl)silyl]oxyproyl]-2-oxo-6,7-dihydro-4H-thiazolo[4,5-c]pyridine-5-carboxylate (10d)

Using the procedure described in example 3 (step 10), *tert*-butyl 7-[allyloxy-(2-nitrophenyl)sulfonyl-amino]-3-[3-[*tert*-butyl(dimethyl)silyl]oxypropyl]-2-oxo-6,7-dihydro-4*H-*thiazolo[4,5-c]pyridine-5-carboxylate (10c) (587 mg, 0.86 mmol) was converted to *tert*-butyl 7-(allyloxyamino)-3-[3-[*tert-*butyl(dimethyl)silyl]oxypropyl]-2-oxo-6,7-dihydro-4*H*-thiazolo[4,5-c]pyridine-5-carboxylate (10d) (334 mg, 0.669 mmol, 78%) after a purification on silica gel (DCM/EtOAc 100/0 to 80/20).
MS *m*/*z* ([M+H]⁺) 500.
¹H NMR (400 MHz, CDCl₃): *δ*(ppm) 0.05 (s, 6H), 0.89 (s, 9H), 1.49 (s, 9H), 1.82-1.89 (m, 2H), 3.37 (m, 1H), 3.60-3.76 (m, 4H), 3.91 (s, 1H), 4.04-4.26 (m, 4H), 4.40-4.70 (m, 1H), 5.18-5.22 (m, 1H), 5.26-5.31 (m, 1H), 5.87-5.97 (m, 1H).

### Step 5: preparation of intermediate 10-Allyloxy-5-(3-hydroxy-propyl)-3-thia-5,8,10-triaza-tricyclo[6.2.1.0^{2,6}]undec-2(6)-ene-4,9-dione (10e)

Using the procedure described in example 3 (step 11), *tert*-butyl 7-(allyloxyamino)-3-[3-[*tert-*butyl(dimethyl)silyl]oxypropyl]-2-oxo-6,7-dihydro-4*H*-thiazolo[4,5-*c*]pyridine-5-carboxylate (10d) (512 mg, 1.02 mmol) was converted to 10-allyloxy-5-(3-hydroxy-propyl)-3-thia-5,8,10-triaza-tricyclo[6.2.1.0^{2,6}]undec-2(6)-ene-4,9-dione (10e) (219 mg, 0.70 mmol, 69%) after a purification on silica gel (DCM/Acetone 100/0 to 60/40).
MS *m*/*z* ([M+H]⁺) 312.
¹H NMR (400 MHz, CDCl₃): *δ*(ppm) 1.77-1.85 (m, 2H), 3.26 (dd, *J=* 11.0, 0.7 Hz, 1H), 3.51-3.64 (m, 2H), 3.65-3.77(m, 3H), 4.06 (d, *J=* 16.7 Hz, 1H), 4.19 (d, *J=* 2.6 Hz, 1H), 4.32 (d, *J* = 16.7 Hz, 1H), 4.36-4.42 (m, 1H), 4.43-4.49 (m, 1H), 5.29-5.39 (m, 2H), 5.94-6.05 (m, 1H).

### Step 6: preparation of intermediate 10-allyloxy-5-(3-methylsulfonyloxypropyl)-3-thia-5,8,10-triaza-tricyclo[6.2.1.0^{2,6}]undec-2(6)-ene-4,9-dione (10f)

Using the procedure described in example 3 (step 12), 10-Allyloxy-5-(3-hydroxy-propyl)-3-thia-5,8,10-triaza-tricyclo[6.2.1,0^{2,6}]undec-2(6)-ene-4,9-dione (10e) (218 mg, 0.70 mmol) was converted to 10-allyloxy-5-(3-methylsulfonyloxypropyl)-3-thia-5,8,10-triaza-tricyclo[6.2.1.0^{2,6}]undec-2(6)-ene-4,9-dione (10f) (272 mg, 0.70 mmol, 100%) without further purification.
MS *m*/*z* ([M+H]⁺) 390.

### Step 7: preparation of intermediate 10-allyloxy-5-(3-(tert-butoxycarbonylamino)propyl)-3-thia-5,8,10-triaza-tricyclo[6.2.1.0^{2,6}]undec-2(6)-ene-4,9-dione (10g)

Using the procedure described in example 3 (step 13), 10-allyloxy-5-(3-methylsulfonyloxypropyl)-3-thia-5,8,10-triaza-tricyclo[6.2.1.0^{2,6}]undec-2(6)-ene-4,9-dione (10f) (272 mg, 0.70 mmol) was converted to 10-allyloxy-5-(3-(*tert*-butoxycarbonylamino)propyl)-3-thia-5,8,10-triaza-tricyclo[6.2.1.0^{2,6}]undec-2(6)-ene-4,9-dione (10g) (189 mg, 0.46 mmol, 66%) after a purification on silica gel (DCM/Acetone 100/0 to 90/10).
MS *m*/*z* ([M+H-Boc]⁺) 311.
¹H NMR (400 MHz, CDCl₃): *δ*(ppm) 1.44 (s, 9H), 1.64-1.74 (m, 1H), 1.78-1.87 (m, 1H), 3.00-3.09 (m, 1H), 3.11-3.18 (m, 1H), 3.28 (d, *J=* 11.1 Hz, 1H), 3.51-3.59 (m, 1H), 3.61-3.70 (m, 2H), 4.05 (d, *J =* 16.6 Hz, 1H), 4.17-4.22 (m, 2H), 4.36-4.42 (m, 1H), 4.43-4.49 (m, 1H), 5.03 (bs, 1H), 5.29-5.39 (m, 2H), 5.94-6.05 (m, 1H).

### Step 8: preparation of sodium [5-(3-(tert-butoxycarbonylamino)propyl)-4,9-dioxo-3-thia-5,8,10-triaza-tricyclo[6.2.1.0^{2,6}]undec-2(6)-en-10-yl] sulfate (10h)

Using the procedure described in example 3 (step 14), 10-allyloxy-5-(3-(*tert-*butoxycarbonylamino)propyl)-3-thia-5,8,10-triaza-tricyclo[6.2.1,0^{2,6}]undec-2(6)-ene-4,9-dione (10g) (186 mg, 0.453 mmol) was converted to sodium [5-(3-(*tert*-butoxycarbonylamino)propyl)-4,9-dioxo-3-thia-5,8,10-triaza-tricyclo[6.2.1.0^{2,6}]undec-2(6)-en-10-yl] sulfate (10h) (105 mg, 0.222 mmol, 49%).
MS *m*/*z* ([M-H]⁻) 449.

### Step 9: preparation of [5-(3-aminopropyl)-4,9-dioxo-3-thia-5,8,1 0-triaza-tricyclo[6.2.1 .0^{2,6}]undec-2(6)-en-10-yl] hydrogen sulfate (Example 10)

TFA (510 µL) was added dropwise to a solution of sodium [5-(3-(*tert-*butoxycarbonylamino)propyl)-4,9-dioxo-3-thia-5,8,10-triaza-tricyclo[6.2.1.0^{2,6}]undec-2(6)-en-10-yl] sulfate (10h) (105 mg, 0.222 mmol) in DCM (1 mL) at 0°C. The mixture was stirred for 15 min at this temperature then concentrated under nitrogen flux. ACN was added to the compound, and after sonication, the precipitate was filtered, and washed twice with ACN to provide [5-(3-aminopropyl)-4,9-dioxo-3-thia-5,8,10-triaza-tricyclo[6.2.1.0^{2,6}]undec-2(6)-en-10-yl] hydrogen sulfate (Example 10) (56 mg, 0.159 mmol, 72%) as a beige solid.
MS *m*/*z* ([M-H]⁻) 349.
¹H NMR (400 MHz, DMSO-*d*₆): *δ*(ppm) 1.74-1.85 (m, 2H), 2.73-2.87 (m, 2H), 3.39 (d, *J =* 11.2 Hz, 1H), 3.49-3.59 (m, 2H), 3.61-3.73 (m, 1H), 4.07 (d, *J =* 16.7 Hz, 1H), 4.22 (d, *J =* 16.7 Hz, 1H), 4.56 (d, *J=* 2.7 Hz, 1H), 7.68 (bs, 3H).

### Example 11: synthesis of sodium (7-oxo-3-pyrazol-1-yl-1,6-diazabicyclo[3.2.1]oct-3-en-6-yl) sulfate

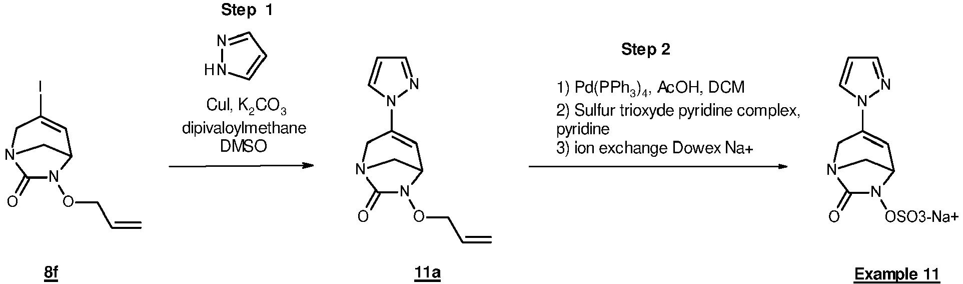

### Step 1: preparation of intermediate 6-allyloxy-3-pyrazol-1-yl-1,6-diazabicyclo[3.2.1]oct-3-en-7-one (11a)

In a wheaton vial under argon atmosphere, 6-allyloxy-3-iodo-1,6-diazabicyclo[3.2.1]oct-3-en-7-one (8f) (200 mg, 0.65 mmol), pyrazole (53 mg, 0.78 mmol), dry Cul (12 mg, 0.065 mmol), K₂CO₃ (181 mg, 1.307 mmol) and dipivaloymethane (27 µL, 0.131 mmol) were dissolved in DMSO (4 mL). The reaction was stirred at 120°C during 6 h, filtered, washed with DCM and concentrated in *vacuo.* The crude was purified by flash chromatography on silica gel (Cyclohexane/EtOAc 50/50) to provide 6-allyloxy-3-pyrazol-1-yl-1,6-diazabicyclo[3.2.1]oct-3-en-7-one (11a) (95 mg, 0.36 mmol, 59 %) as a yellow oil.
MS *m*/*z* ([M+H]⁺) 247.
¹H NMR (400 MHz, CDCl₃): *δ*(ppm) 3.15 (d, *J =* 10.8 Hz, 1H), 3.53 (dd, *J* = 10.8/ 2.2 Hz, 1H), 4.10 (dd, *J* = 5.6/ 2.7 Hz, 1H), 4.22 (dd, *J* = 17.6/ 1.9 Hz, 1H), 4.37-4.50 (m, 3H), 5.28-5.31 (m, 1H), 5.34-5.41 (m, 1H), 5.97-6.08 (m, 1H), 6.35 (dd, *J* = 2.4/ 1.9 Hz, 1H), 6.46 (d, *J* = 5.5 Hz, 1H), 7.57 (d, *J* = 1.7 Hz, 1H), 7.61 (d, *J* = 2.5 Hz, 1H).

### Step 2: preparation of sodium (7-oxo-3-pyrazol-1-yl-1,6-diazabicyclo[3.2.1]oct-3-en-6-yl) sulfate (Example 11)

Using the procedure described in example 9 (step 2), the intermediate 6-allyloxy-3-pyrazol-1-yl-1,6-diazabicyclo[3.2.1]oct-3-en-7-one (11a) (105 mg, 0.427 mmol) was converted into sodium (7-oxo-3-pyrazol-1-yl-1,6-diazabicyclo[3.2.1]oct-3-en-6-yl) sulfate (example 11) (69 mg,0.224 mmol, 52%) as a light yellow solid after lyophilization.
MS *m*/*z* ([M-H]⁻) 285.
¹H NMR (400 MHz, D₂O): *δ*(ppm) 3.48 (d, *J* = 11.3 Hz, 1H), 3.71 (dd, *J* = 11.3/2.8 Hz, 1H), 4.34-4.46 (m, 2H), 4.60 (dd, *J* = 5.6/2.7 Hz, 1H), 6.52 (t, *J=* 2.3 Hz, 1H), 6.63 (d, *J=* 5.6 Hz, 1H), 7.73 (d, *J* = 1.8 Hz, 1H), 7.95 (d, *J* = 2.7 Hz, 1H).

### Example 12: synthesis of sodium [7-oxo-3-(triazol-1-yl)-1,6-diazabicvclo[3.2.1]oct-3-en-6-yl] sulfate

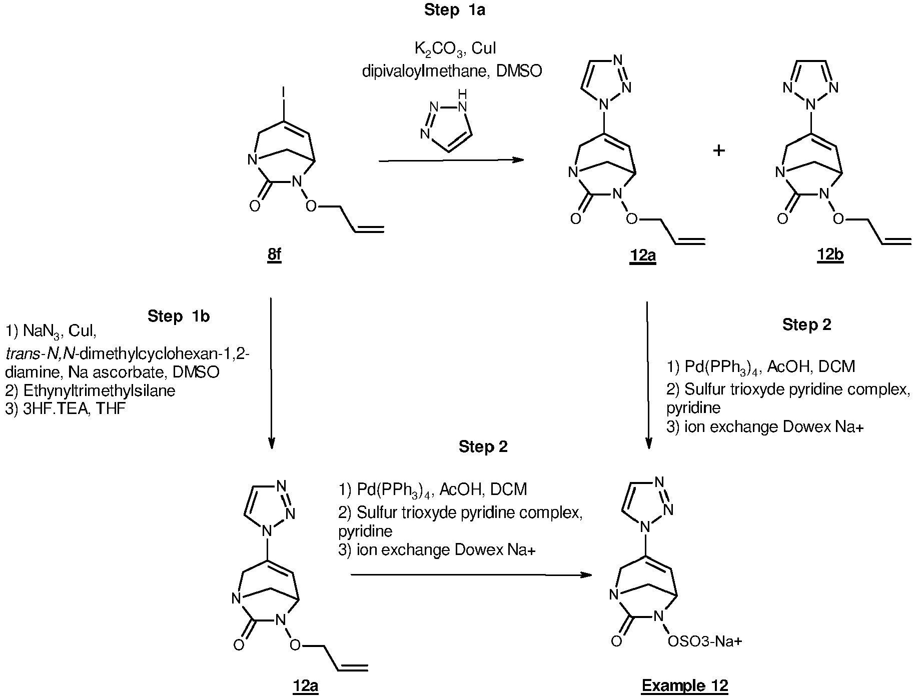

### Step 1a: preparation of intermediates 6-allyloxy-3-(triazol-1-yl)-1,6-diazabicvclo[3.2.1]oct-3-en-7-one (12a) and 6-allyloxy-3-(triazol-2-yl)-1,6-diazabicyclo[3.2.1]oct-3-en-7-one (12b)

In a 20 mL sealed tube under inert atmosphere, 6-allyloxy-3-iodo-1,6-diazabicyclo[3.2.1]oct-3-en-7-one (8f) (629 mg, 2.05 mmol) was diluted with anhydrous DMSO (20 mL). 1*H*-1,2,3-triazol (237 µL, 4.10 mmol), dipivaloylmethane (86 µL, 0.41 mmol), dry K₂CO₃ (567 mg, 4.40 mmol) and Cul (37 mg, 0.20 mmol) were successively added. The blue suspension was heated at 75°C and turned rapidly to brown. After 30 h, the mixture was concentrated to dryness under nitrogen flux. The crude compound was purified by flash chromatography on silica gel (DCM/EtOAc : 100/0 to 0/100) to give 6-allyloxy-3-(triazol-1-yl)-1,6-diazabicyclo[3.2.1]oct-3-en-7-one (12a) (243 mg, 0.982 mmol, 48%) as a yellow oil and 6-allyloxy-3-(triazol-2-yl)-1,6-diazabicyclo[3.2.1]oct-3-en-7-one (12b) (131 mg, 0.530 mmol, 26%) as a yellow oil.
6-allyloxy-3-(triazol-1-yl)-1,6-diazabicyclo[3.2.1]oct-3-en-7-one (12a)
MS *m*/*z* ([M+H]⁺) 248, ([2M+H]⁺) 495.
¹H-NMR (300 MHz, CDCl₃): *δ*(ppm) 3.20 (dd, *J* = 11.0/0.7 Hz, 1H), 3.58 (ddd, *J* = 11.0/2.9/ 1.1 Hz, 1H), 4.16 (dd, *J* = 5.4/2.8 Hz, 1H), 4.30-4.55 (m, 4H), 5.30-5.41 (m, 2H), 5.95-6.08 (m, 1H), 6.64-6.68 (m, 1H), 7.72 (d, *J* = 1.2 Hz, 1H), 7.76 (d, *J* = 1.2 Hz, 1H).
¹³C-NMR (75 MHz, CDCl₃): δ (ppm) 134.10, 133.70, 132.47, 120.62, 120.35, 116.35, 77.31, 75.50, 56.69, 53.13, 49.79.
6-allyloxy-3-(triazol-2-yl)-1 ,6-diazabicyclo[3.2.1]oct-3-en-7-one (12b)
MS *m*/*z* ([M+H]⁺) 248.
¹H NMR (400 MHz, CDCl₃): *δ*(ppm) 3.17 (d, *J=* 10.9 Hz, 1H), 3.52-3.60 (m, 1H), 4.16 (dd, *J* = 5.6/2.7 Hz, 1H), 4.26 (dd, *J* = 17.9/2.0 Hz, 1H), 4.44 (qd, *J* = 12.3/6.3 Hz, 2H), 4.63 (d, *J* = 17.8 Hz, 1H), 5.27-5.43 (m, 2H), 6.03 (ddt, *J* = 16.9/10.3/6.4 Hz, 1H), 6.99 (d, *J =* 5.5 Hz, 1H), 7.69 (s, 2H).
¹³C NMR (75 MHz, CDCl₃): *δ*(ppm) 170.14, 135.62, 135.30, 132.76, 120.26, 114.21,76.72, 60.44, 56.76, 52.72, 50.08.

### Step 1b: preparation of intermediate 6-allyloxy-3-(triazol-1-yl)-1,6-diazabicyclo[3.2.1]oct-3-en-7-one (12a)

In a 100 mL sealed balloon under inert atmosphere, 6-allyloxy-3-iodo-1,6-diazabicyclo[3.2.1]oct-3-en-7-one (8f) (4g, 13.07 mmol) was diluted with anhydrous DMSO (40 mL). Cul (249 mg, 1.31 mmol), sodium azide (1.27g, 19.60 mmol), sodium ascorbate (259 mg, 1.31 mmol) and *trans*-*N*,*N*'-dimethylcyclohexan-1,2-diamine (309 µL, 1.96 mmol) were successively added. The green solution turned rapidly to brown. The mixture was stirred to rt for 30 min until total conversion of starting material. Ethynyltrimethylsilane (2.21mL, 15.68 mmol) was then added to the mixture which was stirred to rt for 30 min until total conversion of intermediate azide. The mixture was diluted with H₂O (400 mL) and extracted with EtOAc (3 x 200 mL). The combined organic layers were dried (Na₂SO₄) and concentrated *in vacuo* to give 4.50 g of a brown oil.

This oil was dissolved in anhydrous THF (87 mL) and 3HF.TEA (2.13 mL, 13.07 mmol) was added to the solution which was stirred 1h at 50°C. The mixture was concentrated *in vacuo* and the crude (6.35 g) was purified by flash chromatography on silica gel (Cyclohexane/EtOAc: 100/0 to 0/100) to give 6-allyloxy-3-(triazol-1-yl)-1,6-diazabicyclo[3.2.1]oct-3-en-7-one (12a) (1.30 g, 5.25 mmol, 40%) as a yellow oil which crystallized as a yellow solid.
MS *m*/*z* ([M+H]⁺) 248, ([2M+H]⁺) 495.
¹H-NMR (300 MHz, CDCl₃): *δ* (ppm) 3.20 (dd, *J=* 11.0/0.7 Hz, 1H), 3.58 (ddd, *J=* 11.0/ 2.9/ 1.1 Hz, 1H), 4.16 (dd, *J* = 5.4/2.8 Hz, 1H), 4.30-4.55 (m, 4H), 5.30-5.41 (m, 2H), 5.95-6.08 (m, 1H), 6.64-6.68 (m, 1H), 7.72 (d, *J* = 1.2 Hz, 1H), 7.76 (d, *J* = 1.2 Hz, 1H).

### Step 2: preparation of sodium [7-oxo-3-(triazol-1-yl)-1,6-diazabicyclo[3.2.1]oct-3-en-6-yl] sulfate (Example 12)

To a solution of 6-allyloxy-3-(triazol-1-yl)-1,6-diazabicyclo[3.2.1]oct-3-en-7-one (12a) (150 mg, 0.607 mmol) in anhydrous DCM (6.1 mL) were added glacial AcOH (69 µL, 1.21 mmol) and Pd(PPh₃)₄ (351 mg, 0.303 mmol). After 45 min of stirring at rt, pyridine (6.1 mL) and sulfur trioxide pyridine complex (483 mg, 3.03 mmol) were added to the reaction mixture. The resulting suspension was protected from light and stirred overnight until the reaction was completed. The reaction mixture was concentrated, then diluted with DCM and filtered. The filtrate was concentrated under *vacuum* and then purified by flash chromatography on silica gel (DCM/acetone: 100/0 to 0/100) to afford triphenyl-(propenyl)-phosphonium 7-oxo-3-(triazol-1-yl)-1,6-diazabicyclo[3.2.1]oct-3-en-6-yl] sulfate (215.7 mg) as a pale yellow foam. This foam was dissolved in a minimum of a mixture H₂O/ACN 20/80 and applied on a Dowex sodium form column (Dowex® 50WX8 hydrogen form stored with an aqueous solution of 2N NaOH and washed until neutral pH with H₂O). The fractions containing the desired compound were combined and concentrated (bath temperature < 30°C). The compound was diluted in H₂O, filtered on Millipore 0.22 µM, frozen and lyophilized to afford sodium [7-oxo-3-(triazol-1-yl)-1,6-diazabicyclo[3.2.1]oct-3-en-6-yl] sulfate (Example 12) (96 mg, 0.310 mmol, 51% over 3 steps, purity 95%) as a beige amorphous solid.
MS *m*/*z* ([M-H]⁻) 286.
¹H-NMR (300 MHz, D₂O): *δ*(ppm) 3.52 (d, *J=* 11.4 Hz, 1H), 3.73 (dd, *J* = 11.5/ 2.8 Hz, 1H), 4.42-4.56 (m, 2H), 4.65 (dd, *J* = 5.6/2.9 Hz, 1H), 6.91-6.93 (m, 1H), 7.85 (d, *J* = 1.3 Hz, 1H), 8.26 (d*, J* = 1.3 Hz, 1H).

### Example 13: synthesis of lithium difluoro-(7-oxo-3-pyrazol-1-yl-1,6-diaza-bicyclo[3.2.1]oct-3-en-6-yloxy)-acetate

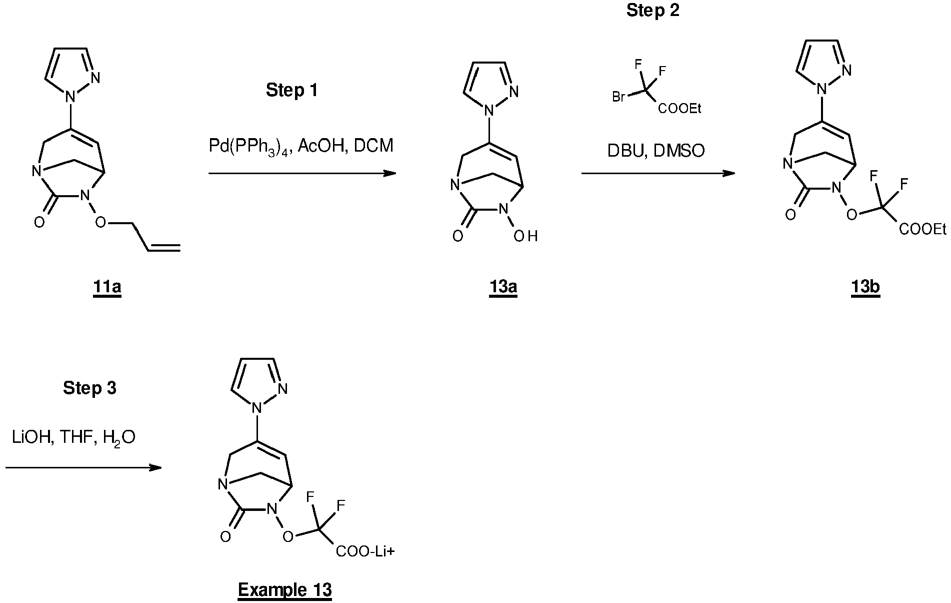

### Step 1: preparation of intermediate 6-hydroxy-3-pyrazol-1-yl-1,6-diaza-bicyclo[3.2.1]oct-3-en-7-one (13a).

A solution of 6-allyloxy-3-pyrazol-1-yl-1,6-diaza-bicyclo[3.2.1]oct-3-en-7-one (11a) (0.100 g, 0.41 mmol) in anhydrous DCM (4 mL) was degazed 10 min under argon atmosphere. AcOH (0.047 mL, 0.81 mmol) and Pd(PPh₃)₄ (0.237 g, 0.205 mmol) were successively added. After stirring for 30 min at rt, the precipitate was filtered and washed with DCM to afford 0.05 mg of white solid. The filtrate was purified by preparative TLC on silica gel (DCM/acetone 60/40) to give additional 0.013 g. The solids were combined to provide 6-hydroxy-3-pyrazol-1-yl-1,6-diaza-bicyclo[3.2.1]oct-3-en-7-one (13a) (0.063 g, 0.31 mmol, 75%).
MS *m*/*z* ([M+H]⁺) 207.
¹H NMR (400 MHz, DMSO): *δ*(ppm): 3.22 (d, *J* = 10.7 Hz, 1H), 3.36 (dd, *J* = 2/10.8 Hz, 1H), 4.02 (dd, *J* = 2.5/5.6 Hz, 1H), 4.18 (d, *J* = 1.1 Hz, 2H), 6.43-6.47 (m, 1H), 6.65 (d, *J* = 5.0 Hz, 1H), 7.64 (d, *J* = 1.5 Hz, 1H), 8.18 (d, *J* = 2.4 Hz, 1H), 9.65 (s, 1H).

### Step 2: preparation of intermediate ethyl difluoro-(7-oxo-3-pyrazol-1-yl-1,6-diaza-bicyclo[3.2.1]oct-3-en-6-yloxy)-acetate (13b).

6-Hydroxy-3-pyrazol-1-yl-1,6-diaza-bicyclo[3.2.1]oct-3-en-7-one (13a) (0.154 g, 0.75 mmol) was solubilised in DMSO (7.5 mL) with DBU (0.123 mL, 0.825 mmol) and ethyl bromo-difluoro-acetate (0.250 mL, 1.94 mmol) and stirred for 30 min. The mixture was washed with NaH₂PO₄ 2M and the product was extracted with ethyl acetate. The organic layer was filtered on a pad of silica then concentrated *in vacuo.* The residue was triturated in Et₂O and filtered on a PTFE membrane to provide ethyl difluoro-(7-oxo-3-pyrazol-1-yl-1,6-diaza-bicyclo[3.2.1]oct-3-en-6-yloxy)-acetate (13b) (0.155 g, 0.47 mmol, 63%).
MS *m*/*z* ([M+H]⁺) 329.
¹H NMR (300 MHz, CDCl₃): *δ*(ppm): 1.38 (t, *J* = 7.2 Hz, 3H), 3.25 (d, *J=* 11.1 Hz, 1H), 3.66 (dd, *J =* 1.7/11.1 Hz, 1H), 4.24-4.44 (m, 4H), 4.59 (dd, *J* = 0.9/17.7 Hz, 1H), 6.39 (dd, *J* = 1.8/2.5 Hz, 1H), 6.40-6.45 (m, 1H), 7.60 (d, *J* = 1.7 Hz, 1H), 7.64 (d, *J* = 2.6 Hz, 1H).

### Step 3: preparation of lithium difluoro-(7-oxo-3-pyrazol-1-yl-1,6-diaza-bicyclo[3.2.1]oct-3-en-6-yloxy)-acetate (Example 13).

To a solution of ethyl difluoro-(7-oxo-3-pyrazol-1-yl-1,6-diaza-bicyclo[3.2.1]oct-3-en-6-yloxy)-acetate (13b) (0.143 g, 0.435 mmol) in THF (4 mL) and H₂O (0.4 mL) at 0°C was dropwise added a 0.1 N LiOH solution (4.8 mL, 0.48 mmol). When monitoring indicated the reaction was completed, the mixture was neutralized with HCI (0.1 N) (0.7 mL) at 0°C. The solution was freezed to evaporate THF on vacuum and lyophilize the aqueous solution. The residue was triturated in Et₂O and filtered on a PTFE membrane. Then the solid was purified on silica gel (*i*PrOH). The fractions containing the desired product were concentrated *in vacuo.* The solid was triturated in Et₂O and the result solid was solubilized on H₂O to lyophilize and provide lithium difluoro-(7-oxo-3-pyrazol-1-yl-1,6-diaza-bicyclo[3.2.1 ]oct-3-en-6-yloxy)-acetate (Example 13) (0.88 g, 0.286 mmol, 66%).
MS *m*/*z*([M+H]⁺) 301.
¹H NMR (300 MHz, D₂O) δ (ppm) : 3.44 (d, *J =* 11.4 Hz, 1H), 3.66 (ddd, *J* = 0.9/2.7/11.3 Hz, 1H), 4.38 (d, *J =* 1.4 Hz, 2H), 4.49 (dd, *J* = 2.5/5.6 Hz, 1H), 6.48 (dd, *J* = 2.0/2.6 Hz, 1H), 6.56-6.61 (m, 1H), 7.69 (d, *J* = 1.8 Hz, 1H), 7.92 (d, *J* = 2.6 Hz, 1H).

### Example 14: synthesis of sodium [7-oxo-3-(1,2,4-triazol-1-yl)-1,6-diazabicyclo[3.2.1]oct-3-en-6-yl] sulfate

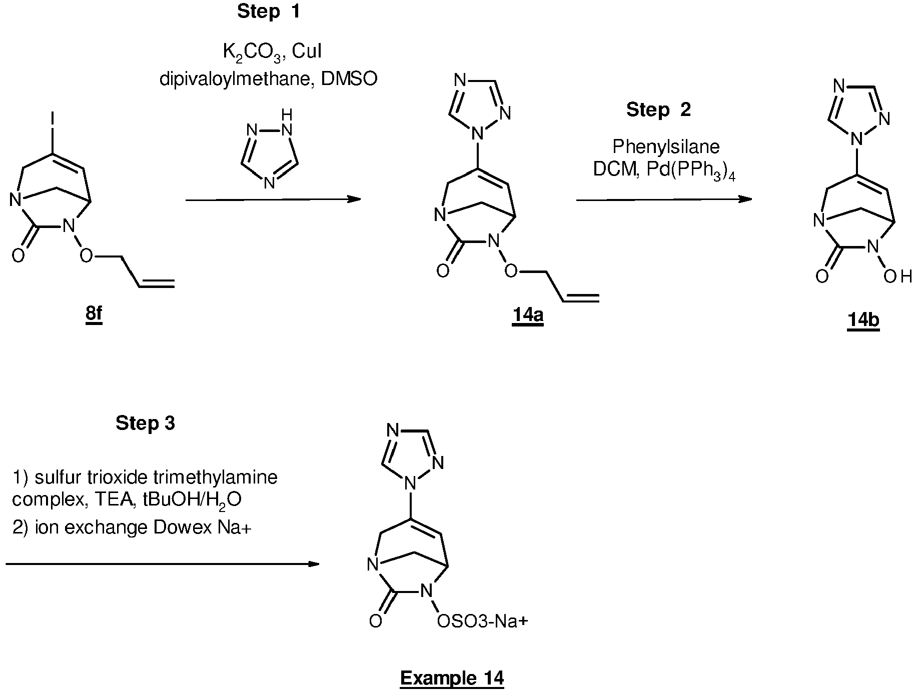

### Step 1: preparation of intermediate 6-allyloxy-3-(1,2,4-triazol-1-yl)-1,6-diazabicyclo[3.2.1]oct-3-en-7-one (14a)

In a 20 mL sealed tube under inert atmosphere, 6-allyloxy-3-iodo-1,6-diazabicyclo[3.2.1]oct-3-en-7-one (8f) (200 mg, 0.65 mmol) was diluted with anhydrous DMSO (4 mL). 1,2,4-triazol (54 mg, 0.78 mmol), dipivaloylmethane (41 µL, 0.196 mmol), dry K₂CO₃ (181 mg, 1.31 mmol) and Cul (12 mg, 0.065 mmol) were successively added. The blue suspension was heated at 100°C. After 3 h, the mixture was diluted in H₂O (10 mL) and DCM (10 mL) was added. The aqueous layer was extract twice with DCM (5 mL) and the the combined organic layers were dried over Na₂SO₄, filtered and concentrated *in vacuo.* The crude compound was purified by flash chromatography on silica gel (DCM/Acetone: 100/0 to 50/50) to give 6-allyloxy-3-(1,2,4-triazol-1-yl)-1,6-diazabicyclo[3.2.1]oct-3-en-7-one (14a) (110 mg, 0.44 mmol, 48%) as an orange oil.
MS *m*/*z* ([M+H]⁺) 248.
¹H NMR (400 MHz, CDCl₃): *δ*(ppm) 3.17 (d, *J=* 10.9 Hz, 1H), 3.57 (dd, *J* = 10.9/2.0 Hz, 1H), 4.14 (dd, *J* = 5.5/2.6 Hz, 1H), 4.18 (dd, *J=* 17.6/2.6 Hz, 1H), 4.35-4.50 (m, 3H), 5.30-5.34 (m, 1H), 5.37 (dq, *J* = 17.2/1.4 Hz, 1H), 5.96-6.07 (m, 1H), 6.68 (d, *J* = 5.4 Hz, 1H), 7.97 (s, 1H), 8.26 (s, 1 H).

### Step 2: preparation of intermediate 6-hydroxy-3-(1,2,4-triazol-1-yl)-1,6-diazabicyclo[3.2.1]oct-3-en-7-one (14b)

Under inert atmosphere, phenylsilane (32 µL, 0.308 mmol) and Pd(PPh₃)₄ (7 mg, 0.006 mmol) were added to a solution of 6-allyloxy-3-(1,2,4-triazol-1-yl)-1,6-diazabicyclo[3.2.1]oct-3-en-7-one (14a) (51 mg, 0.154 mmol) in anhydrous DCM (2 mL). The reaction mixture was stirred at rt for 1 h and filtered. The precipitate was washed with DCM (2 mL) to give 6-hydroxy-3-(1,2,4-triazol-1-yl)-1,6-diazabicyclo[3.2.1]oct-3-en-7-one (14b) (26 mg, 0.125 mmol, 81%) which was used without further purification.
MS *m*/*z* ([M+H]⁺) 208

### Step 3: preparation of sodium [7-oxo-3-(1,2,4-triazol-1-yl)-1,6-diazabicyclo[3.2.1]oct-3-en-6-yl] sulfate (example 14)

6-hydroxy-3-(1,2,4-triazol-1-yl)-1,6-diazabicyclo[3.2.1]oct-3-en-7-one (14b) (13 mg, 0.063 mmol) was dissolved in a mixture of *t*-BuOH (0.3 mL) and H₂O (0.3 mL). TEA (2.2 µL, 0.016 mmol) and sulfur trioxide trimethylamine complex (10 mg, 0.075 mmol) was added. The mixture was stirred at rt for 2 h then concentrated *in vacuo.* The reaction mixture was filtered and the filtrate was concentrated *in vacuo.* The residue was purified by flash chromatography on silica gel (DCM/Acetone: 100/0 to 0/100). The fractions containing the expected intermediate were combined and concentrated *in vacuo.* The residue was dissolved in H₂O and converted after ion exchange (Dowex sodium form column) to sodium [7-oxo-3-(1,2,4-triazol-1-yl)-1,6-diazabicyclo[3.2.1]oct-3-en-6-yl] sulfate (Example 14) (5 mg, 0.016 mmol, 26%).
MS *m*/*z* ([M-H]⁻) 286.
¹H NMR (400 MHz, D₂O): *δ*(ppm): 3.47 (d, *J =* 11.4 Hz, 1H), 3.71 (dd, *J* = 11.4/2.8 Hz, 1H), 4.33 (d, *J =* 17.5 Hz, 1H), 4.41 (dd, *J* = 17.5/1.9 Hz, 1H), 4.61 (dd, *J* = 5.6/2.8 Hz, 1H), 6.85 (d, *J=* 5.6 Hz, 1H), 8.12 (s, 1H), 8.71 (s, 1H).

### Example 15: synthesis of sodium [(5R)-7-oxo-3-pyrazol-1-yl-1,6-diazabicyclo[3.2.1]oct-3-en-6-yl] sulfate

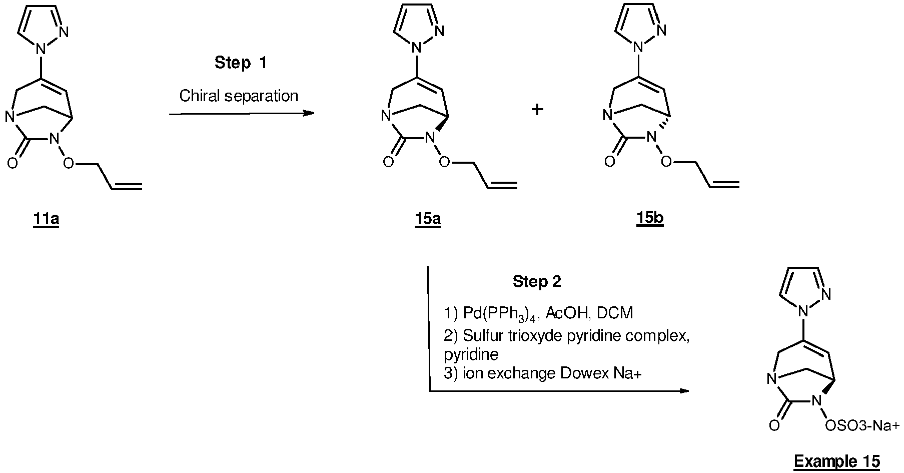

### Step 1: preparation of intermediates (5R)-6-allyloxy-3-pyrazol-1-yl-1,6-diazabicyclo[3.2.1]oct-3-en-7-one (15a) and (5S)-6-allyloxy-3-pyrazol-1-yl-1,6-diazabicyclo[3.2.1]oct-3-en-7-one (15b)

Both enantiomers of 6-allyloxy-3-pyrazol-1-yl-1,6-diaza-bicyclo[3.2.1]oct-3-en-7-one (11a) (4 g, 16.2 mmol) were separated using preparative chiral chromatography (CHIRALPAK® ID 5 µm, 250x30 mm, Heptane/DCM 50/50, 42.5 mL/min) to provide (5*R*)-6-allyloxy-3-pyrazol-1-yl-1,6-diazabicyclo[3.2.1]oct-3-en-7-one (15a) (1.79 g , 7.27 mmol, 44%, 96.8% ee, retention time 23.5 min) and (5*S*)-6-allyloxy-3-pyrazol-1-yl-1,6-diazabicyclo[3.2.1]oct-3-en-7-one (15b) (1.48 g , 6.01 mmol, 37%, 98.1% ee, retention time 14.6 min).
MS *m*/*z* ([M+H]⁺) 247.
¹H NMR (400 MHz, CDCl₃): *δ*(ppm) 3.15 (d, *J=* 10.8 Hz, 1H), 3.53 (dd, *J* = 10.8/ 2.2 Hz, 1H), 4.10 (dd, *J* = 5.6/2.7 Hz, 1H), 4.22 (dd, *J* = 17.6/1.9 Hz, 1H), 4.37-4.50 (m, 3H), 5.28-5.31 (m, 1H), 5.34-5.41 (m, 1H), 5.97-6.08 (m, 1H), 6.35 (dd, *J* = 2.4/1.9 Hz, 1H)

### Preparative method

Column: CHIRALPAK®ID 5 µm - 250 x 30 mm
Mobile phase: Heptane/DCM 50/50
Flow rate: 42.5ml/min
Detection: UV 230 nm
Temperature: 25°C

### Analytical method:

Column: CHIRALPAK® ID 5 µm - 250 x 4.6 mm
Mobile phase: Heptane/DCM 50/50
Flow rate: 1 ml/min
Detection: DAD 275 nm
Temperature: 25°C

### Step 2: preparation of sodium [(5R)-7-oxo-3-pyrazol-1-yl-1,6-diazabicyclo[3.2.1]oct-3-en-6-yl] sulfate (Example 15)

Using the procedure described in example 11 (step 2), the intermediate (5*R*)-6-allyloxy-3-pyrazol-1-yl-1,6-diazabicyclo[3.2.1]oct-3-en-7-one (15a) (1.026 g, 4.17 mmol) was converted into sodium [(5*R*)-7-oxo-3-pyrazol-1-yl-1,6-diazabicyclo[3.2.1]oct-3-en-6-yl] sulfate (Example 15) (0.877 g, 2.84 mmol, 68%) after lyophilization.
MS *m*/*z* ([M-H]⁻) 285.
1H NMR (400 MHz, D₂O): *δ* (ppm) 3.48 (d, *J* = 11.3 Hz, 1H), 3.71 (dd, *J* = 11.3/2.8 Hz, 1H), 4.34-4.46 (m, 2H), 4.60 (dd, *J* = 5.6/2.7 Hz, 1H), 6.52 (t, *J* = 2.3 Hz, 1H), 6.63 (d, *J* = 5.6 Hz, 1H), 7.73 (d, *J* = 1.8 Hz, 1H), 7.95 (d, *J* = 2.7 Hz, 1H).

### Example 16: synthesis of sodium [(5R)-7-oxo-3-(triazol-1-yl)-1,6-diazabicyclo[3.2.1]oct-3-en-6-yl]sulfate

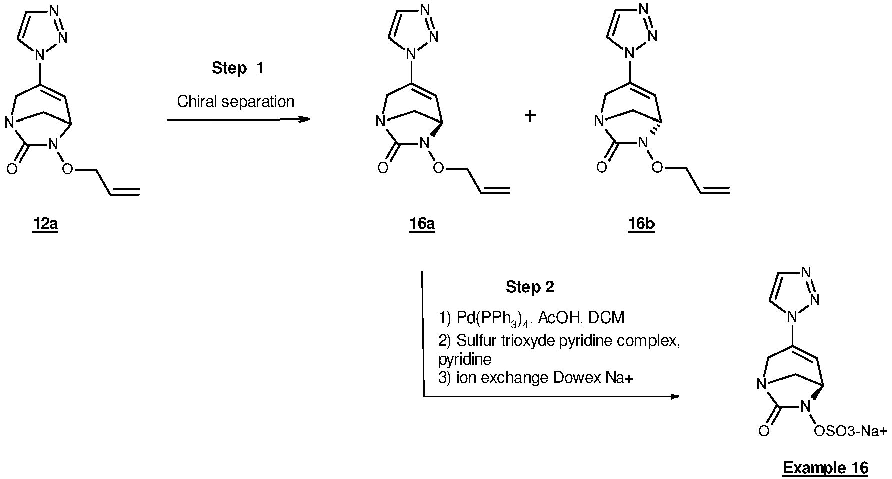

### Step 1: preparation of intermediate (5R)-6-allyloxy-3-(triazol-1-yl)-1,6-diazabicyclo[3.2.1]oct-3-en-7-one (16a) and (5S)-6-allyloxy-3-(triazol-1-yl)-1,6-diazabicyclo[3.2.1]oct-3-en-7-one (16b)

Both enantiomers of 6-allyloxy-3-(triazol-1-yl)-1,6-diazabicyclo[3.2.1]oct-3-en-7-one (12a) (1021 mg, 4.13 mmol) were separated using Supercritical fluid chromatography (LUX C4 5 µm, 250x21.2 mm, iPrOH/CO₂ 35/65, 50 mL/min) to provide (5*R*)-6-allyloxy-3-(triazol-1-yl)-1,6-diazabicyclo[3.2.1]oct-3-en-7-one (16a) (455 mg , 1.84 mmol, 44%, 98.9% ee, retention time 2.23 min) and (5*S*)-6-allyloxy-3-(triazol-1-yl)-1,6-diazabicyclo[3.2.1]oct-3-en-7-one (16b) (482 mg, 1.95 mmol, 47%, 97.6%ee, retention time 2.48 min)
MS *m*/*z* ([M+H]+) 248.
¹H NMR (400 MHz, CDCl3): δ (ppm) 3.20 (d, *J* = 11.0 Hz, 1H), 3.54-3.63 (m, 1H), 4.16 (dd, *J* = 5.5/2.7 Hz, 1H), 4.34 (dd, *J* = 17.9/2.0 Hz, 1H), 4.38-4.57 (m, 3H), 5.30-5.35 (m, 1H), 5.38 (dq, *J* = 17.2/1.4 Hz, 1H), 5.95-6.10 (m, 1H), 6.62-6.70 (m, 1H), 7.73 (d, *J* = 1.2 Hz, 1H), 7.76 (d*, J* = 1.2 Hz, 1H).
¹H NMR (300 MHz, D₂O): *δ* (ppm) 3.49 (d, *J* = 11.4 Hz, 1H), 3.71 (dd, *J* = 11.4, 2.8 Hz, 1H), 4.42 (dd, *J* = 17.7, 1.3 Hz, 1H), 4.51 (dd, *J* = 17.7, 1.9 Hz, 1H), 4.63 (dd, *J* = 5.6, 2.7 Hz, 1H), 6.90 (d, *J* = 5.7 Hz, 1H), 7.82 (d, *J* = 1.3 Hz, 1H), 8.24 (d, *J* = 1.3 Hz, 1H).

### Preparative method

Column: Lux C4 (21.2mm x 250mm, 5µm)
Isocratic Conditions 35:65 IPA:CO₂
Flow rate: 50ml/min
Detection: UV 242 nm
BPR 125 BarG

### Analytical method :

Column: Lux C4 (4.6mm x 250mm, 5µm)
Isocratic Conditions 35:65 IPA:CO₂
Flow rate: 4 ml/min
Detection: 210-400 nm
BPR 125 BarG
Column Temperature 40°C

### Step 2: preparation of sodium [(5R)-7-oxo-3-(triazol-1-yl)-1,6-diazabicyclo[3.2.1]oct-3-en-6-yl] sulfate (Example 16)

Using the procedure described in example 12 (step 2), the intermediate (5*R*)-6-allyloxy-3-(triazol-1-yl)-1,6-diazabicyclo[3.2.1]oct-3-en-7-one (16a) (422 mg, 1.70 mmol) was converted into sodium [(5*R*)-7-oxo-3-(triazol-1-yl)-1,6-diazabicyclo[3.2.1]oct-3-en-6-yl] sulfate (Example 16) (0.243 g, 0.785 mmol, 46%) after lyophilization.
MS *m*/*z* ([M-H]⁻) 286.
¹H NMR (300 MHz, D₂O): *δ* (ppm) 3.49 (d, *J* = 11.4 Hz, 1H), 3.71 (dd, *J* = 11.4, 2.8 Hz, 1H), 4.42 (dd, *J* = 17.7, 1.3 Hz, 1H), 4.51 (dd, *J* = 17.7, 1.9 Hz, 1H), 4.63 (dd, *J* = 5.6, 2.7 Hz, 1H), 6.90 (d, *J* = 5.7 Hz, 1H), 7.82 (d, *J* = 1.3 Hz, 1H), 8.24 (d, *J* = 1.3 Hz, 1H).

### Method 1: MIC of compounds alone and combined with antibacterials (Table 1) against bacterial isolates (Tables 2 and 3)

Compounds of the present invention were assessed against genotyped bacterial strains alone or in combination with an antibacterial. In the assays, MICs of said compounds or combination of antibiotics with fixed concentrations of said compounds (4 or 8 µg/mL) were determined by the broth microdilution method according to the Clinical Laboratory Standards Institute (CLSI - M7-A7). Briefly, compounds alone according to the invention were prepared in DMSO and spotted (2µL each) on sterile polystyrene plates (Corning, 3788). Combinations of compounds and antibiotics dilutions were prepared in DMSO and spotted (1µL each) on sterile polystyrene plates (Corning, 3788). Log phase bacterial suspensions were adjusted to a final density of 5.10⁵ CFU/mL in cation-adjusted Mueller-Hinton broth (ca-MHB; Becton-Dickinson and Company) and added to each well (98µL). Assays including Fosfomycin were supplemented with 25µg/ml of Glucose-6-phosphate. Microplates were incubated for 16-20 h at 35 °C in ambient air. The MIC of the compounds was defined as the lowest concentration of said compounds that prevented bacterial growth as read by visual inspection. The MIC of ATB at each compound concentration was defined as the lowest concentration of ATB that prevented bacterial growth as read by visual inspection. Results are presented in Tables 4 to 11.

**Table 1: Antibacterials used in combination studies**

| **Abbreviations - Antibiotics** | | | |
|---|---|---|---|
| ATB | Antibiotic | FOS | Fosfomycin |
| AMX | Amoxicillin | FUR | Cefuroxime |
| AN | Amikacin | IMI | Imipenem |
| ATM | Aztreonam | LZD | Linezolid |
| CIP | Ciprofloxacin | MEM | Meropenem |
| CLI | Clindamycin | PIP | Piperacillin |
| CM | Chloramphenicol | POD | Cefpodoxime |
| COL | Colistin | RIF | Rifampicine |
| CPO | Cefpirome | SYN | Synercid |
| CTR | Ceftriaxone | TGC | Tigecycline |
| CTX | Cefotaxime | VAN | Vancomycin |
| DAP | Daptomycin | SUL | Sulbactam |
| ERY | Erythromycin | CRO | Ceftaroline |
| FEP | Cefepime | S-266 | Cefiderocol (S-649266) |
| FIX | Cefixime | BAL | BAL30072 |

**Table 2: Bacterial species used in MIC determination**

| Abbreviations - Strains | |
|---|---|
| ECO | *E. coli* |
| KPN | *K. pneumoniae* |
| ECL | *E. cloacae* |
| PAE | *P. aeruginosa* |
| SAU | *S. aureus* |
| SPN | *S. pneumoniae* |
| ABA | *A. baumannii* |

**Table 3: List of the bacterial isolates and their resistance genotype**

| **Strains ID** | | **Resistance genotype** |
|---|---|---|
| ABA | 19606 | - |
| ABA | 107292 | OXA-40 |
| ABA | UFR151 | AmpC wt, ISAbaI- |
| ABA | UFR152 | AmpC |
| ABA | UFR153 | VEB-1, OXA-10, OXA-69, arr-2 |
| ABA | UFR154 | OXA-21 |
| ABA | UFR155 | OXA-25 |
| ABA | UFR156 | GES-11, OXA-23 |
| ABA | UFR157 | GES-14 |
| ABA | UFR158 | NDM-1 |
| ABA | UFR159 | OXA-23, OXA-69, NDM-1 |
| ABA | UFR160 | TEM-1, OXA-23, OXA-69, NDM-1, ADC |
| ABA | UFR161 | NDM-2 |
| ECO | 190317 | TEM-1, SHV-12, CTX-M-15, OXA-1 |
| KPN | UFR68 | TEM-1, SHV-11, CTX-M-15, KPC-3 |
| KPN | 6299 | TEM-1, SHV-11, OXA-163 |
| KPN | BAA-1898 | TEM-1, SHV-11, SHV-12, KPC-2 |
| ECL | P99 | AmpC |
| ECO | UFR39 | CTX-M-15, NDM-1 |
| KPN | UFR42 | SHV-2, CTX-M-15, OXA-1, OXA-181, NDM-1 |
| PAE | 27853 | - |
| PAE | UFR90 | AmpC, oprD- |
| PAE | UFR48 | VIM-2 |
| PAE | 105250 | OXA-15 |
| SAU | 29213 | - |
| SPN | 49619 | - |

**Table 4: MIC of the compounds of the present invention against a set of Gram positive and Gram negative isolates.**

| | MIC (µg/mL) | | | | | | | | | | | | | | |
|---|---|---|---|---|---|---|---|---|---|---|---|---|---|---|---|
| Strain | Example 1 | Example 2 | Example 3 | Example 4 | Example 5 | Example 6 | Example 7 | Example 8 | Example 9 | Example 10 | Example 11 | Example 12 | Example 13 | Example 14 | Example 15 |
| ECO 190317 | 0.031 | 4 | 0.25 | 0.25 | <=0.016 | 1 | 4 | 4 | 0.5 | 0.5 | 1 | 1 | 4 | 1 | 1 |
| KPN BAA-1898 | >32 | >32 | 16 | >32 | 16 | 4 | 8 | 8 | 2 | 16 | 8 | 2 | 16 | 4 | 4 |
| ECL P99 | 0.031 | 16 | 0.5 | 0.063 | <=0.016 | 4 | 8 | 8 | 2 | 2 | 4 | 1 | 16 | 4 | 2 |
| ECO UFR39 | 0.031 | 4 | 0.5 | 0.25 | <=0.016 | 2 | 8 | 4 | 2 | 2 | 4 | 1 | 8 | 2 | 2 |
| KPN UFR42 | >32 | >32 | 16 | >32 | 32 | 4 | 16 | 16 | 8 | >32 | 8 | 4 | 16 | 8 | 4 |
| PAE UFR90 | 0.5 | >128 | 8 | 2 | 0.25 | >32 | >32 | >128 | >128 | 4 | >32 | >32 | - | - | >32 |
| PAE UFR48 | 4 | 64 | 8 | 4 | 2 | >32 | >32 | >128 | 128 | 4 | >32 | >32 | - | - | >32 |
| PAE 105250 | >32 | >32 | 8 | 32 | 4 | >32 | >32 | >32 | >32 | 4 | >32 | >32 | - | - | >32 |
| MSSA 29213 | >32 | >32 | 32 | >32 | >32 | >32 | 32 | 8 | 8 | 32 | 8 | 8 | 32 | 4 | 4 |
| SPN 49619 | >32 | >32 | 1 | 32 | >32 | 16 | 16 | 4 | 8 | 1 | 16 | 8 | >32 | 8 | 8 |

**Table 5: MIC of Antibiotics against a set of Gram negative isolates.**

| | **MIC (µg/mL) / Bacterial strains** | | | | |
|---|---|---|---|---|---|
| **Compounds** | **PAE UFR90** | **PAE UFR48** | **KPN UFR68** | **ECL P99** | **ECO 190317** |
| **MEM** | 2 | 4 | 32 | 0.063 | 0.031 |
| **ATM** | 32 | 256 | >512 | 32 | 128 |
| **PIP** | 256 | 256 | >1024 | 512 | >1024 |
| **FOS** | 16 | 8 | ≥32 | >1024 | 0.5 |
| **FEP** | 16 | 64 | >256 | 2 | 512 |

**Table 6: MIC of the compounds of the present invention against a set of A. baumannii strains.**

| | MIC (µg/mL) | | | | | | | |
|---|---|---|---|---|---|---|---|---|
| Strain | Example 1 | Example 3 | Example 6 | Example 9 | Example 10 | Example 11 | Example 12 | Example 15 |
| ABA 19606 | >32 | 16 | 16 | >32 | 32 | >32 | 32 | >32 |
| ABA 107292 | >32 | >32 | >32 | >32 | >32 | >32 | >32 | >32 |
| ABA UFR151 | >32 | >32 | 32 | >32 | >32 | >32 | >32 | >32 |
| ABA UFR152 | >32 | >32 | 32 | >32 | 32 | >32 | >32 | >32 |
| ABA UFR153 | >32 | >32 | 16 | >32 | 32 | >32 | >32 | >32 |
| ABA UFR154 | >32 | >32 | 32 | >32 | >32 | >32 | >32 | >32 |
| ABA UFR155 | >32 | >32 | 16 | >32 | >32 | >32 | >32 | >32 |
| ABA UFR156 | >32 | >32 | 16 | >32 | >32 | >32 | >32 | >32 |
| ABA UFR157 | >32 | >32 | 8 | >32 | >32 | >32 | >32 | >32 |
| ABA UFR158 | >32 | >32 | 16 | >32 | >32 | >32 | >32 | >32 |
| ABA UFR159 | >32 | >32 | >32 | >32 | >32 | >32 | >32 | >32 |
| ABA UFR160 | >32 | >32 | 16 | >32 | >32 | >32 | >32 | >32 |
| ABA UFR161 | >32 | >32 | 32 | >32 | >32 | >32 | >32 | >32 |

**Table 7: MIC of antibiotics and of Example 1 against a set of P. aeruginosa strains.**

| **MIC (µg/ml)** | PAE 27853 | PAE UFR48 | PAE UFR90 |
|---|---|---|---|
| **FOS** | 32 | 32 | 8 |
| **AN** | 2 | 32 | 2 |
| **CIP** | 0.25 | 8 | 1 |
| **Example 1** | 1 | 4 | 0.5 |

**Table 8: MIC of a list of antibiotics alone, or combined with compounds of the present invention at 4 or 8 µg/mL against a set of bacterial isolates.**

| | | | MIC ATB (µg/mL) combined with : | | | | | | | | |
|---|---|---|---|---|---|---|---|---|---|---|---|
| ATB | Strain | MIC ATB alone (µg/mL) | Example 1 @8µg/ml | Example 9 @4µg/ml | Example 2 @4µg/ml | Example 3 @8µg/ml | Example 4 @4µg/ml | Example 5 @4µg/ml | Example 6 @8µg/ml | Example 7 @8µg/ml | Example 8 @8µg/ml |
| PIP | ECO 190317 | >1024 | <0.031 | <0.031 | <0.031 | <0.031 | <0.031 | <0.031 | <0.031 | <0.031 | <0.031 |
| | KPN 6299 | >1024 | 4 | <0.031 | 2 | 1 | 4 | >32 | <0.031 | 0.25 | 2 |
| | KPN BAA-1898 | >1024 | 8 | <0.031 | >32 | <=0.031 | >32 | 1 | <0.031 | 2 | 1 |
| | ECL P99 | 512 | 0.063 | <0.031 | 2 | <0.031 | <=0.031 | <=0.031 | <0.031 | 0.125 | 0.125 |
| | ECO UFR39 | >1024 | 0.063 | <0.031 | 0.063 | 0.063 | 0.063 | 0.063 | <0.031 | <0.031 | <0.031 |
| | KPN UFR42 | >256 | >32 | 16 | >32 | >32 | >32 | >32 | <=0.031 | >32 | >32 |
| | PAE UFR90 | 256 | <0.031 | 16 | >32 | <0.031 | <0.031 | <0.031 | >32 | >32 | >32 |
| | PAE UFR48 | 256 | <0.031 | 32 | 128 | <0.031 | <0.031 | <0.031 | >32 | >32 | >32 |
| | PAE 105250 | 128 | <0.031 | 32 | >32 | 0.063 | <=0.031 | <0.031 | >32 | >32 | >32 |
| | SAU 29213 | 8 | 5 | <0.031 | 1 | 0.5 | 4 | 2 | 0.063 | 1 | <0.031 |
| | SPN 49619 | 0.5 | 0.5 | <0.031 | 0.5 | <0.031 | 0.5 | 0.5 | <0.031 | 0.5 | <0.031 |

| ATB | Strain | MIC ATB alone (µg/mL | Example 1 @8µg/ml | Example 9 @4µg/ml | Example 2 @4µg/ml | Example 3 @8µg/ml | Example 4 @4µg/ml | Example 5 @4µg/ml | Example 6 @8µg/ml | Example 7 @8µg/ml | Example 8 @8µg/ml |
|---|---|---|---|---|---|---|---|---|---|---|---|
| MEM | ECO 190317 | 0.031 | <0.031 | <0.031 | <0.031 | <0.031 | <0.031 | <0.031 | <0.031 | <0.031 | <0.031 |
| | KPN 6299 | 1 | 0.125 | 0.063 | 0.25 | 0.063 | 0.25 | 0.5 | <0.031 | 0.063 | 0.125 |
| | KPN BAA-1898 | 128 | 0.125 | <0.031 | 0.125 | <=0.031 | 0.5 | 0.063 | <0.031 | <=0.031 | <=0.031 |
| | ECL P99 | 0.063 | <0.031 | <0.031 | 0.0078 | <0.031 | <0.031 | <0.031 | <0.031 | <=0.031 | <=0.031 |
| | ECO UFR39 | 64 | <0.031 | <0.031 | <0.031 | <=0.031 | <0.031 | <=0.031 | <0.031 | <0.031 | <0.031 |
| | KPN UFR42 | 128 | >32 | 4 | >32 | >32 | >32 | >32 | <0.031 | 8 | >32 |
| | PAE UFR90 | 2 | <0.031 | 1 | 1 | <0.031 | <0.031 | <0.031 | 1 | 2 | 2 |
| | PAE UFR48 | 4 | <0.031 | 2 | 4 | <=0.031 | <0.031 | <0.031 | 4 | 4 | 4 |
| | PAE 105250 | 4 | <0.031 | 4 | 2 | 0.063 | <0.031 | <0.031 | 2 | 4 | 2 |
| | SAU 29213 | 0.125 | 0.125 | <0.031 | 0.125 | 0.063 | 0.125 | <=0.031 | <=0.031 | <=0.031 | <0.031 |
| | SPN 49619 | 0.063 | 0.063 | <=0.031 | 0.063 | <0.031 | 0.063 | 0.063 | <0.031 | <=0.031 | <0.031 |
| ATM | ECO 190317 | 128 | <0.031 | <0.031 | <0.031 | <0.031 | <0.031 | <0.031 | <0.031 | <0.031 | <0.031 |
| | KPN 6299 | 32 | 0.063 | <0.031 | 0.063 | <=0.031 | 0.063 | 1 | <0.031 | <=0.031 | <=0.031 |
| | KPN BAA-1898 | >512 | 0.25 | <0.031 | 0.25 | <=0.031 | 8 | 0.25 | <0.031 | 0.063 | <=0.031 |
| | ECL P99 | 32 | <=0.031 | 1 | <=0.125 | <0.031 | <0.031 | <0.031 | <0.031 | <0.031 | <0.031 |
| | ECO UFR39 | >512 | <0.031 | <0.031 | <=0.031 | <0.031 | <0.031 | <0.031 | <0.031 | <0.031 | <0.031 |
| | KPN UFR42 | 256 | 1 | 0.063 | 0.063 | <=0.031 | 1 | 0.5 | <0.031 | <=0.031 | 0.063 |
| | PAE UFR90 | 32 | <0.031 | 128 | 16 | <0.031 | <0.031 | <0.031 | 8 | 16 | 16 |
| | PAE UFR48 | 256 | <0.031 | 128 | 8 | 2 | <0.031 | <0.031 | 8 | >32 | 16 |
| | PAE 105250 | 8 | <0.031 | 8 | 8 | <0.031 | <0.031 | <0.031 | 4 | 8 | 8 |
| | SAU 29213 | >512 | >32 | <=0.031 | >32 | >32 | >32 | >32 | >32 | >32 | <0.031 |
| | SPN 49619 | >64 | >32 | <0.031 | >32 | <0.031 | >32 | >32 | <0.031 | >32 | <=0.031 |

| ATB | Strain | MIC ATB alone (µg/mL) | Example 1 @8µg/ml | Example 9 @4µg/ml | Example 2 @4µg/ml | Example 3 @8µg/ml | Example 4 @4µg/ml | Example 5 @4µg/ml | Example 6 @8µg/ml | Example 7 @8µg/ml | Example 8 @8µg/ml |
|---|---|---|---|---|---|---|---|---|---|---|---|
| FEP | ECO 190317 | 512 | <0.031 | <0.031 | <0.031 | <0.031 | <0.031 | <0.031 | <0.031 | <0.031 | <0.031 |
| | KPN 6299 | 128 | 0.125 | <=0.031 | 0.125 | <=0.031 | 0.125 | 2 | <0.031 | <=0.031 | 0.063 |
| | KPN BAA-1898 | 64 | 0.063 | <0.031 | 0.125 | <=0.031 | 0.5 | <=0.031 | <0.031 | <=0.031 | <0.031 |
| | ECL P99 | 2 | <0.031 | 0.016 | 0.016 | <0.031 | <0.031 | <0.031 | <0.031 | <=0.031 | <0.031 |
| | ECO UFR39 | >128 | <0.031 | <0.031 | <0.031 | <0.031 | <0.031 | <0.031 | <0.031 | <0.031 | <0.031 |
| | KPN UFR42 | 128 | >32 | >32 | 32 | >32 | >32 | >32 | <0.031 | 16 | >32 |
| | PAE UFR90 | 16 | <0.031 | 4 | 8 | <0.031 | <0.031 | <0.031 | 4 | 4 | 4 |
| | PAE UFR48 | 64 | <0.031 | 32 | 32 | <0.031 | <0.031 | <0.031 | 32 | 32 | 32 |
| | PAE 105250 | 8 | <0.031 | 8 | 4 | <0.031 | <=0.031 | <0.031 | 8 | 8 | 4 |
| | SAU 29213 | 4 | 4 | <0.031 | 4 | 1 | 4 | 2 | <=0.031 | 1 | <=0.031 |
| | SPN 49619 | 0.063 | 0.063 | <=0.031 | 0.063 | <0.031 | 0.063 | 0.063 | <0.031 | <=0.031 | <0.031 |
| FOS | ECO 190317 | 0.5 | <0.125 | <0.125 | <0.125 | <0.125 | <0.125 | <0.125 | <0.125 | <0.125 | <0.125 |
| | KPN 6299 | >128 | 64 | 0.5 | 128 | 16 | 64 | 64 | 0.25 | 16 | 64 |
| | KPN BAA-1898 | 64 | 16 | <0.125 | 32 | <0.125 | 32 | 8 | <=0.125 | 8 | 4 |
| | ECL P99 | >1024 | 0.5 | 8 | 16 | <0.125 | <0.125 | <0.125 | <0.125 | 16 | <0.125 |
| | ECO UFR39 | 4 | <0.125 | <0.125 | <0.125 | <0.125 | <0.125 | <0.125 | <0.125 | <0.125 | <0.125 |
| | KPN UFR42 | 128 | 32 | 2 | 32 | 16 | 32 | 16 | <0.125 | 2 | 8 |
| | PAE UFR90 | 8 | <0.125 | 4 | 4 | <0.125 | <0.125 | <0.125 | 8 | 8 | 8 |
| | PAE UFR48 | 16 | 2 | 32 | 16 | 2 | <0.125 | 4 | 32 | 32 | 32 |
| | PAE 105250 | 256 | 4 | >128 | >128 | 16 | 8 | 16 | 128 | >128 | 128 |
| | SAU 29213 | 2 | 1 | <0.125 | 0.5 | <=0.125 | 2 | 0.5 | <=0.125 | 0.5 | 0.25 |
| | SPN 49619 | 32 | 32 | 4 | 16 | <=0.125 | 32 | 16 | <0.125 | 8 | <0.125 |

**Table 9 : MIC of a list of antibiotics alone, or combined with compounds of the present invention at 4 or 8 µg/mL against a set of bacterial isolates.**

| | | | MIC ATB (µg/mL) combined with : | |
|---|---|---|---|---|
| ATB | Strain | MIC ATB alone (µg/mL) | Example 1 @8µg/ml | Example 9 @4µg/ml |
| AN | ECO 190317 | 8 | <0.031 | <0.031 |
| | KPN 6299 | 16 | 16 | <0.031 |
| | KPN BAA-1898 | 1 | 0.25 | <0.031 |
| | ECL P99 | 0.063 | <0.031 | <0.031 |
| | ECO UFR39 | >32 | <=0.031 | <0.031 |
| | KPN UFR42 | >32 | >32 | >32 |
| | PAE UFR90 | 2 | <0.031 | 2 |
| | PAE UFR48 | 32 | <0.031 | 32 |
| | PAE 105250 | 2 | <0.031 | 2 |
| | SAU 29213 | 2 | 4 | <0.031 |
| | SPN 49619 | 32 | 32 | <0.031 |
| TGC | ECO 190317 | 0.25 | <0.031 | <0.031 |
| | KPN 6299 | 1 | 1 | 0.063 |
| | KPN BAA-1898 | 1 | 1 | <0.031 |
| | ECL P99 | 0.5 | <0.031 | <0.031 |
| | ECO UFR39 | 0.5 | <0.031 | <0.031 |
| | KPN UFR42 | 2 | 2 | 0.25 |
| | PAE UFR90 | 16 | <0.031 | 16 |
| | PAE UFR48 | 32 | <0.031 | 32 |
| | PAE 105250 | 8 | 0.5 | 8 |
| | SAU 29213 | 0.25 | 0.125 | <0.031 |
| | SPN 49619 | 0.125 | 0.125 | <0.031 |
| CM | ECO 190317 | 8 | <0.031 | <0.031 |
| | KPN 6299 | >32 | >32 | >32 |
| | KPN BAA-1898 | >32 | >32 | <0.031 |
| | ECL P99 | 512 | <0.031 | <0.031 |
| | ECO UFR39 | 64 | <0.031 | <0.031 |
| | KPN UFR42 | >32 | >32 | >32 |
| | PAE UFR90 | >1024 | <0.031 | >32 |
| | PAE UFR48 | 512 | <0.031 | >32 |
| | PAE 105250 | >32 | >32 | >32 |
| | SAU 29213 | 16 | 16 | <0.031 |
| | SPN 49619 | 4 | 4 | <0.031 |
| COL | ECO 190317 | 0.25 | <0.031 | <0.031 |
| | KPN 6299 | 0.25 | 0.25 | <0.031 |
| | KPN BAA-1898 | 0.25 | 0.25 | <0.031 |
| | ECL P99 | 0.25 | 0.125 | <0.031 |
| | ECO UFR39 | 0.25 | <0.031 | <0.031 |
| | KPN UFR42 | 0.25 | 0.25 | <0.031 |
| | PAE UFR90 | 1 | <0.031 | 1 |
| | PAE UFR48 | 1 | <0.031 | 1 |
| | PAE 105250 | 1 | <0.031 | 1 |
| | SAU 29213 | 256 | >32 | <0.031 |
| | SPN 49619 | 256 | >32 | <0.031 |
| AMX | ECO 190317 | >1024 | <0.031 | <0.031 |
| | KPN 6299 | >1024 | >32 | <=0.5 |
| | KPN BAA-1898 | >1024 | >32 | <0.031 |
| | ECL P99 | >1024 | <0.031 | <0.031 |
| | ECO UFR39 | >1024 | <0.031 | <0.031 |
| | KPN UFR42 | >256 | >32 | >32 |
| | PAE UFR90 | >32 | <0.031 | >32 |
| | PAE UFR48 | >1024 | <0.031 | >32 |
| | PAE 105250 | >1024 | 0.125 | >32 |
| | SAU 29213 | 2 | 0.25 | <0.031 |
| | SPN 49619 | 0.125 | 0.125 | <0.031 |
| IMI | ECO 190317 | 0.125 | <0.031 | <0.031 |
| | KPN 6299 | 0.25 | 1 | <0.031 |
| | KPN BAA-1898 | 64 | 2 | <0.031 |
| | ECL P99 | 0.25 | <0.031 | <0.031 |
| | ECO UFR39 | 16 | <0.031 | <0.031 |
| | KPN UFR42 | 32 | >32 | 32 |
| | PAE UFR90 | 2 | <0.031 | 1 |
| | PAE UFR48 | 8 | <0.031 | 8 |
| | PAE 105250 | 1 | <=0.031 | 1 |
| | SAU 29213 | 0.016 | <=0.031 | <0.031 |
| | SPN 49619 | 0.063 | <=0.031 | <0.031 |
| FUR | ECO 190317 | >512 | <0.031 | <0.031 |
| | KPN 6299 | >512 | 8 | <0.031 |
| | KPN BAA-1898 | >512 | 2 | <0.031 |
| | ECL P99 | >512 | <=0.031 | <0.031 |
| | ECO UFR39 | >32 | >32 | <0.031 |
| | KPN UFR42 | >32 | >32 | >32 |
| | PAE UFR90 | >32 | <0.031 | >32 |
| | PAE UFR48 | >32 | <0.031 | >32 |
| | PAE 105250 | >512 | <0.031 | >32 |
| | SAU 29213 | 2 | 1 | <0.031 |
| | SPN 49619 | 0.25 | 0.25 | <=0.031 |
| FIX | ECO 190317 | 256 | <0.031 | <0.031 |
| | KPN 6299 | 16 | 0.063 | <=0.031 |
| | KPN BAA-1898 | >512 | 0.25 | <0.031 |
| | ECL P99 | >512 | 0.063 | <0.031 |
| | ECO UFR39 | >512 | >32 | <0.031 |
| | KPN UFR42 | >256 | >32 | >32 |
| | PAE UFR90 | >32 | <0.031 | >32 |
| | PAE UFR48 | >512 | <0.031 | >32 |
| | PAE 105250 | >512 | <0.031 | >32 |
| | SAU 29213 | 16 | 16 | <=0.031 |
| | SPN 49619 | 1 | 1 | 0.25 |
| CTR | ECO 190317 | >32 | <0.031 | <0.031 |
| | KPN 6299 | >32 | 0.063 | 0.063 |
| | KPN BAA-1898 | >32 | 0.25 | <0.031 |
| | ECL P99 | >32 | <0.031 | <0.031 |
| | ECO UFR39 | >32 | >32 | <0.031 |
| | KPN UFR42 | >32 | >32 | >32 |
| | PAE UFR90 | >32 | <0.031 | >32 |
| | PAE UFR48 | >32 | <0.031 | >32 |
| | PAE 105250 | >32 | <0.031 | >32 |
| | SAU 29213 | 8 | 4 | <0.031 |
| | SPN 49619 | 0.063 | 0.063 | <=0.031 |
| CTX | ECO 190317 | >128 | <0.031 | <0.031 |
| | KPN 6299 | 128 | 0.25 | <=0.031 |
| | KPN BAA-1898 | >128 | 0.125 | <0.031 |
| | ECL P99 | >128 | <0.031 | <0.031 |
| | ECO UFR39 | >32 | >32 | <0.031 |
| | KPN UFR42 | >32 | >32 | >32 |
| | PAE UFR90 | >32 | <0.031 | >32 |
| | PAE UFR48 | >32 | <0.031 | >32 |
| | PAE 105250 | 128 | <0.031 | 32 |
| | SAU 29213 | 2 | 2 | <0.031 |
| | SPN 49619 | 0.063 | <=0.031 | <=0.031 |
| CPO | ECO 190317 | >256 | <0.031 | <0.031 |
| | KPN 6299 | >256 | 0.5 | <=0.031 |
| | KPN BAA-1898 | >256 | 0.125 | <0.031 |
| | ECL P99 | 16 | <0.031 | <0.031 |
| | ECO UFR39 | >32 | <0.031 | <0.031 |
| | KPN UFR42 | >32 | 16 | <0.031 |
| | PAE UFR90 | >32 | <0.031 | 8 |
| | PAE UFR48 | >32 | <0.031 | >32 |
| | PAE 105250 | 32 | <0.031 | 32 |
| | SAU 29213 | 1 | 1 | <0.031 |
| | SPN 49619 | <=0.031 | <=0.031 | <=0.031 |
| CIP | ECO 190317 | 0.5 | <0.031 | <0.031 |
| | KPN 6299 | 32 | 32 | <=0.031 |
| | KPN BAA-1898 | >32 | 16 | <0.031 |
| | ECL P99 | 0.016 | <0.031 | <0.031 |
| | ECO UFR39 | >256 | <0.031 | <0.031 |
| | KPN UFR42 | >32 | >32 | <0.031 |
| | PAE UFR90 | 1 | <0.031 | 1 |
| | PAE UFR48 | 4 | <0.031 | 4 |
| | PAE 105250 | 0.125 | <0.031 | 0.125 |
| | SAU 29213 | 0.5 | 0.5 | <0.031 |
| | SPN 49619 | 1 | 1 | 0.25 |
| LZD | SAU 29213 | 4 | 2 | <0.0078 |
| | SPN 49619 | 1 | 1 | <=0.0078 |
| ERY | SAU 29213 | 1 | 0.25 | <=0.0020 |
| | SPN 49619 | 0.063 | 0.063 | <0.0020 |
| CLI | SAU 29213 | 0.25 | 0.125 | <0.00098 |
| | SPN 49619 | 0.125 | 0.063 | 0.0039 |
| SYN | SAU 29213 | 0.5 | 0.5 | 0.0039 |
| | SPN 49619 | 0.5 | 0.5 | <=0.0020 |
| VAN | SAU 29213 | 1 | 1 | <0.0039 |
| | SPN 49619 | 0.25 | 0.25 | <0.0039 |
| DAP | SAU 29213 | 0.5 | 0.0078 | <0.0020 |
| | SPN 49619 | 0.125 | 0.125 | <0.0020 |
| RIF | SAU 29213 | 0.0078 | 0.0078 | 0.002 |
| | SPN 49619 | 0.031 | 0.063 | 0.00024 |

**Table 10 : MIC of a list of antibiotics alone, or combined with compounds of the present invention at 4 or 8 µg/mL against a set of Gram negative bacterial isolates.**

| | | | MIC ATB (µg/mL) combined with : | | |
|---|---|---|---|---|---|
| ATB | Strain | MIC ATB alone (µg/mL) | Example 7 @8µg/ml | Example 13 @8µg/ml | Example 15 @4µg/ml |
| AMX | ECO 190317 | >1024 | <0.25 | <0.25 | <0.25 |
| | KPN 6299 | >1024 | 2 | 2 | <0.25 |
| | KPN BAA-1898 | >1024 | >128 | >128 | <0.25 |
| | ECL P99 | >1024 | >128 | 128 | <0.25 |
| | ECO UFR39 | >1024 | <0.25 | <0.25 | <0.25 |
| | KPN UFR42 | >256 | >128 | >128 | <0.25 |
| FUR | ECO 190317 | >512 | <0.25 | <0.25 | <0.25 |
| | KPN 6299 | >512 | 2 | 2 | <=0.25 |
| | KPN BAA-1898 | >512 | 8 | 8 | <0.25 |
| | ECL P99 | >512 | 64 | 16 | <0.25 |
| | ECO UFR39 | >256 | <0.25 | <0.25 | <0.25 |
| | KPN UFR42 | >256 | >128 | >128 | 4 |
| POD | ECO 190317 | >512 | <0.25 | <0.25 | <0.25 |
| | KPN 6299 | 64 | <=0.25 | <=0.25 | <0.25 |
| | KPN BAA-1898 | >512 | 2 | 1 | <0.25 |
| | ECL P99 | >512 | 32 | 4 | <0.25 |
| | ECO UFR39 | >256 | <0.25 | <0.25 | <0.25 |
| | KPN UFR42 | >256 | >128 | >128 | <0.25 |
| FIX | ECO 190317 | >128 | <0.25 | <0.25 | <0.25 |
| | KPN 6299 | 16 | <=0.25 | <=0.25 | <0.25 |
| | KPN BAA-1898 | >128 | <=0.25 | <=0.25 | <0.25 |
| | ECL P99 | >128 | 4 | 8 | <0.25 |
| | ECO UFR39 | >128 | <0.25 | <0.25 | <0.25 |
| | KPN UFR42 | >128 | 128 | >128 | <=0.25 |

**Table 11: MIC of sulbactam, meropenem, cefepime alone, or combined with compounds of the present invention at 4 or 8 µg/mL against a set of A. baumanii isolates**

| | | | MIC ATB (µg/mL) combined with : | | | | | | | |
|---|---|---|---|---|---|---|---|---|---|---|
| ATB | Strain | MIC ATB alone (µg/mL) | Example 1 @8µg/ml | Example 3 @8µg/ml | Example 6 @8µg/ml | Example 9 @4µg/ml | Example 10 @8µg/ml | Example 11 @8µg/ml | Example 12 @8µg/ml | Example 15 @4µg/ml |
| MEM | ABA 19606 | 1 | 1 | <=0.25 | <=0.25 | 2 | <=0.25 | 0.5 | <=0.25 | 0.5 |
| | ABA 107292 | >128 | >128 | >128 | >128 | >128 | 128 | >128 | >128 | >128 |
| | ABA UFR151 | 0.5 | <=0.25 | <=0.25 | <=0.25 | <=0.25 | <=0.25 | 0.5 | <=0.25 | 0.5 |
| | ABA UFR152 | 2 | 4 | 1 | 2 | 1 | 1 | 1 | 1 | 1 |
| | ABA UFR153 | 1 | 1 | <=0.25 | <0.25 | <=0.25 | <=0.25 | 1 | <=0.25 | <=0.25 |
| | ABA UFR154 | 1 | 0.5 | <=0.25 | 0.5 | 0.5 | <=0.25 | 1 | <=0.25 | 0.5 |
| | ABA UFR155 | 32 | 16 | 8 | 16 | 16 | 8 | 16 | 16 | 64 |
| | ABA UFR156 | 64 | 64 | 8 | 16 | 64 | 8 | 64 | 32 | 64 |
| | ABA UFR157 | 32 | 1 | <=0.25 | <=0.25 | 1 | 0.5 | 0.5 | <=0.25 | 0.5 |
| | ABA UFR158 | >128 | >128 | 128 | <0.25 | >128 | 128 | >128 | >128 | >128 |
| | ABA UFR159 | >128 | >128 | >128 | >128 | >128 | >128 | >128 | >128 | >128 |
| | ABA UFR160 | >128 | >128 | 128 | <0.25 | >128 | >128 | >128 | 128 | >128 |
| | ABA UFR161 | >128 | >128 | >128 | >128 | >128 | >128 | >128 | >128 | >128 |
| FEP | ABA 19606 | 16 | 32 | 8 | <=0.25 | 16 | 8 | 16 | 8 | 16 |
| | ABA 107292 | 64 | 64 | 8 | 16 | 16 | 16 | 32 | 8 | 32 |
| | ABA UFR151 | 2 | 2 | 1 | <0.25 | 2 | 2 | 2 | 2 | 2 |
| | ABA UFR152 | 16 | 16 | 8 | 16 | 16 | 8 | 16 | 8 | 16 |
| | ABA UFR153 | >128 | 16 | 8 | <=0.25 | 16 | 8 | 16 | 8 | 16 |
| | ABA UFR154 | 2 | 2 | 1 | 1 | 1 | 0.5 | 1 | 1 | 2 |
| | ABA UFR155 | 128 | 64 | 32 | 16 | 32 | 64 | 64 | 64 | 32 |
| | ABA UFR156 | >128 | 32 | 8 | 8 | 32 | 8 | 32 | 32 | 32 |
| | ABA UFR157 | 32 | 8 | 4 | <0.25 | 8 | 8 | 8 | 4 | 8 |
| | ABA UFR158 | >128 | >128 | >128 | <0.25 | >128 | >128 | >128 | >128 | >128 |
| | ABA UFR159 | >128 | >128 | >128 | >128 | >128 | >128 | >128 | >128 | >128 |
| | ABA UFR160 | >128 | >128 | >128 | <0.25 | >128 | >128 | >128 | >128 | >128 |
| | ABA UFR161 | >128 | >128 | >128 | >128 | >128 | >128 | >128 | >128 | >128 |
| SUL | ABA 19606 | 1 | 1 | <=0.25 | <=0.25 | <=0.25 | <=0.25 | <=0.25 | <=0.25 | <=0.25 |
| | ABA 107292 | 64 | 4 | 2 | 8 | 8 | 4 | 32 | 2 | 4 |
| | ABA UFR151 | 2 | 0.5 | <=0.25 | <0.25 | 0.5 | <=0.25 | <=0.25 | <=0.25 | <=0.25 |
| | ABA UFR152 | 4 | 4 | 1 | 2 | 2 | 1 | 1 | 1 | 2 |
| | ABA UFR153 | 4 | 1 | <=0.25 | <=0.25 | 0.5 | <=0.25 | <=0.25 | <=0.25 | <=0.25 |
| | ABA UFR154 | 8 | 1 | 0.5 | <=0.25 | 0.5 | <=0.25 | <=0.25 | <=0.25 | 0.5 |
| | ABA UFR155 | 16 | 4 | 1 | 2 | 8 | 2 | 4 | 2 | 8 |
| | ABA UFR156 | 64 | 16 | 2 | 4 | 16 | 1 | 8 | 4 | 8 |
| | ABA UFR157 | 64 | 2 | 0.5 | <0.25 | 1 | 1 | 0.5 | 0.5 | 1 |
| | ABA UFR158 | 64 | 16 | 16 | <0.25 | 16 | 16 | 16 | 16 | 32 |
| | ABA UFR159 | 256 | 128 | 128 | 128 | 128 | 128 | 128 | 128 | 128 |
| | ABA UFR160 | >128 | 64 | 64 | <0.25 | 64 | 64 | 64 | 64 | 64 |
| | ABA UFR161 | 128 | 32 | 16 | 16 | 8 | 16 | 8 | 8 | 16 |

### Method 2: Determination of the Fractional Inhibitory Concentration Index

The antibacterial effects of a combination of compounds of the present invention with MEM, ATM, PIP, FOS, and FEP (Table 1) were assessed by the checkerboard assay. Serial 2-fold dilutions of each antibiotic tested were prepared in ca-MHB and mixed in each well of a 96-well microtiter plate so that each row (and column) contained a fixed amount of one agent and increasing amounts of the second agent. The range of drug concentrations used in the checkerboard analysis was such that the dilution range encompassed the MIC for each drug used in the analysis. Broth microdilution plates were inoculated with each strain (Table 3) to yield 5.10⁵ CFU/mL in a 100 µL final volume and incubated for 18 h at 35°C in ambient air. Assays including Fosfomycin were supplemented with 25µg/ml of Glucose-6-phosphate. MICs were determined after overnight incubation, and the fractional inhibitory concentration (FIC) index was calculated for each well. The FIC index (FICl) was calculated for each combination according to the equation FICI = FICa + FICb = (MIC of drug A in combination/MIC of drug A alone) + (MIC of drug B in combination/MIC of drug B alone). The FICl was interpreted as follows: synergy, FICl ≤0.5; indifference, 0.5 < FIC ≤ 4; and antagonism, FICl > 4. The checkerboard assays were performed in duplicate. Results are presented in Table 12.

**Table 12: FIC index of compounds of the present invention combined with antibiotics against Gram negative isolates**

| **Combinations of** | **FIC index min** | | | | |
|---|---|---|---|---|---|
| **Example 1** | **PAE UFR90** | **PAE UFR48** | **KPN UFR68** | **ECL P99** | **ECO 190317** |
| + MEM | 0.75 | 0.27 | ≤0.0098 | 0.56 | 0.63 |
| + ATM | 0.54 | 0.25 | ≤0.023 | 0.50 | 0.51 |
| + PIP | 0.88 | 0.27 | ≤0.012 | 0.50 | ≤0.38 |
| + FOS | 1.0 | 0.62 | ≤0.126 | ≤0.3125 | 0.37 |
| + FEP | 0.50 | 0.53 | ≤0.0088 | 0.47 | 0.49 |
| | | | | | |

| **Combinations of** | **FIC index min** | | | | |
|---|---|---|---|---|---|
| **Example 9** | **PAE UFR90** | **PAE UFR48** | **KPN UFR68** | **ECL P99** | **ECO 190317** |
| + MEM | ≤0.53 | 0.8 | 0.15 | 0.88 | 0.58 |
| + ATM | ≤0.30 | 0.14 | ≤0.27 | 0.53 | 0.20 |
| + PIP | ≤0.23 | 0.32 | ≤0.27 | 0.20 | ≤0.21 |
| + FOS | ≤0.52 | 1.0 | 0.50 | ≤0.19 | 0.69 |
| + FEP | ≤0.27 | 0.50 | ≤0.05 | 0.23 | ≤0.07 |
| | | | | | |

| **Combinations of** | **FIC index min** | | | | |
|---|---|---|---|---|---|
| **Example 3** | **PAE UFR90** | **PAE UFR48** | **KPN UFR68** | **ECL P99** | **ECO 190317** |
| + MEM | 0.75 | 0.75 | 0.16 | 0.54 | 0.56 |
| + ATM | 0.75 | 0.13 | 0.049 | 0.28 | 0.27 |
| + PIP | 0.63 | 0.31 | 0.10 | 0.13 | ≤0.5 |
| + FOS | 0.88 | 0.34 | 0.080 | ≤0.13 | 0.66 |
| + FEP | 0.32 | 0.50 | 0.021 | 0.31 | 0.19 |
| | | | | | |

| **Combinations of** | **FIC index min** | | | | |
|---|---|---|---|---|---|
| **Example 4** | **PAE UFR90** | **PAE UFR48** | **KPN UFR68** | **ECL P99** | **ECO 190317** |
| + MEM | 1.0 | 0.63 | ≤0.023 | 0.44 | 0.87 |
| + ATM | 0.53 | 0.50 | ≤0.023 | 0.63 | 0.57 |
| + PIP | 0.75 | 0.44 | ≤0.063 | 0.56 | ≤0.74 |
| + FOS | 0.63 | 1.20 | ≤0.035 | ≤0.25 | 0.53 |
| + FEP | 0.63 | 0.63 | ≤0.006 | 0.44 | 0.31 |

| **Example 5** | **PAE UFR90** | **PAE UFR48** | **KPN UFR68** | **ECL P99** | **ECO 190317** |
|---|---|---|---|---|---|
| + MEM | 0.81 | 0.63 | 0.020 | 0.63 | 0.76 |
| + ATM | 0.76 | 0.34 | ≤0.008 | 0.23 | 0.52 |
| + PIP | 0.56 | 0.52 | ≤0.035 | 0.32 | ≤0.56 |
| + FOS | 0.69 | 0.88 | 0.203 | 0.16 | 0.66 |
| + FEP | 0.69 | 0.33 | ≤0.0098 | 0.23 | 0.25 |
| | | | | | |

| **Combinations of** | **FIC index min** | | | | |
|---|---|---|---|---|---|
| **Example 2** | **PAE UFR90** | **PAE UFR48** | **KPN UFR68** | **ECL P99** | **ECO 190317** |
| + MEM | ≤0.38 | 0.8 | 0.03 | 0.50 | 0.50 |
| + ATM | ≤0.22 | 0.09 | 0.05 | 0.50 | 0.08 |
| + PIP | ≤0.15 | 0.31 | 0.09 | 0.14 | ≤0.25 |
| + FOS | ≤0.63 | 0.50 | 0.25 | ≤0.14 | 0.56 |
| + FEP | ≤0.28 | 0.63 | ≤0.0098 | 0.14 | ≤0.06 |
| | | | | | |

| **Combinations of** | **FIC index min** | | | | |
|---|---|---|---|---|---|
| **Example 8** | **PAE UFR90** | **PAE UFR48** | **KPN UFR68** | **ECL P99** | **ECO 190317** |
| + MEM | ≤0.56 | 1.0 | ≤0.07 | 0.75 | 0.57 |
| + ATM | ≤0.75 | ≤0.13 | ≤0.07 | 0.53 | 0.13 |
| + PIP | ≤0.25 | ≤0.31 | ≤0.07 | 0.16 | ≤0.14 |
| + FOS | ≤0.62 | ≤0.25 | 0.75 | ≤0.28 | 0.63 |
| + FEP | ≤0.25 | ≤0.5 | ≤0.08 | 0.25 | 0.13 |
| | | | | | |

| **Combinations of** | **FIC index min** | | | | |
|---|---|---|---|---|---|
| **Example 6** | **PAE UFR90** | **PAE UFR48** | **KPN UFR68** | **ECL P99** | **ECO 190317** |
| + MEM | | | 0.50 | | |
| + ATM | | | 1.0 | | |
| + PIP | | | ≤0.5 | | |
| + FOS | | | 0.28 | | |
| + FEP | | | 1.0 | | |

### Method 3: Determination of the bactericidal activity of the compounds alone and combined with antibiotics.

Time-dependent bactericidal activity was determined with the time-kill method standardized by CLSI guidelines. Bacteria (Table 3, inoculum = 5x10⁵ CFU/mL) were grown in ca-MHB for 24h in the presence of the compound of the present invention at defined concentrations or in combination with antibiotics (Table 1). Assays including Fosfomycin were supplemented with 25µg/ml of Glucose-6-phosphate. At time points of 0, 2, 4, 6, and 24 h after addition of the compound of the present invention, a sample was collected from the culture medium, serially diluted, spread onto Tryptic Soy Agar plate (TSA; Becton-Dickinson and Company), and cultured at 35°C in ambient air. The number of colonies grown on the plate was counted after 16-20 h. A bactericidal effect was defined as a decrease of ≥ 3 log₁₀ CFU/mL relative to the level seen with the control sample obtained at 0 h. Results are presented in Figures 1 to 3.

### Method 4: Determination of the spontaneous mutation frequency of the compounds alone and combined with antibiotics.

Bacterial isolates (Table 3) were grown overnight at 35°C on TSA plates (Becton-Dickinson and Company) in ambient air. After 24 h of incubation, isolated bacterial colonies were added to cation-adjusted Mueller-Hinton broth (ca-MHB; Becton-Dickinson and Company) and cultured for approximately 6 h at 35°C. The pre-culture was stopped once bacteria have reached an OD600nm of 0.5 for 200 µL. The bacterial suspension was centrifuged at 4000 rpm for 20 min. The supernatant was then discarded and the bacterial pellet was dissolved in ca-MHB to obtain 10⁹ to 10¹⁰ CFU/mL. Assays including Fosfomycin were supplemented with 25µg/ml of Glucose-6-phosphate. 100 µL of the adjusted bacterial suspension were spread on agar plates containing different concentrations of the compounds of the present invention alone or combined with an antibacterial (Table 1). The plates were incubated for 24-48 h at 35°C in ambient air. CFU were numerated on each agar plate containing compound. The number of resistant colonies obtained was divided by the starting inoculum to give the frequency of spontaneous resistance for a given incubation time (24 and 48 h). Results are presented in Table 13.

**Table 13: Frequency of spontaneous resistance of Example 1 and FOS, AN, CIP alone and combined against P. aeruginosa isolates.**

| **Strain** | **Resistance Mechanism** | **MIC (µg/mL)** | | **Frequency of spontaneous resistance** | | | | | |
|---|---|---|---|---|---|---|---|---|---|
| | | | | **Example 1** | | **AN** | | **AN** / **Example 1** | |
| | | **Example 1** | **AN** | 4.MIC | 8.MIC | 4.MIC | 8.MIC | 4.MIC/4.MIC | 8.MIC/8.MIC |
| PAE 27853 | None | 1 | 2 | **tntbc** | **tntbc** | 6.6E-07 | 1.7E-07 | 4.8E-08 | **<1.3E-09** |
| | | | | | | | | | |

| **Strain** | **Resistance Mechanism** | **MIC (µg/mL)** | | **Frequency of spontaneous resistance** | | | | | |
|---|---|---|---|---|---|---|---|---|---|
| | | | | **Example 1** | | **CIP** | | **CIP** / **Example 1** | |
| | | **Example 1** | **CIP** | 4.MIC | 8.MIC | 4.MIC | 8.MIC | 4.MIC/4.MIC | 8.MIC/8.MIC |
| PAE 27853 | None | 1 | 0.25 | **tntbc** | **tntbc** | 5.1 E-08 | 4.0E-09 | **<1.3E-09** | **<1.3E-09** |
| | | | | | | | | | |

| **Strain** | **Resistance Mechanism** | **MIC (µg/mL)** | | **Frequency of spontaneous resistance** | | | | | |
|---|---|---|---|---|---|---|---|---|---|
| | | | | **Example 1** | | **FOS** | | **FOS / Example 1** | |
| | | **Example 1** | **FOS** | 4.MIC | 8.MIC | 4.MIC | 8.MIC | 4.MIC/4.MIC | 8.MIC/8.MIC |
| PAE 27853 | None | 1 | 32 | **tntbc** | **tntbc** | 1.2E-06 | 1.4E-06 | 1.3E-08 | **<1.3E-09** |
| PAE UFR48 | VIM-2 | 4 | 32 | **tntbc** | **tntbc** | 1.1E-06 | 1.1E-06 | **<1.6E-09** | **<1.6E-09** |
| PAE UFR90 | ampC+, oprD- | 0.5 | 8 | **tntbc** | **tntbc** | 3.0E-07 | 2.0E-07 | 1.2E-07 | **<1.8E-09** |

| | | | | | | | | | |
|---|---|---|---|---|---|---|---|---|---|
| tntbc : Too numerous to be counted | | | | | | | | | |

## Claims

1. Composition comprising :
- at least one antibiotic chosen among: Amikacin, Ciprofloxacin, Clindamycin, Chloramphenicol, Colistin, Daptomycin, Erythromycin, Fosfomycin, Linezolid, Rifampicine, Synercid, Tigecycline, Vancomycin, Sulbactam; and
- at least one compound of formula (I) 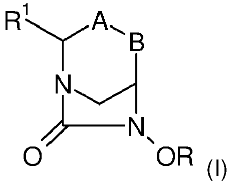 in which
- R is chosen among -SO₃H or CF₂CO₂H;
- R¹ is chosen among H, CH₂-NH₂, CONH₂ or a 5-membered heteroaryl comprising at least two nitrogen;
- A-B is 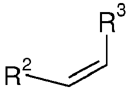 R² is a hydrogen atom or a saturated, partially or totally unsaturated or aromatic 4- to 10-membered heterocycle, comprising at least one nitrogen atom, unsubstituted or substituted by one or more T¹, preferably unsubstituted;
R³ is H, or oxazolyl; T¹, identical or different, independently represents a fluorine atom ; -(CH₂)ₘOQ¹ ; - (CH₂)ₘ-CN ; -(CH₂)ₘ-OC(O)Q¹ ; -(CH₂)ₘ-C(O)OQ¹ ; -(CH₂),-OC(O)OQ¹ ; -(C_{H2})ₘ-OC(O)NQ¹Q² ; -(CH₂)ₘ-C(O)NQ¹Q² ; -(CH₂)ₘ-C(O)ONQ¹Q² ; -(CH₂)ₘ-C(O)NQ¹OQ²; -(CH₂)ₘ-C(O)NQ¹-NQ¹Q² ; -(CH₂)ₘ-NQ¹C(O)Q² ; -(CH₂)ₘ-NQ¹S(O)₂NQ¹Q² ; -(CH₂)ₘ-NQ¹S(O)₂Q² ; -(CH₂)ₘ-S(O)₂NQ¹Q² ; -(CH₂)ₘ-NQ¹C(O)OQ² ; -(CH₂)ₘ-NQ¹C(O)NQ¹Q² ; -(CH₂)ₘ-NQ¹Q² ; -(CH₂)ₘ-NH-C(NHQ³)=NQ⁴ ; -(CH₂)ₘ-NH-CH=NQ³ ; -(CH₂)ₘ-C(NHQ³)=NQ⁴ ; -(X)-(CH₂)ₚOQ¹ ; -(X)-(CH₂)ₙ-CN ; -(X)-(CH₂)ₚ-OC(O)Q¹ ; -(X)-(CH₂)ₙ-C(O)OQ¹ ; -(X)-(C_{H2})ₚ-OC(O)OQ¹ ; -(X)-(CH₂)ₚ-OC(O)NQ¹Q²; -(X)-(CH₂)ₙ-C(O)NQ¹Q² ; -(X)-(CH₂)ₙ-C(O)ONQ¹Q² ; -(X)-(CH₂)ₙ-C(O)NQ¹OQ² ; -(X)-(CH₂)ₙ-C(O)NQ¹-NQ¹Q² ; -(X)-(CH₂)ₚ-NQ¹C(O)Q² ; -(X)-(CH₂)ₚ-NQ¹S(O)2NQ¹Q² ; -(X)-(CH₂)ₚ-NQ¹S(O)₂Q² ; -(X)-(CH₂)ₚ-S(O)₂NQ¹Q² ; -(X)-(CH₂)ₚ-NQ¹C(O)OQ² ; -(X)-(CH₂)ₚ-NQ¹C(O)NQ¹Q² ; -(X)-(CH₂)ₚ-NQ¹Q² ; -(X)-(CH₂)ₚ-NH-C(NHQ³)=NQ⁴ ; -(X)-(CH₂)p-NH-CH=NQ³ ; -(X)-(CH₂)ₙ-C(NHQ³)=NQ⁴ ; -C(O)-(CH₂)ₙOQ¹ ; -C(O)-(CH₂)ₙ-CN ; -C(O)-(CH₂)ₙ-OC(O)Q¹ ; -C(O)-(CH₂)ₙ-C(O)OQ¹ ; -C(O)-(CH₂)ₙ-OC(O)OQ¹ ; - C(O)-(CH₂)ₙ-OC(O)NQ¹Q² ; -C(O)-(CH₂)ₙ-C(O)NQ¹Q² ; -C(O)-(CH₂)ₙ-C(O)ONQ¹Q² ; - C(O)-(CH₂)ₙ-C(O)NQ¹OQ² ; -C(O)-(CH₂)ₙ-C(O)NQ¹-NQ¹Q² ; -C(O)-(CH₂)ₙ-NQ¹C(O)Q² ; -C(O)-(CH₂)ₙ-NQ¹S(O)₂NQ¹Q² ; -C(O)-(CH₂)ₙ-NQ¹S(O)₂Q² ; -C(O)-(CH₂)ₙ-S(O)₂NQ¹Q²; -C(O)-(CH₂)ₙ-NQ¹C(O)OQ² ; -C(O)-(CH₂)ₙ-NQ¹C(O)NQ¹Q² ; - C(O)-(CH₂)ₙ-NQ¹Q² ; -C(O)-(CH₂)ₙ-NH-C(NHQ³)=NQ⁴ ; -C(O)-(CH₂)ₙ-NH-CH=NQ³ ; - C(O)-(CH₂)ₙ-C(NHQ³)=NQ⁴ or T¹, identical or different, independently represents an unsubstituted or substituted by one or more T², -(CH₂)ₘ-(4-, 5- or 6-membered saturated, partially or totally unsaturated or aromatic heterocycle) ; -(X)-(CH₂)ₘ-(4-, 5- or 6-membered saturated, partially or totally unsaturated or aromatic heterocycle) ; (C₁-C₃)-alkyl ; (C₁-C₃)-fluoroalkyl ; -(X)-(C₁-C₃)-alkyl ; -(X)-(C₁-C₃)-fluoroalkyl ; - (CH₂)ₘ-(C₃-C₆)-cycloalkyl ; -(X)-(CH₂)ₘ-(C₃-C₆)-cycloalkyl ; -(CH₂)ₘ-(C₃-C₆)-cyclofluoroalkyl ; -(X)-(CH₂)ₘ-(C₃-C₆)-cyclofluoroalkyl ; -C(O)-(CH₂)ₘ-(4-, 5- or 6-membered saturated, partially or totally unsaturated or aromatic heterocycle) ; - C(O)-(C₁-C₃)-alkyl ; -C(O)-(C₁-C₃)-fluoroalkyl ; -C(O)O-(C₁-C₃)-fluoroalkyl ; -C(O)-(CH₂)ₘ-(C₃-C₆)-cycloalkyl; -C(O)-(CH₂)ₘ-(C₃-C₆)-cycloalkyl ; -C(O)-(CH₂)ₘ-(C₃-C₆)-cyclofluoroalkyl ; -C(O)-(CH₂)ₘ-(C₃-C₆)-cyclofluoroalkyl ;
Q¹ and Q², identical or different, independently represent a hydrogen atom ; -(CH₂)ᵣ-NHQ³ ; -(CH₂)ᵣ-NH-C(NHQ³)=NQ⁴ ; -(CH₂)ᵣ-NH-CH=NQ³ ; (CH₂)ₙ-C(NHQ³)=NQ⁴ ; - (CH₂)ᵣ-OQ³ ; -(CH₂)ₙ-CONHQ³ ; or an unsubstituted or substituted by one or more T², (C₁-C₃)-alkyl ; (C₁-C₃)-fluoroalkyl ; saturated, partially or totally unsaturated or aromatic-(CH₂)ₘ-(4-, 5- or 6-membered heterocycle comprising at least one nitrogen atom) ; or Q¹, Q² and the nitrogen atom to which they are bonded, form together an unsubstituted or substituted by one or more T², saturated or partially unsaturated 4-, 5- or 6-membered heterocycle comprising 1, 2 or 3 heteroatoms ;
Q³ and Q⁴, identical or different, independently represent a hydrogen atom or (C₁-C₃)-alkyl;
T², identical or different, independently represents -OH ; -NH₂ ; -CONH₂;
m, identical or different, independently represents 0, 1, 2 or 3 ;
n, identical or different, independently represents 1, 2 or 3 ;
p, identical or different, independently represents 2 or 3 ;
r is 1, 2 or 3 when the (CH₂)ᵣ is directly linked to a carbon atom or 2 or 3 otherwise, preferably r is 2 or 3;
- X, identical or different, independently represents O ; S ; S(O) ; S(O)₂ or N(Q³);
- at least one of R² and R³ is different from H and R² and R³ are not simultaneously H; and a racemate, an enantiomer, a diastereoisomer, a geometric isomer or a pharmaceutically acceptable salt of the compound of formula (I).

2. Composition comprising :
- at least one antibiotic chosen among: Amoxicillin, Amikacin, Aztreonam, Ciprofloxacin, Clindamycin, Chloramphenicol, Colistin, Cefpirome, Ceftriaxone, Cefotaxime, Daptomycin, Erythromycin, Cefepime, Cefixime, Fosfomycin, Cefuroxime, Imipenem, Linezolid, Meropenem, Piperacillin, Cefpodoxime, Rifampicine, Synercid, Tigecycline, Vancomycin, Sulbactam, Ceftaroline, Cefiderocol (S-649266) or BAL30072; and
- at least one compound selected among :

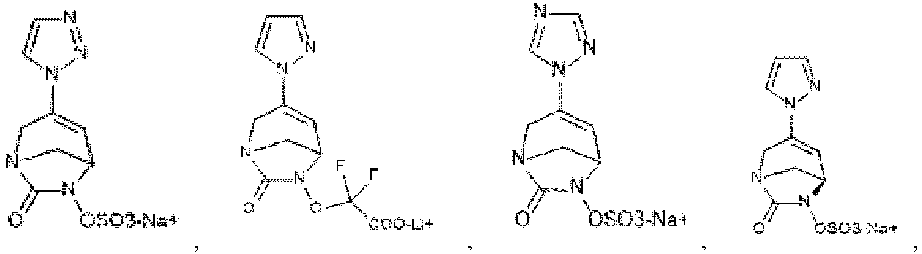

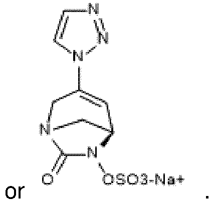

3. Composition according to claim 1 wherein the compound of formula (I) is a compound of formula (I*) 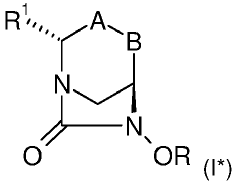 wherein R¹, A, B and R are as defined in claim 1.

4. Composition according to anyone of claims 1 to 3 further comprising a pharmaceutically acceptable excipient.

5. Composition according to anyone of claims 1 , 3 or 4, wherein R² is chosen among H, or a 5 to 6-membered heterocycle saturated, partially or totally unsaturated or aromatic comprising at least one nitrogen atom.

6. Composition according to anyone of claims 1 or 3 to 5, wherein R² is chosen among H or an heterocycle chosen among oxazolyl, pyrazolyl, pyridinyl or triazolyl.

7. Composition according to claim 2 or 4, wherein the antibiotic is chosen among Amoxicillin, Amikacin, Aztreonam, Ciprofloxacin, Clindamycin, Chloramphenicol, Colistin, Cefpirome, Ceftriaxone, Cefotaxime, Daptomycin, Erythromycin, Cefepime, Cefixime, Fosfomycin, Cefuroxime, Imipenem, Linezolid, Meropenem, Piperacillin, Cefpodoxime, Rifampicine, Synercid, Tigecycline, Vancomycin, Sulbactam, Ceftaroline.

8. Composition according to anyone of claims 2 or 4, wherein the antibiotic is chosen among Amoxicillin, Aztreonam, Cefpirome, Ceftriaxone, Cefotaxime, Cefepime, Cefixime, Fosfomycin, Cefuroxime, Imipenem, Meropenem, Piperacillin, Cefpodoxime, Sulbactam.

9. Composition according to anyone of claims 2 or 4, wherein the antibiotic is chosen among Meropenem, Aztreonam, Piperacillin, Fosfomycin, Cefepime, Cefixime or Sulbactam.

10. Composition according to anyone of claims 1 and 3-6, wherein the compounds of formula (I) or (I*) are chosen among : 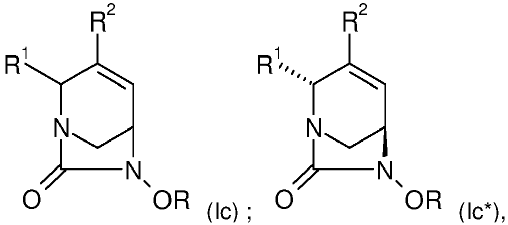 wherein R, R¹ and R² are as defined above, preferably R¹ is CH₂NH₂ or H and preferably R² is , a 5 to 6-membered heterocycle, preferably monocycle, saturated, partially or totally unsaturated or aromatic, preferably aromatic, comprising at least one nitrogen atom, preferably R² is an heterocycle chosen among oxazolyl, pyrazolyl, pyridinyl or triazolyl; or 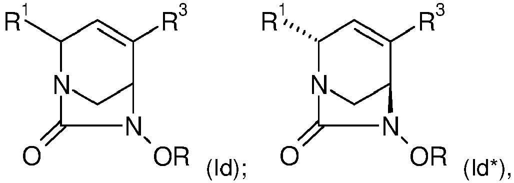 wherein R, R¹ and R³ are as defined above, preferably R¹ is CH₂NH₂.

11. Composition according to anyone of claims 2, 4 and 7 to 10, wherein the compound of formula (I) is chosen among:
sodium and 2,2,2-trifluoroacetate [*trans*-2-(azaniumylmethyl)-4-oxazol-5-yl-7-oxo-1,6-diazabicyclo[3.2.1]oct-3-en-6-yl] sulfate
sodium and 2,2,2-trifluoroacetate [(2*S*,5*R*)-2-(azaniumylmethyl)-7-oxo-3-oxazol-2-yl-1,6-diazabicyclo[3.2.1]oct-3-en-6-yl] sulfate
lithium difluoro-(3-oxazol-3-yl-7-oxo-1,6-diazabicyclo[3.2.1]oct-3-en-6-yloxy]-acetate sodium [3-(3-pyridyl)-7-oxo-1,6-diazabicyclo[3.2.1]oct-3-en-6-yl] sulfate
sodium [(5*R*)-3-(oxazol-2-yl)-7-oxo-1,6-diazabicyclo[3.2.1]oct-3-en-6-yl] sulfate
sodium (7-oxo-3-pyrazol-1-yl-1,6-diazabicyclo[3.2.1]oct-3-en-6-yl) sulfate
sodium [7-oxo-3-(triazol-1-yl)-1,6-diazabicyclo[3.2.1]oct-3-en-6-yl] sulfate
lithium difluoro-(7-oxo-3-pyrazol-1-yl-1,6-diaza-bicyclo[3.2.1]oct-3-en-6-yloxy)-acetate
sodium [7-oxo-3-(1,2,4-triazol-1-yl)-1,6-diazabicyclo[3.2.1]oct-3-en-6-yl] sulfate
sodium [(5*R*)-7-oxo-3-pyrazol-1-yl-1,6-diazabicyclo[3.2.1]oct-3-en-6-yl] sulfate
sodium [(5*R*)-7-oxo-3-(triazol-1-yl)-1,6-diazabicyclo[3.2.1]oct-3-en-6-yl] sulfate.

12. Composition according to anyone of claims 1 to 11 for its use as a medicine.

13. Composition according to anyone of claims 1 to 11 for its use for treating or preventing a bacterial infection, preferably caused by bacteria producing one or more β-lactamase, preferably caused by a gram-positive bacteria or by gram-negative bacteria, preferably a bacterial infection caused by gram-negative bacteria.

14. Composition according to anyone of claims 1 to 11 for its use as an antibacterial agent and/or as a β-lactamase inhibitor.

15. A kit comprising :
- at least one antibiotic chosen among: Amikacin, Ciprofloxacin, Clindamycin, Chloramphenicol, Colistin, Daptomycin, Erythromycin, Fosfomycin, Linezolid, Rifampicine, Synercid, Tigecycline, Vancomycin, Sulbactam; and
- at least one compound of formula (I) 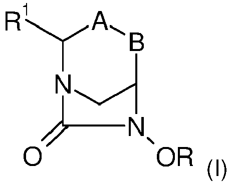 in which
- R is chosen among -SO₃H or CF₂CO₂H;
- R¹ is chosen among H, CH₂-NH₂, CONH₂ or a 5-membered heteroaryl comprising at least two nitrogen;
- A-B is 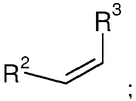 R² is a hydrogen atom or a saturated, partially or totally unsaturated or aromatic 4- to 10-membered heterocycle, comprising at least one nitrogen atom, unsubstituted or substituted by one or more T¹, preferably unsubstituted;
R³ is H, or oxazolyl;
T¹, identical or different, independently represents a fluorine atom ; -(CH₂)ₘOQ¹ ; - (CH₂)ₘ-CN ; -(CH₂)ₘ-OC(O)Q¹ ; -(CH₂)ₘ-C(O)OQ¹ ; -(CH₂)ₘ-OC(O)OQ¹ ; -(CH₂)ₘ-OC(O)NQ¹Q² ; -(CH₂)ₘ-C(O)NQ¹Q² ; -(CH₂)ₘ-C(O)ONQ¹Q² ; -(CH₂)ₘ-C(O)NQ¹OQ²; -(CH₂)ₘ-C(O)NQ¹-NQ¹Q² ; -(CH₂)ₘ-NQ¹C(O)Q² ; -(CH₂)ₘ-NQ¹S(O)₂NQ¹Q² ; -(CH₂)ₘ-NQ¹S(O)₂Q² ; -(CH₂)ₘ-S(O)₂NQ¹Q² ; -(CH₂)ₘ-NQ¹C(O)OQ² ; -(CH₂)ₘ-NQ¹C(O)NQ¹Q² ; -(CH₂)ₘ-NQ¹Q² ; -(CH₂)ₘ-NH-C(NHQ³)=NQ⁴ ; -(CH₂)ₘ-NH-CH=NQ³ ; -(CH₂)ₘ-C(NHQ³)=NQ⁴ ; -(X)-(CH₂)ₚOQ¹ ; -(X)-(CH₂)ₙ-CN ; -(X)-(CH₂)ₚ-OC(O)Q¹ ; -(X)-(CH₂)ₙ-C(O)OQ¹ ; -(X)-(CH₂)ₚ-OC(O)OQ¹ ; -(X)-(CH₂)ₚ-OC(O)NQ¹Q²; -(X)-(CH₂)ₙ-C(O)NQ¹Q² ; -(X)-(CH₂)ₙ-C(O)ONQ¹Q² ; -(X)-(CH₂)ₙ-C(O)NQ¹OQ² ; -(X)-(CH₂)ₙ-C(O)NQ¹-NQ¹Q² ; -(X)-(CH₂)ₚ-NQ¹C(O)Q² ; -(X)-(CH₂)ₚ-NQ¹S(O)₂NQ¹Q² ; -(X)-(CH₂)ₚ-NQ¹S(O)₂Q² ; -(X)-(CH₂)ₚ-S(O)₂NQ¹Q² ; -(X)-(CH₂)ₚ-NQ¹C(O)OQ² ; -(X)-(CH₂)ₚ-NQ¹C(O)NQ¹Q² ; -(X)-(CH₂)ₚ-NQ¹Q² ; -(X)-(CH₂)ₚ-NH-C(NHQ³)=NQ⁴ ; -(X)-(CH₂)ₚ-NH-CH=NQ³ ; -(X)-(CH₂)ₙ-C(NHQ³)=NQ⁴ ; -C(O)-(CH₂)ₙOQ¹ ; -C(O)-(CH₂)ₙ-CN ; -C(O)-(CH₂)ₙ-OC(O)Q¹ ; -C(O)-(CH₂)ₙ-C(O)OQ¹ ; -C(O)-(CH₂)ₙ-OC(O)OQ¹ ; - C(O)-(CH₂)ₙ-OC(O)NQ¹Q² ; -C(O)-(CH₂)ₙ-C(O)NQ¹Q² ; -C(O)-(CH₂)ₙ-C(O)ONQ¹Q² ; - C(O)-(CH₂)ₙ-C(O)NQ¹OQ² ; -C(O)-(CH₂)ₙ-C(O)NQ¹-NQ¹Q² ; -C(O)-(CH₂)ₙ-NQ¹C(O)Q² ; -C(O)-(CH₂)ₙ-NQ¹S(O)₂NQ¹Q² ; -C(O)-(CH₂)ₙ-NQ¹S(O)₂Q² ; -C(O)-(CH₂)ₙ-S(O)₂NQ¹Q²; -C(O)-(CH₂)ₙ-NQ¹C(O)OQ² ; -C(O)-(CH₂)ₙ-NQ¹C(O)NQ¹Q² ; - C(O)-(CH₂)ₙ-NQ¹Q² ; -C(O)-(CH₂)ₙ-NH-C(NHQ³)=NQ⁴ ; -C(O)-(CH₂)ₙ-NH-CH=NQ³ ; - C(O)-(CH₂)ₙ-C(NHQ³)=NQ⁴ or T¹, identical or different, independently represents an unsubstituted or substituted by one or more T², -(CH₂)ₘ-(4-, 5- or 6-membered saturated, partially or totally unsaturated or aromatic heterocycle) ; -(X)-(CH₂)ₘ-(4-, 5- or 6-membered saturated, partially or totally unsaturated or aromatic heterocycle) ; (C₁-C₃)-alkyl ; (C₁-C₃)-fluoroalkyl ; -(X)-(C₁-C₃)-alkyl ; -(X)-(C₁-C₃)-fluoroalkyl ; - (CH₂)ₘ-(C₃-C₆)-cycloalkyl ; -(X)-(CH₂)ₘ-(C₃-C₆)-cycloalkyl ; -(CH₂)ₘ-(C₃-C₆)-cyclofluoroalkyl ; -(X)-(CH₂)ₘ-(C₃-C₆)-cyclofluoroalkyl ; -C(O)-(CH₂)ₘ-(4-, 5- or 6-membered saturated, partially or totally unsaturated or aromatic heterocycle) ; - C(O)-(C₁-C₃)-alkyl ; -C(O)-(C₁-C₃)-fluoroalkyl ; -C(O)O-(C₁-C₃)-fluoroalkyl ; -C(O)-(CH₂)ₘ-(C₃-C₆)-cycloalkyl; -C(O)-(CH₂)ₘ-(C₃-C₆)-cycloalkyl ; -C(O)-(CH₂)ₘ-(C₃-C₆)-cyclofluoroalkyl ; -C(O)-(CH₂)ₘ-(C₃-C₆)-cyclofluoroalkyl ;
Q¹ and Q², identical or different, independently represent a hydrogen atom ; -(CH₂)ᵣ-NHQ³ ; -(CH₂)ᵣ-NH-C(NHQ³)=NQ⁴ ; -(CH₂)ᵣ-NH-CH=NQ³ ; (CH₂)ₙ-C(NHQ³)=NQ⁴ ; - (CH₂)ᵣ-OQ³ ; -(CH₂)ₙ-CONHQ³ ; or an unsubstituted or substituted by one or more T², (C₁-C₃)-alkyl ; (C₁-C₃)-fluoroalkyl ; saturated, partially or totally unsaturated or aromatic-(CH₂)ₘ-(4-, 5- or 6-membered heterocycle comprising at least one nitrogen atom) ; or Q¹, Q² and the nitrogen atom to which they are bonded, form together an unsubstituted or substituted by one or more T², saturated or partially unsaturated 4-, 5- or 6-membered heterocycle comprising 1, 2 or 3 heteroatoms ;
Q³ and Q⁴, identical or different, independently represent a hydrogen atom or (C₁-C₃)-alkyl;
T², identical or different, independently represents -OH ; -NH₂ ; -CONH₂;
m, identical or different, independently represents 0, 1, 2 or 3 ;
n, identical or different, independently represents 1, 2 or 3 ;
p, identical or different, independently represents 2 or 3 ;
r is 1, 2 or 3 when the (CH₂)ᵣ is directly linked to a carbon atom or 2 or 3 otherwise, preferably r is 2 or 3;
- X, identical or different, independently represents O ; S ; S(O) ; S(O)₂ or N(Q³);
- at least one of R² and R³ is different from H and R² and R³ are not simultaneously H; and a racemate, an enantiomer, a diastereoisomer, a geometric isomer or a pharmaceutically acceptable salt of the compound of formula (I)
for the treatment or prevention of bacterial infections by its simultaneous, separate or sequential administration to a patient in need thereof.

16. A kit comprising :
- at least one antibiotic chosen among: Amoxicillin, Amikacin, Aztreonam, Ciprofloxacin, Clindamycin, Chloramphenicol, Colistin, Cefpirome, Ceftriaxone, Cefotaxime, Daptomycin, Erythromycin, Cefepime, Cefixime, Fosfomycin, Cefuroxime, Imipenem, Linezolid, Meropenem, Piperacillin, Cefpodoxime, Rifampicine, Synercid, Tigecycline, Vancomycin, Sulbactam, Ceftaroline; Cefiderocol (S-649266), BAL30072; and
- at least one compound selected among : 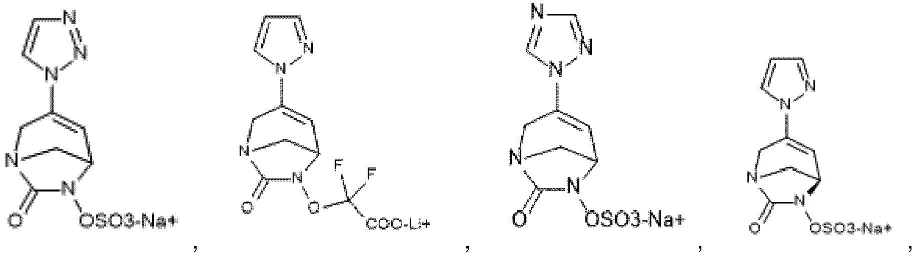 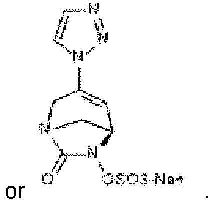 for the treatment or prevention of bacterial infections by its simultaneous, separate or sequential administration to a patient in need thereof.

17. A kit according to claim 15 wherein the compound of formula (I) is a compound of formula (I*) 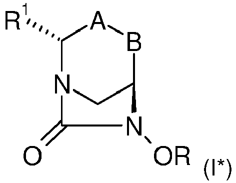 wherein R¹, A, B and R are as defined in claim 15.

## Patentansprüche

1. Zusammensetzung, enthaltend:
- mindestens ein Antibiotikum, ausgewählt aus: Amikacin, Ciprofloxacin, Clindamycin, Chloramphenicol, Colistin, Daptomycin, Erythromycin, Fosfomycin, Linezolid, Rifampicin, Synercid, Tigecyclin, Vancomycin, Sulbactam; und
- mindestens eine Verbindung der Formel (I) 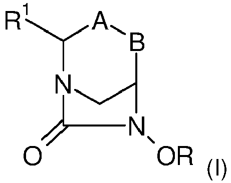 in der
- R ausgewählt ist aus -SO₃H oder CF₂CO₂H;
- R¹ ausgewählt ist aus H, CH₂-NH₂, CONH₂ oder einem 5-gliedrigen Heteroaryl, das mindestens zwei Stickstoffe enthält;
- A-B 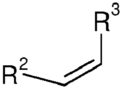 ist
R² ein Wasserstoffatom oder ein gesättigter, teilweise oder vollständig ungesättigter oder aromatischer 4- bis 10-gliedriger Heterozyklus ist, der mindestens ein Stickstoffatom enthält, unsubstituiert oder substituiert durch ein oder mehrere T¹, vorzugsweise unsubstituiert;
R³ H oder Oxazolyl ist;
T¹, gleich oder verschieden, unabhängig ein Fluoratom ; - (CH₂)ₘOQ¹; -(CH₂)ₘ-CN; -(CH₂)ₘ-OC(O)Q¹; -(CH₂)ₘ-C(O)OQ¹; -(CH₂)ₘ-OC(O)OQ¹; -(CH₂)ₘ-OC(O)NQ¹Q²; -(CH₂)ₘ-C(O)NQ¹Q²; -(CH₂)ₘ-C(O)ONQ¹Q²; -(CH₂)ₘ-C(O)NQ¹OQ²; -(CH₂)ₘ-C(O)NQ¹-NQ¹Q²; -(CH₂)ₘ-NQ¹C(O)Q²; -(CH₂)ₘ-NQ¹S(O)₂NQ¹Q²; -(CH₂)ₘ-NQ¹S(O)₂Q²; -(CH₂)ₘ-S(O)₂NQ¹Q²; -(CH₂)ₘ-NQ¹C(O)OQ²; -(CH₂)ₘ-NQ¹C(O)NQ¹Q²; -(CH₂)ₘ-NQ¹Q²; -(CH₂)ₘ-NH-C(NHQ³)=NQ⁴; -(CH₂)ₘ-NH-CH=NQ³; -(CH₂)ₘ-C(NHQ³)=NQ⁴; -(X)-(CH₂)ₚOQ¹; -(X)-(CH₂)ₙ-CN; -(X)-(CH₂)ₚ-OC(O)Q¹; -(X)-(CH₂)ₙ-C(O)OQ¹; -(X)-(CH₂)ₚ-OC(O)OQ¹; -(X)-(CH₂)_{P}-OC(O)NQ¹Q²; -(X)-(CH₂)ₙ-C(O)NQ¹Q²; -(X)-(CH₂)ₙ-C(O)ONQ¹Q²; -(X)-(CH₂)ₙ-C(O)NQ¹OQ²; - (X)-(CH₂)ₙ-C(O)NQ¹-NQ¹Q²; -(X)-(CH₂)ₚ-NQ¹C(O)Q²; -(X)-(CH₂)_{P}-NQ¹S(O)₂NQ¹Q²; -(X)-(CH₂)ₚ-NQ¹S(O)₂Q²; -(X)-(CH₂)ₚ-S(O)₂NQ¹Q²; -(X)-(CH₂)ₚ-NQ¹C(O)OQ²; -(X)-(CH₂)ₚ-NQ¹C(O)NQ¹Q²; -(X)-(CH₂)ₚ-NQ¹Q²; -(X)-(CH₂)_{P}-NH-C(NHQ³)=NQ⁴; -(X)-(CH₂)p-NH-CH=NQ³; -(X)-(CH₂)ₙ-C(NHQ³)=NQ⁴; -C(O)-(CH₂)ₙOQ¹; -C(O)-(CH₂)ₙ-CN; -C(O)-(CH₂)ₙ-OC(O)Q¹; -C(O)-(CH₂)ₙ-C(O)OQ¹; -C(O)-(CH₂)ₙ-OC(O)OQ¹; -C(O)-(CH₂)ₙ-OC(O)NQ¹Q²; -C(O)-(CH₂)ₙ-C(O)NQ¹Q²; -C(O)-(CH₂)ₙ-C(O)ONQ¹Q²; - C(O)-(CH₂)ₙ-C(O)NQ¹OQ²; -C(O)-(CH₂)ₙ-C(O)NQ¹-NQ¹Q²; -C(O)-(CH₂)ₙ-NQ¹C(O)Q²; -C(O)-(CH₂)ₙ-NQ¹S(O)₂NQ¹Q²; -C(O)-(CH₂)ₙ-NQ¹S(O)₂Q²; - C(0)-(CH₂)ₙ-S(O)₂NQ¹Q²; -C(O)-(CH₂)ₙ-NQ¹C(O)OQ²; -C(O)-(CH₂)ₙ-NQ¹C(O)NQ¹Q²; -C(O)-(CH₂)ₙ-NQ¹Q²; -C(O)-(CH₂)ₙ-NH-C(NHQ³)=NQ⁴; - C(O)-(CH₂)ₙ-NH-CH=NQ³; -C(O)-(CH₂)ₙ-C(NHQ³)=NQ⁴; darstellt; oder T¹, gleich oder verschieden, unabhängig einen unsubstituierten oder durch ein oder mehrere T² substituierten, -(CH₂)ₘ-(4-, 5- oder 6-gliedrigen gesättigten, teilweise oder vollständig ungesättigten oder aromatischen Heterozyklus); -(X)-(CH₂)ₘ-(4-, 5- oder 6-gliedrigen gesättigten, teilweise oder vollständig ungesättigten oder aromatischen Heterozyklus); (C₁-C₃)-Alkyl; (C₁-C₃)-Fluoralkyl; -(X)-(C₁-C₃)-Alkyl; -(X)-(C₁-C₃)-Fluoralkyl;-(CH₂)ₘ-(C₃-C₆)-Cycloalkyl ; -(X)-(CH₂)ₘ-(C₃-C₆)-Cycloalkyl; - (CH₂)ₘ-(C₃-C₆)-Cyclofluoralkyl; -(X)-(CH₂)ₘ-(C₃-C₆)-Cyclofluoralkyl; -C(O)-(CH₂)ₘ-(4-, 5- oder 6-gliedrigen gesättigten, teilweise oder vollständig ungesättigten oder aromatischen Heterozyklus); - C(O)-(C₁-C₃)-alkyl; - C(O)-(C₁-C₃)-Fluoralkyl; -C(O)O-(C₁-C₃)-Fluoralkyl; -C(O)-(CH₂)ₘ-(C₃-C₆)-Cycloalkyl; -C(O)-(CH₂)ₘ,-(C₃-C₆)-Cycloalkyl; -C(O)-(CH₂)ₘ-(C₃-C₆)-Cyclofluoralkyl; -C(O)-(CH₂)ₘ-(C₃-C₆)-Cyclofluoralkyl; darstellt;
Q¹ and Q², gleich oder verschieden, unabhängig ein Wasserstoffatom;-(CH₂)ᵣ- NHQ³; -(CH₂)ᵣ-NH-C(NHQ³)=NQ⁴; -(CH₂)ᵣ-NH-CH=NQ³; (CH₂)ₙ-C(NHQ³)=NQ⁴; -(CH₂)ᵣ-OQ³; -(CH₂)ₙ-CONHQ³; oder ein unsubstituiert oder substituiert durch ein oder mehr T², (C₁-C₃)-Alkyl; (C₁-C₃)-Fluoralkyl; gesättigter, vollständig oder teilweise ungesättigter oder aromatischer -(CH₂)ₘ-(4-, 5-oder 6-gliedrigen Heterozyklus enthaltend mindestens ein Stickstoffatom) darstellen; oder Q¹, Q² und das Stickstoffatom, an das sie gebunden sind, bilden zusammen einen unsubstituierten oder durch ein oder mehr T² substituierten, gesättigten oder teilweise ungesättigten 4-, 5- oder 6-gliedrigen Heterozyklus enthaltend 1, 2 oder 3 Heteroatome;
Q³ und Q⁴, gleich oder verschieden, unabhängig ein Wasserstoffatom oder (C₁-C₃)-Alkyl darstellen;
T², gleich oder verschieden, unabhängig -OH, -NH₂, -CONH₂ darstellt;
m, gleich oder verschieden, unabhängig 0, 1, 2 oder 3 darstellt;
n, gleich oder verschieden, unabhängig 1, 2 oder 3 darstellt;
p, gleich oder verschieden, unabhängig 2 oder 3 darstellt;
r 1, 2 oder 3 ist, wenn das (CH₂)ᵣ direkt an ein Kohlenstoffatom gebunden ist, oder ansonsten 2 oder 3 ist, vorzugsweise ist r 2 oder 3;
- X, gleich oder verschieden, unabhängig O; S; S(O); S(O)₂ oder N(Q³) darstellt;
- mindestens eines von R² und R³ von H verschieden ist und R² und R³ nicht gleichzeitig H sind; und ein Racemat, ein Enantiomer, ein Diastereoisomer, ein geometrisches Isomer oder ein pharmazeutisch annehmbares Salz der Verbindung der Formel (I).

2. Zusammensetzung, enthaltend:
- mindestens ein Antibiotikum, ausgewählt aus: Amoxicillin, Amikacin, Aztreonam, Ciprofloxacin, Clindamycin, Chloramphenicol, Colistin, Cefpirom, Ceftriaxon, Cefotaxim, Daptomycin, Erythromycin, Cefepim, Cefixim, Fosfomycin, Cefuroxim, Imipenem, Linezolid, Meropenem, Piperacillin, Cefpodoxim, Rifampicin, Synercid, Tigecyclin, Vancomycin, Sulbactam, Ceftarolin, Cefiderocol (S-649266) oder BAL30072; und
- mindestens eine Verbindung, ausgewählt aus:

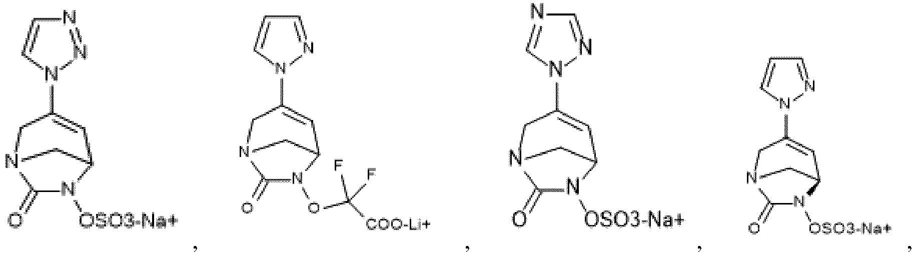

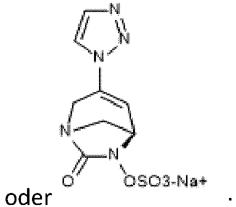

3. Zusammensetzung nach Anspruch 1, wobei die Verbindung der Formel (I) eine Verbindung der Formel (I*) ist 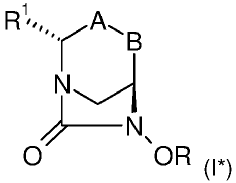 wobei R¹, A, B und R wie in Anspruch 1 definiert sind.

4. Zusammensetzung nach einem der Ansprüche 1 bis 3, ferner einen pharmazeutisch annehmbaren Hilfsstoff enthaltend.

5. Zusammensetzung nach einem der Ansprüche 1, 3 oder 4, wobei R² ausgewählt ist aus H oder einem 5- bis 6-gliedrigen gesättigten, teilweise oder vollständig ungesättigten oder aromatischen Heterozyklus, der mindestens ein Stickstoffatom enthält.

6. Zusammensetzung nach einem der Ansprüche 1 oder 3 bis 5, wobei R² ausgewählt ist aus H oder einem Heterozyklus ausgewählt aus Oxazolyl, Pyrazolyl, Pyridinyl oder Triazolyl.

7. Zusammensetzung nach Anspruch 2 oder 4, wobei das Antibiotikum ausgewählt ist aus Amoxicillin, Amikacin, Aztreonam, Ciprofloxacin, Clindamycin, Chloramphenicol, Colistin, Cefpirom, Ceftriaxon, Cefotaxim, Daptomycin, Erythromycin, Cefepim, Cefixim, Fosfomycin, Cefuroxim, Imipenem, Linezolid, Meropenem, Piperacillin, Cefpodoxim, Rifampicin, Synercid, Tigecyclin, Vancomycin, Sulbactam, Ceftarolin.

8. Zusammensetzung nach einem der Ansprüche 2 oder 4, wobei das Antibiotikum ausgewählt ist unter Amoxicillin, Aztreonam, Cefpirom, Ceftriaxon, Cefotaxim, Cefepim, Cefixim, Fosfomycin, Cefuroxim, Imipenem, Meropenem, Piperacillin, Cefpodoxim, Sulbactam.

9. Zusammensetzung nach einem der Ansprüche 2 oder 4, wobei das Antibiotikum ausgewählt ist aus Meropenem, Aztreonam, Piperacillin, Fosfomycin, Cefepim, Cefixim oder Sulbactam.

10. Zusammensetzung nach einem der Ansprüche 1 und 3-6, wobei die Verbindungen der Formel (I) oder (I*) ausgewählt sind aus: 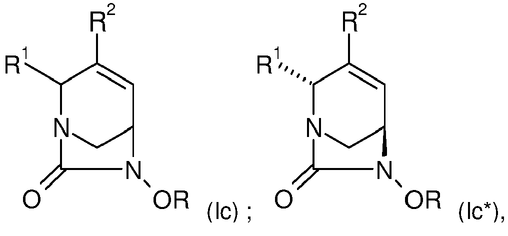 wobei R, R¹ und R² wie oben definiert sind, vorzugsweise R¹ CH₂NH₂ oder H ist und vorzugsweise R² ein 5- bis 6-gliedriger Heterozyklus, vorzugsweise monozyklisch, gesättigt, teilweise oder vollständig ungesättigt oder aromatisch, vorzugsweise aromatisch, ist, der mindestens ein Stickstoffatom enthält, vorzugsweise R² ein Heterozyklus ist, der ausgewählt ist aus Oxazolyl, Pyrazolyl, Pyridinyl oder Triazolyl; oder 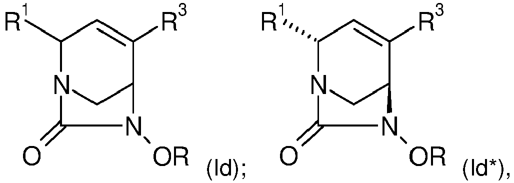 wobei R, R¹ und R³ wie oben definiert sind, vorzugsweise ist R¹ CH₂NH₂.

11. Zusammensetzung nach einem der Ansprüche 2, 4 und 7 bis 10, wobei die Verbindung der Formel (I) ausgewählt ist aus:
Natrium- und 2,2,2-Trifluoracetat*[trans*-*2*-(Azaniumylmethyl)-4-Oxazol-5-yl-7-Oxo-1,6-Diazabicyclo[3.2.1]Oct-3-en-6-yl]-Sulfat
Natrium- und 2,2,2-Trifluoracetat [(2*S*,5*R*)-2-(Azaniumylmethyl)-7-Oxo-3-Oxazol-2-yl-1,6-Diazabicyclo[3.2.1]Oct-3-en-6-yl]-Sulfat
Lithium-Difluor-(3-Oxazol-3-yl-7-Oxo-1,6-Diazabicyclo[3.2.1]Oct-3-en-6-yloxy]-Acetat
Natrium-[3-(3-Pyridyl)-7-Oxo-1,6-Diazabicyclo[3.2.1]Oct-3-en-6-yl]-Sulfat
Natrium-[(5*R*)-3-(Oxazol-2-yl)-7-Oxo-1,6-Diazabicyclo[3.2.1]Oct-3-en-6-yl]-Sulfat
Natrium-(7-Oxo-3-Pyrazol-1-yl-1,6-Diazabicyclo[3.2.1]Oct-3-en-6-yl)-Sulfat
Natrium-[7-Oxo-3-(Triazol-1-yl)-1,6-Diazabicyclo[3.2.1]Oct-3-en-6-yl]-Sulfat
Lithium-Difluor-(7-Oxo-3-Pyrazol-1-yl-1,6-Diaza-Bicyclo[3.2.1]Oct-3-en-6-yloxy)-Acetat
Natrium-[7-Oxo-3-(1,2,4-Triazol-1-yl)-1,6-Diazabicyclo[3.2.1]Oct-3-en-6-yl]-Sulfat
Natrium-[(5*R*)-7-Oxo-3-Pyrazol-1-yl-1,6-Diazabicyclo[3.2.1]Oct-3-en-6-yl]-Sulfat
Natrium-[(5*R*)-7-Oxo-3-(Triazol-1-yl)-1,6-Diazabicyclo[3.2.1]Oct-3-en-6-yl]-Sulfat.

12. Zusammensetzung nach einem der Ansprüche 1 bis 11 zur Verwendung als Arzneimittel.

13. Zusammensetzung nach einem der Ansprüche 1 bis 11 zur Verwendung zur Behandlung oder Vorbeugung einer bakteriellen Infektion, vorzugsweise verursacht durch Bakterien, die eine oder mehrere β-Lactamase(n) produzieren, vorzugsweise verursacht durch ein grampositives Bakterium oder durch gramnegative Bakterien, vorzugsweise eine bakterielle Infektion verursacht durch gramnegative Bakterien.

14. Zusammensetzung nach einem der Ansprüche 1 bis 11 zur Verwendung als antibakterielles Mittel und/oder als β-Lactamase-Inhibitor.

15. Kit, enthaltend:
- mindestens ein Antibiotikum, ausgewählt aus: Amikacin, Ciprofloxacin, Clindamycin, Chloramphenicol, Colistin, Daptomycin, Erythromycin, Fosfomycin, Linezolid, Rifampicin, Synercid, Tigecyclin, Vancomycin, Sulbactam; und
- mindestens eine Verbindung der Formel (I) 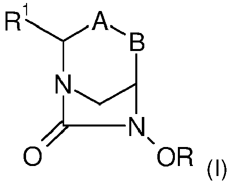 in der
- R ausgewählt ist aus -SO₃H oder CF₂CO₂H;
- R¹ ausgewählt ist aus H, CH₂-NH₂, CONH₂ oder einem 5-gliedrigen Heteroaryl, das mindestens zwei Stickstoffe enthält;
- A-B 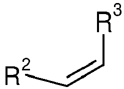 ist; R² ein Wasserstoffatom oder ein gesättigter, teilweise oder vollständig ungesättigter oder aromatischer 4- bis 10-gliedriger Heterozyklus ist, der mindestens ein Stickstoffatom enthält, unsubstituiert oder substituiert durch ein oder mehrere T¹, vorzugsweise unsubstituiert;
R³ H oder Oxazolyl ist;
T¹, gleich oder verschieden, unabhängig ein Fluoratom; - (CH₂)ₘOQ¹; -(CH₂)ₘ-CN; -(CH₂)ₘ-OC(O)Q¹; -(CH₂)ₘ-C(O)OQ¹; -(CH₂)ₘ-OC(O)OQ¹; -(CH₂)ₘ-OC(O)NQ¹Q²; -(CH₂)ₘ-C(O)NQ¹Q²; -(CH₂)ₘ-C(O)ONQ¹Q²; -(CH₂)ₘ-C(O)NQ¹OQ²; -(CH₂)ₘ-C(O)NQ¹-NQ¹Q²; -(CH₂)ₘ-NQ¹C(O)Q²; -(CH₂)ₘ-NQ¹S(O)₂NQ¹Q²; -(CH₂)ₘ-NQ¹S(O)₂Q²; -(CH₂)ₘ-S(O)₂NQ¹Q²; -(CH₂)ₘ-NQ¹C(O)OQ²; -(CH₂)ₘ-NQ¹C(O)NQ¹Q²;-(CH₂)ₘ-NQ¹Q²; -(CH₂)ₘ-NH-C(NHQ³)=NQ⁴; -(CH₂)ₘ-NH-CH=NQ³; -(CH₂)ₘ-C(NHQ³)=NQ⁴; -(X)-(CH₂)ₚOQ¹; -(X)-(CH₂)ₙ-CN; -(X)-(CH₂)ₚ-OC(O)Q¹; -(X)-(CH₂)ₙ-C(O)OQ¹; -(X)-(CH₂)ₚ-OC(O)OQ¹; -(X)-(CH₂)ₚ-OC(O)NQ¹Q²; -(X)-(CH₂)ₙ-C(O)NQ¹Q²; -(X)-(CH₂)ₙ-C(O)ONQ¹Q²; -(X)-(CH₂)ₙ-C(O)NQ¹OQ²; - (X)-(CH₂)ₙ-C(O)NQ¹-NQ¹Q²; -(X)-(CH₂)ₚ-NQ¹C(O)Q²; -(X)-(CH₂)ₚ-NQ¹S(O)₂NQ¹Q²; -(X)-(CH₂)ₚ-NQ¹S(O)₂Q²; -(X)-(CH₂)ₚ-S(O)₂NQ¹Q²; -(X)-(CH₂)ₚ-NQ¹C(O)OQ²; -(X)-(CH₂)ₚ-NQ¹C(O)NQ¹Q²; -(X)-(CH₂)ₚ-NQ¹Q²; -(X)-(CH₂)ₚ-NH-C(NHQ³)=NQ⁴; -(X)-(CH₂)ₚ-NH-CH=NQ³; -(X)-(CH₂)ₙ-C(NHQ³)=NQ⁴; -C(O)-(CH₂)ₙOQ¹; -C(O)-(CH₂)ₙ-CN; -C(O)-(CH₂)ₙ-OC(O)Q¹; -C(O)-(CH₂)ₙ-C(O)OQ¹; -C(O)-(CH₂)ₙ-OC(O)OQ¹; -C(O)-(CH₂)ₙ-OC(O)NQ¹Q²; -C(O)-(CH₂)ₙ-C(O)NQ¹Q²; -C(O)-(CH₂)ₙ-C(O)ONQ¹Q²; - C(O)-(CH₂)ₙ-C(O)NQ¹OQ²; -C(O)-(CH₂)ₙ-C(O)NQ¹-NQ¹Q²; -C(O)-(CH₂)ₙ-NQ¹C(O)Q²; -C(O)-(CH₂)ₙ-NQ¹S(O)₂NQ¹Q²; -C(O)-(CH₂)ₙ-NQ¹S(O)₂Q²; - C(O)-(CH₂)ₙ-S(O)₂NQ¹Q²; -C(O)-(CH₂)ₙ-NQ¹C(O)OQ²; -C(O)-(CH₂)ₙ-NQ¹C(O)NQ¹Q²; -C(O)-(CH₂)ₙ-NQ¹Q²; -C(O)-(CH₂)ₙ-NH-C(NHQ³)=NQ⁴; - C(O)-(CH₂)ₙ-NH-CH=NQ³; -C(O)-(CH₂)ₙ-C(NHQ³)=NQ⁴; darstellt; oder T¹, gleich oder verschieden, unabhängig einen unsubstituierten oder durch ein oder mehrere T² substituierten, -(CH₂)ₘ-(4-, 5- oder 6-gliedriger gesättigter, teilweise oder vollständig ungesättigter oder aromatischer Heterozyklus); -(X)-(CH₂)ₘ-(4-, 5- oder 6-gliedriger gesättigter, teilweise oder vollständig ungesättigter oder aromatischer Heterozyklus); (C₁-C₃)-Alkyl; (C₁-C₃)-Fluoralkyl; -(X)-(C₁-C₃)-Alkyl; -(X)-(C₁-C₃)-Fluoralkyl; -(CH₂)ₘ-(C₃-C₆)-Cycloalkyl; -(X)-(CH₂)ₘ-(C₃-C₆)-Cycloalkyl; - (CH₂)ₘ-(C₃-C₆)-Cyclofluoralkyl; -(X)-(CH₂)ₘ-(C₃-C₆)-Cyclofluoralkyl; -C(O)-(CH₂)ₘ-(4-, 5- oder 6-gliedriger gesättigter, teilweise oder vollständig ungesättigter oder aromatischer Heterozyklus); -C(O)-(C₁-C₃)-Alkyl; - C(O)-(C₁-C₃)-Fluoralkyl; -C(O)O-(C₁-C₃)-Fluoralkyl; -C(O)-(CH₂)ₘ-(C₃-C₆)-Cycloalkyl; -C(O)-(CH₂)ₘ,-(C₃-C₆)-Cycloalkyl;-C(O)-(CH₂)ₘ-(C₃-C₆)-Cyclofluoralkyl; -C(O)-(CH₂)ₘ-(C₃-C6)-Cyclofluoralkyl; darstellt;
Q¹ and Q², gleich oder verschieden, unabhängig ein Wasserstoffatom; -(CH₂)ᵣ-NHQ³; -(CH₂)ᵣ-NH-C(NHQ³)=NQ⁴; -(CH₂)ᵣ-NH-CH=NQ³; (CH₂)ₙ-C(NHQ³)=NQ⁴; -(CH₂)ᵣ-OQ³; -(CH₂)ₙ-CONHQ³; oder einen unsubstituierten oder substituiert durch ein oder mehr T², (C₁-C₃)-Alkyl; (C₁-C₃)-Fluoralkyl gesättigter, vollständig oder teilweise ungesättigter oder aromatischer -(CH₂)ₘ-(4-, 5-oder 6-gliedrigen Heterozyklus enthaltend mindestens ein Stickstoffatom); darstellen; oder Q¹, Q² und das Stickstoffatom, an das sie gebunden sind, zusammen einen unsubstituierten oder durch ein oder mehr T² substituierten, gesättigten oder teilweise ungesättigten 4-, 5- oder 6-gliedrigen Heterozyklus enthaltend 1, 2 oder 3 Heteroatome bilden;
Q³ und Q⁴, gleich oder verschieden, unabhängig ein Wasserstoffatom oder (C₁-C₃)-Alkyl darstellen;
T², gleich oder verschieden, unabhängig -OH, -NH₂, -CONH₂ darstellt;
m, gleich oder verschieden, unabhängig 0, 1, 2 oder 3 darstellt;
n, gleich oder verschieden, unabhängig 1, 2 oder 3 darstellt;
p, gleich oder verschieden, unabhängig 2 oder 3 darstellt;
r 1, 2 oder 3 ist, wenn das (CH₂)ᵣ direkt an ein Kohlenstoffatom gebunden ist, oder ansonsten 2 oder 3 ist, vorzugsweise ist r 2 oder 3;
- X, gleich oder verschieden, unabhängig O; S; S(O); S(O)₂ oder N(Q³) darstellt;
- mindestens eines von R² und R³ von H verschieden ist und R² und R³ nicht gleichzeitig H sind; und ein Racemat, ein Enantiomer, ein Diastereoisomer, ein geometrisches Isomer oder ein pharmazeutisch annehmbares Salz der Verbindung der Formel (I) zur Behandlung oder Vorbeugung von bakteriellen Infektionen durch ihre gleichzeitige, getrennte oder aufeinanderfolgende Verabreichung an einen Patienten, der diese benötigt.

16. Kit, enthaltend:
- mindestens ein Antibiotikum, ausgewählt aus: Amoxicillin, Amikacin, Aztreonam, Ciprofloxacin, Clindamycin, Chloramphenicol, Colistin, Cefpirom, Ceftriaxon, Cefotaxim, Daptomycin, Erythromycin, Cefepim, Cefixim, Fosfomycin, Cefuroxim, Imipenem, Linezolid, Meropenem, Piperacillin, Cefpodoxim, Rifampicin, Synercid, Tigecyclin, Vancomycin, Sulbactam, Ceftarolin; Cefiderocol (S-649266), BAL30072; und
- mindestens eine Verbindung, ausgewählt aus: 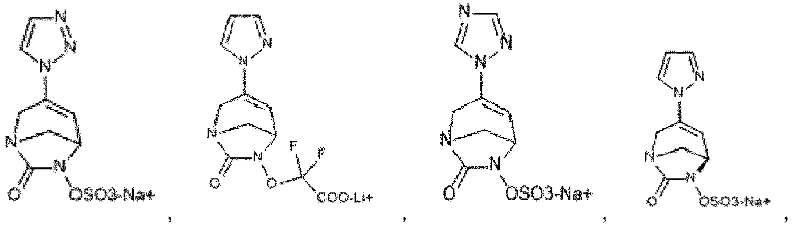 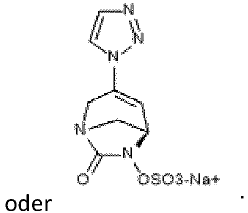 zur Behandlung oder Vorbeugung von bakteriellen Infektionen durch ihre gleichzeitige, getrennte oder aufeinanderfolgende Verabreichung an einen Patienten, der dies benötigt.

17. Kit nach Anspruch 15, wobei die Verbindung der Formel (I) eine Verbindung der Formel (I*) ist 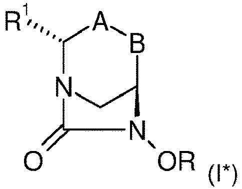 wobei R¹, A, B und R wie in Anspruch 15 definiert sind.

## Revendications

1. Composition comprenant :
- au moins un antibiotique choisi parmi : amikacine, ciprofloxacine, clindamycine, chloramphénicol, colistine, daptomycine, érythromycine, fosfomycine, linézolide, rifampicine, synercid, tigécycline, vancomycine, sulbactam ; et
- au moins un composé de formule (I) 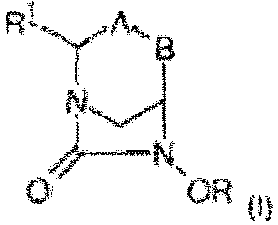 dans laquelle
- R est choisi parmi -SO₃H ou CF₂CO₂H ;
- R¹ est choisi parmi H, CH₂-NH₂, CONH₂ ou un hétéroaryle à 5 chaînons comprenant au moins deux atomes d'azote ;
- A-B est 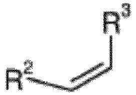 R² est un atome d'hydrogène ou un hétérocycle à 4 à 10 chaînons saturé, partiellement ou totalement insaturé ou aromatique, comprenant au moins un atome d'azote, non substitué ou substitué par un T¹ ou plus, de préférence non substitué ; R³ est H, ou oxazolyle ;
T¹, identique ou différent, représente indépendamment un atome de fluor; - (CH₂)ₘOQ¹; -(CH₂)ₘ-CN; -(CH₂)ₘ-OC(O)Q¹; -(CH₂)ₘ-C(O)OQ¹; -(CH₂)ₘ-OC(O)OQ¹; -(CH₂)ₘ-OC(O)NQ¹Q²; -(CH₂)ₘ-C(O)NQ¹Q²; -(CH₂)ₘ-C(O)ONQ¹Q²; - (CH₂)ₘ-C(O)NQ¹OQ²; -(CH₂)ₘ-C(O)NQ¹-NQ¹Q²; -(CH₂)ₘ-NQ¹C(O)Q² ; -(CH₂)ₘ-NQ¹S(O)₂NQ¹Q²; -(CH₂)ₘ-NQ¹S(O)₂Q²; -(CH₂)ₘ-S(O)₂NQ¹Q²; -(CH₂)ₘ-NQ¹C(O)OQ²; -(CH₂)ₘ-NQ¹C(O)NQ¹Q²; -(CH₂)ₘ-NQ¹Q²; -(CH₂)ₘ-NH-C(NHQ³)=NQ⁴; -(CH₂)ₘ-NH-CH=NQ³; -(CH₂)ₘ-C(NHQ³)=NQ⁴; -(X)-(CH₂)ₚOQ¹; - (X)-(CH₂)ₙ-CN; -(X)-(CH₂)ₚ-OC(O)Q¹; -(X)-(CH₂)ₙ-C(O)OQ¹; -(X)-(CH₂)ₚ-OC(O)OQ¹; -(X)-(CH₂)ₚ-OC(O)NQ¹Q²; -(X)-(CH₂)ₙ-C(O)NQ¹Q²; -(X)-(CH₂)ₙ-C(O)ONQ¹Q²; -(X)-(CH₂)ₙ-C(O)NQ¹OQ²; -(X)-(CH₂)ₙ-C(O)NQ¹-NQ¹Q²; -(X)-(CH₂)ₚ-NQ¹C(O)Q²; -(X)-(CH₂)ₚ-NQ¹S(O)₂NQ¹Q²; -(X)-(CH₂)ₚ-NQ¹S(O)₂Q²; -(X)-(CH₂)ₚ-S(O)₂NQ¹Q²; -(X)-(CH₂)ₚ-NQ¹C(O)OQ²; -(X)-(CH₂)ₚ-NQ¹C(O)NQ¹Q²; -(X)-(CH₂)ₚ-NQ¹Q²; -(X)-(CH₂)ₚ-NH-C(NHQ³)=NQ⁴; -(X)-(CH₂)ₚ-NH-CH=NQ³; -(X)-(CH₂)ₙ-C(NHQ³)=NQ⁴; -C(O)-(CH₂)ₙOQ¹; -C(O)-(CH₂)ₙ-CN; -C(O)-(CH₂)ₙ-OC(O)Q¹; -C(O)-(CH₂)ₙ-C(O)OQ¹; -C(O)-(CH₂)ₙ-OC(O)OQ¹; -C(O)-(CH₂),-OC(O)NQ¹Q²; -C(O)-(CH₂)ₙ-C(O)NQ¹Q²; -C(O)-(CH₂)ₙ-C(O)ONQ¹Q²; -C(O)-(CH₂)ₙ-C(O)NQ¹OQ²; -C(O)-(CH₂)ₙ-C(O)NQ¹-NQ¹Q²; -C(O)-(CH₂)ₙ-NQ¹C(O)Q²; -C(O)-(CH₂)ₙ-NQ¹S(O)₂NQ¹Q²; -C(O)-(CH₂)ₙ-NQ¹S(O)₂Q²; ; -C(O)-(CH₂)ₙ-S(O)₂NQ¹Q²; -C(O)-(CH₂)ₙ-NQ¹C(O)OQ²; -C(O)-(CH₂)ₙ-NQ¹C(O)NQ¹Q²; -C(O)-(CH₂)ₙ-NQ¹Q²; -C(O)-(CH₂)ₙ-NH-C(NHQ³)=NQ⁴; -C(O)-(CH₂)ₙ-NH-CH=NQ³; -C(O)-(CH₂)ₙ-C(NHQ³)=NQ⁴ ou T¹, identique ou différent, représente indépendamment un, non substitué ou substitué par un T² ou plus, -(CH₂)ₘ-(hétérocycle à 4, 5 ou 6 chaînons saturé, partiellement ou totalement insaturé ou aromatique) ; -(X)-(CH₂)ₘ-(hétérocycle à 4, 5 ou 6 chaînons saturé, partiellement ou totalement insaturé ou aromatique) ; (C₁-C₃)-alkyle ; (C₁-C₃)-fluoroalkyle; -(X)-(C₁-C₃)-alkyle; -(X)-(C₁-C₃)-fluoroalkyle; -(CH₂)ₘ-(C₃-C₆)-cycloalkyle; -(X)-(CH₂)ₘ-(C₃-C₆)-cycloalkyle; -(CH₂)ₘ-(C₃-C₆)-cyclofluoroalkyle; - (X)-(CH₂)ₘ-(C₃-C₆)-cyclofluoroalkyle; -C(O)-(CH₂)ₘ-(hétérocycle à 4, 5 ou 6 chaînons saturé, partiellement ou totalement insaturé ou aromatique) ; -C(O)- (C₁-C₃)-alkyle; - C(O)-(C₁-C₃)-fluoroalkyle; -C(O)O-(C₁-C₃)-fluoroalkyle; -C(O)-(CH₂)ₘ-(C₃-C₆)-cycloalkyle; -C(O)-(CH₂)ₘ-(C₃-C₆)-cycloalkyle; -C(O)-(CH₂)ₘ-(C₃-C₆)-cyclofluoroalkyle ; -C(O)-(CH₂)ₘ-(C₃-C₆)-cyclofluoroalkyle;
Q¹ et Q², identiques ou différents, représentent indépendamment un atome d'hydrogène; -(CH₂)ᵣ-NHQ³; -(CH₂)ᵣ-NH-C(NHQ³)=NQ⁴; -(CH₂)ᵣ-NH-CH=NQ³; (CH₂)ₙ-C(NHQ³)=NQ⁴; -(CH₂)ᵣ-OQ³; -(CH₂)ₙ-CONHQ³; ou un, non substitué ou substitué par un T² ou plus, (C₁-C₃)-alkyle; (C₁-C₃)-fluoroalkyle; -(CH₂)ₘ-(hétérocycle à 4, 5 ou 6 chaînons comprenant au moins un atome d'azote, saturé, partiellement ou totalement insaturé ou aromatique) ; ou Q¹, Q² et l'atome d'azote auquel ils sont liés, forment ensemble un hétérocycle à 4, 5 ou 6 chaînons saturé ou partiellement insaturé comprenant 1, 2 ou 3 hétéroatomes, non substitué ou substitué par un T² ou plus ;
Q³ et Q⁴, identiques ou différents, représentent indépendamment un atome d'hydrogène ou (C₁-C₃)-alkyle;
T², identique ou différent, représente indépendamment -OH ; -NH₂; -CONH₂ ;
m, identique ou différent, représente indépendamment 0, 1, 2 ou 3 ;
n, identique ou différent, représente indépendamment 1, 2 ou 3 ;
p, identique ou différent, représente indépendamment 2 ou 3 ;
r vaut 1, 2 ou 3 lorsque (CH₂)ᵣ est directement lié à un atome de carbone ou autrement 2 ou 3, de préférence r vaut 2 ou 3 ;
- X, identique ou différent, représente indépendamment O ; S ; S(O) ; S(O)₂ ou N(Q³) ;
- au moins l'un de R² et R³ est différent de H et R² et R³ ne sont pas simultanément H ; et un racémate, un énantiomère, un diastéréoisomère, un isomère géométrique ou un sel pharmaceutiquement acceptable du composé de formule (I).

2. Composition comprenant :
- au moins un antibiotique choisi parmi : amoxicilline, amikacine, aztréonam, ciprofloxacine, clindamycine, chloramphénicol, colistine, cefpirome, ceftriaxone, céfotaxime, daptomycine, érythromycine, céfépime, céfixime, fosfomycine, céfuroxime, imipénème, linézolide, méropénème, pipéracilline, cefpodoxime, rifampicine, synercid, tigécycline, vancomycine, sulbactam, ceftaroline, céfidérocol (S-649266) ou BAL30072 ; et
- au moins un composé choisi parmi :

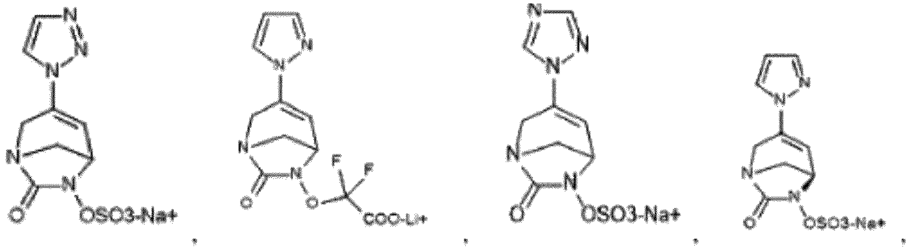

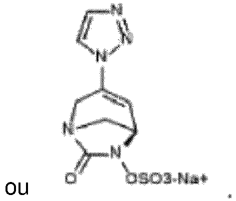

3. Composition selon la revendication 1 dans laquelle le composé de formule (I) est un composé de formule (I*) 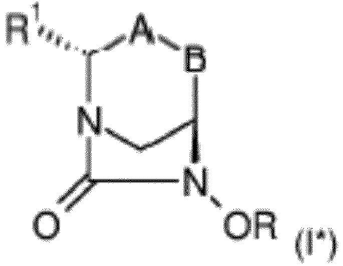 où R¹, A, B et R sont tels que définis dans la revendication 1.

4. Composition selon l'une quelconque des revendications 1 à 3 comprenant en outre un excipient pharmaceutiquement acceptable.

5. Composition selon l'une quelconque des revendications 1, 3 ou 4, dans laquelle R² est choisi parmi H, ou un hétérocycle à 5 à 6 chaînons saturé, partiellement ou totalement insaturé ou aromatique comprenant au moins un atome d'azote.

6. Composition selon l'une quelconque des revendications 1 ou 3 à 5, dans laquelle R² est choisi parmi H ou un hétérocycle choisi parmi oxazolyle, pyrazolyle, pyridinyle ou triazolyle.

7. Composition selon la revendication 2 ou 4, dans laquelle l'antibiotique est choisi parmi amoxicilline, amikacine, aztréonam, ciprofloxacine, clindamycine, chloramphénicol, colistine, cefpirome, ceftriaxone, céfotaxime, daptomycine, érythromycine, céfépime, céfixime, fosfomycine, céfuroxime, imipénème, linézolide, méropénème, pipéracilline, cefpodoxime, rifampicine, synercid, tigécycline, vancomycine, sulbactam, ceftaroline.

8. Composition selon l'une quelconque des revendications 2 ou 4, dans laquelle l'antibiotique est choisi parmi amoxicilline, aztréonam, cefpirome, ceftriaxone, céfotaxime, céfépime, céfixime, fosfomycine, céfuroxime, imipénème, méropénème, pipéracilline, cefpodoxime, sulbactam.

9. Composition selon l'une quelconque des revendications 2 ou 4, dans laquelle l'antibiotique est choisi parmi méropénème, aztréonam, pipéracilline, fosfomycine, céfépime, céfixime ou sulbactam.

10. Composition selon l'une quelconque des revendications 1 et 3 à 6, dans laquelle les composés de formule (I) ou (I*) sont choisis parmi : 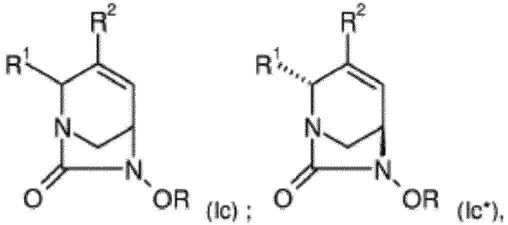 où R, R¹ et R² sont tels que définis ci-dessus, de préférence R¹ est CH₂NH₂ ou H et de préférence R² est, un hétérocycle à 5 à 6 chaînons, de préférence un monocycle, saturé, partiellement ou totalement insaturé ou aromatique, de préférence aromatique, comprenant au moins un atome d'azote, de préférence R² est un hétérocycle choisi parmi oxazolyle, pyrazolyle, pyridinyle ou triazolyle ; ou 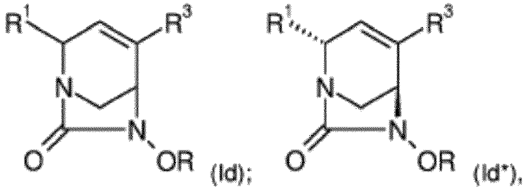 où R, R¹ et R³ sont tels que définis ci-dessus, de préférence R¹ est CH₂NH₂.

11. Composition selon l'une quelconque des revendications 2, 4 et 7 à 10, dans laquelle le composé de formule (I) est choisi parmi :
[*trans*-2-(azaniumylméthyl)-4-oxazol-5-yl-7-oxo-1,6-diazabicyclo[3.2.1]oct-3-èn-6-yl] sulfate de sodium et 2,2,2-trifluoroacétate
[(2*S*,5*R*)-2-(azaniumylméthyl)-7-oxo-3-oxazol-2-yl-1,6-diazabicyclo[3.2.1]oct-3-èn-6-yl] sulfate de sodium et 2,2,2-trifluoroacétate
difluoro-[3-oxazol-3-yl-7-oxo-1,6-diazabicyclo[3.2.1]oct-3-èn-6-yloxy]-acétate de lithium
[3-(3-pyridyl)-7-oxo-1,6-diazabicyclo[3.2.1]oct-3-èn-6-yl] sulfate de sodium
[(5*R*)-3-(oxazol-2-yl)-7-oxo-1,6-diazabicyclo[3.2.1]oct-3-èn-6-yl] sulfate de sodium
(7-oxo-3-pyrazol-1-yl-1,6-diazabicyclo[3.2.1]oct-3-èn-6-yl) sulfate de sodium
[7-oxo-3-(triazol-1-yl)-1,6-diazabicyclo[3.2.1]oct-3-èn-6-yl] sulfate de sodium
difluoro-(7-oxo-3-pyrazol-1-yl-1,6-diaza-bicyclo[3.2.1]oct-3-èn-6-yloxy)-acétate de lithium
[7-oxo-3-(1,2,4-triazol-1-yl)-1,6-diazabicyclo[3.2.1]oct-3-èn-6-yl] sulfate de sodium
[(5*R*)-7-oxo-3-pyrazol-1-yl-1,6-diazabicyclo[3.2.1]oct-3-èn-6-yl] sulfate de sodium
[(5*R*)-7-oxo-3-(triazol-1-yl)-1,6-diazabicyclo[3.2.1]oct-3-èn-6-yl] sulfate de sodium.

12. Composition selon l'une quelconque des revendications 1 à 11 pour son utilisation en tant que médicament.

13. Composition selon l'une quelconque des revendications 1 à 11 pour son utilisation pour le traitement ou la prévention d'une infection bactérienne, de préférence causée par des bactéries produisant au moins une β-lactamase, de préférence causée par des bactéries gram-positives ou par des bactéries gram-négatives, de préférence une infection bactérienne causée par des bactéries gram-négatives.

14. Composition selon l'une quelconque des revendications 1 à 11 pour son utilisation en tant qu'agent antibactérien et/ou en tant qu'inhibiteur de β-lactamase.

15. Kit comprenant :
- au moins un antibiotique choisi parmi : amikacine, ciprofloxacine, clindamycine, chloramphénicol, colistine, daptomycine, érythromycine, fosfomycine, linézolide, rifampicine, synercid, tigécycline, vancomycine, sulbactam ; et
- au moins un composé de formule (I) 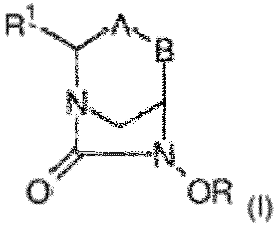 dans laquelle
- R est choisi parmi -SO₃H ou CF₂CO₂H;
- R¹ est choisi parmi H, CH₂-NH₂, CONH₂ ou un hétéroaryle à 5 chaînons comprenant au moins deux atomes d'azote ;
- A-B est 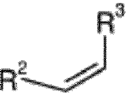 R² est un atome d'hydrogène ou un hétérocycle à 4 à 10 chaînons saturé, partiellement ou totalement insaturé ou aromatique, comprenant au moins un atome d'azote, non substitué ou substitué par un T¹ ou plus, de préférence non substitué ; R³ est H, ou oxazolyle ;
T¹, identique ou différent, représente indépendamment un atome de fluor; - (CH₂)ₘOQ¹; -(CH₂)ₘ-CN; -(CH₂)ₘ-OC(O)Q¹; -(CH₂)ₘ-C(O)OQ¹; -(CH₂)ₘ-OC(O)OQ¹; -(CH₂)ₘ-OC(O)NQ¹Q²; -(CH₂)ₘ-C(O)NQ¹Q²; -(CH₂)ₘ-C(O)ONQ¹Q²; - (CH₂)ₘ-C(O)NQ¹OQ²; -(CH₂)ₘ-C(O)NQ¹-NQ¹Q²; -(CH₂)ₘ-NQ¹C(O)Q²; -(CH₂)ₘ-NQ¹S(O)₂NQ¹Q²; -(CH₂)ₘ-NQ¹S(O)₂Q²; -(CH₂)ₘ-S(O)₂NQ¹Q²; -(CH₂)ₘ-NQ¹C(O)OQ²; -(CH₂)ₘ-NQ¹C(O)NQ¹Q²; -(CH₂)ₘ-NQ¹Q²; -(CH₂)ₘ-NH-C(NHQ³)=NQ⁴; -(CH₂)ₘ-NH-CH=NQ³; -(CH₂)ₘ-C(NHQ³)=NQ⁴; -(X)-(CH₂)ₚOQ¹; - (X)-(CH₂)ₙ-CN; -(X)-(CH₂)ₚ-OC(O)Q¹; -(X)-(CH₂)ₙ-C(O)OQ¹; -(X)-(CH₂)ₚ-OC(O)OQ¹; -(X)-(CH₂)ₚ-OC(O)NQ¹Q²; -(X)-(CH₂)ₙ-C(O)NQ¹Q²; -(X)-(CH₂)ₙ-C(O)ONQ¹Q²; -(X)-(CH₂)ₙ-C(O)NQ¹OQ²; -(X)-(CH₂)ₙ-C(O)NQ¹-NQ¹Q²; -(X)-(CH₂)ₚ-NQ¹C(O)Q²; -(X)-(CH₂)ₚ-NQ¹S(O)₂NQ¹Q²; -(X)-(CH₂)ₚ-NQ¹S(O)₂Q²; -(X)-(CH₂)ₚ-S(O)₂NQ¹Q²; -(X)-(CH₂)ₚ-NQ¹C(O)OQ²; -(X)-(CH₂)ₚ-NQ¹C(O)NQ¹Q²; -(X)-(CH₂)ₚ-NQ¹Q²; -(X)-(CH₂)ₚ-NH-C(NHQ³)=NQ⁴; -(X)-(CH₂)ₚ-NH-CH=NQ³; -(X)-(CH₂)ₙ-C(NHQ³)=NQ⁴; -C(O)-(CH₂)ₙOQ¹; -C(O)-(CH₂)ₙ-CN; -C(O)-(CH₂)ₙ-OC(O)Q¹; -C(O)-(CH₂)ₙ-C(O)OQ¹; -C(O)-(CH₂)ₙ-OC(O)OQ¹; -C(O)-(CH₂)ₙ-OC(O)NQ¹Q²; -C(O)-(CH₂)ₙ-C(O)NQ¹Q²; -C(O)-(CH₂)ₙ-C(O)ONQ¹Q²; -C(O)-(CH₂)ₙ-C(O)NQ¹OQ²; -C(O)-(CH₂)ₙ-C(O)NQ¹-NQ¹Q²; -C(O)-(CH₂)ₙ-NQ¹C(O)Q²; -C(O)-(CH₂)ₙ-NQ¹S(O)₂NQ¹Q²; -C(O)-(CH₂)ₙ-NQ¹S(O)₂Q²; ; -C(O)-(CH₂)ₙ-S(O)₂NQ¹Q²; -C(O)-(CH₂)ₙ-NQ¹C(O)OQ²; -C(O)-(CH₂)ₙ-NQ¹C(O)NQ¹Q²; -C(O)-(CH₂)ₙ-NQ¹Q²; -C(O)-(CH₂)ₙ-NH-C(NHQ³)=NQ⁴; -C(O)-(CH₂)ₙ-NH-CH=NQ³; -C(O)-(CH₂)ₙ-C(NHQ³)=NQ⁴ ou T¹, identique ou différent, représente indépendamment un, non substitué ou substitué par un T² ou plus, -(CH₂)ₘ-(hétérocycle saturé, partiellement ou totalement insaturé ou aromatique à 4, 5 ou 6 chaînons) ; -(X)-(CH₂)ₘ-(hétérocycle à 4, 5 ou 6 chaînons saturé, partiellement ou totalement insaturé ou aromatique) ; (C₁-C₃)-alkyle ; (C₁-C₃)-fluoroalkyle; -(X)-(C₁-C₃)-alkyle; -(X)-(C₁-C₃)-fluoroalkyle; -(CH₂)ₘ-(C₃-C₆)-cycloalkyle ; -(X)-(CH₂)ₘ-(C₃-C₆)-cycloalkyle; -(CH₂)ₘ-(C₃-C₆)-cyclofluoroalkyle; -(X)-(CH₂)ₘ-(C₃-C₆)-cyclofluoroalkyle; -C(O)-(CH₂)ₘ-(hétérocycle saturé, partiellement ou totalement insaturé ou aromatique à 4, 5 ou 6 chaînons) ; -C(O)-(C₁-C₃)-alkyle; - C(O)-(C₁-C₃)-fluoroalkyle; -C(O)O-(C₁-C₃)-fluoroalkyle; -C(O)-(CH₂)ₘ-(C₃-C₆)-cycloalkyle; -C(O)-(CH₂)ₘ-(C₃-C₆)-cycloalkyle; -C(O)-(CH₂)ₘ-(C₃-C₆)-cyclofluoroalkyle ; -C(O)-(CH₂)ₘ-(C₃-C₆)-cyclofluoroalkyle;
Q¹ et Q², identiques ou différents, représentent indépendamment un atome d'hydrogène; -(CH₂)ᵣ-NHQ³; -(CH₂)ᵣ-NH-C(NHQ³)=NQ⁴; -(CH₂)ᵣ-NH-CH=NQ³; (CH₂)ₙ-C(NHQ³)=NQ⁴; -(CH₂)ᵣ-OQ³; -(CH₂)ₙ-CONHQ³; ou un, non substitué ou substitué par un T² ou plus, (C₁-C₃)-alkyle; (C₁-C₃)-fluoroalkyle; -(CH₂)ₘ-(hétérocycle saturé, partiellement ou totalement insaturé ou aromatique à 4, 5 ou 6 chaînons comprenant au moins un atome d'azote) ; ou Q¹, Q² et l'atome d'azote auquel ils sont liés, forment ensemble un hétérocycle saturé ou partiellement insaturé à 4, 5 ou 6 chaînons comprenant 1, 2 ou 3 hétéroatomes, non substitué ou substitué par un T² ou plus ;
Q³ et Q⁴, identiques ou différents, représentent indépendamment un atome d'hydrogène ou (C₁-C₃)-alkyle ;
T², identique ou différent, représente indépendamment -OH ; -NH₂; -CONH₂ ;
m, identique ou différent, représente indépendamment 0, 1, 2 ou 3 ;
n, identique ou différent, représente indépendamment 1, 2 ou 3 ;
p, identique ou différent, représente indépendamment 2 ou 3 ;
r vaut 1, 2 ou 3 lorsque (CH₂)ᵣ est directement lié à un atome de carbone ou autrement 2 ou 3, de préférence r vaut 2 ou 3 ;
- X, identique ou différent, représente indépendamment O ; S ; S(O) ; S(O)₂ ou N(Q³) ;
- au moins l'un de R² et R³ est différent de H et R² et R³ ne sont pas simultanément H ; et un racémate, un énantiomère, un diastéréoisomère, un isomère géométrique ou un sel pharmaceutiquement acceptable du composé de formule (I) pour le traitement ou la prévention d'infections bactériennes par son administration simultanée, séparée ou séquentielle à un patient qui en éprouve le besoin.

16. Kit comprenant :
- au moins un antibiotique choisi parmi : amoxicilline, amikacine aztréonam, ciprofloxacine, clindamycine, chloramphénicol, colistine, cefpirome, ceftriaxone, céfotaxime, daptomycine, érythromycine, céfépime, céfixime, fosfomycine, céfuroxime, imipénème, linézolide, méropénème, pipéracilline, cefpodoxime, rifampicine, synercid, tigécycline, vancomycine, sulbactam, ceftaroline, céfidérocol (S-649266) ou BAL30072 ; et
- au moins un composé choisi parmi : 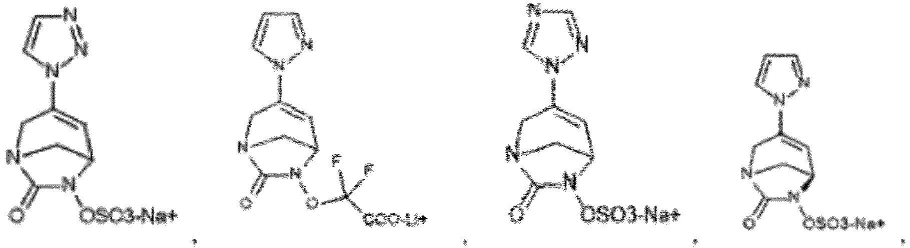 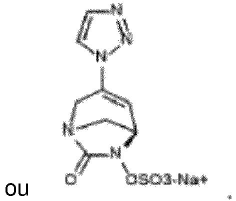 pour le traitement ou la prévention d'infections bactériennes par son administration simultanée, séparée ou séquentielle à un patient qui en éprouve le besoin.

17. Kit selon la revendication 15 dans lequel le composé de formule (I) est un composé de formule (I*) 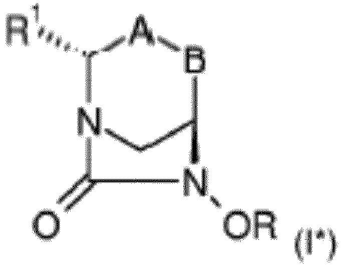 où R¹, A, B et R sont tels que définis dans la revendication 15.
